# FASCICULE DE BREVET EUROPEEN

(11) **EP 2 416 777 B1**
(45) Date de publication et mention de la délivrance du brevet: **12.11.2014**
(21) Numéro de dépôt: 10723222.5
(22) Date de dépôt: 07.04.2010
(51) Int. Cl.: A61K 31/517, A61P 9/00, A61K 9/00

(54) **APPLICATIONS THÉRAPEUTIQUES DANS LE DOMAINE CARDIOVASCULAIRE DE DÉRIVÉS DE QUINAZOLINEDIONE**
THERAPEUTISCHE ANWENDUNGEN VON CHINAZOLINDION-DERIVATEN IM KARDIOVASKULÄREN GEBIET
THERAPEUTIC APPLICATIONS IN THE CARDIOVASCULAR FIELD OF QUINAZOLINEDIONE DERIVATIVES

(30) Priorité: 09.04.2009 FR 0901755
(43) Date de publication de la demande: 15.02.2012
(73) Titulaire: SANOFI, 75008 Paris (FR)
(72) Inventeur: JANIAK, Philip, F-75013 Paris (FR); MARCINIAK, Gilbert, F-75013 Paris (FR); NAVE, Jean-François, F-75013 Paris (FR); VIVIANI, Fabrice, F-75013 Paris (FR)
(74) Mandataire: Veinante, Aude
(86) Numéro de dépôt international: PCT/FR2010/050672
(87) Numéro de publication internationale: WO 2010/116090

(56) Documents cités:
- EP-A1- 0 040 793
- WO-A1-2009/077680
- FR-A1- 2 921 926
- US-A- 3 879 393
- BENDER ANDREW T ET AL: "Cyclic nucleotide phosphodiesterases: Molecular regulation to clinical use" PHARMACOLOGICAL REVIEWS, vol. 58, no. 3, septembre 2006 (2006-09), pages 488-520, XP002552943 ISSN: 0031-6997
- GRASSY G ET AL: "Inhibitory effects on platelet aggregation and cyclic AMP phosphodiesterase of azaindolizine-type compounds" CHEMOMETRICS AND INTELLIGENT LABORATORY SYSTEMS, ELSEVIER SCIENCE PUBLISHERS B.V. AMSTERDAM, NL, vol. 20, no. 1, 1 août 1993 (1993-08-01), pages 71-84, XP026502520 ISSN: 0169-7439 [extrait le 1993-08-01]
- MOVSESIAN M A: "Therapeutic potential of cyclic nucleotide phosphodiesterase inhibitors in heart failure." EXPERT OPINION ON INVESTIGATIONAL DRUGS MAY 2000, vol. 9, no. 5, mai 2000 (2000-05), pages 963-973, XP002552944 ISSN: 1354-3784
- MATSUMOTO TAKAYUKI ET AL: "Phosphodiesterases in the vascular system." JOURNAL OF SMOOTH MUSCLE RESEARCH, vol. 39, no. 4, août 2003 (2003-08), pages 67-86, XP002552945 ISSN: 0916-8737
- OMORI KENJI ET AL: "Overview of PDEs and their regulation" CIRCULATION RESEARCH, vol. 100, no. 3, février 2007 (2007-02), pages 309-327, XP002552946 ISSN: 0009-7330

## Description

L'invention a pour objet l'utilisation de dérivés de quinazolinedione comme médicaments ou pour fabriquer un médicament destiné(s) au traitement et/ou à la prévention d'au moins une maladie cardiovasculaire.

L'invention concerne plus particulièrement l'utilisation de dérivés de quinazolinedione, susceptibles d'agir en tant qu'inhibiteurs de la phosphodiestérase 7 (PDE7), voire, pour certains de ces dérivés, susceptibles d'agir également en tant qu'inhibiteurs de la phosphodiestérase 8 (PDE8), sachant qu'il n'est pas exclu que ces mêmes dérivés de quinazolinedione soient également suceptibles d'agir via d'autres voies biologiques/biochimiques sur le système cardiovasculaire dans le but de traiter une ou des affection(s) cardiovasculaire(s) et/ou prévenir l'apparition de telle(s) affection(s).

Les phosphodiestérases (PDEs) sont des enzymes intracellulaires responsables de l'hydrolyse des messagers secondaires AMPc (adénosine-3',5'-monophosphate cyclique) et GMPc (guanosine-3',5'-monophosphate cyclique) en nucléotides 5'-monophosphates inactifs. L'AMPc et le GMPc jouent un rôle essentiel dans les voies de signalisation cellulaire et interviennent dans de nombreux processus physiologiques.

L'inhibition des phosphodiestérases se traduit par une augmentation des concentrations intracellulaires d'AMPc et de GMPc, produisant l'activation spécifique de voies de phosphorylation impliquées dans des réponses fonctionnelles variées. L'augmentation des concentrations intracellulaires en AMPc ou en GMPc à l'aide d'inhibiteurs sélectifs de phosphodiestérases apparait comme une approche prometteuse pour le traitement de diverses maladies (Bender et Beavo, Pharmacol Rev (2006) 58, 488-520). Les inhibiteurs de phosphodiestérases présentent donc un intérêt comme agents thérapeutiques et comme outils pharmacologiques.

A ce jour, onze familles de phosphodiestérases ont été identifiées. Elles se distinguent par leur structure primaire, leur spécificité de substrat et leur sensibilité vis-à-vis de divers effecteurs et inhibiteurs spécifiques de PDEs. Chaque famille est constituée d'un ou plusieurs gènes qui sont exprimés dans différents tissus sous forme de variants d'épissage (Bender et Beavo, Pharmacol Rev (2006) 58, 488-520; Lugnier, Pharmacol Therapeut (2006) 109, 366-398).

Les PDE4, 7, et 8 hydrolysent spécifiquement l'AMPc et les PDE5, 6 et 9 le GMPc.

La famille PDE7 est représentée par les isoformes PDE7A et PDE7B, provenant de deux gènes distincts.

La PDE7A humaine (Michaeli et al, J Biol Chem (1993) 268, 12925-12932; Han et al, J Biol Chem (1997) 272, 16152-16157 ; Wang et al, Biochem Biophys Res Commun (2000) 276, 1271-1277) et la PDE7B humaine (Sasaki et al, Biochem Biophys Res Commun (2000), 271, 575-583 ; Gardner et al, Biochem Biophys Res Commun (2000) 272, 186-192) hydrolysent sélectivement l'AMPc avec des constantes de Michaelis (Km) de 0.1 à 0.2 µM et de 0.13 à 0.2 µM, respectivement. La partie catalytique de la PDE7B présente environ 67% d'homologie avec celle de la PDE7A.

Trois variants d'épissage sont connus pour la PDE7A. Les PDE7A1 et PDE7A3 sont exprimées principalement dans les cellules du système immunitaire et des poumons alors que la PDE7A2 est surtout exprimée dans les muscles du squelette, le coeur et les reins. Pour la PDE7B, trois variants ont également été récemment identifiés (Giembycz et Smith, Drugs Future (2006) 31, 207-229).

Les profils de distribution tissulaire de PDE7A et PDE7B sont très différents, suggérant que ces deux isoformes ont des fonctions distinctes du point de vue physiologique. La PDE7A est abondamment exprimée dans les cellules hématopoïétiques, les poumons, le placenta, la rate, les muscles du squelette, le coeur, les cellules de Leydig, les tubes collecteurs des reins et les surrénales. Une forte expression de PDE7B est détectée dans le pancréas, le coeur, la thyroïde et les muscles du squelette (Giembycz et Smith, Drugs Future (2006) 31, 207-229 ; Wang et al, Biochem Biophys Res Commun (2000) 276, 1271-1277). Une co-expression des RNA messagers (RNAm) de la PDE7A et de la PDE7B est observée dans certains tissus. C'est le cas dans les ostéoblastes (Ahlstrom et al, Cell Mol Biol Lett (2005) 10, 305-319) et dans certaines régions du cerveau : plusieurs zones du cortex, le gyrus denté, la plupart des composants du système olfactif, le striatum, de nombreux noyaux du thalamus et les cellules pyramidales de l'hippocampe (Miro et al, Synapse (2001) 40, 201-214; Reyes-Irisarri et al, Neuroscience (2005) 132, 1173-1185). Par contre, dans certaines zones du cerveau, seulement une des deux isoformes est exprimée. Ainsi, seuls les RNAm de PDE7A sont présents dans de nombreux noyaux du tronc cérébral. De même, les RNAm de PDE7B sont présents en fortes concentrations dans le noyau accumbens et le noyau dorsal moteur du nerf vague alors que les RNAm de PDE7A n'y sont pas détectés (Miro et al, Synapse (2001) 40, 201-214; Reyes-Irisarri et al, Neuroscience (2005) 132, 1173-1185).

La présente invention a en particulier pour objet des applications thérapeutiques de dérivés de quinazolinedione, pouvant s'avérer de puissants inhibiteurs de PDE7, ou de PDE7 et de PDE8 selon les dérivés ou pouvant agir par d'autres voies biologiques.

L'invention a pour objet des composés de formule générale (I), suivante: 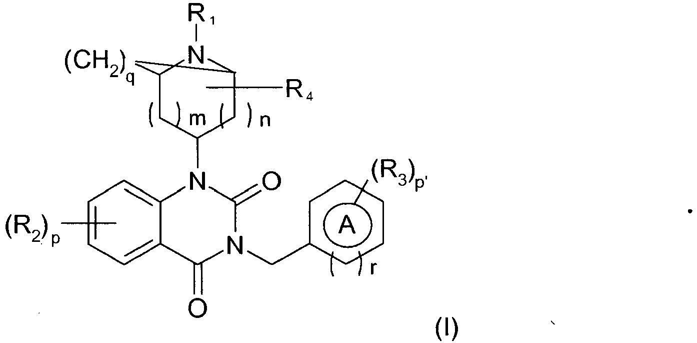 dans laquelle
- A représente un groupe aryle ou un groupe hétéroaryle;
- R₁ représente :
   ▪ un atome d'hydrogène,
   ▪ -C(O)R dans lequel R est un atome d'hydrogène, un groupe (C₁-C₆) alkoxy, un groupe aryle, un groupe (C₃-C₆) cycloalkyle, ou un groupe (C₁-C₆) alkyle, ledit alkyle étant éventuellement substitué par :
      - un ou plusieurs groupe(s) hydroxy,
      - un groupe benzyloxy,
      - un groupe (C₁-C₆) alkoxy, éventuellement substitué par un aryle, ou
      - un groupe (C₃-C₆) cycloalkyle,
   ▪ un groupe (C₁-C₆) alkyle éventuellement substitué;
- R₂ représente :
   ▪ un atome d'hydrogène,
   ▪ un atome d'halogène,
   ▪ un groupe cyano,
   ▪ un groupe nitro,
   ▪ un groupe (C₁-C₆) alkyle éventuellement substitué par un -NH₂, ou bien par un groupe -NHC(O)Rb, avec Rb tel que défini plus bas,
   ▪ un groupe -ORa dans lequel Ra représente :
      - un atome d'hydrogène,
      - un groupe (C₁-C₆) alkyle éventuellement substitué par un ou plusieurs atome(s) d'halogène, par un ou plusieurs groupe(s) hydroxy, par un groupe aryle et/ou par un ou plusieurs groupe(s) cyano,
      - un groupe (C₂-C₆) alcynyle,
      - un groupe aryle ;
- R₃ représente :
   ▪ un atome d'hydrogène,
   ▪ un atome d'halogène,
   ▪ un groupe hydroxy,
   ▪ un groupe cyano,
   ▪ un groupe -SCF₃,
   ▪ un groupe nitro,
   ▪ un groupe -S(O)₀₋₂-alkyle, un groupe -S(O)₀₋₂-hétérocycloalkyle, un groupe-O- SO₂-aryle éventuellement substitué par un ou plusieurs atome(s) d'halogène ;
   ▪ un groupe -alkyl-amino-alkyle ou -cycloalkyl-amino-alkyle, chacun éventuellement substitué sur l'alkyle terminal,
   ▪ un groupe sulfonamide éventuellement substitué,
   ▪ un groupe aryle ou un groupe hétéroaryle, ledit groupe étant monocyclique ou polycyclique et étant de plus éventuellement substitué par un groupe (C₁-C₆) alkyle, par un ou plusieurs atome(s) d'halogène, ou par un groupe (C₁-C₆) alkoxy,
   ▪ un groupe hétérocycloalkyle éventuellement substitué par un groupe (C₁-C₆) alkyle,
   ▪ un groupe (C₁-C₆) alkyle éventuellement substitué par :
      - un ou plusieurs atome(s) d'halogène,
      - un groupe aryle pouvant être substitué par un ou plusieurs atome(s) d'halogène, ou par un ou plusieurs groupe(s) hydroxy,
      - un groupe hétéroaryle,
      - un ou plusieurs groupe(s) hydroxy pouvant être substitué(s) par un groupe aryle lui-même éventuellement substitué par un ou plusieurs atome(s) d'halogène, ou
      - un groupe hétérocycloalkyle éventuellement sunstitué par un groupe CO(O)Ra, ou par un groupe (C₁-C₆) alkyle, Ra étant tel que défini précédemment ;
   ▪ un groupe -C(O)NRbRc, avec Rb et Rc étant tels que définis plus bas,
   ▪ un groupe -C(O)ORc, ou un groupe -O-C(O)ORc, avec Rc étant tel que défini plus bas,
   ▪ un groupe (C₁-C₆) alkoxy, éventuellement substitué par
      - un groupe amino-alkyle,
      - un groupe amino-cycloalkyle,
      - un groupe cycloalkyle,
      - un groupe hétérocycloalkyle
      - un groupe hétéroaryle monocyclique ou polycyclique,
      - un ou plusieurs groupement(s) hydroxy,
      - un ou plusieurs atome(s) d'halogène,
      - un groupe (C₁-C₆) alkoxy,
      - un groupe -C(O)ORc, avec Rc étant tel que défini plus bas,
      - un groupe -C(O)NRbRc, avec Rb et Rc étant tels que définis plus bas, et/ou
      - un groupe aryle, lui-même éventuellement substitué par un ou plusieurs atome(s) d'halogène, un groupe cyano, un groupe (C₁-C₆) alkoxy, un groupe -O- halogénoalkyle et/ou par un groupe halogénoalkyle,
   ▪ un groupe -O-cycloalkyle, un groupe -O-aryle, ou un groupe -O-hétérocycloalkyle, chacun éventuellement substitué par
      - un groupe aryle, lui-même éventuellement substitué par un ou plusieurs atome(s) d'halogène, ou par un groupe (C₁-C₆) alkyle,
      - un ou plusieurs atome(s) d'halogène, et/ou
      - un groupe (C₁-C₆) alkyle, lui-même pouvant être substitué par un groupe aryle
   ▪ un groupe -NH-CO-NH-aryle, un groupe -NH-CO-NH-hétéroaryle, ou un groupe -NH-CO-NH-(C₁-C₆) alkyle, chacun étant éventuellement substitué par un ou plusieurs atome(s) d'halogène, par un groupe cyano, par un groupe nitro, par un ou plusieurs groupe(s) hydroxy, ou par un groupe (C₁-C₆) alkoxy,
   ▪ un groupe -N-(C₁-C₆) alkyle, le groupe (C₁-C₆) alkyle pouvant être substitué par
      un ou plusieurs groupe(s) aryle éventuellement substitué(s) par un ou plusieurs atome(s) d'halogène et/ou par un groupe SO₂,
   ▪ un groupe -NH-CO-aryle, un groupe -NH-CO-hétéroaryle, chacun étant éventuellement substitué par un ou plusieurs atome(s) d'halogène ;
   ou bien R₃ forme avec A un groupe hétéroaryle polycyclique éventuellement substitué par un groupe (C₁-C₆) alkoxy, un groupe (C₁-C₆) alkyle éventuellement substitué par un groupe aryle pouvant lui-même être substitué par un ou plusieurs atome(s) d'halogène ;
- R₄ représente un atome d'hydrogène ou un groupe (C₁-C₆) alkyle ;
- Rb représente :
   - un atome d'hydrogène,
   - un groupe (C₁-C₆) alkyle éventuellement substitué par un ou plusieurs atome(s) d'halogène, par un ou plusieurs groupe(s) hydroxy, cyano, amino, hétérocycloalkyle, (C₁-C₆) alkoxy, ou par un groupe aryle éventuellement substitué par un ou plusieurs atome(s) d'halogène,
   - un groupe (C₃-C₆) cycloalkyle,
   - un groupe (C₂-C₆) alcynyle,
   - un groupe (C₁-C₆) alkoxy,
   - un groupe aryle éventuellement substitué par un ou plusieurs atome(s) d'halogène;
- Rc représente un atome d'hydrogène, ou un groupe (C₁-C₆) alkyle éventuellement substitué par un ou plusieurs atome(s) d'halogène ;
ou alors Rb et Rc forment ensemble avec l'atome d'azote auquel ils sont attachés un groupe hétéroaryle polycyclique, ou un groupe hétérocycloalkyle ;
- m et n représentent indépendamment l'un de l'autre la valeur 0, 1 ou 2, étant entendu que m+n ≤ 3 ;
- p et p' représentent indépendamment l'un de l'autre la valeur 1, 2 ou 3, étant entendu que lorsque p est supérieur ou égal à 2, alors les groupes R₂ sont sur des atomes de carbone distincts et peuvent être différents les uns des autres, et lorsque p' est supérieur ou égal à 2 alors les groupes R₃ sont sur des atomes de carbone distincts et peuvent être différents les uns des autres;
- q représente la valeur 0 ou 2, étant entendu que lorsque q = 0, alors le groupe hétérocyclique azoté rattaché à l'azote situé en position 1 du noyau 2,4-dioxo-1,2,3,4 tétrahydroquinazoline n'est plus ponté et est du type : 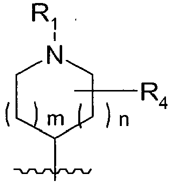 avec R1, R4, m et n tels que définis précédemment,
- r représente la valeur 0 ou 1,
pour le traitement d'au moins une maladie cardio-vasculaire et/ou à prévenir l'apparition d'au moins une maladie cardio-vasculaire.

Les composés de formule générale (I) peuvent comporter un ou plusieurs carbones asymétriques. Ils peuvent donc exister sous forme d'énantiomères ou de diastéréoisomères. Ces énantiomères, diastéréoisomères, ainsi que leurs mélanges, y compris les mélanges racémiques, font partie de l'invention.

De par leur structure, les composés de formule générale (I) peuvent également exister sous forme d'isomères de type rotamère ou d'atropoisomère.

Les composés de formule (I) peuvent également exister à l'état de bases ou de sels d'addition à des acides. De tels sels d'addition font partie de l'invention.

Ces sels sont avantageusement préparés avec des acides pharmaceutiquement acceptables, mais les sels d'autres acides utiles, par exemple, pour la purification ou la séparation des composés de formule générale (I) font également partie de l'invention.

Les composés de formule générale (I) peuvent également se trouver sous forme cristalline, amorphe ou huileuse, ces formes faisant partie de l'invention.

Les composés de formule générale (I) peuvent, en outre, se trouver sous forme d'hydrates ou de solvats, à savoir sous forme d'associations ou de combinaisons avec une ou plusieurs molécules d'eau ou avec un solvant. De tels hydrates et solvats font également partie de l'invention.

Selon la présente invention, les N-oxydes des composés comportant une amine font également partie de l'invention.

Les composés de formule (I), conformes à l'invention, comprennent également ceux dans lesquels un ou plusieurs atomes d'hydrogène, de carbone ou d'halogène, notamment de chlore ou de fluor ont été remplacés par leurs isotopes radioactifs, par exemple le tritium pour remplacer l'hydrogène ou le carbone 14 pour remplacer le carbone 12. De tels composés marqués sont utiles dans des travaux de recherche, de métabolisme ou de pharmacocinétique, dans des essais biologiques et pharmacologiques en tant qu'outils.

Dans le cadre de l'invention, on définit ce qui suit :
- dans (C₁-C₆), les indices numériques déterminent le nombre possible d'atomes de carbone présents dans une chaîne ou un cycle. Ainsi, à titre d'exemple, C₁-C₆ représente une chaîne carbonée pouvant avoir de 1 à 6 atomes de carbone. De même, à titre d'exemple, (C₁-C₅) représente une chaîne carbonée qui peut avoir de 1 à 5 atomes de carbone, ou encore (C₃-C₆) peut représenter un cycle carboné saturé pouvant avoir de 3 à 6 atomes de carbone ;
- alkoxy: un groupe -O-alkyle à chaîne aliphatique saturée, linéaire ou ramifiée ;
- alcynyle : un groupe aliphatique mono- ou poly-insaturé, linéaire ou ramifié, comprenant par exemple une ou deux insaturations acétyléniques. Par exemple, un groupe (C₂-C₆) alcynyle peut représenter un ethynyle, propynyle ...;
- alkyle : un groupe aliphatique saturé, linéaire ou ramifié; par exemple, un groupe (C₁-C₆) alkyle représente une chaîne carbonée de 1 à 6 atomes de carbone, linéaire ou ramifiée, notamment un méthyle, éthyle, propyle, isopropyle, butyle, isobutyle, secbutyle, tertbutyle, pentyle ;
- aminoalkyle : un groupe -NH(C₁-C₆) alkyle, ou encore -N((C₁-C₆)alkyle)₂ ;
- aryle : un système aromatique monocyclique éventuellement substitué comprenant de 5 à 14 chaînons par cycle, de préférence de 5 à 10 chaînons par cycle. A titre d'exemples de groupes aryles monocycliques, on peut citer le phényle, ou le naphtyle ; le groupe aryle peut être substitué par un groupe pouvant être un ou plusieurs atome(s) d'halogène, un groupe hydroxy, un groupe cyano, un groupe trifluorométhylthio, un groupe nitro, un groupe alkyle, un groupe alkoxy, un groupe alkylthio, un groupe méthylsulfonyle, un groupe alkyl-amino-alkyle ou alkyl-amino-cycloalkyle éventuellement substitué, un groupe alkyl-amino-alkoxy ou cycloalkyl-amino-alkoxy ou un groupe sulfonamide, par exemple ;
- aryle polycyclique : un système aromatique polycyclique éventuellement substitué comprenant de 5 à 14 chaînons par cycle, de préférence de 5 à 10 chaînons par cycle, et comprenant de 2 à 10 cycles, dont au moins un des cycles est aromatique. A titre d'exemple de groupe aryles polycycliques, on peut citer l'acéanthrylène, l'anthracène, l'azulène, le coronène, le rubicène, le naphtalène ; le groupe aryle polycyclique peut être substitué par un groupe pouvant être un ou plusieurs atome(s) d'halogène, un groupe hydroxy, un groupe cyano, un groupe trifluorométhylthio, un groupe nitro, un groupe alkyle, un groupe alkoxy, un groupe alkylthio, un groupe méthylsulfonyle, un groupe alkyl-amino-alkyle ou alkyl-amino-cycloalkyle éventuellement substitué, un groupe alkyl-amino-alkoxy ou cycloalkyl-amino-alkoxy ou un groupe sulfonamide, par exemple ;
- un cycle ponté : une structure bicyclique, comprenant selon l'invention un atome d'azote, dans laquelle au moins 2 atomes de carbone sont reliés par une liaison simple ou une chaîne carbonée pouvant comporter 2 atomes de carbone. A titre d'exemple, le cycle précité est du type : 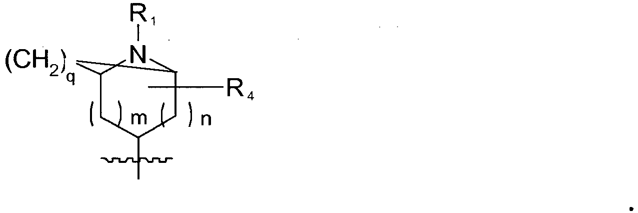 avec q = 1 ou 2 et avec les autres groupes et indices tels que définis plus haut ;
- cycloalkyle : un groupe aliphatique cyclique saturé comprenant de 3 à 8 atomes de carbone. A titre d'exemple, on peut citer les groupes cyclopropyle, méthylcyclopropyle, cyclobutyle, cyclopentyle, cyclohexyle ;
- halogène : un fluor, un chlore, un brome ou un iode ;
- halogénoalkyle : (C₁-C₆) alkyle substitué par un à trois atome(s) d'halogène;
- hétéroaryle : un système aromatique monocyclique comprenant de 5 à 14 chaînons, de préférence de 5 à 10 chaînons et comprenant un à plusieurs hétéroatomes tels que les atomes d'azote, d'oxygène ou de soufre. Les atomes d'azote peuvent être sous forme de N-oxydes. Par exemple, un hétérocycle monocyclique peut être un pyrane, une pyrazine, un pyrazole, une pyridazine, une pyridine, une pyrimidine, un pyrrole, un isothiazole, un isoxazole, un furane, un imidazole, une morpholine, un thiophène, une pipérazine, une diazétidine, une dihydropyrrolidine, une pipéridine, ou une azépine, etc ; un hétérocycle bicyclique peut être une isoquinoléine, une ptéridine, un chromane, etc ; un hétérocycle tricyclique peut être une phénanthroline, un xanthène, etc
- hétéroaryle polycyclique : un système aromatique polycyclique éventuellement substitué comprenant de 5 à 14 chaînons par cycle, de préférence de 5 à 10 chaînons par cycle, et comprenant de 2 à 10 cycles, comprenant de plus un à plusieurs hétéroatomes tels que les atomes d'azote, d'oxygène ou de soufre sur au moins l'un des cycles, et dont au moins des cycles est aromatique. A titre d'exemple de groupe hétéroaryles polycycliques, on peut citer l'indole, le benzofurane, le benzimidazole, le benzothiophène, le benzotriazole, le benzothiazole, la benzoxazole, la quinoline, l'isoquinoline, l'indazole, la quinazoline, la phthalazine, la quinoxaline, la naphtyridine, le 2,3-dihydro-1H-indole, le 2,3-dihydro-benzofurane, le 2,3-dihydro-indène, la tétrahydroquinoline, la tétrahydroisoquinoline, la tétrahydroisoquinazoline ;
- un hétérocycloalkyle : un cycle saturé, éventuellement substitué, comprenant de 3 à 8 atomes et comprenant un à plusieurs hétéroatomes tels que les atomes d'azote, d'oxygène ou de soufre sur au moins l'un des cycles, ou plusieurs hétéroatomes identiques ou différents entre eux. Par exemple, un hétérocycloalkyle peut être une pyrrolidine, une morpholine, une pipérazine, une diazétidine, une dihydropyrrolidine, une pipéridine, une pipéradine, une azépane, une imidazolidine, thiomorpholine, tétrahydropyrane, tétrahydrothiopyrane, pipérazine, diazépane, etc ;
- hydroxy : un groupe -OH ;
- nitro : un groupe -NO₂ ;
- oxo : un groupe -C(O)- ;
- sulfonamide : groupe répondant à la formule SO₂-N-alkyl ou SO₂-N-cycloalkyl, alkyl et cycloalkyl étant tels que définis ci-dessus ;
- trifluorométhylthio est défini par la formule -S-CF₃.

Il est de plus entendu que dans la présente description, lorsqu'un atome ou un groupe est substitué ou éventuellement substitué par un ou plusieurs groupe(s) ou atome(s) définis, les substituants peuvent être identiques ou différents entre eux, et être portés le cas échéant par le même atome ou des atomes différents.

Parmi les composés objet de l'invention, on peut citer un groupe de composés de formule (I) dans laquelle A représente un groupe aryle, en particulier un groupe phényle ou hétéroaryle, en particulier un groupe pyridyle, et tous les autres substituants et indices sont tels que définis dans la formule générale (I) définie plus haut.

Parmi les composés conformes à l'invention, on peut citer un groupe de composés de formule (I) dans laquelle q = 0, m et n représente chacun 1, et tous les autres substituants et indices sont tels que définis dans la formule générale (I) définie plus haut.

Parmi les composés conformes à l'invention, on peut citer un groupe de composés de formule (I), dans laquelle R₂ représente un groupe (C₁-C₆) alkyle, en particulier un méthyle, substitué par un groupe -NHC(O)Rb dans lequel Rb, les autres substituants et indices sont tels que définis pour le composé de formule générale (I) définie plus haut.

Parmi les composés conformes à l'invention, on peut citer un groupe de composés de formule générale (I), dans laquelle R₂ représente un groupe -ORa, le groupe Ra et tous les autres substituants et indices étant tels que définis dans la formule générale (I) définie plus haut.

Parmi les composés conformes à l'invention, on peut citer un groupe de composés de formule générale (I), dans laquelle R₂ est un atome d'halogène, ou un cyano, ou un hydrogène, ou un hydroxyle, ou un (C₁-C₆) alkyle éventuellement substitué par un -NH₂, ou bien par un groupe -NHC(O)Rb, Rb et les autres substituants et indices étant tels que définis dans la formule générale (I) définie plus haut.

Parmi les composés conformes à l'invention, on peut citer un groupe de composés de formule générale (I), dans laquelle A est un phényle, R₁ est un groupe-C(O)R dans lequel R représente un atome d'hydrogène, q est égal à 0, n et m valent chacun 1, et R₂ représente -ORa, Ra et les autres substituants et indices étant tels que définis dans la formule générale (I) définie plus haut.

Parmi les composés conformes à l'invention, on peut citer un groupe de composés de formule générale (I), dans laquelle A est un phényle, R₁ est un groupe-C(O)R dans lequel R représente un atome d'hydrogène, q est égal à 0, n et m valent chacun 1, p est égal à 2, l'un des R₂ est -ORa et l'autre des R₂ est un atome d'halogène, Ra et les autres substituants et indices étant tels que définis dans la formule générale (I) définie plus haut.

Parmi les composés conformes à l'invention, on peut citer un groupe de composés de formule générale (I), dans laquelle A est un phényle, R₁ est un groupe-C(O)R dans lequel R représente un atome d'hydrogène, q est égal à 0, n et m valent chacun 1, p=1 et R₂ est un méthyle substitué par un groupe -NH-CO-Rb, Rb et les autres substituants et indices étant tels que définis dans la formule générale (I) définie plus haut.

Avantageusement, dans les composés de formule (I), le groupe R₂ est en position 6 du noyau 2,4-dioxo-1,2,3,4 tétrahydroquinazoline. Les composés de formule (I) peuvent également avoir un groupe R2 en position 7 du noyau 2,4-dioxo-1,2,3,4 tétrahydroquinazoline. Les groupes R2 en position 6 et 7 peuvent être identiques ou différents.

Avantageusement, dans les composés de formule (I), p est égal à 1 ou 2.

Les composés de formule générale (I) peuvent être à l'état de base, d'hydrate, de solvat, d'isomères ou de leurs mélanges.

Dans un mode particulier, lorsque p' = 2, alors les deux groupes R₃ sont en position 3 et 4 du noyau A et peuvent être différents l'un de l'autre.

Les combinaisons des groupes de composés conformes à l'invention précités font également partie de l'invention.

A titre d'exemple de composés préférés conformes à l'invention, on peut citer les composés suivants :
n°1 : 2-{[3-(3,4-diméthoxybenzyl)-1-(1-formylpipéridin-4-yl)-2,4-dioxo-1,2,3,4-tétrahydroquinazolin-6-yl]oxy}propanenitrile
n°2 : 1-(1-acétylpipéridin-4-yl)-3-(3,4-diméthoxybenzyl)-6-hydroxyquinazoline-2,4(1H,3H)-dione
n°3 : {[1-(1-acétylpipéridin-4-yl)-3-(3,4-diméthoxybenzyl)-2,4-dioxo-1,2,3,4-tétrahydroquinazolin-6-yl]oxy}acétonitrile
n°4 : 2-{[1-(1-acétylpipéridin-4-yl)-3-(3,4-diméthoxybenzyl)-2,4-dioxo-1,2,3,4-tétrahydroquinazolin-6-yl]oxy}propanenitrile
n°5 : {[3-(3,4-diméthoxybenzyl)-1-(1-formylpipéridin-4-yl)-2,4-dioxo-1,2,3,4-tétrahydroquinazolin-6-yl]oxy}acétonitrile
n°11 : 4-[3-(3,4-diméthoxybenzyl)-6-[2-fluoro-1-(fluorométhyl)éthoxy]-2,4-dioxo-3,4-dihydroquinazolin-1 (2H)-yl]pipéridine-1-carbaldéhyde
n°12 :1-(1-acétylpipéridin-4-yl)-3-(3,4-diméthoxybenzyl)-6-[2-fluoro-1-(fluorométhyl)éthoxy]quinazoline-2,4(1H,3H)-dione
n°13 : 4-[3-(3,4-diméthoxybenzyl)-2,4-dioxo-6-(2,2,2-trifluoroéthoxy)-3,4-dihydroquinazolin-1(2H)-yl]pipéridine-1-carbaldéhyde
n°14 : 1-(1-acétylpipéridin-4-yl)-6-(2,2-difluoroéthoxy)-3-(3,4-diméthoxybenzyl)quinazoline-2,4(1H,3H)-dione
n°16 :4-[6-(2,2-difluoroéthoxy)-3-(3,4-diméthoxybenzyl)-2,4-dioxo-3,4-dihydroquinazolin-1(2H)-yl]pipéridine-1-carbaldéhyde
n°20 : N-{[3-(3,4-diméthoxybenzyl)-1-(1-formylpipéridin-4-yl)-2,4-dioxo-1,2,3,4-tétrahydroquinazolin-6-yl]méthyl}acétamide
n°22 : chlorhydrate de 1-(1-acétylpipéridin-4-yl)-6-(aminométhyl)-3-(3,4-diméthoxybenzyl)quinazoline-2,4(1H,3H)-dione
n°23 : N-{[3-(3,4-diméthoxybenzyl)-1-(1-formylpipéridin-4-yl)-2,4-dioxo-1,2,3,4-tétrahydroquinazolin-6-yl]méthyl}formamide
n°24 : N-{[1-(1-acétylpipéridin-4-yl)-3-(3,4-diméthoxybenzyl)-2,4-dioxo-1,2,3,4-tétrahydroquinazolin-6-yl]méthyl}formamide
n°25 : N-{[1-(1-acétylpipéridin-4-yl)-3-(3,4-diméthoxybenzyl)-2,4-dioxo-1,2,3,4-tétrahydroquinazolin-6-yl]méthyl}acétamide
n°32 : 4-[6-(2,2-difluoroéthoxy)-2,4-dioxo-3,4-dihydroquinazolin-1 (2H)-yl]pipéridine-1-carbaldéhyde
n° 33 4-[3-(3,4-dichlorobenzyl)-6-(2,2-difluoroéthoxy)-2,4-dioxo-3,4-dihydroquinazolin-1(2H)-yl]pipéridine-1-carbaldéhyde
n°34 : 4-[3-(4-chlorobenzyl)-6-(2,2-difluoroéthoxy)-2,4-dioxo-3,4-dihydroquinazolin-1(2H)-yl]pipéridine-1-carbaldéhyde
n°35 : 4-{[6-(2,2-difluoroéthoxy)-1-(1-formylpipéridin-4-yl)-2,4-dioxo-1,4-dihydroquinazolin-3(2H)-yl]méthyl}benzoate de méthyle
n°36 : acide 4-{[6-(2,2-difluoroéthoxy)-1-(1-formylpipéridin-4-yl)-2,4-dioxo-1,4-dihydroquinazolin-3(2H)-yl]méthyl}benzoïque
n°37 : 4-{[6-(2,2-difluoroéthoxy)-1-(1-formylpipéridin-4-yl)-2,4-dioxo-1,4-dihydroquinazolin-3(2H)-yl]méthyl}-N-(2-méthoxyéthyl)benzamide
n°38 : 4-[3-(3,4-diméthoxybenzyl)-6-méthyl-2,4-dioxo-3,4-dihydroquinazolin-1(2H)-yl]pipéridine-1-carbaldéhyde
n°39 : 4-[6-(2,2-difluoroéthoxy)-3-(3-hydroxy-4-méthoxybenzyl)-2,4-dioxo-3,4-dihydroquinazolin-1 (2H)-yl]pipéridine-1-carbaldéhyde
n°40 : 4-[6-(2,2-difluoroéthoxy)-3-[3-(2-hydroxyéthoxy)-4-méthoxybenzyl]-2,4-dioxo-3,4-dihydroquinazolin-1(2H)-yl]pipéridine-1-carbaldéhyde
n°41 : 4-[6-(2,2-difluoroéthoxy)-3-(3-éthoxy-4-méthoxybenzyl)-2,4-dioxo-3,4-dihydroquinazolin-1 (2H)-yl]pipéridine-1-carbaldéhyde
n°42 : 4-[6-(2,2-difluoroéthoxy)-3-[4-méthoxy-3-(2-méthoxyéthoxy)benzyl]-2,4-dioxo-3,4-dihydroquinazolin-1(2H)-yl]pipéridine-1-carbaldéhyde
n°43 : 4-[6-(2,2-difluoroéthoxy)-3-(3,4-diméthoxybenzyl)-2,4-dioxo-3,4-dihydroquinazolin-1 (2H)-yl]azépane-1-carbaldéhyde
n°47 : 4-[6-(2,2-difluoroéthoxy)-3-[3-(3-hydroxypropoxy)-4-méthoxybenzyl]-2,4-dioxo-3,4-dihydroquinazolin-1(2H)-yl]pipéridine-1-carbaldéhyde
n°48 : 4-[5-chloro-3-(3,4-diméthoxybenzyl)-2,4-dioxo-3,4-dihydroquinazolin-1(2H)-yl]pipéridine-1-carbaldéhyde
n°49 : 4-{3-[3-(cyclopentyloxy)-4-méthoxybenzyl]-6-(2,2-difluoroéthoxy)-2,4-dioxo-3,4-dihydroquinazolin-1(2H)-yl}pipéridine-1-carbaldéhyde
n°50 : 2-(5-{[6-(2,2-difluoroéthoxy)-1-(1-formylpipéridin-4-yl)-2,4-dioxo-1,4-dihydroquinazolin-3(2H)-yl]méthyl}-2-méthoxyphénoxy)acétamide
n°51 : 4-[6-(2,2-difluoroéthoxy)-3-(3,4-diméthoxybenzyl)-2,4-dioxo-3,4-dihydroquinazolin-1(2H)-yl]-3-méthylpipéridine-1-carbaldéhyde
n°52 : 3-[6-(2,2-difluoroéthoxy)-3-(3,4-diméthoxybenzyl)-2,4-dioxo-3,4-dihydroquinazolin-1(2H)-yl]-8-azabicyclo[3.2.1]octane-8-carbaldéhyde
n°56 : 4-{3-[4-(cyclopentyloxy)-3-méthoxybenzyl]-6-[2-fluoro-1-(fluorométhyl)éthoxy]-2,4-dioxo-3,4-dihydroquinazolin-1(2H)-yl}pipéridine-1-carbaldéhyde
n°57 : 4-[3-(3-chlorobenzyl)-6-[2-fluoro-1-(fluorométhyl)éthoxy]-2,4-dioxo-3,4-dihydroquinazolin-1(2H)-yl]pipéridine-1-carbaldéhyde
n°58 : 4-[3-(4-chlorobenzyl)-6-[2-fluoro-1-(fluorométhyl)éthoxy]-2,4-dioxo-3,4-dihydroquinazolin-1 (2H)-yl]pipéridine-1-carbaldéhyde
n°59 : 4-{3-[3-(cyclopentyloxy)-4-méthoxybenzyl]-6-[2-fluoro-1-(fluorométhyl)éthoxy]-2,4-dioxo-3,4-dihydroquinazolin-1(2H)-yl}pipéridine-1-carbaldéhyde
n°72 : 4-[3-(3,4-diméthoxybenzyl)-6-(2-hydroxyéthoxy)-2,4-dioxo-3,4-dihydroquinazolin-1(2H)-yl]pipéridine-1-carbaldéhyde
n°74 : 4-[3-(3,4-dichlorobenzyl)-6-[2-fluoro-1-(fluorométhyl)éthoxy]-2,4-dioxo-3,4-dihydroquinazolin-1(2H)-yl]pipéridine-1-carbaldéhyde
n°76 : 4-{3-[(6-chloropyridin-3-yl)méthyl]-6-[2-fluoro-1-(fluorométhyl)éthoxy]-2,4-dioxo-3,4-dihydroquinazolin-1(2H)-yl}pipéridine-1-carbaldéhyde
n°78 : 4-[3-(3-chloro-4-méthoxybenzyl)-6-[2-fluoro-1-(fluorométhyl)éthoxy]-2,4-dioxo-3,4-dihydroquinazolin-1(2H)-yl]pipéridine-1-carbaldéhyde
n°79 : 4-[3-(3,4-diméthoxybenzyl)-6-(2-fluoroéthoxy)-2,4-dioxo-3,4-dihydroquinazolin-1(2H)-yl]pipéridine-1-carbaldéhyde
n°89: 2-[5-({6-[2-fluoro-1-(fluorométhyl)éthoxy]-1-(1-formylpipéridin-4-yl)-2,4-dioxo-1,4-dihydroquinazolin-3(2H)-yl}méthyl)-2-méthoxyphénoxy]acétamide
n°90 : 4-{6-[2-fluoro-1-(fluorométhyl)éthoxy]-3-(3-hydroxy-4-méthoxybenzyl)-2,4-dioxo-3,4-dihydroquinazolin-1(2H)-yl}pipéridine-1-carbaldéhyde
n°91: 4-[3-(3,4-diméthoxybenzyl)-6-éthoxy-2,4-dioxo-3,4-dihydroquinazolin-1(2H)-yl]pipéridine-1-carbaldéhyde
n°97 : 4-[5,7-dichloro-3-(3,4-diméthoxybenzyl)-2,4-dioxo-3,4-dihydroquinazolin-1(2H)-yl]pipéridine-1-carbaldéhyde
n°102: 4-[7-chloro-3-(3,4-diméthoxybenzyl)-2,4-dioxo-3,4-dihydroquinazolin-1(2H)-yl]pipéridine-1-carbaldéhyde
n°108 : 4-{6-[2-fluoro-1-(fluorométhyl)éthoxy]-3-(3-fluoro-4-méthoxybenzyl)-2,4-dioxo-3,4-dihydroquinazolin-1(2H)-yl}pipéridine-1-carbaldéhyde
n°111 : 4-[6-(difluorométhoxy)-3-(3,4-diméthoxybenzyl)-2,4-dioxo-3,4-dihydroquinazolin-1(2H)-yl]pipéridine-1-carbaldéhyde
n°112 : 4-[3-(3,4-diméthoxybenzyl)-6-(1-méthyléthoxy)-2,4-dioxo-3,4-dihydroquinazolin-1(2H)-yl]pipéridine-1-carbaldéhyde
n°114 : 4-{6-[2-fluoro-1-(fluorométhyl)éthoxy]-3-[4-méthoxy-3-(1-méthyléthoxy)benzyl]-2,4-dioxo-3,4-dihydroquinazolin-1(2H)-yl}pipéridine-1-carbaldéhyde
n°116 : 4-{6-[2-fluoro-1-(fluorométhyl)éthoxy]-3-(3-méthoxybenzyl)-2,4-dioxo-3,4-dihydroquinazolin-1(2H)-yl}pipéridine-1-carbaldéhyde
n°117 : 4-{3-[3,5-bis(trifluorométhyl)benzyl]-6-[2-fluoro-1-(fluorométhyl)éthoxy]-2,4-dioxo-3,4-dihydroquinazolin-1(2H)-yl}pipéridine-1-carbaldéhyde
n°118 : 4-[3-(3-éthoxybenzyl)-6-[2-fluoro-1-(fluorométhyl)éthoxy]-2,4-dioxo-3,4-dihydroquinazolin-1(2H)-yl]pipéridine-1-carbaldéhyde
n°124 :4-{3-[3-chloro-4-(2-méthoxyéthoxy)benzyl]-6-[2-fluoro-1-(fluorométhyl)éthoxy]-2,4-dioxo-3,4-dihydroquinazolin-1(2H)-yl}pipéridine-1-carbaldéhyde
n°130 : 4-[3-(3,4-diéthoxybenzyl)-6-[2-fluoro-1-(fluorométhyl)éthoxy]-2,4-dioxo-3,4-dihydroquinazolin-1(2H)-yl]pipéridine-1-carbaldéhyde
n°131 : 4-[3-(4-éthoxy-3-méthoxybenzyl)-6-[2-fluoro-1-(fluorométhyl)éthoxy]-2,4-dioxo-3,4-dihydroquinazolin-1(2H)-yl]pipéridine-1-carbaldéhyde
n°133 : 4-{6-[2-fluoro-1-(fluorométhyl)éthoxy]-3-(4-méthoxy-3-méthylbenzyl)-2,4-dioxo-3,4-dihydroquinazolin-1(2H)-yl}pipéridine-1-carbaldéhyde
n°134 : 4-{6-[2-fluoro-1-(fluorométhyl)éthoxy]-2,4-dioxo-3-[4-(trifluorométhyl)benzyl]-3,4-dihydroquinazolin-1(2H)-yl}pipéridine-1-carbaldéhyde
n°135 : 4-{6-[2-fluoro-1-(fluorométhyl)éthoxy]-2,4-dioxo-3-[4-(trifluorométhyl)benzyl]-3,4-dihydroquinazolin-1(2H)-yl}pipéridine-1-carbaldéhyde
n°143 : 4-{3-[4-(benzyloxy)-3-méthoxybenzyl]-6-[2-fluoro-1-(fluorométhyl)éthoxy]-2,4-dioxo-3,4-dihydroquinazolin-1(2H)-yl}pipéridine-1-carbaldéhyde
n°145 : 4-{6-[2-fluoro-1-(fluorométhyl)éthoxy]-3-(3-méthoxy-4-nitrobenzyl)-2,4-dioxo-3,4-dihydroquinazolin-1(2H)-yl}pipéridine-1-carbaldéhyde
n°155 : 4-[3-(4-éthoxybenzyl)-6-[2-fluoro-1-(fluorométhyl)éthoxy]-2,4-dioxo-3,4-dihydroquinazolin-1 (2H)-yl]pipéridine-1-carbaldéhyde
n°158 : 4-{6-[2-fluoro-1-(fluorométhyl)éthoxy]-3-[4-(morpholin-4-ylméthyl)benzyl]-2,4-dioxo-3,4-dihydroquinazolin-1(2H)-yl}pipéridine-1-carbaldéhyde
n°160 : 4-{6-[2-fluoro-1-(fluorométhyl)éthoxy]-3-(4-morpholin-4-ylbenzyl)-2,4-dioxo-3,4-dihydroquinazolin-1(2H)-yl}pipéridine-1-carbaldéhyde
n°165 : 4-[3-(biphényl-4-ylméthyl)-6-[2-fluoro-1-(fluorométhyl)éthoxy]-2,4-dioxo-3,4-dihydroquinazolin-1 (2H)-yl]pipéridine-1-carbaldéhyde
n°166 : 4-{6-[2-fluoro-1-(fluorométhyl)éthoxy]-3-[4-(méthylsulfanyl)benzyl]-2,4-dioxo-3,4-dihydroquinazolin-1(2H)-yl}pipéridine-1-carbaldéhyde
n°167 : 4-{6-[2-fluoro-1-(fluorométhyl)éthoxy]-2,4-dioxo-3-(4-pyridin-3-ylbenzyl)-3,4-dihydroquinazolin-1 (2H)-yl}pipéridine-1-carbaldéhyde
n°170 : 4-{6-[2-fluoro-1-(fluorométhyl)éthoxy]-3-(3-méthoxy-4-méthylbenzyl)-2,4-dioxo-3,4-dihydroquinazolin-1(2H)-yl}pipéridine-1-carbaldéhyde
n°175 : 2-[2-(cyclopentyloxy)-5-({6-[2-fluoro-1-(fluorométhyl)éthoxy]-1-(1-formylpipéridin-4-yl)-2,4-dioxo-1,4-dihydroquinazolin-3(2H)-yl}méthyl)phénoxy]acétamide
n°178 : 4-{6-[2-fluoro-1-(fluorométhyl)éthoxy]-3-(3-méthoxy-4-propoxybenzyl)-2,4-dioxo-3,4-dihydroquinazolin-1(2H)-yl}pipéridine-1-carbaldéhyde
n°183 : 2-[2-(cyclopentyloxy)-5-({6-[2-fluoro-1-(fluorométhyl)éthoxy]-1-(1-formylpipéridin-4-yl)-2,4-dioxo-1,4-dihydroquinazolin-3(2H)-yl}méthyl)phénoxy]-N-méthylacétamide
n°184 : 2-[2-(cyclopentyloxy)-5-({6-[2-fluoro-1-(fluorométhyl)éthoxy]-1-(1-formylpipéridin-4-yl)-2,4-dioxo-1,4-dihydroquinazolin-3(2H)-yl}méthyl)phénoxy]-N,N-diméthylacétamide
n°185 : 2-[2-(cyclopentyloxy)-5-({6-[2-fluoro-1-(fluorométhyl)éthoxy]-1-(1-formylpipéridin-4-yl)-2,4-dioxo-1,4-dihydroquinazolin-3(2H)-yl}méthyl)phénoxy]-N-méthoxy-N-méthylacétamide
n°186 : :4-{3-[4-(cyclopentyloxy)-3-éthoxybenzyl]-6-[2-fluoro-1-(fluorométhyl)éthoxy]-2,4-dioxo-3,4-dihydroquinazolin-1(2H)-yl}pipéridine-1-carbaldéhyde
n°188 : 4-{3-[4-(cyclopentyloxy)-3-(1-méthyléthoxy)benzyl]-6-[2-fluoro-1-(fluorométhyl)éthoxy]-2,4-dioxo-3,4-dihydroquinazolin-1(2H)-yl}pipéridine-1-carbaldéhyde
n°189 : 4-{3-[4-(cyclopentyloxy)-3-propoxybenzyl]-6-[2-fluoro-1-(fluorométhyl)éthoxy]-2,4-dioxo-3,4-dihydroquinazolin-1(2H)-yl}pipéridine-1-carbaldéhyde
n°190 : 4-{3-[4-(cyclopentyloxy)-3-hydroxybenzyl]-6-[2-fluoro-1-(fluorométhyl)éthoxy]-2,4-dioxo-3,4-dihydroquinazolin-1(2H)-yl}pipéridine-1-carbaldéhyde
n°193 : 4-{3-[4-(difluorométhoxy)-3-méthoxybenzyl]-6-[2-fluoro-1-(fluorométhyl)éthoxy]-2,4-dioxo-3,4-dihydroquinazolin-1(2H)-yl}pipéridine-1-carbaldéhyde
n°194 : :4-{3-[4-(difluorométhoxy)-3-éthoxybenzyl]-6-[2-fluoro-1-(fluorométhyl)éthoxy]-2,4-dioxo-3,4-dihydroquinazolin-1 (2H)-yl}pipéridine-1-carbaldéhyde
n°200 : 4-{6-[2-fluoro-1-(fluorométhyl)éthoxy]-2,4-dioxo-3-(4-thiophén-3-ylbenzyl)-3,4-dihydroquinazolin-1(2H)-yl}pipéridine-1-carbaldéhyde
n°201 : 4-{6-[2-fluoro-1-(fluorométhyl)éthoxy]-2,4-dioxo-3-(4-pyridin-4-ylbenzyl)-3,4-dihydroquinazolin-1(2H)-yl}pipéridine-1-carbaldéhyde
n°203 : 4-{6-[2-fluoro-1-(fluorométhyl)éthoxy]-3-[(1-méthyl-1H-indol-6-yl)méthyl]-2,4-dioxo-3,4-dihydroquinazolin-1(2H)-yl}pipéridine-1-carbaldéhyde
n°206 : 4-{3-[4-(cyclopropylméthoxy)-3-méthoxybenzyl]-6-[2-fluoro-1-(fluorométhyl)éthoxy]-2,4-dioxo-3,4-dihydroquinazolin-1(2H)-yl}pipéridine-1-carbaldéhyde
n°207 : 2-[4-({6-[2-fluoro-1-(fluorométhyl)éthoxy]-1-(1-formylpipéridin-4-yl)-2,4-dioxo-1,4-dihydroquinazolin-3(2H)-yl}méthyl)-2-méthoxyphénoxy]-N-méthylacétamide
n°212 : 4-{6-[2-fluoro-1-(fluorométhyl)éthoxy]-2,4-dioxo-3-[4-(1H-pyrazol-1-yl)benzyl]-3,4-dihydroquinazolin-1(2H)-yl}pipéridine-1-carbaldéhyde
n°213 : 4-{6-[2-fluoro-1-(fluorométhyl)éthoxy]-2,4-dioxo-3-(4-pyridin-2-ylbenzyl)-3,4-dihydroquinazolin-1(2H)-yl}pipéridine-1-carbaldéhyde
n °215 : 4-{6-[2-fluoro-1-(fluorométhyl)éthoxy]-2,4-dioxo-3-(4-thiophén-2-ylbenzyl)-3,4-dihydroquinazolin-1(2H)-yl}pipéridine-1-carbaldéhyde
n°216 : 4-{6-[2-fluoro-1-(fluorométhyl)éthoxy]-2,4-dioxo-3-(quinoléin-7-ylméthyl)-3,4-dihydroquinazolin-1(2H)-yl}pipéridine-1-carbaldéhyde
n°218 : 4-{6-[2-fluoro-1-(fluorométhyl)éthoxy]-3-[(6-méthoxynaphtalén-2-yl)méthyl]-2,4-dioxo-3,4-dihydroquinazolin-1(2H)-yl}pipéridine-1-carbaldéhyde
n°223 : 4-{3-[4-(1H-benzimidazol-1-yl)benzyl]-6-[2-fluoro-1-(fluorométhyl)éthoxy]-2,4-dioxo-3,4-dihydroquinazolin-1(2H)-yl}pipéridine-1-carbaldéhyde
n°224 : 4-{6-[2-fluoro-1-(fluorométhyl)éthoxy]-3-[3-méthoxy-4-(2-méthylpropoxy)benzyl]-2,4-dioxo-3,4-dihydroquinazolin-1(2H)-yl}pipéridine-1-carbaldéhyde
n °226 : 4-{6-[2-fluoro-1-(fluorométhyl)éthoxy]-3-[3-méthoxy-4-(tétrahydrofuran-3-yloxy)benzyl]-2,4-dioxo-3,4-dihydroquinazolin-1(2H)-yl}pipéridine-1-carbaldéhyde
n °228 : 4-[3-{4-[(1-benzylpyrrolidin-3-yl)oxy]-3-méthoxybenzyl}-6-[2-fluoro-1-(fluorométhyl)éthoxy]-2,4-dioxo-3,4-dihydroquinazolin-1(2H)-yl]pipéridine-1-carbaldéhyde
n°230 : 4-[3-(1-benzothiophén-5-ylméthyl)-6-[2-fluoro-1-(fluorométhyl)éthoxy]-2,4-dioxo-3,4-dihydroquinazolin-1(2H)-yl]pipéridine-1-carbaldéhyde
n°232 : 4-{6-[2-fluoro-1-(fluorométhyl)éthoxy]-3-[3-méthoxy-4-(1-méthyléthoxy)benzyl]-2,4-dioxo-3,4-dihydroquinazolin-1(2H)-yl}pipéridine-1-carbaldéhyde
n°233 : 4-[3-(3,4-diméthoxybenzyl)-7-fluoro-6-[2-fluoro-1-(fluorométhyl)éthoxy]-2,4-dioxo-3,4-dihydroquinazolin-1(2H)-yl]pipéridine-1-carbaldéhyde
n°234 : 4-[3-{4-[(1-acétylpyrrolidin-3-yl)oxy]-3-méthoxybenzyl}-6-[2-fluoro-1-(fluorométhyl)éthoxy]-2,4-dioxo-3,4-dihydroquinazolin-1(2H)-yl]pipéridine-1-carbaldéhyde
n°239 : 4-[3-{4-[(4-fluorobenzyl)oxy]-3-méthoxybenzyl}-6-[2-fluoro-1-(fluorométhyl)éthoxy]-2,4-dioxo-3,4-dihydroquinazolin-1(2H)-yl]pipéridine-1-carbaldéhyde
n°240 : 4-[3-{4-[(4-chlorobenzyl)oxy]-3-méthoxybenzyl}-6-[2-fluoro-1-(fluorométhyl)éthoxy]-2,4-dioxo-3,4-dihydroquinazolin-1(2H)-yl]pipéridine-1-carbaldéhyde
n°242 : 4-[3-{4-[(3-chlorobenzyl)oxy]-3-méthoxybenzyl}-6-[2-fluoro-1-(fluorométhyl)éthoxy]-2,4-dioxo-3,4-dihydroquinazolin-1(2H)-yl]pipéridine-1-carbaldéhyde
n°243 : 4-{6-[2-fluoro-1-(fluorométhyl)éthoxy]-2,4-dioxo-3-(3-thiophén-3-ylbenzyl)-3,4-dihydroquinazolin-1 (2H)-yl}pipéridine-1-carbaldéhyde
n°245 : 4-[3-(4-éthoxy-3-méthoxybenzyl)-6-(2-hydroxyéthoxy)-2,4-dioxo-3,4-dihydroquinazolin-1(2H)-yl]pipéridine-1-carbaldéhyde
n°246 : 4-[3-{4-[2-(2,3-dihydro-1H-indol-1-yl)-2-oxoéthoxy]-3-méthoxybenzyl]-6-[2-fluoro-1-(fluorométhyl)éthoxy]-2,4-dioxo-3,4-dihydroquinazolin-1(2H)-yl]pipéridine-1-carbaldéhyde
n°250 : 4-[3-{4-[(3,4-dichlorobenzyl)oxy]-3-méthoxybenzyl}-6-[2-fluoro-1-(fluorométhyl)éthoxy]-2,4-dioxo-3,4-dihydroquinazolin-1(2H)-yl]pipéridine-1-carbaldéhyde
n°251 : 4-{6-[2-fluoro-1-(fluorométhyl)éthoxy]-3-[3-méthoxy-4-(2-oxo-2-pipéridin-1-yléthoxy)benzyl]-2,4-dioxo-3,4-dihydroquinazolin-1(2H)-yl}pipéridine-1-carbaldéhyde
n°254 : 4-{3-[3-éthoxy-4-(thiophén-2-ylméthoxy)benzyl]-6-[2-fluoro-1-(fluorométhyl)éthoxy]-2,4-dioxo-3,4-dihydroquinazolin-1(2H)-yl}pipéridine-1-carbaldéhyde
n°258 : 4-[3-(3,4-diméthoxybenzyl)-6-[2-fluoro-1-(hydroxyméthyl)éthoxy]-2,4-dioxo-3,4-dihydroquinazolin-1(2H)-yl]pipéridine-1-carbaldéhyde
n°263 : acide (2R)-2-[2-(cyclopentyloxy)-5-({6-[2-fluoro-1-(fluorométhyl)éthoxy]-1-(1-formylpipéridin-4-yl)-2,4-dioxo-1,4-dihydroquinazolin-3(2H)-yl}méthyl)phénoxy]propanoïque
n°264 : 4-{6-[2-fluoro-1-(fluorométhyl)éthoxy]-3-[(1-méthyl-3-thiophén-2-yl-1H-pyrazol-5-yl)méthyl]-2,4-dioxo-3,4-dihydroquinazolin-1(2H)-yl}pipéridine-1-carbaldéhyde
n°270 : 4-{6-[2-fluoro-1-(fluorométhyl)éthoxy]-3-[4-(5-méthyl-1,2,4-oxadiazol-3-yl)benzyl]-2,4-dioxo-3,4-dihydroquinazolin-1(2H)-yl}pipéridine-1-carbaldéhyde
n°275 : 4-{6-[2-fluoro-1-(fluorométhyl)éthoxy]-2,4-dioxo-3-(4-pyrimidin-5-ylbenzyl)-3,4-dihydroquinazolin-1(2H)-yl}pipéridine-1-carbaldéhyde
n°276 : 4-{6-[2-fluoro-1-(fluorométhyl)éthoxy]-3-[(1-méthyl-3-phényl-1H-pyrazol-5-yl)méthyl]-2,4-dioxo-3,4-dihydroquinazolin-1(2H)-yl}pipéridine-1-carbaldéhyde
n°278 : 4-{6-[2-fluoro-1-(fluorométhyl)éthoxy]-2,4-dioxo-3-{[6-(1H-pyrazol-1-yl)pyridin-3-yl]méthyl}-3,4-dihydroquinazolin-1(2H)-yl}pipéridine-1-carbaldéhyde
n°279 : 4-{6-[2-fluoro-1-(fluorométhyl)éthoxy]-2,4-dioxo-3-[(2-thiophén-2-ylpyrimidin-5-yl)méthyl]-3,4-dihydroquinazolin-1(2H)-yl}pipéridine-1-carbaldéhyde
n°280 : 4-{6-[2-fluoro-1-(fluorométhyl)éthoxy]-3-[4-(1-méthyl-1H-pyrazol-3-yl)benzyl]-2,4-dioxo-3,4-dihydroquinazolin-1(2H)-yl}pipéridine-1-carbaldéhyde
n°282 : 4-{6-[2-fluoro-1-(fluorométhyl)éthoxy]-3-[4-(3-méthyl-1,2,4-oxadiazol-5-yl)benzyl]-2,4-dioxo-3,4-dihydroquinazolin-1(2H)-yl}pipéridine-1-carbaldéhyde
n°283 : acide [2-(cyclopentyloxy)-5-({6-[2-fluoro-1-(fluorométhyl)éthoxy]-1-(1-formylpipéridin-4-yl)-2,4-dioxo-1,4-dihydroquinazolin-3(2H)-yl}méthyl)phénoxy]acétique
n°285 : 4-{6-[2-fluoro-1-(fluorométhyl)éthoxy]-2,4-dioxo-3-(thiéno[2,3-b]pyridin-2-ylméthyl)-3,4-dihydroquinazolin-1(2H)-yl}pipéridine-1-carbaldéhyde
n°286 : 4-{6-[2-fluoro-1-(fluorométhyl)éthoxy]-2,4-dioxo-3-[(6-phénylpyridin-3-yl)méthyl]-3,4-dihydroquinazolin-1(2H)-yl}pipéridine-1-carbaldéhyde
n°287 : 4-{6-[2-fluoro-1-(fluorométhyl)éthoxy]-3-[(6-morpholin-4-ylpyridin-3-yl)méthyl]-2,4-dioxo-3,4-dihydroquinazolin-1(2H)-yl}pipéridine-1-carbaldéhyde
n°289 : 4-{6-[2-fluoro-1-(fluorométhyl)éthoxy]-2,4-dioxo-3-[(6-thiophén-2-ylpyridin-3-yl)méthyl]-3,4-dihydroquinazolin-1(2H)-yl}pipéridine-1-carbaldéhyde
n°292 : 4-{6-[2-fluoro-1-(fluorométhyl)éthoxy]-3-[(1-méthyl-5-phényl-1H-pyrazol-3-yl)méthyl]-2,4-dioxo-3,4-dihydroquinazolin-1(2H)-yl}pipéridine-1-carbaldéhyde
n°294 : 4-({6-[2-fluoro-1-(fluorométhyl)éthoxy]-1-(1-formylpipéridin-4-yl)-2,4-dioxo-1,4-dihydroquinazolin-3(2H)-yl}méthyl)biphényl-2-carbonitrile
n°295 : (2R)-2-[2-(cyclopentyloxy)-5-({6-[2-fluoro-1-(fluorométhyl)éthoxy]-1-(1-formylpipéridin-4-yl)-2,4-dioxo-1,4-dihydroquinazolin-3(2H)-yl}méthyl)phénoxy]-N-méthylpropanamide
n°297 : 4-{7-fluoro-6-[2-fluoro-1-(fluorométhyl)éthoxy]-2,4-dioxo-3-(4-thiophén-2-ylbenzyl)-3,4-dihydroquinazolin-1(2H)-yl}pipéridine-1-carbaldéhyde
n°298 : 4-{6-[2-fluoro-1-(fluorométhyl)éthoxy]-3-[3-méthoxy-4-(morpholin-4-ylméthyl)benzyl]-2,4-dioxo-3,4-dihydroquinazolin-1(2H)-yl}pipéridine-1-carbaldéhyde
n°299 : 4-{6-[2-fluoro-1-(fluorométhyl)éthoxy]-3-[3-méthoxy-4-(pipéridin-1-ylméthyl)benzyl]-2,4-dioxo-3,4-dihydroquinazolin-1(2H)-yl}pipéridine-1-carbaldéhyde
n°300 :4-[3-{4-[(3,4-dichlorobenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde
n°301 : 2-[2-(cyclopentyloxy)-5-({6-[2-fluoro-1-(fluorométhyl)éthoxy]-1-(1-formylpipéridin-4-yl)-2,4-dioxo-1,4-dihydroquinazolin-3(2H)-yl}méthyl)phénoxy]-N-éthylacétamide
n°302 : acide (2S)-2-[2-(cyclopentyloxy)-5-({6-[2-fluoro-1-(fluorométhyl)éthoxy]-1-(1-formylpipéridin-4-yl)-2,4-dioxo-1,4-dihydroquinazolin-3(2H)-yl}méthyl)phénoxy]propanoïque
n°305 : 4-{6-[2-fluoro-1-(fluorométhyl)éthoxy]-3-(3-méthoxy-4-{[(3R)-2-oxo-1-phénylpyrrolidin-3-yl]oxy}benzyl)-2,4-dioxo-3,4-dihydroquinazolin-1(2H)-yl}pipéridine-1-carbaldéhyde
n°306 : 4-{3-[4-(cyclobutylméthoxy)-3-méthoxybenzyl]-6-[2-fluoro-1-(fluorométhyl)éthoxy]-2,4-dioxo-3,4-dihydroquinazolin-1(2H)-yl}pipéridine-1-carbaldéhyde
n°307 : 4-{3-[4-(benzyloxy)-3-méthoxybenzyl]-7-fluoro-6-[2-fluoro-1-(fluorométhyl)éthoxy]-2,4-dioxo-3,4-dihydroquinazolin-1(2H)-yl}pipéridine-1-carbaldéhyde
n °308 : 4-{7-fluoro-6-[2-fluoro-1-(fluorométhyl)éthoxy]-3-(4-hydroxy-3-méthoxybenzyl)-2,4-dioxo-3,4-dihydroquinazolin-1(2H)-yl}pipéridine-1-carbaldéhyde
n°309 : 4-{3-[4-(cyclopropylméthoxy)-3-méthoxybenzyl]-7-fluoro-6-[2-fluoro-1-(fluorométhyl)éthoxy]-2,4-dioxo-3,4-dihydroquinazolin-1(2H)-yl}pipéridine-1-carbaldéhyde
n °310 : 4-{7-fluoro-6-[2-fluoro-1-(fluorométhyl)éthoxy]-3-[3-méthoxy-4-(2-méthylpropoxy)benzyl]-2,4-dioxo-3,4-dihydroquinazolin-1(2H)-yl}pipéridine-1-carbaldéhyde
n°311 : 4-{7-fluoro-6-[2-fluoro-1-(fluorométhyl)éthoxy]-3-[3-méthoxy-4-(1-méthyléthoxy)benzyl]-2,4-dioxo-3,4-dihydroquinazolin-1(2H)-yl}pipéridine-1-carbaldéhyde
n°312 : 4-[3-(4-éthoxy-3-méthoxybenzyl)-7-fluoro-6-[2-fluoro-1-(fluorométhyl)éthoxy]-2,4-dioxo-3,4-dihydroquinazolin-1(2H)-yl]pipéridine-1-carbaldéhyde
n°315 : 4-{7-fluoro-6-[2-fluoro-1-(fluorométhyl)éthoxy]-3-{[6-(3-méthoxyphényl)pyridin-3-yl]méthyl}-2,4-dioxo-3,4-dihydroquinazolin-1(2H)-yl}pipéridine-1-carbaldéhyde
n°316 : 4-{7-fluoro-6-[2-fluoro-1-(fluorométhyl)éthoxy]-3-{[6-(2-fluorophényl)pyridin-3-yl]méthyl}-2,4-dioxo-3,4-dihydroquinazolin-1(2H)-yl}pipéridine-1-carbaldéhyde
n°317 : 4-{7-fluoro-6-[2-fluoro-1-(fluorométhyl)éthoxy]-3-{[6-(4-fluorophényl)pyridin-3-yl]méthyl}-2,4-dioxo-3,4-dihydroquinazolin-1(2H)-yl}pipéridine-1-carbaldéhyde
n°318 : 4-{7-fluoro-6-[2-fluoro-1-(fluorométhyl)éthoxy]-3-{[6-(4-méthoxyphényl)pyridin-3-yl]méthyl}-2,4-dioxo-3,4-dihydroquinazolin-1(2H)-yl}pipéridine-1-carbaldéhyde
n°319 : 4-{7-fluoro-6-[2-fluoro-1-(fluorométhyl)éthoxy]-2,4-dioxo-3-[(6-thiophén-2-ylpyridin-3-yl)méthyl]-3,4-dihydroquinazolin-1(2H)-yl}pipéridine-1-carbaldéhyde
n°320 : 4-{3-[3-éthoxy-4-(thiophén-2-ylméthoxy)benzyl]-7-fluoro-6-[2-fluoro-1-(fluorométhyl)éthoxy]-2,4-dioxo-3,4-dihydroquinazolin-1(2H)-yl}pipéridine-1-carbaldéhyde
n°321 : 4-{7-fluoro-6-[2-fluoro-1-(fluorométhyl)éthoxy]-3-[4-(1-méthyl-1H-pyrazol-3-yl)benzyl]-2,4-dioxo-3,4-dihydroquinazolin-1(2H)-yl}pipéridine-1-carbaldéhyde
n°322 : 4-{7-fluoro-6-[2-fluoro-1-(fluorométhyl)éthoxy]-2,4-dioxo-3-(4-pyrimidin-5-ylbenzyl)-3,4-dihydroquinazolin-1(2H)-yl}pipéridine-1-carbaldéhyde
n°323 : 4-{7-fluoro-6-[2-fluoro-1-(fluorométhyl)éthoxy]-3-[(1-méthyl-3-thiophén-2-yl-1H-pyrazol-5-yl)méthyl]-2,4-dioxo-3,4-dihydroquinazolin-1(2H)-yl}pipéridine-1-carbaldéhyde
n°324 : 4-{7-fluoro-6-[2-fluoro-1-(fluorométhyl)éthoxy]-3-[3-méthoxy-4-(2-oxo-2-pipéridin-1-yléthoxy)benzyl]-2,4-dioxo-3,4-dihydroquinazolin-1 (2H)-yl}pipéridine-1-carbaldéhyde
n°325 : 4-[3-{4-[2-(2,3-dihydro-1H-indol-1-yl)-2-oxoéthoxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde
n°326 : 4-{7-fluoro-6-[2-fluoro-1-(fluorométhyl)éthoxy]-3-[4-(5-méthyl-1,2,4-oxadiazol-3-yl)benzyl]-2,4-dioxo-3,4-dihydroquinazolin-1(2H)-yl}pipéridine-1-carbaldéhyde
n°327 : 4-[3-{4-[(3-chlorobenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde
n°328 : 4-[3-{[6-(3,5-dichlorophényl)pyridin-3-yl]méthyl}-6-[2-fluoro-1-(fluorométhyl)éthoxy]-2,4-dioxo-3,4-dihydroquinazolin-1(2H)-yl]pipéridine-1-carbaldéhyde
n°329 : 4-({7-fluoro-6-[2-fluoro-1-(fluorométhyl)éthoxy]-1-(1-formylpipéridin-4-yl)-2,4-dioxo-1,4-dihydroquinazolin-3(2H)-yl}méthyl)biphényl-2-carbonitrile
n°330 : 4-{7-fluoro-6-[2-fluoro-1-(fluorométhyl)éthoxy]-2,4-dioxo-3-[4-(1H-pyrazol-1-yl)benzyl]-3,4-dihydroquinazolin-1(2H)-yl}pipéridine-1-carbaldéhyde
n°331 :4-{7-fluoro-6-[2-fluoro-1-(fluorométhyl)éthoxy]-3-{[6-(3-fluorophényl)pyridin-3-yl]méthyl}-2,4-dioxo-3,4-dihydroquinazolin-1(2H)-yl}pipéridine-1-carbaldéhyde
n°332 : 3-[5-({7-fluoro-6-[2-fluoro-1-(fluorométhyl)éthoxy]-1-(1-formylpipéridin-4-yl)-2,4-dioxo-1,4-dihydroquinazolin-3(2H)-yl}méthyl)pyridin-2-yl]benzonitrile
n°333 : 4-[3-(3,4-diéthoxybenzyl)-7-fluoro-6-[2-fluoro-1-(fluorométhyl)éthoxy]-2,4-dioxo-3,4-dihydroquinazolin-1(2H)-yl]pipéridine-1-carbaldéhyde
n°334 : 4-[3-{4-[(4-chlorobenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde
n°335 : 4-{7-fluoro-6-[2-fluoro-1-(fluorométhyl)éthoxy]-3-[4-(morpholin-4-ylméthyl)benzyl]-2,4-dioxo-3,4-dihydroquinazolin-1(2H)-yl}pipéridine-1-carbaldéhyde
n°336 : 4-{7-fluoro-6-[2-fluoro-1-(fluorométhyl)éthoxy]-2,4-dioxo-3-{[6-(1H-pyrazol-1-yl)pyridin-3-yl]méthyl}-3,4-dihydroquinazolin-1(2H)-yl}pipéridine-1-carbaldéhyde
n°337 : 4-{7-fluoro-6-[2-fluoro-1-(fluorométhyl)éthoxy]-3-(4-morpholin-4-ylbenzyl)-2,4-dioxo-3,4-dihydroquinazolin-1(2H)-yl}pipéridine-1-carbaldéhyde
n°338 : 4-{7-fluoro-6-[2-fluoro-1-(fluorométhyl)éthoxy]-3-(3-méthoxy-4-propoxybenzyl)-2,4-dioxo-3,4-dihydroquinazolin-1(2H)-yl}pipéridine-1-carbaldéhyde
n°339 : 4-{3-[4-(1 H-benzimidazol-1-yl)benzyl]-7-fluoro-6-[2-fluoro-1-(fluorométhyl)éthoxy]-2,4-dioxo-3,4-dihydroquinazolin-1(2H)-yl}pipéridine-1-carbaldéhyde
n°340 : 5-({7-fluoro-6-[2-fluoro-1-(fluorométhyl)éthoxy]-1-(1-formylpipéridin-4-yl)-2,4-dioxo-1,4-dihydroquinazolin-3(2H)-yl}méthyl)-2-méthoxybenzonitrile
n°341 : 3-(3,4-diméthoxybenzyl)-6-[2-fluoro-1-(fluorométhyl)éthoxy]-1-(1-formylpipéridin-4-yl)-2,4-dioxo-1,2,3,4-tétrahydroquinazoline-7-carbonitrile
n°342 : 4-[3-(4-bromobenzyl)-7-fluoro-6-[2-fluoro-1-(fluorométhyl)éthoxy]-2,4-dioxo-3,4-dihydroquinazolin-1(2H)-yl]pipéridine-1-carbaldéhyde
n°343 : 4-[3-{4-[(3,4-dichlorobenzyl)oxy]-3-(2-méthoxyéthoxy)benzyl}-7-fluoro-6-[2-fluoro-1-(fluorométhyl)éthoxy]-2,4-dioxo-3,4-dihydroquinazolin-1(2H)-yl]pipéridine-1-carbaldéhyde
n°344 : 4-{3-[4-(benzyloxy)benzyl]-7-fluoro-6-[2-fluoro-1-(fluorométhyl)éthoxy]-2,4-dioxo-3,4-dihydroquinazolin-1(2H)-yl}pipéridine-1-carbaldéhyde
n°345 : 4-[3-{4-[(3,4-dichlorobenzyl)oxy]-3-éthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde
n°349 : 4-[3-{4-[(3,4-dichlorobenzyl)oxy]-3-(2-fluoroéthoxy)benzyl}-7-fluoro-6-[2-fluoro-1-(fluorométhyl)éthoxy]-2,4-dioxo-3,4-dihydroquinazolin-1(2H)-yl]pipéridine-1-carbaldéhyde
n°350 : 4-[3-{4-[(2-chloro-4-fluorobenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde
n°351 : 4-[3-{4-[(2,4-dichlorobenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde
n°352 : 4-[3-{4-[(2-chloro-6-fluorobenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde
n°353 : 4-[3-{4-[(2,6-dichlorobenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde
n°354 : 4-[3-{4-[(2-chlorobenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde
n°355 : 4-[7-fluoro-3-{4-[(2-fluorobenzyl)oxy]-3-méthoxybenzyl}-6-[2-fluoro-1-(fluorométhyl)éthoxy]-2,4-dioxo-3,4-dihydroquinazolin-1(2H)-yl]pipéridine-1-carbaldéhyde
n°357 : 2-[(3,4-dichlorobenzyl)oxy]-5-({7-fluoro-6-[2-fluoro-1-(fluorométhyl)éthoxy]-1-(1-formylpipéridin-4-yl)-2,4-dioxo-1,4-dihydroquinazolin-3(2H)-yl}méthyl)benzonitrile
n°358 : 4-[3-{4-[(3,4-dichlorophénoxy)méthyl]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde
n°360 : 4-{7-fluoro-6-[2-fluoro-1-(fluorométhyl)éthoxy]-2,4-dioxo-3-[4-(2-phényléthyl)benzyl]-3,4-dihydroquinazolin-1(2H)-yl}pipéridine-1-carbaldéhyde
n°362 : 4-[3-{4-[(4,5-dichloro-2-fluorobenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde n°369 : 4-[3-{4-[(4-chlorophénoxy)méthyl]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde
n°371 : 4-[3-{3-chloro-4-[(4-chlorobenzyl)oxy]-5-éthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde
n°373 : 4-[3-{3-chloro-4-[(2,4-dichlorobenzyl)oxy]-5-éthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde
n°375 : 4-[7-fluoro-3-{4-[(4-fluorobenzyl)oxy]-3-méthoxybenzyl}-6-[2-fluoro-1-(fluorométhyl)éthoxy]-2,4-dioxo-3,4-dihydroquinazolin-1(2H)-yl]pipéridine-1-carbaldéhyde
n°376 : 4-[3-{4-[(3,5-dichlorobenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde
n°377 : 4-[3-(4-{[4-chloro-3-(trifluorométhyl)benzyl]oxy}-3-méthoxybenzyl)-7-fluoro-6-[2-fluoro-1-(fluorométhyl)éthoxy]-2,4-dioxo-3,4-dihydroquinazolin-1(2H)-yl]pipéridine-1-carbaldéhyde n°379 : 4-[3-{4-[(3-chlorophénoxy)méthyl]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde
n°380 : 4-[3-{4-[(3,5-difluorobenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde
n°381 : 4-{3-[4-(benzyloxy)-3-méthoxybenzyl]-7-fluoro-6-[2-fluoro-1-(fluorométhyl)éthoxy]-2,4-dioxo-3,4-dihydroquinazolin-1(2H)-yl}pipéridine-1-carbaldéhyde
n°382 : 4-[3-{4-[(3-chloro-5-fluorobenzyl)oxy]-3-méthoxybenzyl}-7-fluoro-6-[2-fluoro-1-(fluorométhyl)éthoxy]-2,4-dioxo-3,4-dihydroquinazolin-1 (2H)-yl]pipéridine-1-carbaldéhyde
n°383 : 4-{7-fluoro-6-[2-fluoro-1-(fluorométhyl)éthoxy]-3-(3-méthoxy-4-{[4-(trifluorométhyl)benzyl]oxy}benzyl)-2,4-dioxo-3,4-dihydroquinazolin-1(2H)-yl}pipéridine-1-carbaldéhyde
n°384 : 4-[3-{4-[(2,5-dichlorobenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde
n°385 : 4-{[4-({7-fluoro-6-[2-fluoro-1-(fluorométhyl)éthoxy]-1-(1-formylpipéridin-4-yl)-2,4-dioxo-1,4-dihydroquinazolin-3(2H)-yl}méthyl)-2-méthoxyphénoxy]méthyl}benzonitrile
n°386 3-{[4-({7-fluoro-6-[2-fluoro-1-(fluorométhyl)éthoxy]-1-(1-formylpipéridin-4-yl)-2,4-dioxo-1,4-dihydroquinazolin-3(2H)-yl}méthyl)-2-méthoxyphénoxy]méthyl}benzonitrile
n°387 : 4-[3-{4-[(4-chloro-2-fluorobenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde
n°388 : 4-[3-{4-[1-(3,4-dichlorophényl)éthoxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde
n°389 : 4-{7-fluoro-6-[2-fluoro-1-(fluorométhyl)éthoxy]-3-{4-[(3-hydroxybenzyl)oxy]-3-méthoxybenzyl}-2,4-dioxo-3,4-dihydroquinazolin-1(2H)-yl}pipéridine-1-carbaldéhyde
n°390 : 4-[7-fluoro-3-{4-[(3-fluorobenzyl)oxy]-3-méthoxybenzyl}-6-[2-fluoro-1-(fluorométhyl)éthoxy]-2,4-dioxo-3,4-dihydroquinazolin-1(2H)-yl]pipéridine-1-carbaldéhyde
n°391 : 4-[3-{4-[(3,4-difluorobenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde
n°392 : 4-{3-[4-(5,6-dichloro-1H-benzimidazol-1-yl)-3-méthoxybenzyl]-7-fluoro-6-[2-fluoro-1-(fluorométhyl)éthoxy]-2,4-dioxo-3,4-dihydroquinazolin-1(2H)-yl}pipéridine-1-carbaldéhyde
n°393 : 3,4-dichlorobenzènesulfonate de 4-({7-fluoro-6-[2-fluoro-1-(fluorométhyl)éthoxy]-1-(1-formylpipéridin-4-yl)-2,4-dioxo-1,4-dihydroquinazolin-3(2H)-yl}méthyl)phényle
n°394 : 3,4-dichlorobenzènesulfonate de 4-({7-fluoro-6-[2-fluoro-1-(fluorométhyl)éthoxy]-1-(1-formylpipéridin-4-yl)-2,4-dioxo-1,4-dihydroquinazolin-3(2H)-yl}méthyl)-2-méthoxyphényle
n°403 : 3,4-dichloro-N-[4-({7-fluoro-6-[2-fluoro-1-(fluorométhyl)éthoxy]-1-(1-formylpipéridin-4-yl)-2,4-dioxo-1,4-dihydroquinazolin-3(2H)-yl}méthyl)-2-méthoxyphényl]benzamide

Les composés conformes à l'invention peuvent être préparés par les méthodes illustrées dans les schémas 1 à 4 qui suivent.

Dans ce qui suit, on entend par groupe partant, un groupe pouvant être facilement substitué, avec départ d'une paire électronique, par rupture d'une liaison hétérolytique. Ce groupe peut ainsi être remplacé facilement par un autre groupe, lors d'une réaction de substitution par exemple. De tels groupes partants sont, par exemple, les halogènes, ou un groupe hydroxy activé tel qu'un mésylate, tosylate, triflate, etc. Des exemples de groupes partants ainsi que des références pour leur préparation sont donnés dans « Advanced Organic Chemistry », J. March, 3rd Edition, Wiley Interscience, p 310-316.

On entend par groupe protecteur PG, un groupe permettant d'empêcher la réactivité d'une fonction ou position, lors d'une réaction chimique pouvant l'affecter, et qui restitue la molécule après clivage selon des méthodes connues de l'homme du métier.

Par groupe protecteur temporaire des amines ou alcools, on entend les groupes protecteurs tels que ceux décrits dans Protective Groups in Organic Synthesis, Greene T.W. et Wuts P.G.M., Ed. Willey Intersciences 1999 et dans Protecting Groups, Kocienski P.J., 1994, Georg Thieme Verlag.

On peut citer par exemple des groupements protecteurs temporaires des amines : benzyles, carbamates, (tels que tert-butyloxycarbonyle clivable en milieu acide, benzyloxycarbonyle clivable par hydrogénolyse), des groupements protecteurs temporaires d'acides carboxyliques : esters d'alkyle (tels que méthyle ou éthyle, *tert-*butyle hydrolysables en milieu basique ou acide) et benzyliques hydrogénolysables, des groupements protecteurs temporaires d'alcools ou de phénols tels que les éthers de tétrahydropyranyle, métyloxyméthyle ou méthyléthoxyméthyle, tert-butyle et benzyle, des groupements protecteurs temporaires de dérivés carbonylés tels que les acétals linéaires ou cycliques comme par exemple 1,3-dioxane-2-yle ou 1,3-dioxolan-2-yle ; et se référer aux méthodes générales bien connues décrites dans Protective Groups, cité ci-dessus.

Selon les cas, l'homme de l'art sera à même de choisir les groupes protecteurs appropriés. Les composés de formule (I) peuvent comporter des groupes précurseurs d'autres fonctions qui sont générées ultérieurement en une ou plusieurs autres étapes.

Dans les schémas généraux de synthèse qui suivent, les composés de départ et les réactifs, quand leur mode de préparation n'est pas décrit, sont disponibles dans le commerce ou décrits dans la littérature, ou bien peuvent être préparés selon des méthodes qui y sont décrites ou qui sont connues de l'homme du métier.

Les énantiomères purs des composés conformes à l'invention peuvent être obtenus à partir de précurseurs énantiomèriquement purs ou bien par chromatographie sur des phases chirales ou bien, lorsque les composés comportent des fonctions acides ou des amines par cristallisations sélectives de sels diastéréoisomèriques obtenus par réaction des composés (I) avec, respectivement, des amines ou des acides chiraux.

Les composés de formule générale (I) peuvent être obtenus selon les Schémas 1 à 4 qui suivent. Par souci de clarté, on a choisi le groupe R4 comme étant un hydrogène, p et p' représentent 1 et 2 respectivement et on a fixé les groupes R₂ et R₃ tels qu'indiqués dans les schémas. Cependant, il s'entend que R₄ peut être tel que défini dans la formule générale de (I), que R₂ et R₃ peuvent avoir les positions indiquées dans la formule générale (I), et que p et p' peuvent être tels que définis dans la formule générale de (I).

Les voies de synthèse décrites ci-après servent uniquement à titre illustratif et ne sont en aucun cas limitatives. L'homme du métier pourra appliquer sans difficultés l'enseignement ci-dessous aux composés de formule (I) pour lesquels R, R₁, R₂, R₃, R₄, Ra, Rb, Rc, m, n, p, p' et q sont tels que définis dans la formule générale (I).

Selon le schéma 1, le composé de formule (IV) est obtenu par une réaction d'amination réductrice en faisant réagir un composé de formule (II) dans lequel R' représente un groupe (C₁-C₆) alkyle, et R₂ est tel que défini pour le composé de formule (I), avec un composé de formule (III) en milieu acide et en présence d'un agent réducteur tel que le tri-acétoxy-borohydrure de sodium. Le groupe GP du composé de formule (III) est un groupe protecteur de la fonction amine, qui peut avantageusement être du tert-butyloxycarbonyle (boc). Le composé de formule (IV) ainsi formé est ensuite acylé selon les méthodes bien connues de l'homme du métier, avec un chloroformate d'alkyle ou d'aryle pour donner le composé de formule (V) dans lequel R" représente un groupe (C₁-C₆) alkyle ou un groupe aryle substitué. Une réaction d'hydrolyse en milieu basique permet d'obtenir les composés de formule (VI) qui par une réaction de couplage avec un composé de formule (VII), dans lequel R₃ est tel que défini pour le composé de formule (I), conduit aux composés de formule (VIII). Une réaction de cyclisation intra-moléculaire en milieu basique permet d'obtenir les dérivés de quinazolinedione de formule (IX). Le groupe GP protecteur de la fonction amine est ensuite clivé, en milieu acide par exemple lorsque GP est un boc, pour donner les composés de formule (la) qui par une réaction d'acylation donne les composés de formule (Ib).

Les composés de formule (la) sont des composés de formule (I) et peuvent servir d'intermédiaire à d'autres composés de formule (I), tels que les composés de formule (Ib).

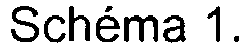

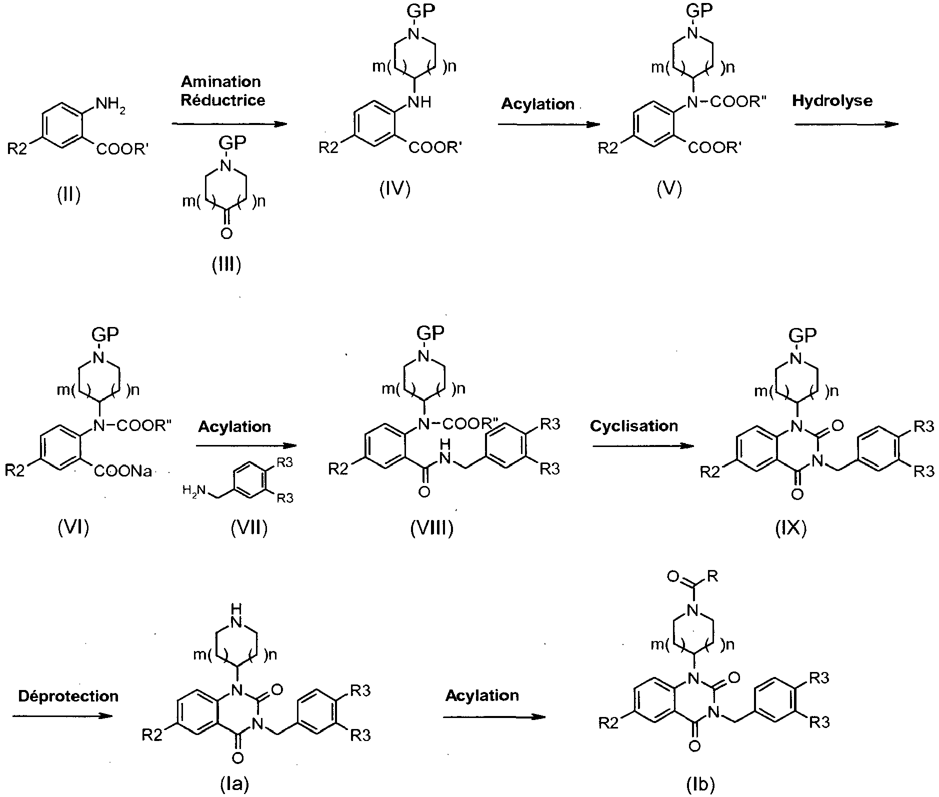

Les composés de formule (I) pour lesquels R₂ représente -ORa, Ra étant tel que défini pour le composé de formule (I), répondent à la formule (Id). Ils peuvent être obtenus à partir des composés de formule (X) selon le schéma 2 suivant. Les composés de formule (Ic), obtenus par une réaction d'hydrogénolyse des composés de formule (X), sont soumis par exemple à une réaction d'alkylation avec un agent alkylant de type Ra-X dans lequel Ra est tel que défini pour le composé de formule (I) et X représente un groupement partant (tel qu'un atome d'halogène par exemple) en présence d'une base telle que du carbonate de césium (Cs₂CO₃), ou encore à une réaction de Mitsunobu (Synthesis 1981, 1) avec un alcool de type Ra-OH, Ra étant tel que défini pour le composé de formule (I), pour donner des composés de formule (Id).

Les composés de formule (X) ainsi que les composés de formule (Ic) sont des composés de formule (I) et peuvent servir d'intermédiaire à d'autres composés de formule (I), tels que les composés de formule (Id).

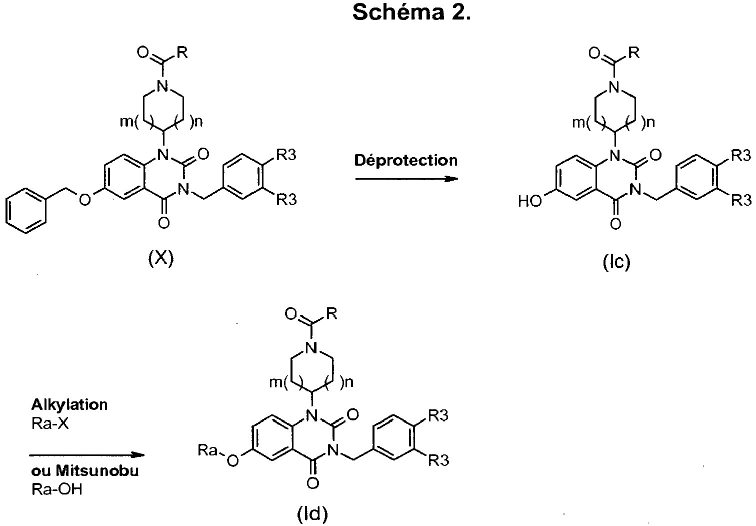

Alternativement les composés de formule (Id) peuvent être obtenus en suivant le procédé décrit dans le schéma 3.

Les composés de formule (XII) sont obtenus par une réaction de substitution nucléophile aromatique impliquant un composé de formule (XI) dans lequel R' est tel que défini précédemment et un alcool de type Ra-OH, dans lequel Ra est tel que défini pour le composé de formule (I), en présence d'une base. La réduction du groupement nitro des composés de formule (XII) conduit aux dérivés anilino correspondants (XIII). Une réaction d'amination réductrice avec un composé de formule (III), dans lequel GP
est un groupe protecteur de fonctions amines, tel par exemple que le boc, conduit aux composés de formule (XIV). L'obtention des composés de formule (XV) se fait par réaction d'un composé de formule (XIV) avec de l'isocyanate de potassium (KNCO) en milieu acide. Une réaction de cyclisation intra-moléculaire en milieu basique permet d'obtenir les composés de formule (XVI). Le groupement protecteur GP est clivé par des méthodes bien connues de l'homme de l'art pour donner les composés de formule (XVII). Une réaction d'acylation conduit aux composés de formule (XVIII). Finalement, on obtient les composés de formule (Id) soit par réaction d'alkylation avec un dérivé de type (XIX) dans lequel X représente un groupement partant tel qu'un atome d'halogène en présence d'une base comme par exemple le carbonate de césium, soit encore par une réaction de Mitsunobu avec un alcool benzylique de type (XX). Dans les composés (XIX) et (XX), R₃ est tel que défini précédemment.

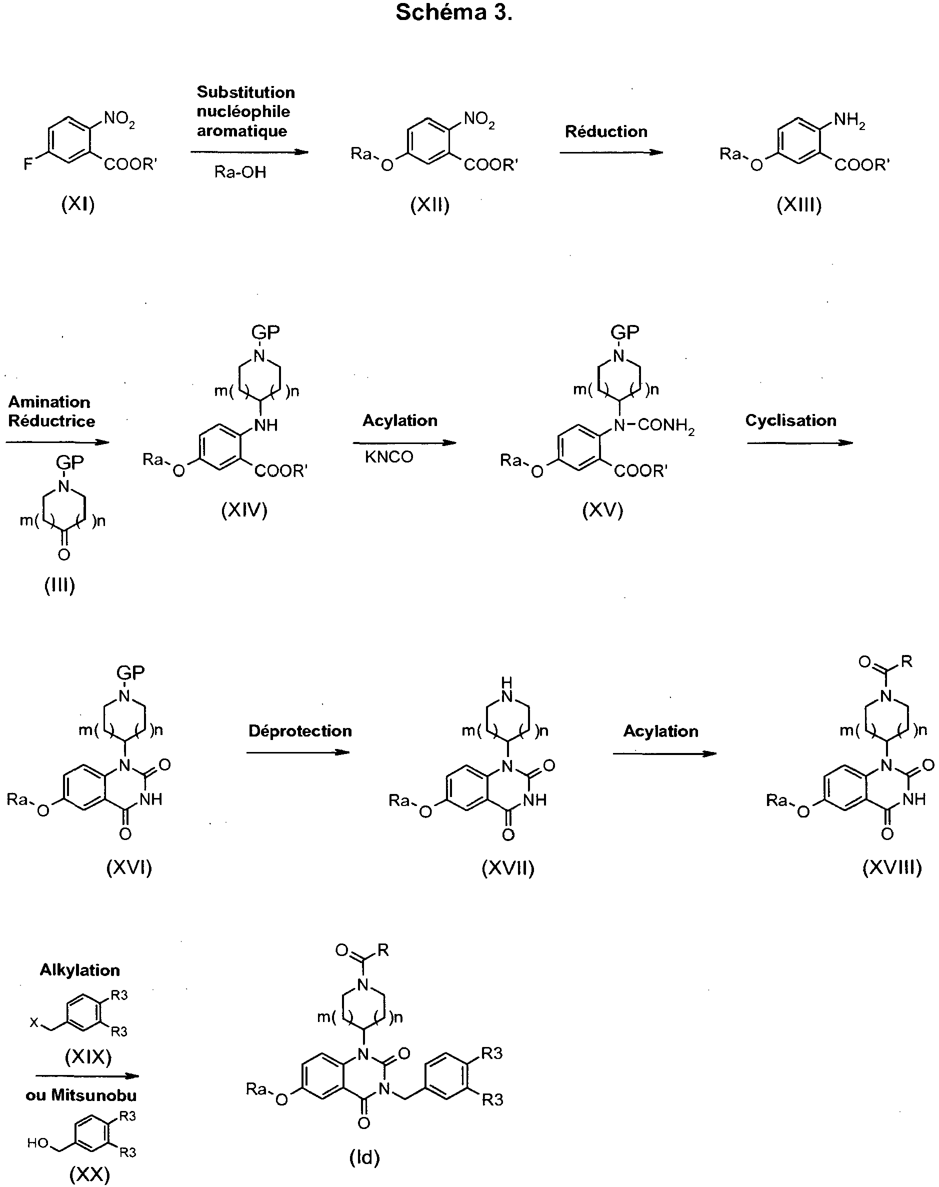

Les composés de formule (le) et (If) dans lesquels R₂ représente plus particulièrement un groupe de type -CH₂-NHC(O)Rb, Rb étant défini comme dans le composé de formule (I) peuvent être préparés selon le Schéma 4 suivant.

Il est entendu que dans le schéma 3, le groupement R2 illustré est de type -O-Ra et est en position 6 de la structure quinazoline-dione (voir par exemple composé (XVIII)), mais qu'il est également possible d'avoir un deuxième groupement R2 tel que déini dans la formule générale de (I) en position 7 du même groupement quinazoline-dione.

La réduction du groupement nitro des composés de type (XXI), dans lequel R' et GP' sont tels que définis précédemment, le groupe GP' étant avantageusement du boc, conduit aux dérivés anilino correspondants (XXII), qui par une réaction d'amination réductrice en faisant réagir en milieu acide et aventageusement en présence d'un agent réducteur tel que le tri-acétoxy-borohydrure de sodium, avec un composé de formule (III) dans lequel GP représente un groupement protecteur d'amine benzyloxycarbonyl, donnent des composés de formule (XXIII). Une réaction d'acylation avec un chloroformate d'alkyle ou d'aryle dans lequel R" représente un groupe (C₁-C₆) alkyle ou un groupe aryle substitué conduit aux composés de formule (XXIV). Les analogues de quinazolinedione de formule (XXV) peuvent être obtenus par une réaction d'hydrolyse en milieu basique puis par une réaction de couplage avec un composé de formule (VII) dans lequel R₃ est tel que défini pour le composé de formule (I), suivie par une réaction de cyclisation intra-moléculaire en milieu basique. Le groupe GP' (préférablement un boc) est ensuite clivé en milieu acide pour conduire aux composés de formule (XXVI), qui par acylation donnent des composés de formule (XXVII), dans lesquels Rb est tel que défini pour le composé de formule (I). Le groupe protecteur GP de (XXVII) est clivé par une réaction d'hydrogénolyse pour donner les composés de formule (le). Finalement les composés de formule (If) sont obtenus par une réaction d'acylation des composés de formule (le).

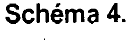

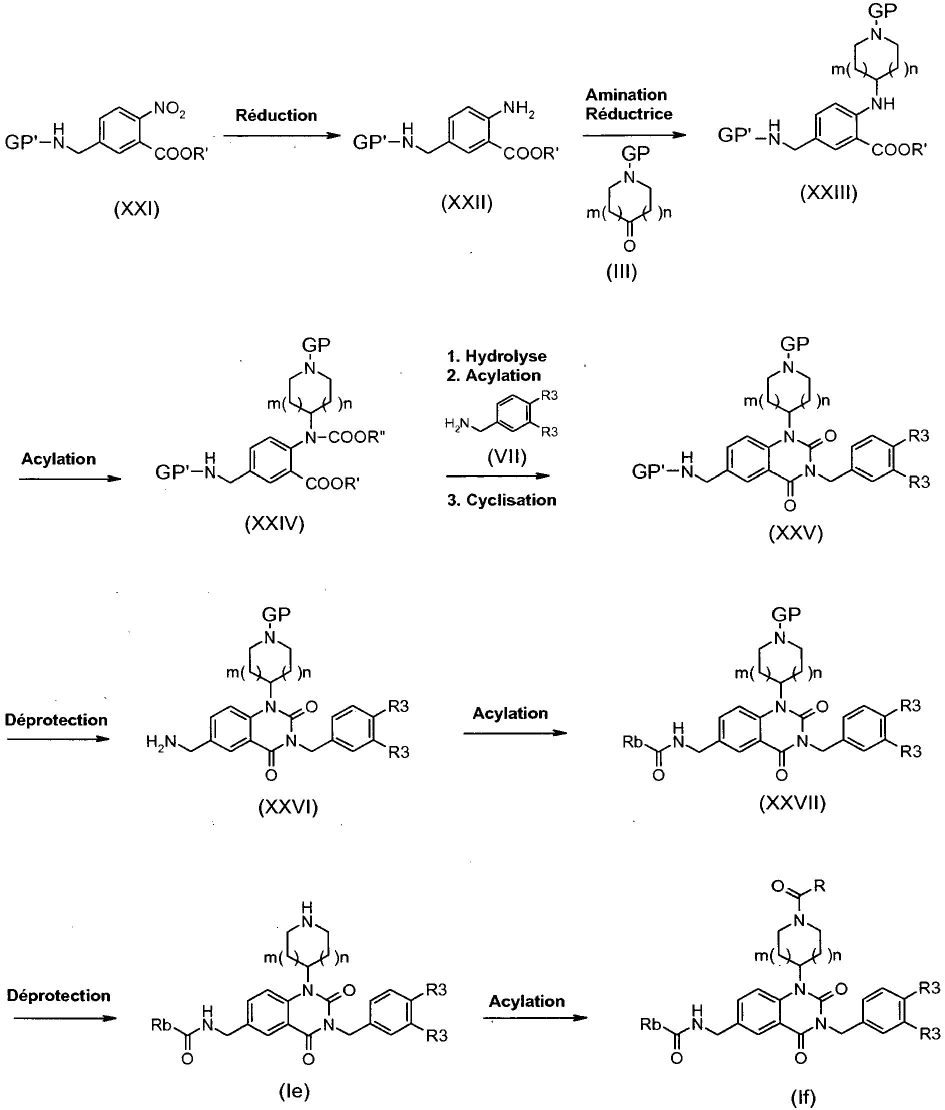

Il va de soi que l'homme du métier sera à même de choisir, à la lumière de ses connaissances et de la littérature, d'autres groupements protecteurs appropriés permettant l'introduction de tous les groupements décrits dans la formule générale (I).

Lorsque le composé de formule (I) comporte un cycle ponté, il peut être indifféremment obtenu par l'une des voies de synthèse décrites ci-dessus.

Les modes opératoires et exemples suivants décrivent la préparation de certains composés conformes à l'invention. Ces modes opératoires et exemples ne sont pas limitatifs et ne font qu'illustrer la présente invention.

Dans les modes opératoires et exemples ci-dessous :
- Les spectres de masse sont réalisés sur un spectromètre quadripolaire de type Platform LCZ (WATERS) ou de type ZQ 4000 (WATERS) en mode d'ionisation par électrospray positif ;
- Les spectres de RMN (résonnance magnétique nucléaire) sont réalisés sur un spectromètre à transformée de Fourier (BRUKER), à la température de 300°K (protons échangeables non enregistrés) ;
- s = singulet,
- d = doublet,
- m = multiplet,
- br = signal large (broad signal)
- t = triplet,
- q = quadruplet
- DMSO-d₆ = diméthylsulfoxyde deutéré
- CDCl₃ = chloroforme deutéré ;

Les mélanges de solvants sont quantifiés en rapports volumétriques ;

Les spectres RMN et spectres de masse confirment les structures des composés obtenus selon les exemples ci-dessous.

Dans les exemples qui suivent, on utilise les abréviations suivantes :
ACN : acétonitrile
AcOEt : acétate d'éthyle
AcOH : acide acétique
DBU : 1,8-diazabicyclo[5.4.0]undec-7-ène
DCM : dichlorométhane
DCE : 1,2-dichloroéthane
DIAD : diisopropyl azodicarboxylate
DIEA : di-isopropylamine
DMF : N,N-diméthylformamide
EtOH : éthanol
HBTU : O-(benzotriazol-1-yl)-N,N,N',N', tetraméthyluronium hexafluorophosphate
IBCF : isobutylchloroformate
MeOH : méthanol
NaBH(OAc)₃ : tri-acétoxy-borohydrure de sodium
TA : température ambiante
min : minute
THF : tétrahydrofurane
NEt₃ = triéthylamine
TFA : acide trifluoroacétique

### EXEMPLES

Les exemples suivants décrivent la préparation de certains composés conformes à l'invention. Ces exemples ne sont pas limitatifs et ne font qu'illustrer la présente invention. Les numéros des composés exemplifiés renvoient à ceux donnés dans le tableau ci-après, qui illustre les structures chimiques et les propriétés physiques de quelques composés conformes à l'invention.

### EXEMPLE 1 : composé n°6

### Préparation du {[3-(3,4-diméthoxybenzyl)-1-(1-formylpipéridin-4-yl)-2,4-dioxo-1,2,3,4-tétrahydroquinazolin-6-yl]oxy}acétonitrile

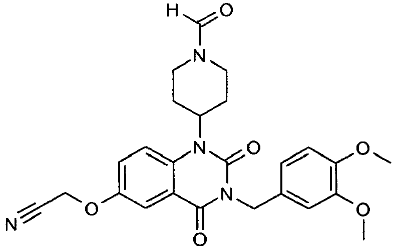

### Etape 1.1 :

### 4-{[4-(Benzyloxy)-2-(méthoxycarbonyl)phényl]amino}pipéridine-1-carboxylate de 1,1-diméthyléthyle

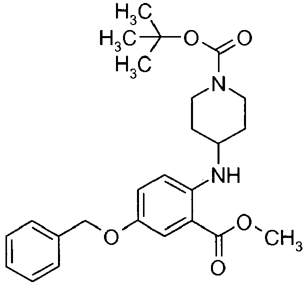

On irradie sous champ de micro-ondes (Biotage Initiator Sixty) pendant 20 min à 110°C, un mélange de 2 g de 2-amino-5-(benzyloxy)benzoate de méthyle, 3,1g de 4-oxopipéridine-1-carboxylate de 1,1-diméthyléthyle et 3,29 g de NaBH(OAc)₃ dans 10 ml de AcOH. On répète la même réaction avec 2 autres lots de 2 g de 2-amino-5-(benzyloxy)benzoate de méthyle. On rassemble les 3 milieux réactionnels. On reprend dans l'AcOEt. On lave la phase organique à l'eau, par une solution saturée de NH₄Cl, par une solution saturé de NaHCO₃, sèche sur Na₂SO₄, filtre et évapore le solvant sous pression réduite. On chromatographie le résidu sur gel de silice en éluant par un mélange AcOEt/Heptane (5/95, v/v) jusqu'à (30/70, v/v) pour donner 10,2 g du produit attendu.

### Etape 1.2 :

### 4-{[4-(Benzyloxy)-2-(méthoxycarbonyl)phényl][(2-méthylpropoxy)carbonyl]amino}pipéridine-1-carboxylate de 1,1-diméthyléthyle

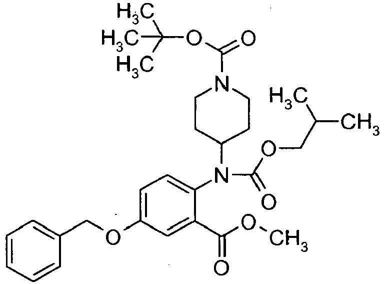

On irradie sous champ de micro-ondes pendant 30 min à 80°C, un mélange de 2 g de 4-{[4-(Benzy)oxy)-2-(méthoxycarbonyl)phényl]amino}pipéridine-1-carboxylate de 1,1-diméthyléthyle obtenu à l'étape 1.1, de 0,87 ml de DIEA, de 1,78 ml d'IBCF, 1g de NaOH dans 10 ml de DCE. On répète la même réaction avec 4 autres lots de 2 g de 4-{[4-(Benzyloxy)-2-(méthoxycarbonyl)phényl]amino}pipéridine-1-carboxylate de 1,1-diméthyléthyle. On rassemble les 5 milieux réactionnels. On reprend dans l'AcOEt, filtre et évapore le filtrat sous pression réduite. On chromatographie le résidu sur gel de silice en éluant par un mélange AcOEt/Heptane (10/90, v/v) jusqu'à (50/50, v/v) pour donner 9,3 g du produit attendu.

### Etape 1.3 :

Sel de sodium de l'acide 5-(benzyloxy)-2-({1-[(1,1-diméthyléthoxy)carbonyl] pipéridin-4-yl}[(2-méthylpropoxy)carbonyl]amino)benzoïque

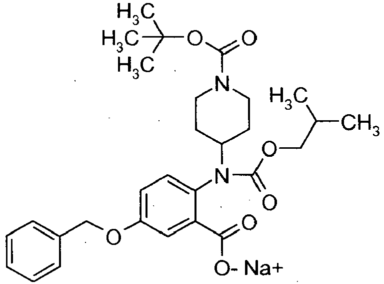

On chauffe 3h00 à 100°C un mélange de 9,3 g de 4-{[4-(Benzyloxy)-2-(méthoxycarbonyl)phényl][(2-méthylpropoxy)carbonyl]amino}pipéridine-1-carboxylate de 1,1-diméthyléthyle obtenu à l'étape 1.2, de 34,4 ml de NaOH 2N dans 57ml de MeOH. On évapore la solution sous pression réduite et on ajoute du DCM. On sèche sur Na₂SO₄, filtre et évapore le solvant sous pression réduite pour donner 8,7 g du produit attendu.

### Etape 1.4 :

### 4-[{4-(Benzyloxy)-2-[(3,4-diméthoxybenzyl)carbamoyl]phényl} (isobutoxycarbonyl)amino]pipéridine-1-carboxylate de 1,1-diméthyléthyle

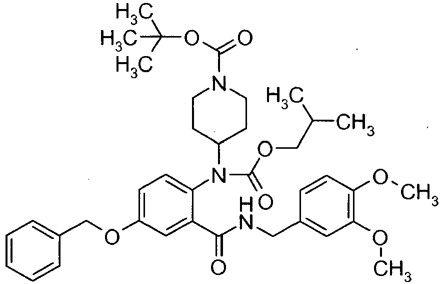

On agite 15 min à TA un mélange de 6g de sel de sodium de l'acide 5-(benzyloxy)-2-({1-[(1,1-diméthyléthoxy)carbonyl]pipéridin-4-yl}[(2-méthylpropoxy)carbonyl] amino)benzoïque obtenu à l'étape 1.3, de 4,42 g de DIEA dans 250 ml de DMF. On ajoute de 6,48 g d'HBTU et on laisse agiter pendant 30 min. On ajoute 2,48 g de vératrylamine et on agite le mélange réactionnel pendant 48h00. On évapore sous pression réduite, reprend le résidu dans de l'AcOEt, lave avec une solution saturée de NH₄Cl, par une solution saturé de NaHCO₃, sèche sur Na₂SO₄, filtre et évapore le solvant sous pression réduite. On chromatographie le résidu sur gel de silice en éluant par un mélange AcOEt/Heptane (20/80, v/v) jusqu'à (60/40, v/v) pour donner 7,5 g du produit attendu.

### Etape 1.5 :

### 4-[6-(Benzyloxy)-3-(3,4-diméthoxybenzyl)-2,4-dioxo-3,4-dihydroquinazolin-1(2H)-yl]pipéridine-1-carboxylate de 1,1-diméthyléthyle

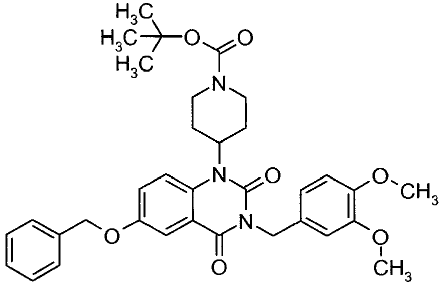

On irradie sous champ de micro-ondes pendant 30 min à 110°C, un mélange de 2.5g de 4-[{4-(benzyloxy)-2-[(3,4-diméthoxybenzyl)carbamoyl]phényl}(isobutoxycarbonyl) amino]pipéridine-1-carboxylate de 1,1-diméthyléthyle obtenu à l'étape 1.4, 7,4 g de NaOH dans 18,5 ml de DCE. On répète la même réaction avec 2 autres lots de 2,5 g de 4-[{4-(benzyloxy)-2-[(3,4-diméthoxybenzyl)carbamoyl]phényl} (isobutoxycarbonyl)amino]pipéridine-1-carboxylate de 1,1-diméthyléthyl. On rassemble les 3 milieux réactionnels. On reprend dans du DCM, lave à l'eau, sèche sur Na₂SO₄, filtre et évapore le solvant sous pression réduite pour donner 6,6 g du produit attendu.

### Etape 1.6 :

### 6-(Benzyloxy)-3-(3,4-diméthoxybenzyl)-1-pipéridin-4-ylquinazoline-2,4(1H,3H)-dione

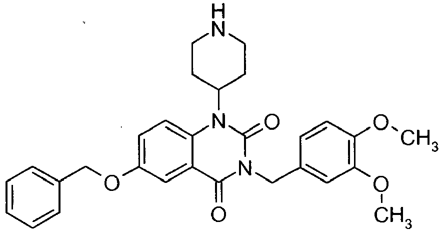

On agite pendant 2h00 à TA un mélange de 3,5 g de 4-[6-(benzyloxy)-3-(3,4-diméthoxybenzyl)-2,4-dioxo-3,4-dihydroquinazolin-1(2H)-yl]pipéridine-1-carboxylate de 1,1-diméthyléthyle obtenu à l'étape 1.5 et 25 ml de TFA dans 50 ml de DCM. On neutralise avec du K₂CO₃. On filtre, évapore le filtrat sous pression réduite. On reprend dans du DCM, lave avec une solution saturée de NaHCO₃, puis par une solution de NaOH à 8%. On sèche sur Na₂SO₄, filtre et évapore le solvant sous pression réduite pour donner 2,67 g du produit attendu.

### Etape 1.7 :

### 4-[6-(Benzyloxy)-3-(3,4-diméthoxybenzyl)-2,4-dioxo-3,4-dihydroquinazolin-1(2H)-yl]pipéridine-1-carbaldéhyde

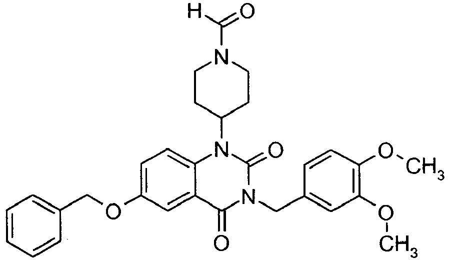

On irradie sous champ de micro-ondes pendant 1h00 à 140°C un mélange de 0,6 g de 6-(benzyloxy)-3-(3,4-diméthoxybenzyl)-1-pipéridin-4-ylquinazoline-2,4(1H,3H)-dione obtenu à l'étape 1.7, de 0,113 g de formiate d'ammonium dans 5 ml d'ACN. On filtre, évapore le filtrat sous pression réduite pour donner 0,62 g du produit attendu.

### Etape 1.8 : composé No.5 :

### 4-[3-(3,4-Diméthoxybenzyl)-6-hydroxy-2,4-dioxo-3,4-dihydroquinazolin-1(2H)-yl]pipéridine-1-carbaldéhyde

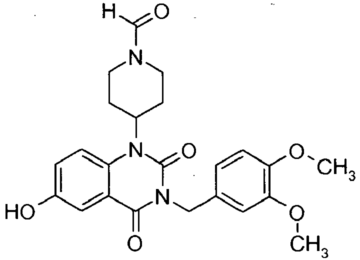

On irradie sous champ de micro-ondes pendant 2h00 à 80°C un mélange de 0,618 g de 4-[6-(benzyloxy)-3-(3,4-diméthoxybenzyl)-2,4-dioxo-3,4-dihydroquinazolin-1(2H)-yl]pipéridine-1-carbaldéhyde obtenu à l'étape 1.7, de 0,44 g de formiate d'ammonium et de 0,124 g de Pd/C (10%) dans 10ml d'EtOH préalablement purgé avec de l'azote. On filtre, évapore le filtrat sous pression réduite pour donner 0,513 g du produit attendu.

### Etape 1.9 : composé No.6

### {[3-(3,4-Diméthoxybenzyl)-1-(1-formylpipéridin-4-yl)-2,4-dioxo-1 ,2,3,4-tétrahydroquinazolin-6-yl]oxy}acétonitrile

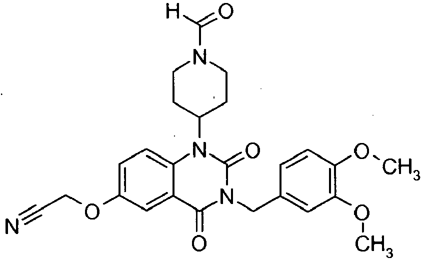

On agite à TA pendant 15 min 0,17 g de 4-[3-(3,4-diméthoxybenzyl)-6-hydroxy-2,4-dioxo-3,4-dihydroquinazolin-1(2H)-yl]pipéridine-1-carbaldéhyde obtenu à l'étape 1.8, de 0,25 g de Cs₂CO₃ dans 3ml de DMF. On ajoute 0,056 g de bromoacétonitrile et on irradie ensuite le mélange réactionnel sous champ de micro-ondes pendant 15 min à 100°C. On filtre, évapore sous pression réduite. On chromatographie le résidu sur gel de silice en éluant par un mélange MeOH/DCM (1/99, v/v) jusqu'à (4/96, v/v) pour donner 0,112 g du produit attendu.

### EXEMPLE 2 : Composé No.3

### Préparation du {[1-(1-acétylpipéridin-4-yl)-3-(3,4-diméthoxybenzyl)-2,4-dioxo-1,2,3,4-tétrahydroquinazolin-6-yl]oxy}acétonitrile

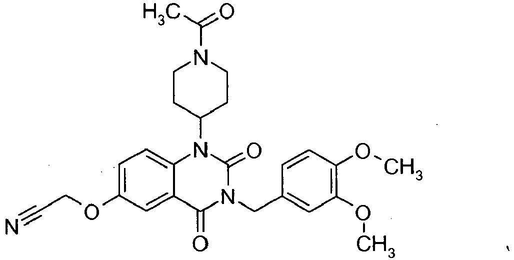

### Etape 2.1 :

### 1-(1-Acétylpipéridin-4-yl)-6-(benzyloxy)-3-(3,4-diméthoxybenzyl)quinazoline-2,4(1H,3H)-dione

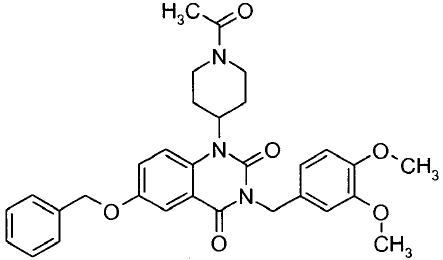

On ajoute 0,14 g de chlorure d'acétyle à un mélange de 0,6 g de 6-(benzyloxy)-3-(3,4-diméthoxybenzyl)-1-pipéridin-4-ylquinazoline-2,4(1H,3H)-dione obtenu selon l'étape 1.6, de 0,24 g de NEt₃ dans 10 ml de DCM refroidit à 0°C. On agite à TA pendant toute la nuit. On lave 2 fois avec une solution saturée de NH₄Cl, filtre, puis évapore le filtrat sous pression réduite pour donner 0,64 g du produit attendu.

### Etape 2.2 : Composé No.2

### 1-(1-Acétylpipéridin-4-yl)-3-(3,4-diméthoxybenzyl)-6-hydroxyquinazoline-2,4(1H,3H)-dione

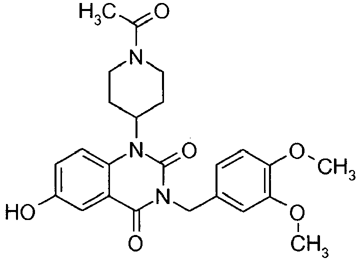

On irradie sous champ de micro-ondes pendant 2h00 à 80°C un mélange de 0,64 g de 1-(1-Acétylpipéridin-4-yl)-6-(benzyloxy)-3-(3,4-diméthoxybenzyl)quinazoline-2,4(1*H*,3*H*)-dione obtenu à l'étape 2.1, de 0,44 g de formiate d'ammonium et de 0,125 g de Pd/C (10%) dans 10 ml d'EtOH préalablement purgé avec de l'azote. On filtre, évapore le filtrat sous pression réduite pour donner 0,48 g du produit attendu.

### Etape 2.3 : Composé No.3

### {[1-(1-Acétylpipéridin-4-yl)-3-(3,4-diméthoxybenzyl)-2,4-dioxo-1 ,2,3,4-tétrahydroquinazolin-6-yl]oxy}acétonitrile

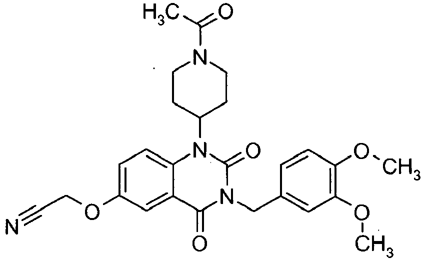

On agite à TA pendant 15min 0,12 g de 1-(1-acétylpipéridin-4-yl)-3-(3,4-diméthoxybenzyl)-6-hydroxyquinazoline-2,4(1H,3H)-dione obtenu à l'étape 2.2, de 0,172 g de Cs₂CO₃ dans 3ml de DMF. On ajoute 0,038 g de bromoacétonitrile et on irradie ensuite le mélange réactionnel sous champ de micro-ondes pendant 15 min à 100°C. On filtre, évapore le filtrat sous pression réduite. On chromatographie le résidu sur gel de silice en éluant par un mélange MeOH/DCM (1/99, v/v) jusqu'à (4/96, v/v) pour donner 0,094 g du produit attendu.

### EXEMPLE 3 : Composé n°34

### Synthèse du 4-[3-(4-chlorobenzyl)-6-(2,2-difluoroéthoxy)-2,4-dioxo-3,4-dihydroquinazolin-1(2H)-yl]pipéridine-1-carbaldéhyde

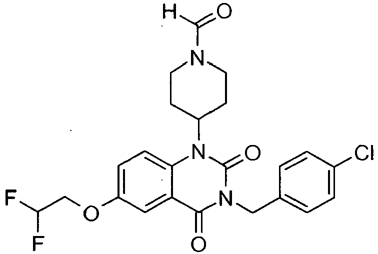

### Etape 3.1 :

### 5-(2,2-Difluoroéthoxy)-2-nitrobenzoate de méthyle

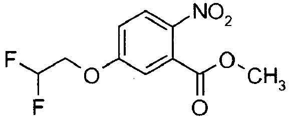

On ajoute 8,53 g de 2,2-difluoroéthanol à une solution de 17,31 g de 5-fluoro-2-nitrobenzoate de méthyle, de 9,64 g de NEt₃, de 32,71 g de 2,8,9-triisobutyl-2,5,8,9-tetraaza-1-phosphabicyclo[3.3.3]undecane dans 250 ml de THF anhydre. On agite 30 min à TA. On évapore le solvant sous pression réduite. On ajoute de l'eau et on extrait à l'AcOEt. On lave avec une solution aqueuse d'HCl 1 N, à l'eau puis avec une solution saturée de NaCl. On sèche sur MgSO₄, filtre, évapore le solvant sous pression réduite pour donner 21 g du produit attendu.

### Etape 3.2 :

### 2-Amino-5-(2,2-difluoroéthoxy)benzoate de méthyle

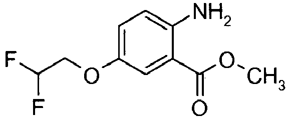

On agite sous atmosphère d'hydrogène pendant 24h00 à TA 21 g de 5-(2,2-difluoroéthoxy)-2-nitrobenzoate de méthyle obtenu à l'étape 3.1 et de 1 g de Pd/C (10%) dans un mélange de 300 ml d'AcOEt, de 50 ml d'EtOH et de 5 ml d'AcOH.

On filtre, évapore sous pression réduite pour donner 18,6 g du produit attendu.

### Etape 3.3 :

### 4-{[4-(2,2-Difluoroéthoxy)-2-(méthoxycarbonyl)phényl]amino}pipéridine-1-carboxylate de 1,1-diméthyléthyle

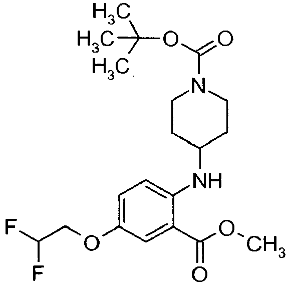

On chauffe à 90°C pendant 10 min un mélange de 4 g de 2-amino-5-(2,2-difluoroéthoxy)benzoate de méthyle, de 6,88 g de 4-oxopipéridine-1-carboxylate de 1,1-diméthyléthyle obtenu à l'étape 3.2 dans 15 ml d'AcOH. On laisse refroidir à TA et on ajoute 7,3 g de NaBH(OAc)₃. On laisse agiter 12h00 à TA. On extrait à l'AcOEt, on lave avec une solution saturée de K₂CO₃, puis à l'eau. On sèche sur MgSO₄, filtre, évapore sous pression réduite pour donner 6,63 g du produit attendu.

### Etape 3.4 :

### 4-{Carbamoyl[4-(2,2-difluoroéthoxy)-2-(méthoxycarbonyl)phényl] amino}pipéridine-1-carboxylate de 1,1-diméthyléthyle

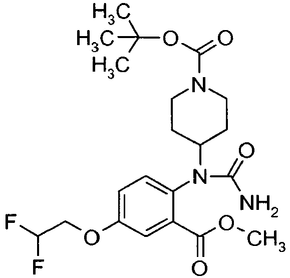

On ajoute 1,95 g d'isocyanate de potassium en solution dans 4 ml d'eau à une solution de 6,63 g de 4-{[4-(2,2-difluoroéthoxy)-2-(méthoxycarbonyl)phényl] amino}pipéridine-1-carboxylate de 1,1-diméthyléthyle obtenu à l'étape 3.3 dans 40 ml d'AcOH. On agite 12h00 à TA. On extrait à l'AcOEt, on lave avec une solution saturée de K₂CO₃, puis à l'eau. On sèche sur MgSO₄, filtre, évapore sous pression réduite pour donner 6,95 g du produit attendu.

### Etape 3.5 :

### 4-[6-(2,2-Difluoroéthoxy)-2,4-dioxo-3,4-dihydroquinazolin-1(2H)-yl]pipéridine-1-carboxylate de 1,1-diméthyléthyle

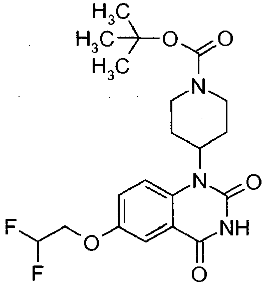

On irradie sous champ de micro-ondes pendant 30 min à 130°C, 2.5g de 4-{carbamoyl[4-(2,2-difluoroéthoxy)-2-(méthoxycarbonyl)phényl]amino}pipéridine-1-carboxylate de 1,1-diméthyléthyle obtenu à l'étape 3.4 en solution dans un mélange de 10 ml de dioxane et de 5ml d'une solution aqueuse de NaOH 1N. On extrait à l'AcOEt, on neutralise avec une solution aqueuse d'HCl 1N, lave à l'eau, sèche sur MgSO₄, filtre, évapore sous pression réduite. Le résidu obtenu est trituré dans un mélange AcOEt/pentane pour donner le produit attendu. La même réaction est reproduite avec 2 autres lots de 2,5 g de 4-{carbamoyl[4-(2,2-difluoroéthoxy)-2-(méthoxycarbonyl)phényl]amino} pipéridine-1-carboxylate de 1,1-diméthyléthyle obtenu à l'étape 3.4 pour donner au total 5,63 g du produit attendu.

### Etape 3.6 :

### 6-(2,2-Difluoroéthoxy)-1-pipéridin-4-ylquinazoline-2,4(1H,3H)-dione

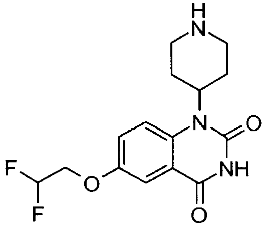

On agite 2h00 à TA une solution de 5,63 g de 4-[6-(2,2-difluoroéthoxy)-2,4-dioxo-3,4-dihydroquinazolin-1(2H)-yl]pipéridine-1-carboxylate de 1,1-diméthyléthyle obtenu à l'étape 3.5 dans 70 ml d'acide formique. On évapore le solvant sous pression réduite pour donner 6,13 g du produit attendu sous forme de sel d'acide formique.

### Etape 3.7 :

### 4-[6-(2,2-Difluoroéthoxy)-2,4-dioxo-3,4-dihydroquinazolin-1(2H)-yl]pipéridine-1-carbaldéhyde

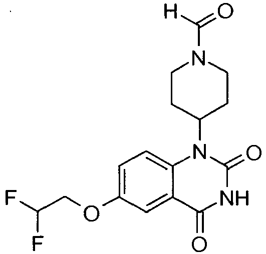

On irradie sous champ de micro-ondes pendant 1h00 à 140°C, un mélange de 6,13 g de 6-(2,2-difluoroéthoxy)-1-pipéridin-4-ylquinazoline-2,4(1H,3H)-dione obtenu à l'étape 3.6, de 3,12 g de formiate d'ammonium dans 28 ml d'ACN et 28ml de dioxane. On verse le mélange réactionnel dans de l'eau. On filtre, lave le précipité à l'eau puis à l'éther pour donner 4,47 g du produit attendu.

### Etape 3.8 : Composé n°34

### 4-[3-(4-Chlorobenzyl)-6-(2,2-difluoroéthoxy)-2,4-dioxo-3,4-dihydroquinazolin-1(2H)-yl]pipéridine-1-carbaldéhyde

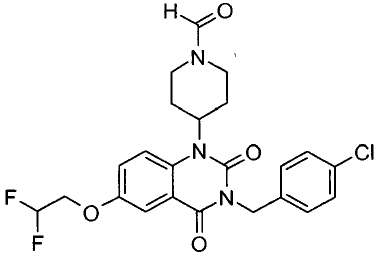

On agite 1h00 à TA un mélange de 0,15 g de 4-[6-(2,2-difluoroéthoxy)-2,4-dioxo-3,4-dihydroquinazolin-1(2*H*)-yl]pipéridine-1-carbaldéhyde obtenu à l'étape 3.7, 0,096 g de 1-(bromométhyl)-4-chlorobenzène et de 0,3 g de Cs₂CO₃ dans 3 ml de DMF. On ajoute de l'AcOEt, lave à l'eau puis avec une solution saturée de NaCl. On sèche sur MgSO₄, filtre, évapore sous pression réduite. On chromatographie le résidu sur gel de silice en éluant à l'AcOEt pour donner 0,116 g du produit attendu.

### Exemple 4 : Composé n°49

### Synthèse du 4-{3-[3-(cyclopentyloxy)-4-méthoxybenzyl]-6-(2,2-difluoroéthoxy)-2,4-dioxo-3,4-dihydroquinazolin-1(2H)-yl}pipéridine-1-carbaldéhyde

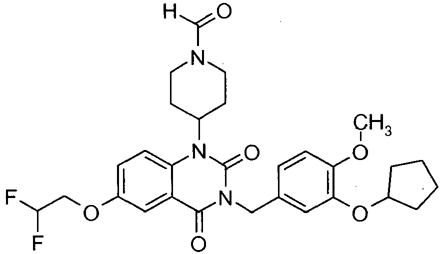

On ajoute 0,172 g de DIAD à une solution de 0,15 g de 4-[6-(2,2-difluoroéthoxy)-2,4-dioxo-3,4-dihydroquinazolin-1(2*H*)-yl]pipéridine-1-carbaldéhyde obtenu à l'étape 3.7, de 0,142 g de [4-(cyclopentyloxy)-3-méthoxyphényl]méthanol et de 0,223 g de PPh₃ dans 3 ml de THF anhydre. On agite 12h00 à TA puis 1h00 à 60°C. On évapore sous pression réduite et on purifie le résidu sur gel de silice en éluant à l'AcOEt pour donner 0,083 g du produit attendu.

### EXEMPLE 5 : Composé n°20

### Synthèse du N-{[3-(3,4-diméthoxybenzyl)-1-(1-formylpipéridin-4-yl)-2,4-dioxo-1,2,3,4-tétrahydroquinazolin-6-yl]méthyl}acétamide

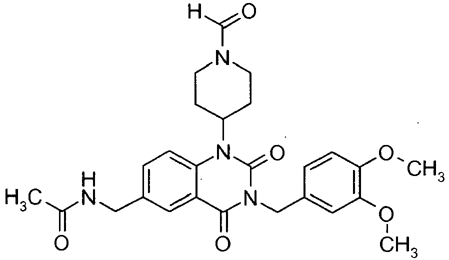

### Etape 5.1 :

### 2-Amino-5-({[(1,1-diméthyléthoxy)carbonyl]amino}méthyl)benzoate de méthyle

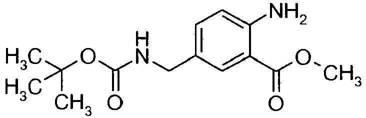

On irradie sous champ de micro-ondes pendant 5 min à 120°C un mélange de 0,273 g de 5-{[(tert-butoxycarbonyl)amino]méthyl}-2-nitrobenzoate de méthyle, 0,166 g de formiate d'ammonium et de 0,094 g de Pd/C (10%) dans 10 ml d'EtOH préalablement purgé avec de l'azote. On filtre, évapore le filtrat sous pression réduite. On chromatographie le résidu sur gel de silice en éluant par un mélange AcOEt/Heptane (5/95, v/v) jusqu'à (30/70, v/v) pour donner 0,2 g du produit attendu.

### Etape 5.2 :

### 4-{[4-({[(1,1-Diméthyléthoxy)carbonyl]amino}méthyl)-2-(méthoxycarbonyl)phényl]amino}pipéridine-1-carboxylate de benzyle

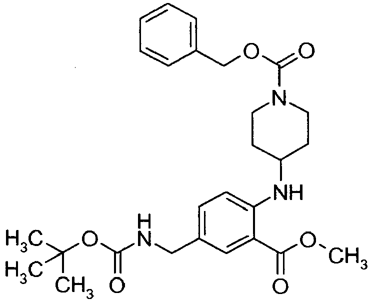

On ajoute goutte à goutte à TA une solution de 1,66 g de 4-oxopipéridine-1-carboxylate de benzyle et de 1g de 2-amino-5-({[(1,1-diméthyléthoxy) carbonyl]amino}méthyl)benzoate de méthyle obtenu à l'étape 5.1 dans 20 ml de DCM à une suspension de 2,04 g de NaBH(OAc)₃ dans un mélange de 20 ml de DCM et de 0,41 ml d'AcOH. On agite 15h00 à TA puis on rajoute 2,04 g de NaBH(OAc)₃. Après 6h00 d'agitation, on ajoute 1,66 g de 4-oxopipéridine-1-carboxylate de benzyle et on agite 48h00 à TA. On ajoute une solution saturée de NaHCO₃, on extrait au DCM. La phase organique est lavée avec une solution saturée de NaHCO₃, 2 fois avec une solution saturée de NH₄Cl. On sèche sur Na₂SO₄, filtre, évapore le filtrat sous pression réduite. On chromatographie le résidu sur gel de silice en éluant par un mélange AcOEt/Heptane (5/95, v/v) jusqu'à (40/60, v/v) pour donner 1,6 g du produit attendu.

### Etape 5.3 :

### 4-{[4-({[(1,1-Diméthyléthoxy)carbonyl]amino}méthyl)-2-(méthoxycarbonyl)phényl](éthoxycarbonyl)amino}pipéridine-1-carboxylate de benzyle

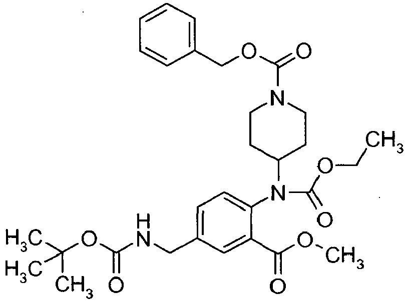

On ajoute 2,08 g de DIEA puis 1,745 g de chloroformate d'éthyle à une solution de 1,6 g de 4-{[4-({[(1,1-Diméthyléthoxy)carbonyl]amino}méthyl)-2-(méthoxycarbonyl) phényl]amino}pipéridine-1-carboxylate de benzyle obtenu à l'étape 5.2 dans 11 ml de DCM. On agite à TA pendant 4 jours. On évapore sous pression réduite. On reprend le résidu dans 10 ml de pyridine (10 ml) et on ajoute 0,7 g de chloroformate d'éthyle. On agite 4h00 à TA. On évapore sous pression réduite. On chromatographie le résidu sur gel de silice en éluant par un mélange AcOEt/Heptane (10/90, v/v) jusqu'à (30/70, v/v) pour donner 0,875 g du produit attendu.

### Etape 5.4 :

### 4-[3-(3,4-Diméthoxybenzyl)-6-({[(1,1-diméthyléthoxy)carbonyl]amino}méthyl)-2,4-dioxo-3,4-dihydroquinazolin-1(2H)-yl]pipéridine-1-carboxylate de benzyle

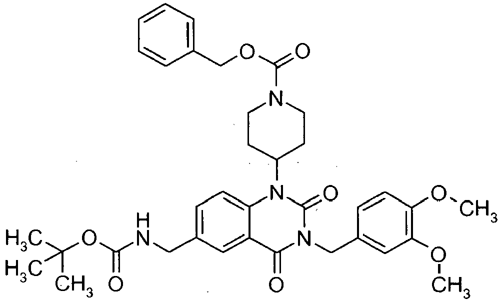

On agite 15h00 à TA un mélange de 0,165 g de 4-{[4-({[(1,1-Diméthy)éthoxy) carbonyl]amino}méthyl)-2-(méthoxycarbonyl)phényl](éthoxycarbonyl) amino}pipéridine-1-carboxylate de benzyle obtenu à l'étape 5.3, de 0,028 g de LiOH dans 5 ml de THF/H20 (70/30). On irradie ensuite sous champ de micro-ondes pendant 1h00 à 100°C. On filtre et on évapore le filtrat sous pression réduite. On reprend le résidu dans 5 ml de DMF. On ajoute 0,108 g de DIEA et on agite 10min à TA. On ajoute 0,159 g de HBTU et on agite 30 min à TA. On ajoute ensuite 0,061 g de vératrylamine et on agite 1h00 à TA. On ajoute 0,5 ml de DBU et on agite 48h00 à TA. On évapore sous pression réduite, reprend le résidu dans de l'AcOEt. On lave 3 fois avec une solution saturée de NH₄Cl et 2 fois à l'eau. On sèche sur Na₂SO₄, filtre, évapore le filtrat sous pression réduite. On chromatographie le résidu sur gel de silice en éluant par un mélange AcOEt/DCM (10/90, v/v) jusqu'à (20/80, v/v) pour donner 0,104 g du produit attendu.

### Etape 5.5 :

### 4-[6-(Aminométhyl)-3-(3,4-diméthoxybenzyl)-2,4-dioxo-3,4-dihydroquinazolin-1(2H)-yl]pipéridine-1-carboxylate de benzyle

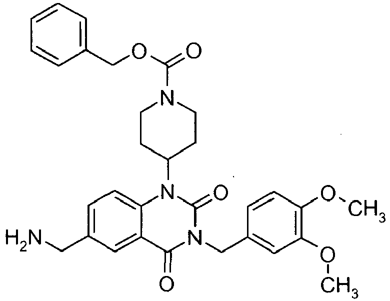

On agite 2h00 à TA une solution de 0,102 g de 4-[3-(3,4-Diméthoxybenzyl)-6-({[(1,1-diméthyléthoxy)carbonyl]amino}méthyl)-2,4-dioxo-3,4-dihydroquinazolin-1(2H)-yl]pipéridine-1-carboxylate de benzyle obtenu à l'étape 5.4 et de 0,5 ml de TFA dans 9,5 ml de DCM. On ajoute une solution saturée de NaHCO₃. On sèche la phase organique sur Na₂SO₄, filtre, évapore le filtrat sous pression réduite pour donner 0,09 g du produit attendu.

### Etape 5.6 :

### 4-{6-[(Acétylamino)méthyl]-3-(3,4-diméthoxybenzyl)-2,4-dioxo-3,4-dihydroquinazolin-1(2H)-yl}pipéridine-1-carboxylate de benzyle

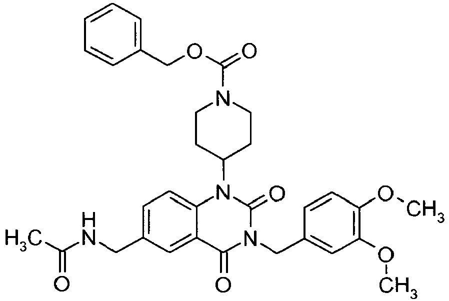

On ajoute 0,049 g d'anhydride acétique à une solution de 0,09 g de 4-[6-(aminométhyl)-3-(3,4-diméthoxybenzyl)-2,4-dioxo-3,4-dihydroquinazolin-1(2*H*)-yl]pipéridine-1-carboxylate de benzyle obtenu à l'étape 5.5, de 0,09 ml de NEt₃ dans 3 ml de DCM et on agite 1h00 à TA. On ajoute du DCM et on lave avec une solution saturée de NH₄Cl, puis par une solution HCl 1N, NaOH 2N puis à l'eau. On sèche la phase organique sur Na₂SO₄, filtre, évapore le filtrat sous pression réduite pour donner 0,104 g du produit attendu.

### Etape 5.7 :

### N-{[3-(3,4-Diméthoxybenzyl)-2;4-dioxo-1-pipéridin-4-yl-1,2,3,4-tétrahydroquinazolin-6-yl]méthyl}acétamide

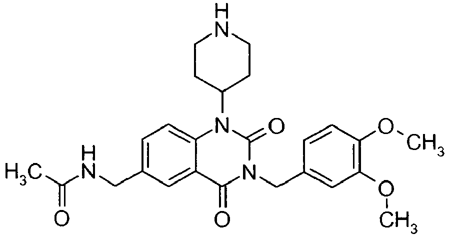

On irradie sous champ de micro-ondes pendant 30 min à 80°C un mélange de 0,1 g de 4-{6-[(acétylamino)méthyl]-3-(3,4-diméthoxybenzyl)-2,4-dioxo-3,4-dihydroquinazolin-1(2*H*)-yl}pipéridine-1-carboxylate de benzyle obtenu à l'étape 5.6, de 0,016 g de formiate d'ammonium et 0,018 g de Pd/C (10%) dans 2 ml d'EtOH préalablement purgé avec de l'azote. On filtre, évapore le filtrat sous pression réduite pour donner 0,077 g du produit attendu.

### Etape 5.8 : Composé n°20

### N-{[3-(3,4-Diméthoxybenzyl)-1-(1-formylpipéridin-4-yl)-2,4-dioxo-1,2,3,4-tétrahydroquinazolin-6-yl]méthyl}acétamide

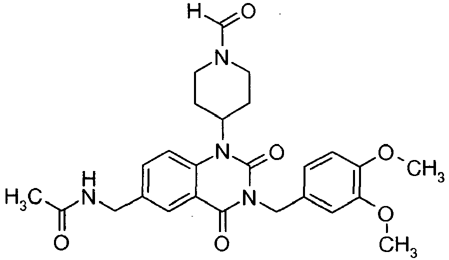

On irradie sous champ de micro-ondes pendant 1h00 à 140°C un mélange de 0,070 g de *N*-{[3-(3,4-diméthoxybenzyl)-2,4-dioxo-1-pipéridin-4-yl-1,2,3,4-tétrahydroquinazolin-6-yl]méthyl}acétamide obtenu à l'étape 5.7, de 0,028 g de formiate d'ammonium dans 2 ml d'ACN. On filtre, évapore le filtrat sous pression réduite. On chromatographie le résidu sur gel de silice en éluant par un mélange MeOH/DCM (0.5/99.5, v/v) jusqu'à (7/93, v/v) pour donner 0,035 g du produit attendu. Le tableau suivant illustre les structures chimiques et les propriétés physiques de composés répondant à la formule générale (I) selon l'invention, ainsi que de certains de leurs intermédiaires (notamment les composés 32, 55, 120 et 257)

| ***Composé N°*** | ***STRUCTURE*** | ***NOMENCLATURE*** | ***RMN ou MASSE*** |
|---|---|---|---|
| 1 | 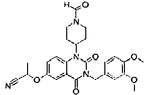 | 2-{[3-(3,4-diméthoxybenzyl)-1-(1-formylpipéridin-4-yl)-2,4-dioxo-1,2,3,4-tétrahydroquinazolin-6-yl]oxy}propanenitrile | ¹H NMR (500 MHz, DMSO-*d*₆) δ ppm 1.71(d, 3H) 1.79(brdd, 2H) 2.41(dq, 1H) 2.53(dq, 1H) 2.80(td, 1H) 3.25(t, 1H) 3.70(s, 3H) 3.71 (s, 3H) 3.80(d, 1H) 4.31 (d, 1H) 4.77(brs, 1H) 5.03(s, 2H) 5.60(q, 1H) 6.83(dd, 1H) 6.85 (d, 1H) 6.98(s, 1H) 7.51 (dd, 1H) 7.74(d, 1H) 7.84(d, 1H) 8.03(s, 1H) |
| 2 | 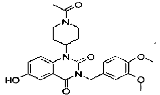 | 1-(1-acétylpipéridin-4-yl)-3-(3,4-diméthoxybenzyl)-6-hydroxyquinazoline-2,4(1 H,3H)-dione | ¹H NMR (500 MHz, DMSO-*d*₆) δ ppm 1.69(d, 1H) 1.74(d, 1H) 2.05(s, 3H) 2.42(dq, 1H) 2.57(dq, 1H) 2.70(t, 1H) 3.24(t, 1H) 3.70(br s, 3H) 3.71 (s, 3H) 3.92(d, 1H) 4.51 (d, 1H) 4.69(brs, 1H) 5.02(s, 2H) 6.81 (dd, 1H) 6.86(d, 1H) 6.97(d, 1H) 7.21(dd, 1H) 7.44(d, 1H) 7.65(d, 1H) 9.88(brs, 1H) |
| 3 | 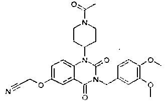 | {[1-(1-acétylpipéridin-4-yl)-3-(3,4-diméthoxybenzyl)-2,4-dioxo-1,2,3,4-tétrahyd roquinazolin-6-yl]oxy}acétonitrile | ¹H NMR (500 MHz, DMSO-*d*₆) δ ppm 1.71(d, 1H) 1.76(d, 1H) 2.04(s, 3H) 2.41 (dq, 1H) 2.56(dq, 1H) 2.70(t, 1H) 3.24(t, 1H) 3.70(s, 3H) 3.71 (s, 3H) 3.92(d, 1H) 4.51 (d, 1H) 4.73(brs,1H) 5.04(s, 2H) 5.29(s, 2H) 6.82(dd, 1H) 6.85(d, 1H) 6.98(d, 1H) 7.50(dd, 1H) 7.70(d, 1H) 7.83(d, 1H) |
| 4 | 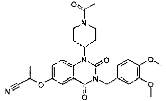 | 2-{[1-(1-acétylpipéridin-4-yl)-3-(3,4-diméthoxybenzyl)-2,4-dioxo-1,2,3,4-tétrahydroquinazolin-6-yl]oxy}propanenitrile | ¹H NMR (500 MHz, DMSO-*d*₆) δ ppm 1.71(d, 3H) 1.72(d, 2H) 1.77(d, 1H) 2.04(s, 3H) 2.41 (qd, 1H) 2.56(dq, 1H) 2.70(d, 1H) 3.24(t, 1H) 3.70(s, 3H) 3.71 (s, 3H) 3.92(d, 1H) 4.51(d, 1H) 4.73(brs, 1H) 5.03(s, 2H) 5.60(q, 1H) 6.83(d, 1H) 6.85(m, 1H) 6.98(d, 1H) 7.51 (dd, 1H) 7.74(d, 1H) 7.83(d, 1H) |
| 5 | 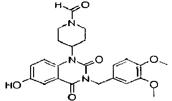 | 4-[3-(3,4-diméthoxybenzyl)-6-hydroxy-2,4-dioxo-3,4-dihydroquinazolin-1 (2H)-yl]pipéridine-1-carbaldéhyde | ¹H NMR (500 MHz, DMSO-*d*₆) δ ppm 1.75(d, 1H) 1.78(d, 1H) 2.41 (qd, 1H) 2.53(qd, 1H) 2.79(td, 1H) 3.25(td, 1H) 3.70(s, 3H) 3.71 (s, 3H) 3.80(d, 1H) 4.31 (d, 1H)4.72 (brs, 1H) 5.02(s, 2H) 6.82(dd, 1H) 6.86(d, 1H) 6.97(d, 1H) 7.21 (dd, 1H) 7.44(d, 1H) 7.66(d, 1H) 8.03(s, 1H) 9.85(brs, 1H) |
| 6 | 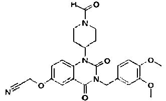 | {[3-(3,4-diméthoxybenzyl)-1-(1-formylpipéridin-4-yl)-2,4-dioxo-1,2,3,4-tétrahydroquinazolin-6-yl]oxy}acétonitrile | ¹H NMR (500 MHz, DMSO-*d*₆) δ ppm 1.78(d, 1H) 1.81(d, 1H) 2.42(qd, 1H) 2.54(dt, 1H) 2.81 (td, 4H) 3.26(dt, 1H) 3.71 (s, 3H) 3.72(s, 3H) 3.81(d, 1H) 4.32(d, 1H) 4.77(brs, 1H) 5.05(s, 2H) 5.30(s, 2H) 6.84(dd, 1H) 6.87(d, 1H) 6.99(d, 1H) 7.51 (dd, 1H) 7.71 (d, 1H) 7.85(d, 1H) 8.04(s, 1H) |
| 7 | 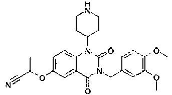 | 2-{[3-(3,4-diméthoxybenzyl)-2,4-dioxo-1-pipéridin-4-yl-1,2,3,4-tétrahydroquinazolin-6-yl]oxy}propanenitrile | ¹H NMR (500 MHz, DMSO-*d*₆) δ ppm1.69(d, 3H) 1.75(d, 2H) 2.65(td, 2H) 2.87(t, 2H) 3.21 (d, 2H) 3.69(s, 3H) 3.70(s, 3H) 4.68(brs, 1H) 5.04(s, 2H) 5.59(q, 1H) 6.84(m, 2H) 6.98(d, 1H) 7.50(dd, 1H) 7.73(d, 1H) 7.80(d, 1H) |
| 8 | 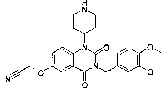 | {[3-(3,4-diméthoxybenzyl)-2,4-dioxo-1-pipéridin-4-yl-1,2,3,4-tétrahydroquinazolin-6-yl]oxy}acétonitrile | ¹H NMR (600 MHz, DMSO-*d*₆) δ ppm 1.77(d, 2H) 2.67(dt, 2H) 2.89(t, 2H) 3.23(d, 2H) 3.69(s, 3H) 3.70(s, 3H) 4.69(brs, 1H) 5.05(s, 2H) 5.28(s, 2H) 6.84(m, 2H) 6.99(s, 1H) 7.49(dd, 1H) 7.70(d, 1H) 7.80(d, 1H) |
| 9 | 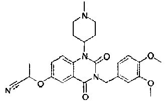 | 2-{[3-(3,4-diméthoxybenzyl)-1-(1-méthylpipéridin-4-yl)-2,4-dioxo-1,2,3,4-tétrahyd roq u inazoli n-6-yl]oxy}propanenitrile | ¹H NMR (500 MHz, DMSO-*d*₆) δ ppm 1.59(d, 2H) 1.66(d, 3H) 2.04(t, 2H) 2.16(s, 3H) 2.62(dt, 2H) 2.82(d, 2H) 3.66(s, 3H) 3.67(s, 3H) 4.43(brs, 1H) 5.01 (s, 2H) 5.54(q, 1H) 6.79(m, 1H) 6.82(d, 1H) 6.95(d, 1H) 7.45(dd, 1H) 7.68(d, 1H) 7.70(d, 1H) |
| 10 | 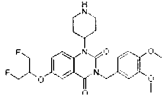 | 3-(3,4-d iméthoxybenzyl)-6-[2-fluoro-1-(fluorométhyl)éthoxy]-1-pipéridin-4-ylquinazoline-2,4(1H,3H)-dione | ¹H NMR (500 MHz, DMSO-*d*₆) δ ppm 1.85(d, 2H) 2.78(td, 2H) 3.04(t, 2H) 3.33(d, 2H) 3.71 (s, 6H) 4.61-4.82(m, 4H) 5.04(brt, 1H) 5.05(s, 2H) 6.86(m, 2H) 6.99(s, 1H) 7.49(dd, 1H) 7.68(d, 1H) 7.76(d, 1H) 8.05(brs, 1H) |
| 11 | 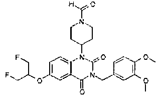 | 4-[3-(3,4-diméthoxybenzyl)-6-[2-fluoro-1-(fluorométhyl)éthoxy]-2,4-dioxo-3,4-dihydroquinazolin-1(2H)-yl]pipéridine-1-carbaldéhyde | ¹H NMR (500 MHz, DMSO-*d*₆) δ ppm 1.76(d, 1H) 1.80(d, 1H) 2.41 (qd, 1H) 2.53(qd, 1H) 2.80(td, 1H) 3.25(dt, 1H) 3.70(s, 6H) 3.80(d, 1H) 4.31 (d, 1H) 4.61-4.83(m, 5H) 5.02(m, 1H) 5.03(s, 2H) 6.83(dd, 1H) 6.85(d, 1H) 6.97(d, 1H) 7.47(dd, 1H) 7.67(d, 1H) 7.77(d, 1H) 8.03(s, 1H) |
| 12 | 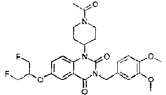 | 1-(1-acétylpipéridin-4-yl)-3-(3,4-diméthoxybenzyl)-6-[2-fluoro-1-(fluorométhyl)éthoxy]quinazolin e-2,4(1H,3H)-dione | ¹H NMR (500 MHz, DMSO-*d*₆) δ ppm 1.70(d, 1H) 1.76(d, 1H) 2.04(s, 3H) 2.41 (dq, 1 H) 2.56(dq, 1H) 2.70(t, 1H) 3.25(t, 1H) 3.70(s, 3H) 3.71 (s, 3H) 3.92(d, 1H) 4.51 (d, 1H) 4.61-4.83(m, 5H) 5.02(m, 1H) 5.03(s, 2H) 6.82(dd, 1H) 6.85(m, 1H) 6.97(d, 1H) 7.47(dd, 1H) 7.67(d, 1H) 7.77(d, 1H) |
| 13 | 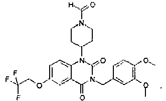 | 4-[3-(3,4-diméthoxybenzyl)-2,4-dioxo-6-(2, 2,2-trifluoroéthoxy)-3,4-dihydroquinazolin-1 (2H)-yl]pipéridine-1-carbaldéhyde | ¹H NMR (600 MHz, DMSO-*d*₆) δ ppm 1.75(d, 1H) 1.79(d, 1H) 2.40(dq, 1H)) 2.52(dq, 1H) 2.79(td, 1H) 3.25(td, 1H) 3.69(s, 6H) 3.79(d, 1H) 4.30(d, 1H) 4.75(brs, 1H) 4.88(q, 2H) 5.02(s, 2H) 6.82(d, 1H) 6.85(d, 1H) 6.97(s, 1H) 7.49(dd, 1H) 7.66(d, 1H) 7.79(d, 1H) 8.02(s, 1H) |
| 14 | 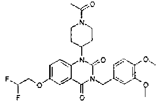 | 1-(1-acétylpipéridin-4-yl)-6-(2,2-difluoroéthoxy)-3-(3,4-diméthoxybenzyl)quinazoline-2,4(1H,3H)-dione | ¹H NMR (500 MHz, DMSO-*d*₆) δ ppm 1.70(d, 1H) 1.74(d, 1H) 2.04(s, 3H) 2.41 (qd, 1 H) 2.56(qd, 1H) 2.70(t, 1H) 3.24(t, 1H) 3.70(s, 3H) 3.70(s, 3H) 3.91 (d, 1H) 4.43(td, 2H) 4.50(d, 1H) 4.73(brs,1H) 5.03 (s, 2H) 6.41 (tt, 1H) 6.82(dd, 1H) 6.85(d, 1H) 6.97(d, 1H) 7.46(dd, 1H) 7.60(d, 1H) 7.79(d, 1H) |
| 15 | 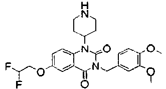 | 6-(2,2-difluoroéthoxy)-3-(3,4-diméthoxybenzyl)-1-pipéridin-4-ylquinazoline-2,4(1H,3H)-dione | ¹H NMR (500 MHz, DMSO-*d*₆) δ ppm 1.83(d, 2H) 2.75(td, 2H) 3.00(t, 2H) 3.30(d, 2H) 3.71 (s, 6H) 4.44(td, 2H) 4.75(b. s, 1H) 5.06(s, 2H) 6.42(tt, 1H) 6.83-6.89(m, 2H) 7.00(s, 1H) 7.48(dd, 1H) 7.62(d, 1H) 7.78(d, 1H) |
| 16 | 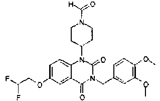 | 4-[6-(2,2-difluoroéthoxy)-3-(3,4-diméthoxybenzyl)-2,4-dioxo-3,4-dihydroquinazolin-1(2H)-yl]pipéridine-1-carbaldéhyde | ¹H NMR (500 MHz, DMSO-*d*₆) δ ppm 1.76(d, 1H) 1.80(d, 1H) 2.41 (qd, 1H) 2.53(qd, 1H) 2.81 (td, 1H) 3.26(td, 1H) 3.71 (s, 6H) 3.80(d, 1H) 4.31 (d, 1H) 4.44(td, 2H) 4.77(brs, 1H) 5.04(s, 2H) 6.42(tt, 1H) 6.83(m, 1H) 6.86(d, 1H) 6.86(d, 1H) 6.98(d, 1H) 7.47(dd, 1H) 7.61 (d, 1H) 7.80(d, 1H) 8.03(s, 1H) |
| 17 | 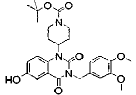 | **4-[3-(3,4-diméthoxybenzyl)-6-**hydroxy-2,4-dioxo-3,4-dihydroquinazolin-1 (2H)-yl]pipéridine-1-carboxylate de 1,1-diméthyléthyle | ¹H NMR (500 MHz, DMSO-*d*₆) δ ppm 1.43(s, 9H) 1.67(d, 2H) 2.48(m, 2H) 2.92(brs, 2H) 3.70(s, 3 H) 3.71 (s, 3H) 4.05(br s, 2H) 4.61 (brs, 1H) 5.02(s, 2H) 6.80(dd, 1H) 6.86(d, 1H) 6.97(d, 1H) 7.20(dd, 1H) 7.43(d, 1H) 7.62(d, 1H) 9.95(brs, 1H) |
| 18 | 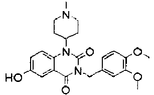 | 3-(3,4-diméthoxybenzyl)-6-hydroxy-1-(1-méthylpipéridin-4-yl)quinazoline-2,4(1H,3H)-dione | ¹H NMR (500 MHz, DMSO-*d*₆) δ ppm 1.60(d, 2H) 2.06(td, 2H) 2.19(s, 3H) 2.65(qd, 2H) 2.85(d, 2H) 3.70(s, 3 H) 3.71(s, 3H) 4.43(br.s, 1H) 5.02(s, 2H) 6.81(m, 1H) 6.85(d, 1H) 6.98(d, 1H) 7.19(dd, 1H) 7.41(d, 1H) 7.55(d, 1H) 9.82(s, 1H) |
| 19 | 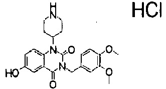 | chlorhydrate de 3-(3,4-diméthoxybenzyl)-6-hydroxy-1-pipéridin-4-ylquinazoline-2,4(1H,3H)-dione | ¹H NMR (500 MHz, DMSO-*d*₆) δ ppm 1.86(d, 2H) 2.83(m, 2H) 3.12(t, 2H) 3.37(m, 2H) 3.70(s, 6H) 4.76(brs, 1H) 5.03(s, 2H) 6.83(dd, 1H) 6.85(d, 1H) 6.98(d, 1H) 7.21 (dd, 1H) 7.44(d, 1H) 7.70(d, 1H) 8.70(brs, 2H) 9.91 (s, 1H) |
| 20 | 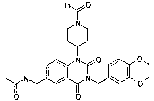 | N-{[3-(3,4-diméthoxybenzyl)-1-(1-formylpipéridin-4-yl)-2,4-dioxo-1,2,3,4-tétrahydroquinazolin-6-yl]méthyl}acétamide | ¹H NMR (500 MHz, DMSO-*d*₆) δ pm1.75(d, 1H) 1.78(d, 1H) 1.86(s, 3H) 2.40(qd, 1H) 2.53(qd, 1H) 2.79(t, 1H) 3.25(t, 1H) 3.69(s, 3H) 3.70(s, 3H) 3.80(d, 1H) 4.29(d, 2H) 4.31 (d., 1H) 4.77(brs, 1H) 5.02(s, 2H) 6.80(dd, 1H) 6.85(d, 1H) 6.96(d, 1H) 7.65(dd, 1H) 7.76(d, 1H) 7.96(d, 1H) 8.02(s, 1H) 8.46(t, 1H) |
| 21 | 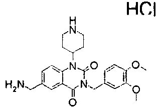 | chlorhydrate de 6-(aminométhyl)-3-(3,4-diméthoxybenzyl)-1-pipéridin-4-ylquinazoline-2,4(1 H,3H)-dione | ¹H NMR (500 MHz, DMSO-*d*₆) δ ppm 1.89(d, 2H) 2.83-2.92(m, 2H) 3.16(brs, 2H) 3.38-3.42(m, 2H) 3.71(s, 6H) 4.13(brs, 2H) 4.82-4.97(m, 1H) 5.07(s, 2H) 6.83-6.88(m, 2H) 7.00(s, 1H) 7.91-7.99(m, 2H) 8.24(s, 1H) 8.42(br s, 3H) 9.16(brs, 1H) |
| 22 | 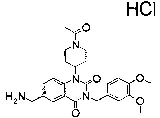 | chlorhydrate de 1-(1-acétylpipéridin-4-yl)-6-(aminométhyl)-3-(3,4-diméthoxybenzyl)quinazoline-2,4(1H,3H)-dione | ¹H NMR (500 MHz, DMSO-*d*₆) δ ppm 1.67-1.79(m, 2H) 2.05(s, 3H) 2.36-2.47(m, 1H) 2.51-2.61 (m, 1H) 2.73(t, 1H) 3.27(t, 1H) 3.71(s, 6H) 3.93(d, 1H) 4.11-4.15(m, 2H) 4.52(d, 1H) 4.78(brs, 1H) 5.05(s, 2H) 6.82(d, 1H) 6.86(d, 1H) 6.98(s, 1H) 7.87(s, 2H) 8.24(s, 1H) 8.32(brs, 3H) |
| 23 | 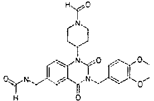 | N-{[3-(3,4-diméthoxybenzyl)-1-(1-formylpipéridin-4-yl)-2,4-dioxo-1,2,3,4-tétrahydroquinazolin-6-yl]méthyl}formamide | ¹H NMR (500 MHz, DMSO-*d*₆) δ ppm 1.73-1.83(m, 2H) 2.36-2.45(m., 1H) 2.48-2.57(m, 1H) 2.77-2.84(m, 1H) 3.23-3.29(m, 1H) 3.70(s, 1H) 3.71 (s, 3H) 3.81 (d, 1H) 4.31 (d, 1H) 4.37(d, 2H) 4.77(brs, 1H) 5.04(s, 2H) 6.82(dd, 1H) 6.86(d, 1H) 6.98(d, 1H) 7.67(dd, 1H) 7.78(d, 1H) 7.99(d, 1H) 8.03(s, 1H) 8.16(s, 1H) 8.62(t, 1H) |
| 24 | 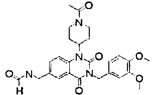 | N-{[1-(1-acétylpipéridin-4-yl)-3-(3,4-diméthoxybenzyl)-2,4-dioxo-1,2,3,4-tétrahydroquinazolin-6-yl]méthyl}formamide | ¹H NMR (500 MHz, DMSO-*d*₆) δ ppm 1.67-1.78(m, 2H) 2.05(s, 3H) 2.35-2.48(m, 1H) 2.53-2.62(m, 1H) 2.72(t, 1H) 3.25(t, 1H) 3.70(s, 3H) 3.71 (s, 3H) 3.93(d, 1H) 4.37(d, 2H) 4.52(d, 1H) 4.75(brs, 1H) 5.04(s, 2H) 6.82(dd, 1H) 6.86(d, 1H) 6.98(d, 1H) 7.67(dd,1H) 7.78(d,1H) 7.99(d, 1H) 8.16(s, 1H) 8.63(t, 1H) |
| 25 | 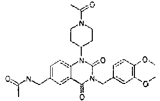 | N-{[1-(1-acétylpipéridin-4-yl)-3-(3,4-diméthoxybenzyl)-2,4-dioxo-1,2,3,4-tétrahydroquinazolin-6-yl]méthyl}acétamide | ¹H NMR (500 MHz, DMSO-*d*₆) δ ppm 1.63-1.81 (m, 2H) 1.87(s, 3H) 2.05(s, 3H) 2.32-2.42(m, 1H) 2.54-2.63(m, 1H) 2.71 (dt, 1H) 3.25(dt, 1H) 3.70(s, 3H) 3.71 (s, 3H) 3.93(d, 1H) 4.30(d, 2H) 4.52(d, 1H) 4.76(brs, 1H) 5.04(s, 2H) 6.81 (dd 1H) 6.86(d, 1H) 6.97(d, 1H) 7.65(dd, 1H) 7.76(d, 1H) 7.97(d, 1H) 8.47(t, 1H) |
| 26 | 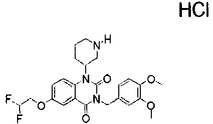 | chlorhydrate de 6-(2,2-difluoroéthoxy)-3-(3,4-diméthoxybenzyl)-1-pipéridin-3-ylquinazoline-2,4(1H,3H)-dione | ¹H NMR (600 MHz, DMSO-*d*₆) δ ppm 1.86-1.97(m, 3H) 2.54-2.62(m, 1H) 2.85-2.92(m, 1H) 3.31-3.35(m, 1H) 3.37-3.42(m, 1H) 3.71 (s, 3H) 3.72(s, 3H) 3.73-3.78(m,1H) 3.76(t, 1H) 4.45(td, 2H) 4.87(br s, 1H) 5.05(s, 2H) 6.41 (tt, 1H) 6.83-6.88(m, 2H) 6.99(s, 1H) 7.52(dd, 1H) 7.63(d, 1H) 7.72(d, 1H) 8.95(brs, 1H) |
| 27 | 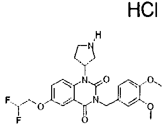 | chlorhydrate de 6-(2,2-difluoroéthoxy)-3-(3,4-diméthoxybenzyl)-1-pyrrolidin-3-ylquinazoline-2,4(1H,3H)-dione | ¹H NMR (500 MHz, DMSO-*d*₆) δ ppm 2.25-2.32(m, 1H) 2.42-2.48(m, 1H) 3.11-3.19(m, 1H) 3.38-3.45(m, 1H) 3.58-3.64(m, 1H) 3.65-3.72(m, 1H) 3.73(s, 3H) 3.71 (s, 3H) 4.45(td, 2H) 5.05-5.12(m, 2H) 5.47-5.54(m, 1H) 6.42(tt, 1H) 6.82-6.88(m, 2H) 7.01 (s, 1H) 7.55(dd, 1H) 7.62(d, 1H) 7.71 (d, 1H) 8.87(brs, 1H) |
| 28 | 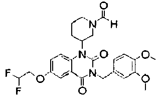 | 3-[6-(2,2-difluoroéthoxy)-3-(3,4-diméthoxybenzyl)-2,4-dioxo-3,4-dihydroquinazolin-1 (2H)-yl]pipéridine-1-carbaldéhyde | ¹H NMR (600 MHz, DMSO-*d*₆) mélange de deux isomères δ ppm 1.52-1.62(m, 0.5H) 1.64-1.73(m, 0.5H) 1.75-1.84(m, 1H) 1.84-1.90(m, 1H) 2.54-2.67(m, 1.5H) 3.05(t, 0.5H) 3.59(t, 0.5H) 3.70(s, 3.5H) 3.70(s, 3H) 3.77(dd, 0.5H) 4.06(t, 0.5H) 4.17-4.27(m, 1H) 4.31 (brs, 0.5H) 4.42(td, 2H) 4.44(brs, 0.5H) 5.04(s, 2H) 6.38(tt, 1H) 6.80-6.84(m, 1H) 6.84-6.88(m, 1H) 6.98(brs, 1H) 7.43(td, 1H) 7.60(brs, 1H) 7.62(d, 0.5H) 7.79(d, 0.5H) 7.99(s, 0.5H) 8.09(s, 0.5H) |
| 29 | 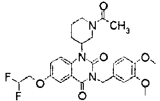 | 1-(1-acétylpipéridin-3-yl)-6-(2,2-difluoroéthoxy)-3-(3,4-diméthoxybenzyl)quinazoline-2,4(1H,3H)-dione | 1H NMR (500 MHz, DMSO-*d*₆) mélange de deux isomères δ ppm 1.55-1.65(m, 0.5H) 1.67-1.89(m, 2.5H) 1.99(s, 1.5H) 2.06(s, 1.5H) 2.52-2.65(m, 1H) 3.06(t, 0.5H) 3.30-3.37(m, 0.5H) 3.52(t, 0.5H) 3.70(s, 3H) 3.71 (s, 3H) 3.52(t, 0.5H) 3.84(d, 0.5H) 3.91 (d, 0.5H) 4.09(t, 0.5H) 4.28(brs, 0.5H) 4.39-4.48(m, 3H) 4.49(brs, 0.5H) 5.05(d, 2H) 6.41 (tt, 1H) 6.81-6.89(m, 2H) 6.99(d, 1H) 7.44-7.48(m, 1H) 7.59-7.62(m, 1H) 7.64(d, 0.5H) 7.82(d, 0.5H) |
| 30 | 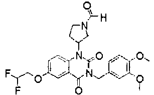 | 3-[6-(2,2-difluoroéthoxy)-3-(3,4-diméthoxybenzyl)-2,4-dioxo-3,4-dihydroquinazolin-1 (2H)-yl]pyrrolidine-1-carbaldéhyde | 1H NMR (500 MHz, DMSO-*d*₆) mélange de deux isomères δ ppm 2.18-2.27(m, 1H) 2.36-2.46(m, 1H) 3.41-3.48(m, 0.5H) 3.53-3.60(m, 0.5H) 3.63-3.73(m, 1.5H) 3.85-3.95(m, 1.5H) 4.43(td, 2H) 5.06(s, 2H) 5.38-5.45(m, 0.5H) 5.46-5.54(m, 0.5H) 6.41 (tt, 1H) 6.82-6.88(m, 2H) 7.00(s, 1H) 7.46 - 7.51(m, 1H) 7.61 (d, 1H) 7.66(d, 0.5H) 7.72(d, 0.5H) 8.16(s, 0.5H) 8.20(s, 0.5H) |
| 31 | 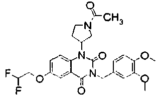 | 1-(1-acétylpyrrolidin-3-yl)-6-(2,2-difluoroéthoxy)-3-(3,4-diméthoxybenzyl)quinazoline-2,4(1H,3H)-dione | ¹H NMR (500 MHz, DMSO-*d*₆) mélange de deux isomères δ ppm1.98(s, 1.5H) 2.04(s, 1.5H) 2.17-2.25(m, 0.5H) 2.27-2.35(m, 0.5H) 2.57-2.66(m, 1 H) 3.40-3.45(m, 0.5H) 3 56-3.61(m, 0.5H) 3.70-3.78(m, 7.5H) 3.82-3.95(m, 1.5H) 4.48(td, 2H) 5.10(d, 2H) 5.45-5.58(m, 1H) 6.46(tt, 1H) 6.87-6.92(m, 2H) 7.05(dd, 1H) 7.53(td, 1H) 7.66(t, 1H) 7.72 (dd, 1H) |
| 32 | 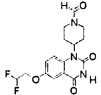 | 4-[6-(2,2-difluoroéthoxy)-2,4-dioxo-3,4-dihydroquinazolin-1(2H)-yl]pipéridine-1-carbaldéhyde | ¹H NMR (600 MHz, DMSO-*d*₆) δ ppm 1.72-1.82(m, 2H) 2.35-2.44(m, 1H) 2.47-2.55(m, 1H) 2.79(td, 1H) 3.25(td, 1H) 3.80(d, 1H) 4.32(d, 1H) 4.42(td, 2H) 4.71(brs, 1H) 6.40(tt, 1H) 7.43(dd, 1H) 7.55(d, 1H) 7.74(d, 1H) 8.03(s, 1H) |
| 33 | 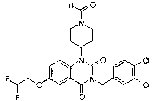 | 4-[3-(3,4-dichlorobenzyl)-6-(2,2-difluoroéthoxy)-2,4-dioxo-3,4-dihydroquinazolin-1 (2H)-yl]pipéridine-1-carbaldéhyde | ¹H NMR (500 MHz, DMSO-*d*₆) δ ppm 1.80(dd, 2H) 2.32-2.43(m, 1H) 2.44-2.52(m, 1H) 2.80(td, 1H) 3.26(dt, 1H) 3.80(brd, 1H) 4.31 (br d, 1H) 4.44(td, 2H) 4.78(brs, 1H) 5.09(s, 2H) 6.41 (tt, 1H) 7.30(dd, 1H) 7.49(dd, 1H) 7.58(d, 1H) 7.60(d, 1H) 7.62(d, 1H) 7.81(d, 1H) 8.03(s, 1H) |
| 34 | 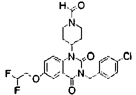 | 4-[3-(4-chlorobenzyl)-6-(2,2-difluoroéthoxy)-2,4-dioxo-3,4-dihydroquinazolin-1(2H)-yl]pipéridine-1-carbaldéhyde | ¹H NMR (500 MHz, DMSO-*d*₆) δ ppm 1.72-1.83(m, 2H) 2.32-2.44(m, 1H) 2.45-2.53(m, 1H) 2.80(dt, 1H) 3.25-3.30(dt, 1H) 3.80 (d, 1H) 4.30 (d,1H) 4.44 (td, 2H) 4.78(brs, 1H) 5.09(s, 2H) 6.42 (tt, 1H) 7.32-7.40(m, 4H) 7.48(dd, 1H) 7.61(d, 1H) 7.81(d, 1H) 8.03(s, 1H) |
| 35 | 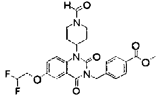 | 4-{[6-(2,2-difluoroéthoxy)-1-(1-formylpipéridin-4-yl)-2,4-dioxo-1,4-dihydroquinazolin-3(2H)-yl]méthyl}benzoate de méthyle | ¹H NMR (500 MHz, DMSO-*d*₆) δ ppm 1.78(dd, 1H) 2.33-2.42(m, 1H) 2.44-2.52(m, 1H) 2.80(dt, 1H) 3.26(dt, 1H) 3.79(brd, 1H) 3.84(s, 3H) 4.30(brd, 1H) 4.45(td, 2H) 4.79(brs, 1H) 5.18(s, 2 H) 6.42(tt, 1 H) 7.43(d, 2H) 7.49(dd, 1H) 7.62(d, 1H) 7.83(d, 1H) 7.91 (d, 2H) 8.02(s, 1H) |
| 36 | 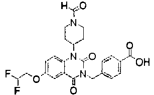 | acide 4-{[6-(2,2-difluoroéthoxy)-1-(1-formylpipéridin-4-yl)-2,4-dioxo-1,4-dihydroquinazolin-3(2H)-yl]méthyl}benzoïque | ¹H NMR (500 MHz, DMSO-*d*₆) δ ppm 1.79(dd, 2H) 2.38(qd, 1H) 2.49(qd, 1H) 2.80(dt, 1H) 3.26(dt, 1H) 3.79(d, 1H) 4.31 (d, 1H) 4.45(td, 2H) 4.79(brs, 1H) 5.17(s, 2H) 6.42(tt, 1H) 7.40(d, 2H) 7.49(dd, 1H) 7.62(d, 1H) 7.83(d, 1H) 7.88(d, 2H) 8.02(s, 1H) 12.94(brs, 1H) |
| 37 | 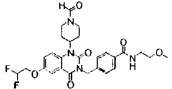 | 4-{[6-(2,2-difluoroéthoxy)-1-(1-formylpipéridin-4-yl)-2,4-dioxo-1,4-dihydroquinazolin-3(2H)-yl]méthyl}-N-(2-méthoxyéthyl)benzamide | 1H NMR (500 MHz, DMSO-*d*₆) δ ppm 1.72-1.82(m, 2H) 2.32-2.42(m, 1H) 2.44-2.54(m, 1H) 2.75-2.83(m, 1H) 3.21-3.29(m, 4H) 3.37-3.45(m, 4H) 3.77(br.d., 1H) 4.29(br.d., 1H) 4.44(td, 2H) 4.78(br.s., 1H) 5.14(s, 2H) 6.41(tt, 1H) 7.35(m, 2H) 7.48(dd, 1H) 7.61(d, 1H) 7.77(m, 2H) 7.81(d, 1H) 8.01(s, 1H) 8.47(t, 1H) |
| 38 | 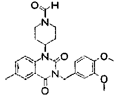 | 4-[3-(3,4-diméthoxybenzyl)-6-méthyl-2,4-dioxo-3,4-dihydroquinazolin-1 (2H)-yl]pipéridine-1-carbaldéhyde | ¹H NMR (500 MHz, DMSO-*d*₆) δ ppm 1.76(dd, 2H) 2.37(s, 3H) 2.39-2.42(m, 1H) 2.50-2.54(m, 1H) 2.77-2.82(m, 1H) 3.19-3.30(m, 1H) 3.70(s, 6H) 3.79(d, 1H) 4.30(d, 1H) 4.76(brs, 1H) 5.02(s, 2H) 6.82(dd, 1H) 6.85(d, 1H) 6.97(d, 1H) 7.60(dd, 1H) 7.71 (d, 1H) 7.89(s, 1H) 8.03(s, 1H) |
| 39 | 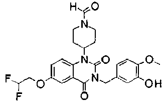 | 4-[6-(2,2-difluoroéthoxy)-3-(3-hydroxy-4-méthoxybenzyl)-2,4-dioxo-3,4-dihydroquinazolin-1(2H)-yl]pipéridine-1-carbaldéhyde | ¹H NMR (500 MHz, DMSO-*d*₆) δ ppm 1.70-1,80(m, 2H) 2.35-1,45(m, 1H) 2.47-2.56(m, 1H) 2.80(dt, 1H) 3.25(dt, 1H) 3.70(s, 3H) 3.79(brd, 1H) 4.30(brd, 1H) 4.43(td, 2H) 4.77(brs , 1H) 4.96(s, 2H) 6.41 (tt, 1H) 6.73(d, 1H) 6.78-6.82(m, 2H) 7.46(dd, 1H) 7.60(d, 1H) 7.79(d, 1H) 8.03(s, 1H) 8.95 (s, 1 H) |
| 40 | 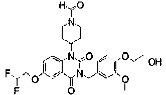 | 4-[6-(2,2-difluoroéthoxy)-3-[3-(2-hydroxyéthoxy)-4-méthoxybenzyl]-2,4-dioxo-3,4-dihydroquinazolin-1(2H)-yl]pipéridine-1-carbaldéhyde | 1H NMR (600 MHz, DMSO-d6) δ ppm 1.76(dd, 2H) 2.35-2.45(m, 1H) 2.50-2.54(m, 1H) 2.79(dt, 1H) 3.25(dt, 1H) 3.68(dt, 2H) 3.70(s, 3H) 3.79(brd, 1H) 3.90(t, 2H) 4.30(brd, 1H) 4.42(td, 2H) 4.75(brs, 1H) 4.77(t, 1H) 5.01 (s, 2H) 6.39(tt, 1H) 6.85(m, 2H) 6.97(s, 1H) 7.45(dd, 1H) 7.60(d, 1H) 7.77(d, 1H) 8.02(s, 1H) |
| 41 | 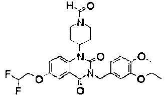 | 4-[6-(2,2-difluoroéthoxy)-3-(3-éthoxy-4-méthoxybenzyl)-2,4-dioxo-3,4-dihydroquinazolin-1(2H)-yl]pipéridine-1-carbaldéhyde | ¹H NMR (600 MHz, DMSO-*d*₆) δ ppm 1.29(t, 3H) 1.76(dd, 2H) 2.33-2.45(m, 1H) 2.50-2.56(m, 1H) 2.76-2.82(m, 1H) 3.21-3.31 (m, 1H) 3.70(s, 3H) 3.79(brd, 1H) 3.94(q, 2H) 4.30(brd,1 H) 4.41(dt, 2H) 4.75(brs, 1H) 5.01(s, 2H) 6.39(tt, 1H) 6.81-6.86(m, 2H) 6.96(s, 1H) 7.45(dd, 1H) 7.60(d, 1H) 7.77(d, 1H) 8.02(s, 1H) |
| 42 | 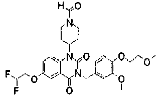 | 4-[6-(2,2-difluoroéthoxy)-3-[4-méthoxy-3-(2-méthoxyéthoxy)benzyl]-2,4-dioxo-3,4-dihydroquinazolin-1(2H)-yl]pipéridine-1-carbaldéhyde | ¹H NMR (600 MHz, DMSO-*d*₆) δ ppm 1.69-1.82(m, 2H) 2.35-2.45(m, 1H) 2.45-2.55(m, 1H) 2.78(t, 1H) 3.21-3.28(m, 1H) 3.61 (brs, 2H) 3.70(s, 3H) 3.78(d, 1H) 4.00(brs, 2H) 4.30(d, 1H) 4.42(t, 2H) 4.75(brs, 1H) 5.01 (s, 2H) 6.39(t, 1H) 6.82- 6.88(m, 2H) 6.97(s, 1H) 7.45(d, 1H) 7.59(s, 1H) 7.77(d, 1H) 8.02(s, 1H) |
| 43 | 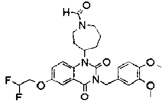 | 4-[6-(2,2-difluoroéthoxy)-3-(3,4-diméthoxybenzyl)-2,4-dioxo-3,4-dihydroquinazolin-1(2H)-yl]azépane-1-carbaldéhyde | ¹H NMR (500 MHz, DMSO-*d*₆) mélange de deux isomères δ ppm 1.72-1.81 (m, 1H) 1.85(brd, 1H) 1.86-2.03(m, 2H) 2.47(brs, 2H) 3.30-3.40(m, 1H) 3.42-3.52(m, 1H) 3.58-3.64(m, 2H) 3.70(s, 3H) 3.71 (s, 3H) 4.42(td, 2H) 4.45(brs, 0.5H) 5.04(brs, 2H) 5.21(brs, 0.5H) 6.40(tt, 1H) 6.78-6.83(m, 1H) 6.86(d, 1H) 6.98(s, 1H) 7.38-7.48(m, 1H) 7.59(brs, 1H) 7.64-7.74(m, 1H) 8.08(brs, 0.5H) 8.12(s, 0.5H) |
| 44 | 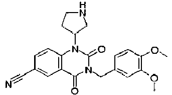 | 3-(3,4-diméthoxybenzyl)-2,4-dioxo-1-pyrrolidin-3-yl-1,2,3,4-tétrahydroquinazoline-6-carbonitrile | ¹H NMR (600 MHz, DMSO-*d*₆) δ ppm 1.96-2.09(m, 2H) 2.78-2.84(m, 1H) 3.05-3.12(m, 2H) 3.18-3.27(m, 1H) 3.70(s, 3H) 3.71 (s, 3H) 5.04(s, 2H) 5.47-5.53(m, 1H) 6.83(brs, 2H) 6.99(s, 1H) 8.15(dd, 1H) 8.20(d, 1H) 8.47(d, 1H) |
| 45 | 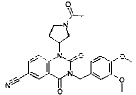 | 1-(1-acétylpyrrolidin-3-yl)-3-(3,4-diméthoxybenzyl)-2,4-dioxo-1,2,3,4-tétrahydroquinazoline-6-carbonitrile | ¹H NMR (600 MHz, DMSO-*d*₆) mélange de deux isomères δ ppm 1.91(s, 1.5H) 1.98(s, 1.5H) 2.14-2.22(m, 0.5H) 2.25-2.32(m, 0.5H) 2.43-2.50(m, 0.5H) 2.52-2.58(m, 0.5H) 3.31-3.39(m, 0.5H) 3.50-3.55(m, 0.5H) 3.63-3.73(m, 7.5H) 3.79-3.87(m, 1.5H) 5.00-5.06(m, 2H) 5.43-5.55(m, 1H) 6.82-6.90(m, 2H) 6.98(d, 1H) 7.84(dd, 1H) 8.17(dt, 1H) 8.43(dd, 1H) |
| 46 | 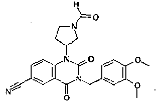 | 3-(3,4-diméthoxybenzyl)-1-(1-formylpyrrolidin-3-yl)-2,4-dioxo-1,2,3,4-tétrahydroquinazoline-6-carbonitrile | ¹H NMR (500 MHz, DMSO-*d*₆) mélange de deux isomères δ ppm 2.19-2.27(m, 1H) 2.36-2.43(m, 1H) 3.43-3.47(m, 0.4H) 3.53-3.59(m, 0.6) 3.62-3.73(m, 1.6H) 3.70(s, 3H) 3.71 (s, 3H) 3.84-3.94(m, 1.4H) 5.04(s, 2H) 5.42-5.49(m, 0.4H) 5.50-5.56(m, 0.6H) 6.86(s, 2H) 6.99(s, 1H) 7.85(d, 0.6H) 7.90(d, 0.4H) 8.15-8.22(m, 2H) 8.45(s, 1H) |
| 47 | 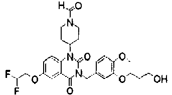 | 4-[6-(2,2-difluoroéthoxy)-3-[3-(3-hydroxypropoxy)-4-méthoxybenzyl]-2,4-dioxo-3,4-dihydroquinazolin-1(2H)-yl]pipéridine-1-carbaldéhyde | ¹H NMR (600 MHz, DMSO-*d*₆) δ ppm 1.72-1.79(m, 2H) 1.82(q, 2H) 2.36-2.44(m, 1H) 2.49-2.55(m, 1H) 2.78(td, 1H) 3.25(td, 1H) 3.51-3.54(m, 2H) 3.70(s, 3H) 3.76-3.80(m, 1H) 3.95(t, 2H) 4.28-4.32(m, 1H) 4.41 (td, 2H) 4.47(t, 1H) 4.75(s, 1H) 5.01(s, 2H) 6.39(tt, 1H) 6.81-6.86(m, 2H) 6.96-6.97(m, 1H) 7.45(dd, 1H) 7.59(d, 1H) 7.77(d, 1H) 8.02(s, 1H) |
| 48 | 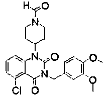 | 4-[5-chloro-3-(3,4-diméthoxybenz 2,4-dioxo-3,4-dihydroquinazolin-1(2H)-yl]pipéridine-1-carbaldéhyd | ¹H NMR (500 MHz, DMSO-*d*₆) δ ppm 1.78-1.88(m, 2H) 2.34-2.44(m, 1H) 2.46-2.56(m, 1H) 2.80(t, 1H) 3.25(t, 1H) 3.71 (s, 6H) 3.80(d, 1H) 4.31(d, 1H) 4.71(brs, 1H) 5.00(s, 2H) 6.83(d, 1H) 6.87(d, 1H) 6.97(s, 1H) 7.37(d, 1H) 7.69(dd, 1H) 7.77(d, 1 H) 8.03(s, 1H) |
| 49 | 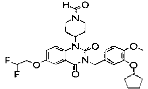 | 4-{3-[3-(cyclopentyloxy)-4-méthoxybenzyl]-6-(2,2-difluoroéthoxy)-2,4-dioxo-3,4-dihydroquinazolin-1(2H)-yl}pipéridine-1-carbaldéhyde | 1H NMR (500 MHz, DMSO-*d*₆) δ ppm 1.5-1.6(m, 2H) 1.63-1.71 (m, 3H) 1.68-1.78(m, 1H) 1.78-1.98(m, 3H) 2.35-2.46(m, 1H) 2.47-2.57(m, 1H) 2.79(dt, 1H) 3.26(dt, 1H) 3.69(s, 3H) 3.79(br.d., 1H) 4.30(br.d., 1H) 4.43(td, 2H) 4.66-4.70(m, 1 H) 4.76(br.s., 1H) 5.01 (s, 2H) 6.41 (tt, 1H) 6.82 - 6.86(m, 2H) 6.96(s, 1H) 7.46(dd, 1H) 7.6(d, 1H) 7.80(d, 1H) 8.03(s, 1H) |
| 50 | 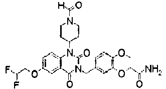 | 2-(5-{[6-(2,2-difluoroéthoxy)-1-(1-formylpipéridin-4-yl)-2,4-dioxo-1,4 dihydroquinazolin-3(2H)-yl]méthyl méthoxyphénoxy)acétamide | ¹H NMR (500 MHz, DMSO-*d*₆) δ ppm 1.72-1.82(m, 2H) 2.35-2.45(m, 1H) 2.46-2.56(m, 1H) 2.79(td, 1H) 3.25(td, 1H) 3.74(s, 3H) 3.79(d, 1H) 4.31 (d, 1H) 4.35(s, 2H) 4.43(td, 2H) 4.76(brs, 1H) 5.00(s, 2H) 6.41 (tt, 1H) 6.88-6.95(m, 3H) 7.31(brs, 1H) 7.37(brs, 1H) 7.46(dd, 1H) 7.59(d, 1H) 7.79(d, 1H) 8.02(s, 1H) |
| 51 | 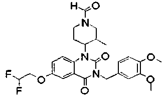 | 4-[6-(2,2-difluoroéthoxy)-3-(3,4-diméthoxybenzyl)-2,4-dioxo-3,4-dihydroquinazolin-1 (2H)-yl]-3-méthylpipéridine-1-carbaldéhyde | ¹H NMR (500 MHz, DMSO-*d*₆) mélange de deux isomères δ ppm 0.92(t, 3H) 1.72-1.82(m, 1H) 2.23(brs, 1H) 2.70-2.77(m, 0.5H) 3.00-3.10(m, 0.5H) 3.12-3.20(m, 1.5H) 3.44(d, 0.5H) 3.63(dd, 0.5H) 3.69(s, 6H) 3.80(d, 0.5H) 4.04(d, 0.5H) 4.29(d, 0.5H) 4.43(td, 2H) 4.62-4.70(m, 1H) 5.03(s, 2H) 6.41 (tt, 1H) 6.80(dd, 1H) 6.85(d, 1H) 6.97(d, 1H) 7.46(dd, 1H) 7.60(d, 1H) 7.64(dd, 1H) 7.97(s, 0.5H) 8.11 (s, 0.5H) |
| 52 | 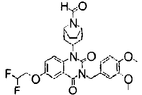 | 3-[6-(2, 2-difluoroéthoxy)-3-(3,4-diméthoxybenzyl)-2,4-dioxo-3,4-dihydroquinazolin-1(2H)-yl]-8-azabicyclo[3.2.1]octane-8-carbaldéhyde | 1H NMR (600 MHz, DMSO-*d*₆) δ ppm 1.85-1.95(m, 4H) 2.18(t, 2H) 2.27-2.33(m, 1H) 2.34-2.41(m, 1H) 3.70(s, 1H) 3.71(s, 1H) 4.25-4.29(m, 1H) 4.40(td, 2H) 4.50-4.54(m , 1H) 4.55(br.s., 1H) 5.05(s, 2H) 6.38(tt, H) 6.80(d, 1H) 6.85(d, 1H) 6.98(s, 1H) 7.45(dd, 1H) 7.56(d, 1H) 7.58(d, 1H) 8.12(s, 1H) |
| 53 | 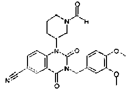 | 3-(3,4-diméthoxybenzyl)-1-(1-formylpipéridin-3-yl)-2,4-dioxo-1,2,3,4-tétrahydroquinazoline-6-carbonitrile | ¹H NMR (500 MHz, DMSO-*d*₆) mélange de deux isomères δ ppm 1.52-1.63(m, 0.5H) 1.65-1.73(m, 0.5H) 1.74-1.83(m, 1H) 1.87-1.93(m, 1H) 2.54-2.64(m, 1.5H) 3.06(dt, 0.5H) 3.57(t, 0.5H) 3.70(s, 3H) 3.71 (s, 3H) 3.79(dd, 0.5H) 4.03(t, 0.5H) 4.20(d, 0.5H) 4.30(d, 0.5H) 4.38(brs, 0.5H) 4.53(brs, 0.5H) 4.99-5.07(m, 2H) 6.84-6.88(m, 2H) 6.98(s, 1H) 7.86(d, 0.5H) 8.01 (d, 0.5H) 7.98(s, 0.5H) 8.08(s, 0.5H) 8.14-8.18(m, 1H) 8.44(s, 1H) |
| 54 | 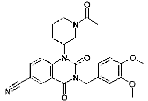 | 1-(1-acétylpipéridin-3-yl)-3-(3,4-diméthoxybenzyl)-2,4-dioxo-1,2,3,4-tétrahydroquinazoline-6-carbonitrile | ¹H NMR (500 MHz, DMSO-*d*₆) mélange de deux isomères δ ppm 1.56-1.66(m, 0.5H) 1.70-1.82(m, 1.5H) 1.87(brd, 1H) 1.97(s, 1.5H) 2.05(s, 1.5H) 2.54(dd, 1H) 3.05(brt, 0.5H) 3.48(t, 0.5H) 3.70-3.72(m, 6H) 3.83(d, 0.5H) 3.94(brd, 0.5H) 4.04(t, 0.5H) 4.33(brs, 0.5H) 4.45(dd, 1H) 4.53(brs, 0.5H) 4.99-5.07(m, 2H) 6.83-6.87(m, 2H) 6.98(d, 1H) 7.84(d, 0.5H) 8.02(d, 0.5H) 8.16(dt, 1H) 8.44(dd, 1H) |
| 55 | 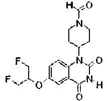 | 4-{6-[2-fluoro-1-(fluorométhyl)éthoxy]-2,4-dioxo-3,4-dihydroquinazolin-1(2H)-yl}pipéridine-1-carbaldéhyde | 1H NMR (600 MHz, DMSO-*d*₆) δ ppm 1.73-1.82(m, 2H) 2.36-2.44(m, 1H) 2.44-2.55(m, 1H) 2.79(td, 1H) 3.25(td, 1H) 3.80(brd, 1H) 4.32(brd, 1H) 4.62-4.67(m, 1H) 4.68-4.76(m, 3H) 4.76-4.82(m, 1H) 4.96-5.06(m, 1H) 7.44(dd, 1H) 7.61 (d, 1H) 7.73(d, 1H) 8.03(s, 1H) 11.46(brs, 1H) |
| 56 | 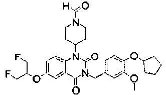 | 4-{3-[4-(cyclopentyloxy)-3-méthoxybenzyl]-6-[2-fluoro-1-(fluorométhyl)éthoxy]-2,4-dioxo-3,4-dihydroquinazolin-1(2H)-yl}pipéridine-1-carbaldéhyde | LCMS m/z [MH]+ 572 |
| 57 | 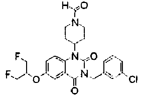 | 4-[3-(3-chlorobenzyl)-6- [2-fluoro-1-(fluorométhyl)éthoxy]-2,4-dioxo-3,4-dihydroquinazolin-1(2H)-yl]pipéridine-1-carbaldéhyde | LCMS m/z [MH]+ 492 |
| 58 | 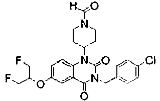 | 4-[3-(4-chlorobenzyl)-6-[2-fluoro-1-(fluorométhyl)éthoxy]-2,4-dioxo-3,4-dihydroquinazolin-1(2H)-yl]pipéridine-1-carbaldéhyde | LCMS m/z [MH]+ 492 |
| 59 | 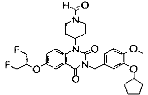 | 4-{3-[3-(cyclopentyloxy)-4-méthoxybenzyl]-6-[2-fluoro-1-(fluorométhyl)éthoxy]-2,4-dioxo-3,4-dihydroquinazolin-1(2H)-yl}pipéridine-1-carbaldéhyde | LCMS m/z [MH]+ 572 |
| 60 | 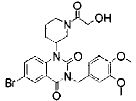 | 6-bromo-3-(3,4-diméthoxybenzyl)-1-[1-(hydroxyacétyl)pipéridin-3-yl]quinazoline-2,4(1 H,3H)-dione | LCMS m/z [MH]+ 532 |
| 61 | 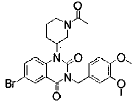 | 1-(1-acétylpipéridin-3-yl)-6-bromo-3-(3,4-diméthoxybenzyl)quinazoline-2,4(1H,3H)-dione | LCMS m/z [MH]+ 516 |
| 62 | 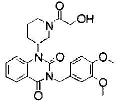 | 3-(3,4-diméthoxybenzyl)-1-[1-(hydroxyacétyl)pipéridin-3-yl]quinazoline-2,4(1 H,3H)-dione | LCMS m/z [MH]+ 454 |
| 63 | 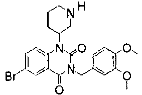 | 6-bromo-3-(3,4-diméthoxybenzyl)-1-[pipéridin-3-yl]quinazoline-2,4(1H,3H)-dione | LCMS m/z [MH]+ 474 |
| 64 | 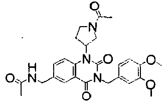 | N-{[1-(1-acétylpyrrolidin-3-yl)-3-(3,4-diméthoxybenzyl)-2,4-dioxo-1,2,3,4-tétrahydroquinazolin-6-yl]méthyl}acétamide | LCMS m/z [MH]+ 495 |
| 65 | 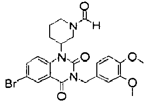 | 3-[6-bromo-3-(3,4-diméthoxybenzyl)-2,4-dioxo-3,4-dihydroquinazolin-1 (2H)-yl]pipéridine-1-carbaldéhyde | LCMS m/z [MH]+ 502 |
| 66 | 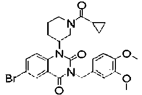 | 6-bromo-1-[1-(cyclopropylcarbonyl)pipéridi n-3-yl]-3-(3,4-diméthoxybenzyl)quinazoline -2,4(1H,3H)-dione | LCMS m/z [MH]+ 542 |
| 67 | 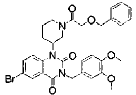 | 1-{1-[(benzyloxy)acétyl]pipéridin-3-yl}-6-bromo-3-(3,4-diméthoxybenzyl)quinazoline-2,4(1H,3H)-dione | LCMS m/z [MH]+ 622 |
| 68 | 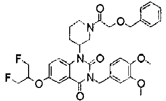 | 1-{1-[(benzyloxy)acétyl]pipéridin-3-yl}-3-(3,4-diméthoxybenzyl)-6-[2-fluoro-1-(fluorométhyl)éthoxy]quinazoline -2,4(1H,3H)-dione | LCMS m/z [MH]+ 638 |
| 69 | 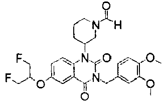 | 3-[3-(3,4-diméthoxybenzyl)-6-[2-fluoro-1-(fluorométhyl)éthoxy]-2,4-dioxo-3,4-dihydroquinazolin-1(2H)-yl]pipéridine-1-carbaldéhyde | LCMS m/z [MH]+ 518 |
| 70 | 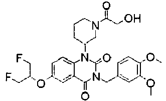 | 3-(3,4-diméthoxybenzyl)-6-[2-fluoro-1-(fluorométhyl)éthoxy]-1-[1-(hydroxyacétyl)pipéridin-3-yl]quinazoline-2,4(1 H,3H)-dione | LCMS m/z [MH]+ 548 |
| 71 | 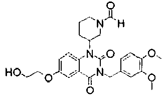 | 3-[3-(3,4-diméthoxybenzyl)-6-(2-hydroxyéthoxy)-2,4-dioxo-3,4-dihydroquinazolin-1 (2H)-yl]pipéridine-1-carbaldéhyde | LCMS m/z [MH]+ 484 |
| 72 | 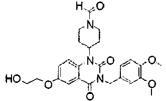 | 4-[3-(3,4-diméthoxybenzyl)-6-(2-hydroxyéthoxy)-2,4-dioxo-3,4-dihydroquinazolin-1 (2H)-yl]pipéridine-1-carbaldéhyde | LCMS m/z [MH]+484 |
| 73 | 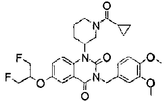 | 1-[1-(cyclopropylcarbonyl)pipéridin-3-yl]-3-(3,4-diméthoxybenzyl)-6-[2-fluoro-1-(fluorométhyl)éthoxy]quinazoli ne-2,4(1H,3H)-dione | LCMS m/z [MH]+ 558 |
| 74 | 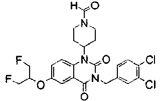 | 4-[3-(3,4-dichlorobenzyl)-6-[2-fluoro-1-(fluorométhyl)éthoxy]-2,4-dioxo-3,4-dihydroquinazolin-1 (2H)-yl]pipéridine-1-carbaldéhyde | LCMS m/z [MH]+ 526 |
| 75 | 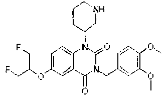 | 3-(3,4-diméthoxybenzyl)-6-[2-fluoro-1-(fluorométhyl)éthoxy]-1-pipéridin-3-ylquinazoline-2,4(1H,3H)-dione | LCMS m/z [MH]+ 490 |
| 76 | 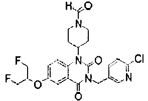 | 4-{3-[(6-chloropyridin-3-yl)méthyl]-6-[2-fluoro-1-(fluorométhyl)éthoxy]-2,4-dioxo-3,4-dihydroquinazolin-1(2H)-yl}pipéridine-1-carbaldéhyde | LCMS m/z [MH]+ 493 |
| 77 | 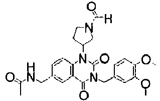 | N-{[3-(3,4-diméthoxybenzyl)-1-(1-formylpyrrolidin-3-yl)-2,4-dioxo-1,2,3,4-tétrahydroquinazolin-6-yl]méthyl}acétamide | LCMS m/z [MH]+ 481 |
| 78 | 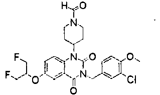 | 4-[3-(3-chloro-4-méthoxybenzyl)-6-[2-fluoro-1-(fluorométhyl)éthoxy]-2,4-dioxo-3,4-dihydroquinazolin-1(2H)-yl]pipéridine-1-carbaldéhyde | LCMS m/z [MH]+ 522 |
| 79 | 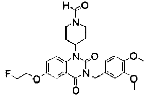 | 4-[3-(3,4-diméthoxybenzyl)-6-(2-fluoroéthoxy)-2,4-dioxo-3,4-dihydroquinazolin-1 (2H)-yl]pipéridine-1-carbaldéhyde | LCMS m/z [MH]+ 486 |
| 80 | 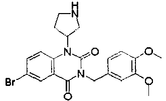 | 6-bromo-3-(3,4-diméthoxybenzyl)-1-[pyrrolidin-3-yl]quinazoline-2,4(1 H,3H)-dione | LCMS m/z [MH]+ 460 |
| 81 | 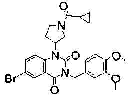 | 6-bromo-1-[1-(cyclopropylcarbonyl)pyrrolidin-3-yl]-3-(3,4-diméthoxybenzyl)quinazoline-2,4(1H,3H)-dione | LCMS m/z [MH]+ 528 |
| 82 | 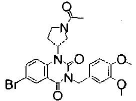 | 1-(1-acétylpyrrolidin-3-yl)-6-bromo-3-(3,4-diméthoxybenzyl)quinazoline-2,4(1H,3H)-dione | LCMS m/z [MH]+ 502 |
| 83 | 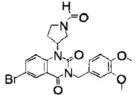 | 3-[6-bromo-3-(3,4-diméthoxybenzyl)-2,4-dioxo-3,4-dihydroquinazolin-1 (2H)-yl]pyrrolidine-1-carbaldéhyde | LCMS m/z [MH]+ 488 |
| 84 | 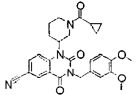 | 1-[1-(cyclopropylcarbonyl)pipéridin-3-yl]-3-(3,4-diméthoxybenzyl)-2,4-dioxo-1,2,3,4-tétrahydroquinazoline-6-carbonitrile | LCMS m/z [MH]+ 489 |
| 85 | 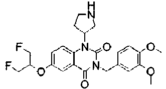 | 3-(3,4-diméthoxybenzyl)-6-[2-fluoro-1-(fluorométhyl)éthoxy]-1-pyrrolidin-3-ylquinazoline-2,4(1H,3H)-dione | LCMS m/z [MH]+ 476 |
| 86 | 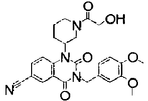 | 3-(3,4-diméthoxybenzyl)-1-[1-(hydroxyacétyl)pipéridin-3-yl]-2,4-dioxo-1,2,3,4-tétrahydroquinazoline-6-carbonitrile | LCMS m/z [MH]+ 479 |
| 87 | 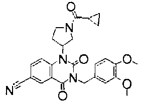 | 1-[1-(cyclopropylcarbonyl)pyrrolidin-3-yl]-3-(3,4-diméthoxybenzyl)-2,4-dioxo-1,2,3,4-tétrahydroquinazoline-6-carbonitrile | LCMS m/z [MH]+ 475 |
| 88 | 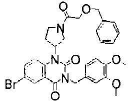 | 1-{1-[(benzyloxy)acétyl]pyrrolidin-3-yl}-6-bromo-3-(3,4-diméthoxybenzyl)quinazoline-2,4(1H,3H)-dione | LCMS m/z [MH]+ 608 |
| 89 | 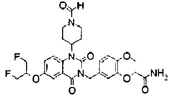 | 2-[5-({6-[2-fluoro-1-(fluorométhyl)éthoxy]-1-(1-formylpipéridin-4-yl)-2,4-dioxo-1,4-dihydroquinazolin-3(2H)-yl}méthyl)-2-méthoxyphénoxy]acétamide | LCMS m/z [MH]+ 561 |
| 90 | 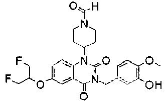 | 4-{6-[2-fluoro-1-(fluorométhyl)éthoxy]-3-(3-hydroxy-4-méthoxybenzyl)-2,4-dioxo-3,4-dihydroquinazolin-1(2H)-yl}pipéridine-1-carbaldéhyde | LCMS m/z [MH]+ 504 |
| 91 | 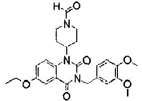 | 4-[3-(3,4-diméthoxybenzyl)-6-éthoxy-2,4-dioxo-3,4-dihydroquinazolin-1 (2H)-yl]pipéridine-1-carbaldéhyde | LCMS m/z [MH]+ 468 |
| 92 | 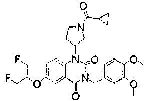 | 1-[1-(cyclopropylcarbonyl)pyrrolidin -3-yl]-3-(3,4-diméthoxybenzyl)-6-[2-fluoro-1-(fluorométhyl)éthoxy]quinazoli ne-2,4(1H,3H)-dione | LCMS m/z [MH]+ 544 |
| 93 | 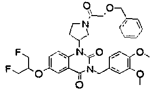 | 1-{1-[(benzyloxy)acétyl] pyrrolidin-3-yl}-3-(3,4-diméthoxybenzyl)-6-[2-fluoro-1-(fluorométhyl)éthoxyJquinazoline-2,4(1H,3H)-dione | LCMS m/z [MH]+ 624 |
| 94 | 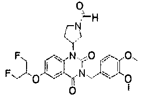 | 3-[3-(3,4-diméthoxybenzyl)-6-[2-fluoro-1-(fluorométhyl)éthoxy]-2,4-dioxo-3,4-dihydroquinazolin-1(2H)-yl]pyrrolidine-1-carbaldéhyde | LCMS m/z [MH]+ 504 |
| 95 | 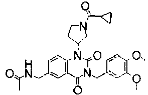 | N-({1-[1-(cyclopropylcarbonyl)pyrrolidin-3-yl]-3-(3,4-diméthoxybenzyl)-2,4-dioxo-1,2,3,4-tétrahyd roquinazolin 6-yl}méthyl)acétamide | LCMS m/z [MH]+ 521 |
| 96 | 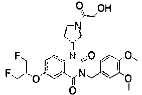 | 3-(3,4-diméthoxybenzyl)-6-[2-fluoro-1-(fluorométhyl)éthoxy]-1-[1-(hydroxyacétyl)pyrrolidin-3-yl]quinazoline-2,4(1 H,3H)-dione | LCMS m/z [M+Na]+ 534 |
| 97 | 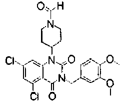 | 4-[5,7-dichloro-3-(3,4-diméthoxybenzyl)-2,4-dioxo-3,4-dihydroquinazolin-1 (2H)-yl]pipéridine-1-carbaldéhyde | LCMS m/z [MH]+ 492 |
| 98 | 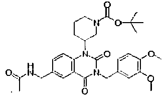 | 3-{6-[(acétylamino)méthyl]-3-(3,4-diméthoxybenzyl)-2,4-dioxo-3,4-dihydroquinazolin-1 (2H)-yl}pipéridine-1-carboxylate de 1,1-diméthyléthyle | LCMS m/z [M+Na]+ 589 |
| 99 | 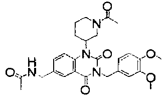 | N-{[3-(3,4-diméthoxybenzyl)-1-(1-formylpipéridin-3-yl)-2,4-dioxo-1,2,3,4-tétrahydroquinazoli n-6-yl]méthyl}acétamide | LCMS m/z [MH]+ 495 |
| 100 | 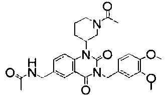 | N-{[1-(1-acétylpipéridin-3-yl)-3-(3,4-diméthoxybenzyl)-2,4-dioxo-1,2,3,4-tétrahyd roquinazolin-6-yl]méthyl}acétamide | LCMS m/z [MH]+ 509 |
| 101 | 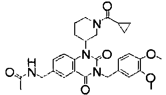 | N-({1-[1-(cyclopropylcarbonyl)pipéridin-3-yl]-3-(3,4-diméthoxybenzyl)-2,4-dioxo-1,2,3,4-tétrahydroquinazolin-6-yl}méthyl)acétamide | LCMS m/z [MH]+ 535 |
| 102 | 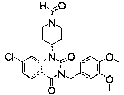 | 4-[7-chloro-3-(3,4-diméthoxybenzyl)-2,4-dioxo-3,4-dihydroquinazolin-1 (2H)-yl]pipéridine-1-carbaldéhyde | LCMS m/z [MH]+ 458 |
| 103 | 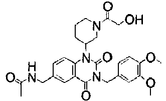 | N-({3-(3,4-diméthoxybenzyl)-1-[1-(hydroxyacétyl)pipéridin-3-yl]-2,4-dioxo-1,2,3,4-tétrahydroquinazolin-6-yl}méthyl)acétamide | LCMS m/z [MH]+ 525 |
| 104 | 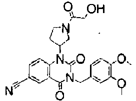 | 3-(3,4-diméthoxybenzyl)-1-[1-(hydroxyacétyl)pyrrolidin-3-yl]-2,4-dioxo-1,2,3,4-tétrahydroquinazoline-6-carbonitrile | LCMS m/z [MH]+ 465 |
| 105 | 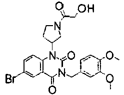 | 6-bromo-3-(3,4-diméthoxybenzyl)-1-[1-(hydroxyacétyl)pyrrolidin-3-yl]quinazoline-2,4(1 H,3H)-dione | LCMS m/z [MH]+ 518 |
| 106 | 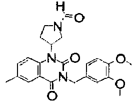 | 3-[3-(3,4-diméthoxybenzyl)-6-méthyl-2,4-dioxo-3,4-dihydroquinazolin-1 (2H)-yl]pyrrolidine-1-carbaldéhyde | LCMS m/z [MH]+ 424 |
| 107 | 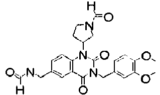 | N-{[3-(3,4-diméthoxybenzyl)-1-(1-formylpyrrolidin-3-yl)-2,4-dioxo-1,2,3,4-tétrahydroquinazolin-6-yl]méthyl}formamide | LCMS m/z [MH]+ 467 |
| 108 | 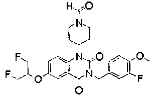 | 4-{6-[2-fluoro-1-(fluorométhyl)éthoxy]-3-(3-fluoro-4-méthoxybenzyl)-2,4-d ioxo-3,4-dihydroquinazolin-1 (2H)-yl}pipéridine-1-carbaldéhyde | LCMS m/z [MH]+ 506 |
| 109 | 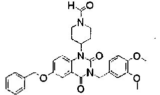 | 4-[6-(benzyloxy)-3-(3,4-diméthoxybenzyl)-2,4-dioxo-3,4-dihydroquinazolin-1 (2H)-yl]pipéridine-1-carbaldéhyde | LCMS m/z [MH]+ 530 |
| 110 | 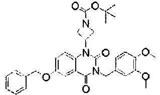 | 3-[6-(benzyloxy)-3-(3,4-diméthoxybenzyl)-2,4-dioxo-3,4-dihydroquinazolin-1 (2H)-yl]azétidine-1-carboxylate de 1,1-diméthyléthyle | LCMS m/z [MH]+ 574 |
| 111 | 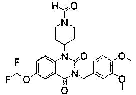 | 4-[6-(difluorométhoxy)-3-(3,4-d i méthoxybenzyl)-2,4-d ioxo-3,4-dihydroquinazolin-1 (2H)-yl]pipéridine-1-carbaldéhyde | LCMS m/z [MH]+ 490 |
| 112 | 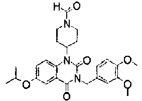 | 4-[3-(3,4-diméthoxybenzyl)-6-(1-méthyléthoxy)-2,4-dioxo-3,4-dihydroquinazolin-1 (2H)-yl]pipéridine-1-carbaldéhyde | LCMS m/z [MH]+ 482 |
| 113 | 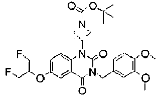 | 3-[3-(3,4-diméthoxybenzyl)-6-[2-fluoro-1-(fluorométhyl)éthoxy]-2,4-dioxo-3,4-dihydroquinazolin-1(2H)-yl]azétidine-1-carboxylate de 1,1-diméthyléthyle | LCMS m/z [MH]+ 562 |
| 114 | 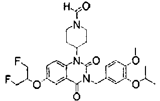 | 4-{6-[2-fluoro-1-(fluorométhyl)éthoxy]-3-[4-méthoxy-3-(1-méthyléthoxy)benzyl]-2,4-dioxo-3,4-dihydroquinazolin-1 (2H)-yl}pipéridine-1-carbaldéhyde | LCMS m/z [MH]+ 546 |
| 115 | 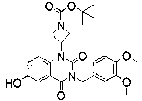 | 3-[3-(3,4-diméthoxybenzyl)-6-hydroxy-2,4-dioxo-3,4-dihydroquinazolin-1 (2H)-yl]azétidine-1-carboxylate de 1,1-diméthyléthyle | LCMS m/z [MH]+ 484 |
| 116 | 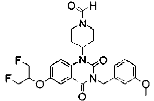 | 4-{6-[2-fluoro-1-(fluorométhyl)éthoxy]-3-(3-méthoxybenzyl)-2,4-dioxo-3,4-dihydroquinazolin-1 (2H)-yl}pipéridine-1-carbaldéhyde | LCMS m/z [MH]+ 488 |
| 117 | 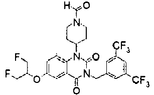 | 4-{3-[3,5-bis(trifluorométhyl)benzyl]-6-[2-fluoro-1-(fluorométhyl)éthoxy]-2,4-dioxo-3,4-dihydroquinazolin-1(2H)-yl}pipéridine-1-carbaldéhyde | LCMS m/z [MH]+ 564 |
| 118 | 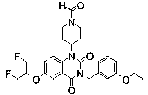 | 4-[3-(3-éthoxybenzyl)-6-[2-fluoro-1-(fluorométhyl)éthoxy]-2,4-dioxo-3,4-dihydroquinazolin-1 (2H)-yl]pipéridine-1-carbaldéhyde | LCMS m/z [MH]+ 502 |

| ***Composé No*** | ***STRUCTURE*** | ***NOMENCLATURE*** | ***MASSE LCUVMS MH+*** |
|---|---|---|---|
| 119 | 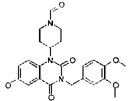 | 4-[3-(3,4-diméthoxybenzyl)-6-hydroxy-2,4-dioxo-3,4-dihydroquinazolin-1(2H)-yl]pipéridine-1-carbaldéhyde | 440 |
| 120 | 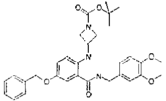 | 3-({4-(benzyloxy)-2-[(3,4-diméthoxybenzyl)carbamoyl]phényl}amino)azétidine-1-carboxylate de 1,1-diméthyléthyle | 548 |
| 121 | 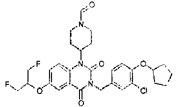 | 4-{3-[3-chloro-4-(cyclopentyloxy)benzyl]-6-[2-fluoro-1-(fluorométhyl)éthoxy]-2,4-dioxo-3,4-dihydroquinazolin-1(2H)-yl}pipéridine-1-carbaldéhyde | 576 |
| 122 | 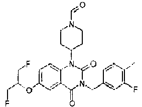 | 4-{6-[2-fluoro-1-(fluorométhyl)éthoxy]-3-(3-fluoro-4-méthylbenzyl)-2,4-dioxo-3,4-dihydroquinazolin-1(2H)-yl}pipéridine-1-carbaldéhyde | 490 |
| 123 | 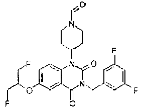 | 4-[3-(3,5-difluorobenzyl)-6-[2-fluoro-1-(fluorométhyl)éthoxy]-2,4-dioxo-3,4-dihydroquinazolin-1(2H)-yl]pipéridine-1-carbaldéhyde | 494 |
| 124 | 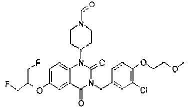 | 4-{3-[3-chloro-4-(2-méthoxyéthoxy)benzyl]-6-[2-fluoro-1-(fluorométhyl)éthoxy]-2,4-dioxo-3,4-dihydroquinazolin-1(2H)-yl}pipéridine-1-carbaldéhyde | 566 |
| 125 | 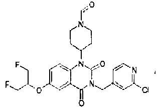 | 4-{3-[(2-chloropyridin-4-yl)méthyl]-6-[2-fluoro-1-(fluorométhyl)éthoxy]-2,4-dioxo-3,4-dihydroquinazolin-1(2H)-yl}pipéridine-1-carbaldéhyde | 493 |
| 126 | 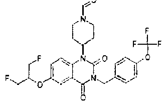 | 4-{6-[2-fluoro-1-(fluorométhyl)éthoxy]-2,4-dioxo-3-[4-(trifluorométhoxy)benzyl]-3,4-dihydroquinazolin-1(2H)-yl}pipéridine-1-carbaldéhyde | 542 |
| 127 | 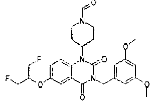 | 4-[3-(3,5-diméthoxybenzyl)-6-[2-fluoro-1-(fluorométhyl)éthoxy]-2,4-dioxo-3,4-dihydroquinazolin-1(2H)-yl]pipéridine-1-carbaldéhyde | 518 |
| 128 | 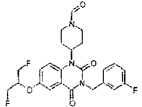 | 4-[3-(3-fluorobenzyl)-6-[2-fluoro-1-(fluorométhyl)éthoxy]-2,4-dioxo-3,4-dihydroquinazolin-1(2H)-yl]pipéridine-1-carbaldéhyde | 476 |
| 129 | 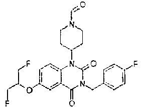 | 4-[3-(4-fluorobenzyl)-6-[2-fluoro-1-(fluorométhyl)éthoxy]-2,4-dioxo-3,4-dihydroquinazolin-1(2H)-yl]pipéridine-1-carbaldéhyde | 476 |
| 130 | 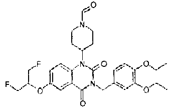 | 4-[3-(3,4-diéthoxybenzyl)-6-[2-fluoro-1-(fluorométhyl)éthoxy]-2,4-dioxo-3,4-dihydroquinazolin-1(2H)-yl]pipéridine-1-carbaldéhyde | 546 |
| 131 | 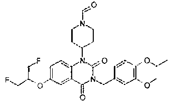 | 4-[3-(4-éthoxy-3-méthoxybenzyl)-6-[2-fluoro-1-(fluorométhyl)éthoxy]-2,4-dioxo-3,4-dihydroquinazolin-1(2H)-yl]pipéridine-1-carbaldéhyde | 532 |
| 132 | 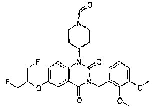 | 4-[3-(2,3-diméthoxybenzyl)-6-[2-fluoro-1-(fluorométhyl)éthoxy]-2,4-dioxo-3,4-dihydroquinazolin-1(2H)-yl]pipéridine-1-carbaldéhyde | 518 |
| 133 | 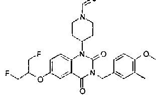 | 4-{6-[2-fluoro-1-(fluorométhyl)éthoxy]-3-(4-méthoxy-3-méthylbenzyl)-2 ,4-dioxo-3,4-dihydroquinazolin-1(2H)-yl}pipéridine-1-carbaldéhyde | 502 |
| 134 | 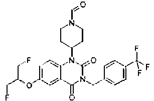 | 4-{6-[2-fluoro-1-(fluorométhyl)éthoxy]-2,4-dioxo-3-[4-(trifluorométhyl)benzyl]-3,4-dihydroquinazolin-1(2H)-yl}pipéridine-1-carbaldéhyde | 526 |
| 135 | 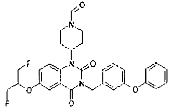 | 4-{6-[2-fluoro-1-(fluorométhyl)éthoxy]-2,4-dioxo-3-(3-phénoxybenzyl)-3,4-dihydroquinazolin-1(2H)-yl}pipéridine-1-carbaldéhyde | 550 |
| 136 | 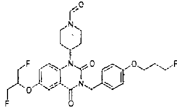 | 4-{6-[2-fluoro-1-(fluorométhyl)éthoxy]-3-[4-(3-fluoropropoxy)benzyl]-2,4-dioxo-3,4-dihydroquinazolin-1(2H)-yl}pipéridine-1-carbaldéhyde | 534 |
| 137 | 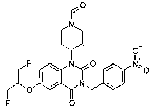 | 4-{6-[2-fluoro-1-(fluorométhyl)éthoxy]-3-(4-nitrobenzyl)-2,4-dioxo-3,4-dihydroquinazolin-1(2H)-yl}pipéridine-1-carbaldéhyde | 503 |
| 138 | 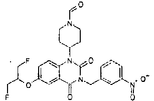 | 4-{6-[2-fluoro-1-(fluorométhyl)éthoxy]-3-(3-nitrobenzyl)-2,4-dioxo-3,4-dihydroquinazolin-1(2H)-yl}pipéridine-1-carbaldéhyde | 503 |
| 139 | 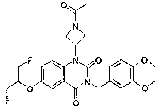 | 1-(1-acétylazétidin-3-yl)-3-(3,4-diméthoxybenzyl)-6-[2-fluoro-1-(fluorométhyl)éthoxy]quinazoline-2,4(1H,3H)-dione | 504 |
| 140 | 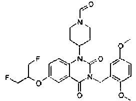 | 4-[3-(2,5-diméthoxybenzyl)-6-[2-fluoro-1-(fluorométhyl)éthoxy]-2,4-dioxo-3,4-dihydroquinazolin-1(2H)-yl]pipéridine-1-carbaldéhyde | 518 |
| 141 | 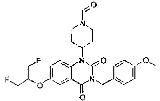 | 4-{6-[2-fluoro-1-(fluorométhyl)éthoxy]-3-(4-méthoxybenzyl)-2,4-dioxo-3,4-dihydroquinazolin-1(2H)-yl}pipéridine-1-carbaldéhyde | 488 |
| 142 | 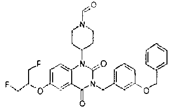 | 4-{3-[3-(benzyloxy)benzyl]-6-[2-fluoro-1-(fluorométhyl)éthoxy]-2,4-dioxo-3,4-dihydroquinazolin-1(2H)-yl}pipéridine-1-carbaldéhyde | 564 |
| 143 | 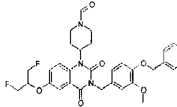 | 4-{3-[4-(benzyloxy)-3-méthoxybenzyl]-6-[2-fluoro-1-(fluorométhyl)éthoxy]-2,4-dioxo-3,4-dihydroquinazolin-1(2H)-yl}pipéridine-1-carbaldéhyde | 594 |
| 144 | 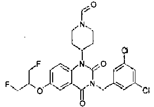 | 4-[3-(3,5-dichlorobenzyl)-6-[2-fluoro-1-(fluorométhyl)éthoxy]-2,4-dioxo-3,4-dihydroquinazolin-1(2H)-yl]pipéridine-1-carbaldéhyde | 526 |
| 145 | 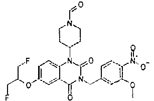 | 4-{6-[2-fluoro-1-(fluorométhyl)éthoxy]-3-(3-méthoxy-4-nitrobenzyl)-2,4-dioxo-3,4-dihydroquinazolin-1(2H)-yl}pipéridine-1-carbaldéhyde | 533 |
| 146 | 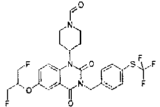 | 4-{6-[2-fluoro-1-(fluorométhyl)éthoxy]-2,4-dioxo-3-{4-[(trifluorométhyl)sulfanyl]benzyl}-3,4-dihydroquinazolin-1(2H)-yl}pipéridine-1-carbaldéhyde | 558 |
| 147 | 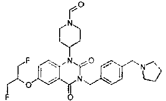 | 4-{6-[2-fluoro-1-(fluorométhyl)éthoxy]-2,4-dioxo-3-[4-(pyrrolidin-1-ylméthyl)benzyl]-3,4-dihydroquinazolin-1(2H)-yl}pipéridine-1-carbaldéhyde | 541 |
| 148 | 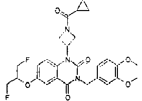 | 1-[1-(cyclopropylcarbonyl)azétidin-3-yl]-3-(3,4-diméthoxybenzyl)-6-[2-fluoro-1-(fluorométhyl)éthoxy]quinazoline-2,4(1H,3H)-dione | 530 |
| 149 | 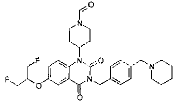 | 4-{6-[2-fluoro-1-(fluorométhyl)éthoxy]-2,4-dioxo-3-[4-(pipéridin-1-ylméthyl)benzyl]-3,4-dihydroquinazolin-1(2H)-yl}pipéridine-1-carbaldéhyde | 555 |
| 150 | 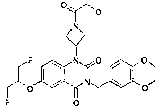 | 3-(3,4-diméthoxybenzyl)-6-[2-fluoro-1-(fluorométhyl)éthoxy]-1-[1-(hydroxyacétyl)azétidin-3-yl]quinazoline-2,4(1H,3H)-dione | 520 |
| 151 | 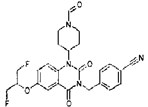 | 4-({6-[2-fluoro-1-(fluorométhyl)éthoxy]-1-(1-formylpipéridin-4-yl)-2,4-dioxo-1,4-dihydroquinazolin-3(2H)-yl}méthyl)benzonitrile | 483 |
| 152 | 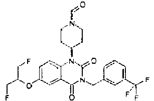 | 4-{6-[2-fluoro-1-(fluorométhyl)éthoxy]-2,4-dioxo-3-[3-(trifluorométhyl)benzyl]-3,4-dihydroquinazolin-1(2H)-yl}pipéridine-1-carbaldéhyde | 526 |
| 153 | 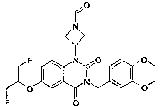 | 3-[3-(3,4-diméthoxybenzyl)-6-[2-fluoro-1-(fluorométhyl)éthoxy]-2,4-dioxo-3,4-dihydroquinazolin-1(2H)-yl]azétidine-1-carbaldéhyde | 490 |
| 154 | 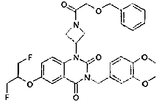 | 1-{1-[(benzyloxy)acétyl]azétidin-3-yl}-3-(3,4-diméthoxybenzyl)-6-[2-fluoro-1-(fluorométhyl)éthoxy]quinazoline-2,4(1H,3H)-dione | 610 |
| 155 | 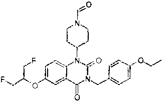 | 4-[3-(4-éthoxybenzyl)-6-[2-fluoro-1-(fluorométhyl)éthoxy]-2,4-dioxo-3,4-dihydroquinazolin-1(2H)-yl]pipéridine-1-carbaldéhyde | 502 |
| 156 | 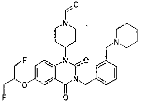 | 4-{6-[2-fluoro-1-(fluorométhyl)éthoxy]-2,4-dioxo-3-[3-(pipéridin-1-ylméthyl)benzyl]-3,4-dihydroquinazolin-1(2H)-yl}pipéridine-1-carbaldéhyde | 555 |
| 157 | 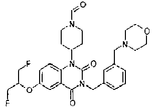 | 4-{6-[2-fluoro-1-(fluorométhyl)éthoxy]-3-[3-(morpholin-4-ylméthyl)benzyl]-2,4-dioxo-3,4-dihydroquinazolin-1(2H)-yl}pipéridine-1-carbaldéhyde | 557 |
| 158 | 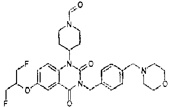 | 4-{6-[2-fluoro-1-(fluorométhyl)éthoxy]-3-[4-(morpholin-4-ylméthyl)benzyl]-2,4-dioxo-3,4-dihydroquinazolin-1(2H)-yl}pipéridine-1-carbaldéhyde | 557 |
| 159 | 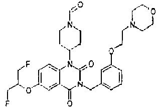 | 4-{6-[2-fluoro-1-(fluorométhyl)éthoxy]-3-[3-(2-morpholin-4-yléthoxy)benzyl]-2,4-dioxo-3,4-dihydroquinazolin-1(2H)-yl}pipéridine-1-carbaldéhyde | 587 |
| 160 | 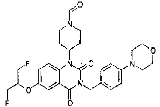 | 4-{6-[2-fluoro-1-(fluorométhyl)éthoxy]-3-(4-morpholin-4-ylbenzyl)-2,4-dioxo-3,4-dihydroquinazolin-1(2H)-yl}pipéridine-1-carbaldéhyde | 543 |
| 161 | 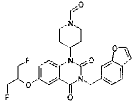 | 4-[3-(1-benzofur-6-ylméthyl)-6-[2-fluoro-1-(fluorométhyl)éthoxy]-2,4-dioxo-3,4-dihydroquinazolin-1(2H)-yl]pipéridine-1-carbaldéhyde | 498 |
| 162 | 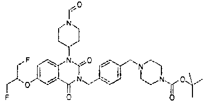 | 4-[4-({6-[2-fluoro-1-(fluorométhyl)éthoxy]-1-(1-formylpipéridin-4-yl)-2,4-dioxo-1,4-dihydroquinazolin-3(2H)-yl}méthyl)benzyl]pipérazine-1-carboxylate de 1,1-diméthyléthyle | 656 |
| 163 | 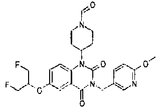 | 4-{6-[2-fluoro-1-(fluorométhyl)éthoxy]-3-[(6-méthoxypyridin-3-yl)méthyl]-2,4-dioxo-3,4-dihydroquinazolin-1(2H)-yl}pipéridine-1-carbaldéhyde | 489 |
| 164 | 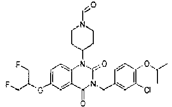 | 4-{3-[3-chloro-4-(1-méthyléthoxy)benzyl]-6-[2-fluoro-1-(fluorométhyl)éthoxy]-2,4-dioxo-3,4-dihydroquinazolin-1(2H)-yl}pipéridine-1-carbaldéhyde | 550 |
| 165 | 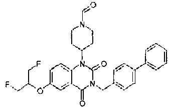 | 4-[3-(biphényl-4-ylméthyl)-6-[2-fluoro-1-(fluorométhyl)éthoxy]-2,4-dioxo-3,4-dihydroquinazolin-1(2H)-yl]pipéridine-1-carbaldéhyde | 534 |
| 166 | 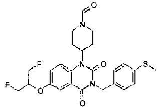 | 4-{6-[2-fluoro-1-(fluorométhyl)éthoxy]-3-[4-(méthylsulfanyl)benzyl]-2,4-dioxo-3,4-dihydroquinazolin-1(2H)-yl}pipéridine-1-carbaldéhyde | 504 |
| 167 | 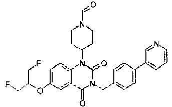 | 4-{6-[2-fluoro-1-(fluorométhyl)éthoxy]-2,4-dioxo-3-(4-pyridin-3-ylbenzyl)-3,4-dihydroquinazolin-1(2H)-yl}pipéridine-1-carbaldéhyde | 535 |
| 168 | 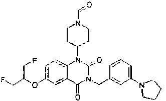 | 4-{6-[2-fluoro-1-(fluorométhyl)éthoxy]-2,4-dioxo-3-(3-pyrrolidin-1-ylbenzyl)-3,4-dihydroquinazolin-1(2H)-yl}pipéridine-1-carbaldéhyde | 527 |
| 169 | 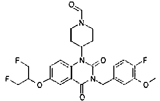 | 4-{6-[2-fluoro-1-(fluorométhyl)éthoxy]-3-(4-fluoro-3-méthoxybenzyl)-2,4-dioxo-3,4-dihydroquinazolin-1(2H)-yl}pipéridine-1-carbaldéhyde | 506 |
| 170 | 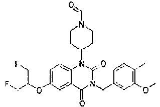 | 4-{6-[2-fluoro-1-(fluorométhyl)éthoxy]-3-(3-méthoxy-4-méthylbenzyl)-2,4-dioxo-3,4-dihydroquinazolin-1(2H)-yl}pipéridine-1-carbaldéhyde | 502 |
| 171 | 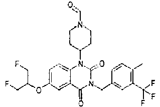 | 4-{6-[2-fluoro-1-(fluorométhy)éthoxy]-3-[4-méthyl-3-(trifluorométhyl)benzyl]-2,4-dioxo-3,4-dihydroquinazolin-1(2H)-yl}pipéridine-1-carbaldéhyde | 540 |
| 172 | 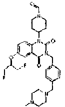 | 4-{6-[2-fluoro-1-(fluorométhyl)éthoxy]-3-{4-[(4-méthylpipérazin-1-yl)méthyl]benzyl}-2,4-dioxo-3,4-dihydroquinazolin-1(2H)-yl}pipéridine-1-carbaldéhyde | 570 |
| 173 | 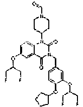 | 4-[3-{4-(cyclopentyloxy)-3-[2-fluoro-1-(fluorométhyl)éthoxy]benzyl}-6-[2-fluoro-1-(fluorométhyl)éthoxy]-2,4-dioxo-3,4-dihydroquinazolin-1(2H)-yl]pipéridine-1-carbaldéhyde | 636 |
| 174 | 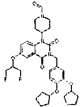 | 3-[3,4-bis(cyclopentyloxy)benzyl]-1-(1-éthénylpipéridin-4-yl)-6-[2-fluoro-1-(fluorométhyl)éthoxy]quinazoline-2,4(1H,3H)-dione | 626 |
| 175 | 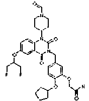 | 2-[2-(cyclopentyloxy)-5-({6-[2-fluoro-1-(fluorométhyl)éthoxy]-1-(1-formylpipéridin-4-yl)-2,4-dioxo-1,4-dihydroquinazolin-3(2H)-yl}méthyl)phénoxy]acétamide | 615 |
| 176 | 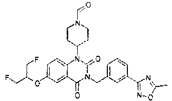 | 4-{6-[2-fluoro-1-(fluorométhyl)éthoxy]-3-[3-(5-méthyl-1,2,4-oxadiazol-3-yl)benzyl]-2,4-dioxo-3,4-dihydroquinazolin-1(2H)-yl}pipéridine-1-carbaldéhyde | 540 |
| 177 | 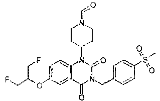 | 4-{6-[2-fluoro-1-(fluorométhyl)éthoxy]-3-[4-(méthylsulfonyl)benzyl]-2,4-dioxo-3,4-dihydroquinazolin-1(2H)-yl}pipéridine-1-carbaldéhyde | 536 |
| 178 | 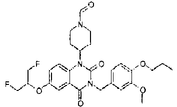 | 4-{6-[2-fluoro-1-(fluorométhyl)éthoxy]-3-(3-méthoxy-4-propoxybenzyl)-2,4-dioxo-3,4-dihydroquinazolin-1(2H)-yl}pipéridine-1-carbaldéhyde | 546 |
| 179 | 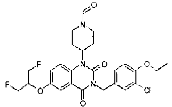 | 4-[3-(3-chloro-4-éthoxybenzyl)-6-[2-fluoro-1-(fluorométhyl)éthoxy]-2,4-dioxo-3,4-dihydroquinazolin-1(2H)-yl]pipéridine-1-carbaldéhyde | 536 |
| 180 | 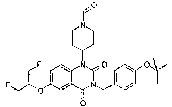 | 4-{3-[4-(1,1-diméthyléthoxy)benzyl]-6-[2-fluoro-1-(fluorométhyl)éthoxy]-2,4-dioxo-3,4-dihydroquinazolin-1(2H)-yl}pipéridine-1-carbaldéhyde | 530 |
| 181 | 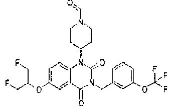 | 4-{6-[2-fluoro-1-(fluorométhyl)éthoxy]-2,4-dioxo-3-[3-(trifluorométhoxy)benzyl]-3,4-dihydroquinazolin-1(2H)-yl}pipéridine-1-carbaldéhyde | 542 |
| 182 | 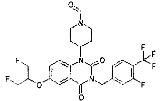 | 4-{6-[2-fluoro-1-(fluorométhyl)éthoxy]-3-[3-fluoro-4-(trifluorométhyl)benzyl]-2,4-dioxo-3,4-dihydroquinazolin-1(2H)-yl}pipéridine-1-carbaldéhyde | 544 |
| 183 | 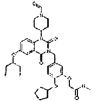 | 2-[2-(cyclopentyloxy)-5-({6-[2-fluoro-1-(fluorométhyl)éthoxy]-1-(1-formylpipéridin-4-yl)-2,4-dioxo-1,4-dihydroquinazolin-3(2H)-yl}méthyl)phénoxy]-N-méthylacétamide | 629 |
| 184 | 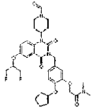 | 2-[2-(cyclopentyloxy)-5-({6-[2-fluoro-1-(fluorométhyl)éthoxy]-1-(1-formylpipéridin-4-yl)-2,4-dioxo-1,4-dihydroquinazolin-3(2H)-yl}méthyl)phénoxy]-N,N-diméthylacétamide | 643 |
| 185 | 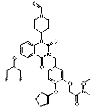 | 2-[2-(cyclopentyloxy)-5-({6-[2-fluoro-1-(fluorométhyl)éthoxy]-1-(1-formylpipéridin-4-yl)-2,4-dioxo-1,4-dihydroquinazolin-3(2H)-yl}méthyl)phénoxy]-N-méthoxy-N-méthylacétamide | 659 |
| 186 | 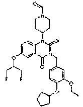 | 4-{3-[4-(cyclopentyloxy)-3-éthoxybenzyl]-6-[2-fluoro-1-(fluorométhyl)éthoxy]-2,4-dioxo-3,4-dihydroquinazolin-1(2H)-yl}pipéridine-1-carbaldéhyde | 586 |
| 187 | 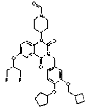 | 4-{3-[3-(cyclobutylméthoxy)-4-(cyclopentyloxy)benzyl]-6-[2-fluoro-1-(fluorométhyl)éthoxy]-2,4-dioxo-3,4-dihydroquinazolin-1(2H)-yl}pipéridine-1-carbaldéhyde | 626 |
| 188 | 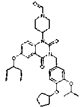 | 4-{3-[4-(cyclopentyloxy)-3-(1-méthyléthoxy)benzyl]-6-[2-fluoro-1-(fluorométhyl)éthoxy]-2,4-dioxo-3,4-dihydroquinazolin-1(2H)-yl}pipéridine-1-carbaldéhyde | 600 |
| 189 | 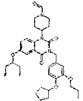 | 4-{3-[4-(cyclopentyloxy)-3-propoxybenzyl]-6-[2-fluoro-1-(fluorométhyl)éthoxy]-2,4-dioxo-3,4-dihydroquinazolin-1(2H)-yl}pipéridine-1-carbaldéhyde | 600 |
| 190 | 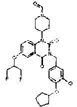 | 4-{3-[4-(cyclopentyloxy)-3-hydroxybenzyl]-6-[2-fluoro-1-(fluorométhyl)éthoxy]-2,4-dioxo-3,4-dihydroquinazolin-1(2H)-yl}pipéridine-1-carbaldéhyde | 558 |
| 191 | 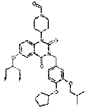 | 4-{3-[4-(cyclopentyloxy)-3-(2-méthylpropoxy)benzyl]-6-[2-fluoro-1-(fluorométhyl)éthoxy]-2,4-dioxo-3,4-dihydroquinazolin-1(2H)-yl}pipéridine-1-carbaldéhyde | 614 |
| 192 | 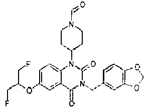 | 4-[3-(1,3-benzodioxol-5-ylméthyl)-6-[2-fluoro-1-(fluorométhyl)éthoxy]-2,4-dioxo-3,4-dihydroquinazolin-1(2H)-yl]pipéridine-1-carbaldéhyde | 502 |
| 193 | 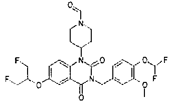 | 4-{3-[4-(difluorométhoxy)-3-méthoxybenzyl]-6-[2-fluoro-1-(fluorométhyl)éthoxy]-2,4-dioxo-3,4-dihydroquinazolin-1(2H)-yl}pipéridine-1-carbaldéhyde | 554 |
| 194 | 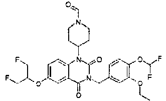 | 4-{3-[4-(difluorométhoxy)-3-éthoxybenzyl]-6-[2-fluoro-1-(fluorométhyl)éthoxy]-2,4-dioxo-3,4-dihydroquinazolin-1(2H)-yl}pipéridine-1-carbaldéhyde | 568 |
| 195 | 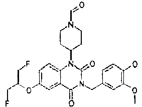 | 4-{6-[2-fluoro-1-(fluorométhyl)éthoxy]-3-(4-hydroxy-3-méthoxybenzyl)-2,4-d ioxo-3,4-dihydroquinazolin-1(2H)-yl}pipéridine-1-carbaldéhyde | 504 |
| 196 | 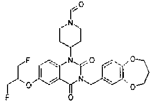 | 4-[3-(3,4-dihydro-2H-1,5-benzodioxépin-7-ylméthyl)-6-[2-fluoro-1-(fluorométhyl)éthoxy]-2,4-dioxo-3,4-dihydroquinazolin-1(2H)-yl]pipéridine-1-carbaldéhyde | 530 |
| 197 | 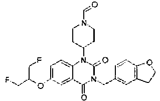 | 4-[3-(2,3-dihydro-1-benzofur-5-ylméthyl)-6-[2-fluoro-1-(fluorométhyl)éthoxy]-2,4-dioxo-3,4-dihydroquinazolin-1(2H)-yl]pipéridine-1-carbaldéhyde | 500 |
| 198 | 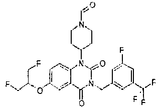 | 4-{6-[2-fluoro-1-(fluorométhyl)éthoxy]-3-[3-fluoro-5-(trifluorométhyl)benzyl]-2,4-dioxo-3,4-dihydroquinazolin-1(2H)-yl}pipéridine-1-carbaldéhyde | 544 |
| 199 | 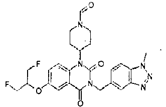 | 4-{6-[2-fluoro-1-(fluorométhyl)éthoxy]-3-[(1-méthyl-1H-benzotriazol-5-yl)méthyl]-2,4-dioxo-3,4-dihydroquinazolin-1(2H)-yl}pipéridine-1-carbaldéhyde | 513 |
| 200 | 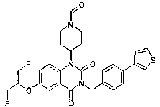 | 4-{6-[2-fluoro-1-(fluorométhyl)éthoxy]-2,4-dioxo-3-(4-thiophén-3-ylbenzyl)-3,4-dihydroquinazolin-1(2H)-yl}pipéridine-1-carbaldéhyde | 540 |
| 201 | 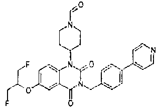 | 4-{6-[2-fluoro-1-(fluorométhyl)éthoxy]-2,4-dioxo-3-(4-pyridin-4-ylbenzyl)-3,4-dihydroquinazolin-1(2H)-yl}pipéridine-1-carbaldéhyde | 535 |
| 202 | 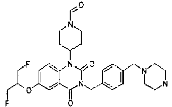 | 4-{6-[2-fluoro-1-(fluorométhyl)éthoxy]-2,4-dioxo-3-[4-(pipérazin-1-ylméthyl)benzyl]-3,4-dihydroquinazolin-1(2H)-yl}pipéridine-1-carbaldéhyde | 555 |
| 203 | 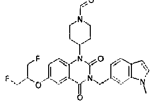 | 4-{6-[2-fluoro-1-(fluorométhyl)éthoxy]-3-[(1-méthyl-11H-indol-6-yl)méthyl]-2,4-dioxo-3,4-dihydroquinazolin-1(2H)-yl}pipéridine-1-carbaldéhyde | 511 |
| 204 | 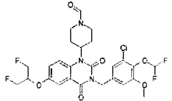 | 4-{3-[3-chloro-4-(difluorométhoxy)-5-méthoxybenzyl]-6-[2-fluoro-1-(fluorométhyl)éthoxy]-2,4-dioxo-3,4-dihydroquinazolin-1(2H)-yl}pipéridine-1-carbaldéhyde | 588 |
| 205 | 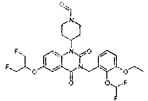 | 4-{3-[2-(difluorométhoxy)-3-éthoxybenzyl]-6-[2-fluoro-1-(fluorométhyl)éthoxy]-2,4-dioxo-3,4-dihydroquinazolin-1(2H)-yl}pipéridine-1-carbaldéhyde | 568 |
| 206 | 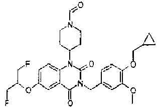 | 4-{3-[4-(cyclopropylméthoxy)-3-méthoxybenzyl]-6-[2-fluoro-1-(fluorométhyl)éthoxy]-2,4-dioxo-3,4-dihydroquinazolin-1(2H)-yl}pipéridine-1-carbaldéhyde | 558 |
| 207 | 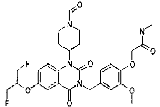 | 2-[4-({6-[2-fluoro-1-(fluorométhyl)éthoxy]-1-(1-formylpipéridin-4-yl)-2,4-dioxo-1,4-dihydroquinazolin-3(2H)-yl}méthyl)-2-méthoxyphénoxy]-N-méthylacétamide | 575 |
| 208 | 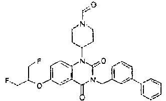 | 4-[3-(biphényl-3-ylméthyl)-6-[2-fluoro-1-(fluorométhyl)éthoxy]-2,4-dioxo-3,4-dihydroquinazolin-1(2H)-yl]pipéridine-1-carbaldéhyde | 534 |
| 209 | 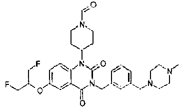 | 4-{6-[2-fluoro-1-(fluorométhyl)éthoxy]-3-{3-[(4-méthylpipérazin-1-yl)méthyl]benzyl}-2,4-dioxo-3,4-dihydroquinazolin-1(2H)-yl}pipéridine-1-carbaldéhyde | 570 |
| 210 | 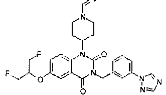 | 4-{6-[2-fluoro-1-(fluorométhyl)éthoxy]-2,4-dioxo-3-[3-(1H-1,2,4-triazol-1-yl)benzyl]-3,4-dihydroquinazolin-1(2H)-yl}pipéridine-1-carbaldéhyde | 525 |
| 211 | 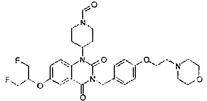 | 4-{6-[2-fluoro-1-(fluorométhyl)éthoxy]-3-[4-(2-morpholin-4-yléthoxy)benzyl]-2,4-dioxo-3,4-dihydroquinazolin-1(2H)-yl}pipéridine-1-carbaldéhyde | 587 |
| 212 | 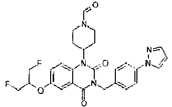 | 4-{6-[2-fluoro-1-(fluorométhyl)éthoxy]-2,4-dioxo-3-[4-(1H-pyrazol-1-yl)benzyl]-3,4-dihydroquinazolin-1(2H)-yl}pipéridine-1-carbaldéhyde | 524 |
| 213 | 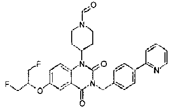 | 4-{6-[2-fluoro-1-(fluorométhyl)éthoxy]-2,4-dioxo-3-(4-pyridin-2-ylbenzyl)-3,4-dihydroquinazolin-1(2H)-yl}pipéridine-1-carbaldéhyde | 535 |
| 214 | 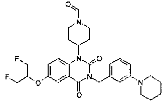 | 4-{6-[2-fluoro-1-(fluorométhyl)éthoxy]-2,4-dioxo-3-(3-pipéridin-1-ylbenzyl)-3,4-dihydroquinazolin-1(2H)-yl}pipéridine-1-carbaldéhyde | 541 |
| 215 | 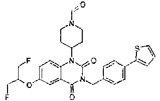 | 4-{6-[2-fluoro-1-(fluorométhyl)éthoxy]-2,4-dioxo-3-(4-thiophén-2-ylbenzyl)-3,4-dihydroquinazolin-1(2H)-yl}pipéridine-1-carbaldéhyde | 540 |
| 216 | 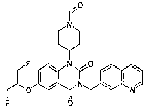 | 4-{6-[2-fluoro-1-(fluorométhyl)éthoxy]-2,4-dioxo-3-(quinoléin-7-ylméthyl)-3,4-dihydroquinazolin-1(2H)-yl}pipéridine-1-carbaldéhyde | 509 |
| 217 | 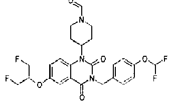 | 4-{3-[4-(difluorométhoxy)benzyl]-6-[2-fluoro-1-(fluorométhyl)éthoxy]-2,4-dioxo-3,4-dihydroquinazolin-1(2H)-yl}pipéridine-1-carbaldéhyde | 524 |
| 218 | 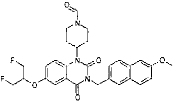 | 4-{6-[2-fluoro-1-(fluorométhyl)éthoxy]-3-[(6-méthoxynaphtalén-2-yl)méthyl]-2,4-dioxo-3,4-dihydroquinazolin-1(2H)-yl}pipéridine-1-carbaldéhyde | 538 |
| 219 | 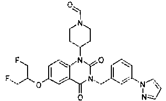 | 4-{6-[2-fluoro-1-(fluorométhyl)éthoxy]-2,4-dioxo-3-[3-(1H-pyrazol-1-yl)benzyl]-3,4-dihydroquinazolin-1(2H)-yl}pipéridine-1-carbaldéhyde | 524 |
| 220 | 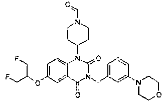 | 4-{6-[2-fluoro-1-(fluorométhyl)éthoxy]-3-(3-morpholin-4-ylbenzyl)-2,4-dioxo-3,4-dihydroquinazolin-1(2H)-yl}pipéridine-1-carbaldéhyde | 543 |
| 221 | 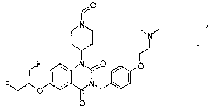 | 4-[3-{4-[2-(diméthylamino)éthoxy]benzyl}-6-[2-fluoro-1-(fluorométhyl)éthoxy]-2,4-dioxo-3,4-dihydroquinazolin-1(2H)-yl]pipéridine-1-carbaldéhyde | 545 |
| 222 | 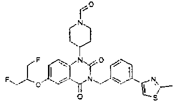 | 4-{6-[2-fluoro-1-(fluorométhyl)éthoxy]-3-[3-(2-méthyl-1,3-thiazol-4-yl)benzyl]-2,4-dioxo-3,4-dihydroquinazolin-1(2H)-yl}pipéridine-1-carbaldéhyde | 555 |
| 223 | 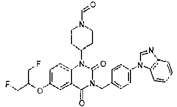 | 4-{3-[4-(1H-benzimidazol-1-yl)benzyl]-6-[2-fluoro-1-(fluorométhyl)éthoxy]-2,4-dioxo-3,4-dihydroquinazolin-1(2H)-yl}pipéridine-1-carbaldéhyde | 574 |
| 224 | 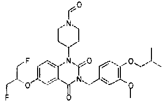 | 4-{6-[2-fluoro-1-(fluorométhyl)éthoxy]-3-[3-méthoxy-4-(2-méthylpropoxy)benzyl]-2,4-dioxo-3,4-dihydroquinazolin-1(2H)-yl}pipéridine-1-carbaldéhyde | 560 |
| 225 | 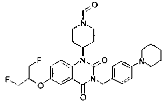 | 4-{6-[2-fluoro-1-(fluorométhyl)éthoxy]-2,4-dioxo-3-(4-pipéridin-1-ylbenzyl)-3,4-dihydroquinazolin-1(2H)-yl}pipéridine-1-carbaldéhyde | 541 |
| 226 | 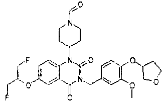 | 4-{6-[2-fluoro-1-(fluorométhyl)éthoxy]-3-[3-méthoxy-4-(tétrahydrofuran-3-yloxy)benzyl]-2,4-dioxo-3,4-dihydroquinazolin-1(2H)-yl}pipéridine-1-carbaldéhyde | 574 |
| 227 | 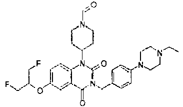 | 4-{3-[4-(4-éthylpipérazin-1-yl)benzyl]-6-[2-fluoro-1-(fluorométhyl)éthoxy]-2,4-dioxo-3,4-dihydroquinazolin-1(2H)-yl}pipéridine-1-carbaldéhyde | 570 |
| 228 | 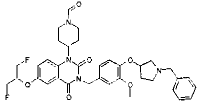 | 4-[3-{4-[(1-benzylpyrrolidin-3-yl)oxy]-3-méthoxybenzyl}-6-[2-fluoro-1-(fluorométhyl)éthoxy]-2,4-dioxo-3,4-dihydroquinazolin-1(2H)-yl]pipéridine-1-carbaldéhyde | 663 |
| 229 | 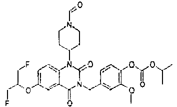 | carbonate de 4-({6-[2-fluoro-1-(fluorométhyl)éthoxy]-1-(1-formylpipéridin-4-yl)-2,4-dioxo-1,4-dihydroquinazolin-3(2H)-yl}méthyl)-2-méthoxyphényle et de 1-méthyléthyle | 590 |
| 230 | 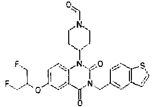 | 4-[3-(1-benzothiophén-5-ylméthyl)-6-[2-fluoro-1-(fluorométhyl)éthoxy]-2,4-dioxo-3,4-dihydroquinazolin-1(2H)-yl]pipéridine-1-carbaldéhyde | 514 |
| 231 | 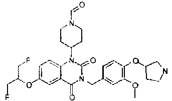 | 4-{6-[2-fluoro-1-(fluorométhyl)éthoxy]-3-[3-méthoxy-4-(pyrrolidin-3-yloxy)benzyl]-2,4-dioxo-3,4-dihydroquinazolin-1(2H)-yl}pipéridine-1-carbaldéhyde | 573 |
| 232 | 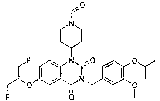 | 4-{6-[2-fluoro-1-(fluorométhyl)éthoxy]-3-[3-méthoxy-4-(1-méthyléthoxy)benzyl]-2,4-dioxo-3,4-dihydroquinazolin-1(2H)-yl}pipéridine-1-carbaldéhyde | 546 |
| 233 | 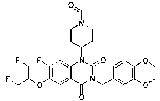 | 4-[3-(3,4-diméthoxybenzyl)-7-fluoro-6-[2-fluoro-1-(fluorométhyl)éthoxy]-2,4-dioxo-3,4-dihydroquinazolin-1(2H)-yl]pipéridine-1-carbaldéhyde | 536 |
| 234 | 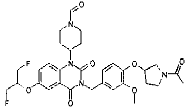 | 4-[3-{4-[(1-acétylpyrrolidin-3-yl)oxy]-3-méthoxybenzyl}-6-[2-fluoro-1-(fluorométhyl)éthoxy]-2,4-dioxo-3,4-dihydroquinazolin-1(2H)-yl]pipéridine-1-carbaldéhyde | 615 |
| 235 | 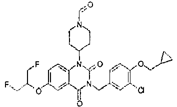 | 4-{3-[3-chloro-4-(cyclopropylméthoxy)benzyl]-6-[2-fluoro-1-(fluorométhyl)éthoxy]-2,4-dioxo-3,4-dihydroquinazolin-1(2H)-yl}pipéridine-1-carbaldéhyde | 562 |
| 236 | 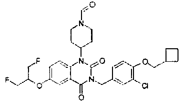 | 4-{3-[3-chloro-4-(cyclobutylméthoxy)benzyl]-6-[2-fluoro-1-(fluorométhyl)éthoxy]-2,4-dioxo-3,4-dihydroquinazolin-1(2H)-yl}pipéridine-1-carbaldéhyde | 576 |
| 237 | 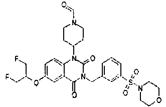 | 4-{6-[2-fluoro-1-(fluorométhyl)éthoxy]-3-[3-(morpholin-4-ylsulfonyl)benzyl]-2,4-dioxo-3,4-dihydroquinazolin-1(2H)-yl}pipéridine-1-carbaldéhyde | 607 |
| 238 | 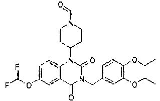 | 4-[3-(3,4-diéthoxybenzyl)-6-(difluorométhoxy)-2,4-dioxo-3,4-dihydroquinazolin-1(2H)-yl]pipéridine-1-carbaldéhyde | 518 |
| 239 | 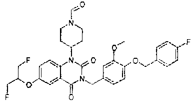 | 4-[3-{4-[(4-fluorobenzyl)oxy]-3-méthoxybenzyl}-6-[2-fluoro-1-(fluorométhyl)éthoxy]-2,4-dioxo-3,4-dihydroquinazolin-1(2H)-yl]pipéridine-1-carbaldéhyde | 612 |
| 240 | 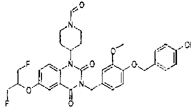 | 4-[3-{4-[(4-chlorobenzyl)oxy]-3-méthoxybenzyl}-6-[2-fluoro-1-(fluorométhyl)éthoxy]-2,4-dioxo-3,4-dihydroquinazolin-1(2H)-yl]pipéridine-1-carbaldéhyde | 628 |
| 241 | 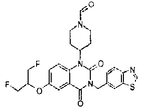 | 4-[3-(1,3-benzothiazol-6-ylméthyl)-6-[2-fluoro-1-(fluorométhyl)éthoxy]-2,4-dioxo-3,4-dihydroquinazolin-1(2H)-yl]pipéridine-1-carbaldéhyde | 515 |
| 242 | 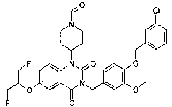 | 4-[3-{4-[(3-chlorobenzyl)oxy]-3-méthoxybenzyl}-6-[2-fluoro-1-(fluorométhyl)éthoxy]-2,4-dioxo-3,4-dihydroquinazolin-1(2H)-yl]pipéridine-1-carbaldéhyde | 628 |
| 243 | 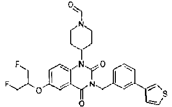 | 4-{6-[2-fluoro-1-(fluorométhyl)éthoxy]-2,4-dioxo-3-(3-thiophén-3-ylbenzyl)-3,4-dihydroquinazolin-1(2H)-yl}pipéridine-1-carbaldéhyde | 540 |
| 244 | 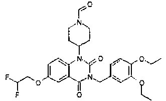 | 4-[3-(3,4-diéthoxybenzyl)-6-(2,2-difluoroéthoxy)-2,4-dioxo-3,4-dihydroquinazolin-1(2H)-yl]pipéridine-1-carbaldéhyde | 532 |
| 245 | 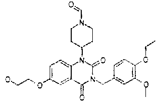 | 4-[3-(4-éthoxy-3-méthoxybenzyl)-6-(2-hydroxyéthoxy)-2,4-dioxo-3,4-dihydroquinazolin-1(2H)-yl]pipéridine-1-carbaldéhyde | 498 |
| 246 | 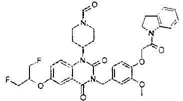 | 4-[3-{4-[2-(2,3-dihydro-1H-indol-1-yl)-2-oxoéthoxy]-3-méthoxybenzyl}-6-[2-fluoro-1-(fluorométhyl)éthoxy]-2,4-dioxo-3,4-dihydroquinazolin-1(2H)-yl]pipéridine-1-carbaldéhyde | 663 |
| 247 | 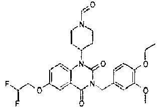 | 4-[6-(2,2-difluoroéthoxy)-3-(4-éthoxy-3-méthoxybenzyl)-2,4-d ioxo-3,4-dihydroquinazolin-1(2H)-yl]pipéridine-1-carbaldéhyde | 518 |
| 248 | 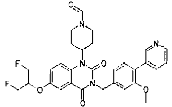 | 4-{6-[2-fluoro-1-(fluorométhyl)éthoxy]-3-(3-méthoxy-4-pyridin-3-ylbenzyl)-2,4-dioxo-3,4-dihydroquinazolin-1(2H)-yl}pipéridine-1-carbaldéhyde | 565 |
| 249 | 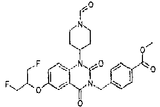 | 4-({6-[2-fluoro-1-(fluorométhyl)éthoxy]-1-(1-formylpipéridin-4-yl)-2,4-dioxo-1,4-dihydroquinazolin-3(2H)-yl}méthyl)benzoate de méthyle | 516 |
| 250 | 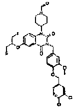 | 4-[3-{4-[(3,4-dichlorobenzyl)oxy]-3-méthoxybenzyl}-6-[2-fluoro-1-(fluorométhyl)éthoxy]-2,4-dioxo-3,4-dihydroquinazolin-1(2H)-yl]pipéridine-1-carbaldéhyde | 662 |
| 251 | 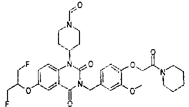 | 4-{6-[2-fluoro-1-(fluorométhyl)éthoxy]-3-[3-méthoxy-4-(2-oxo-2-pipéridin-1-yléthoxy)benzyl]-2,4-dioxo-3,4-dihydroquinazolin-1(2H)-yl}pipéridine-1-carbaldéhyde | 629 |
| 252 | 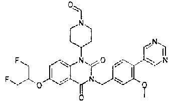 | 4-{6-[2-fluoro-1-(fluorométhyl)éthoxy]-3-(3-méthoxy-4-pyrimidin-5-ylbenzyl)-2,4-dioxo-3,4-dihydroquinazolin-1(2H)-yl}pipéridine-1-carbaldéhyde | 566 |
| 253 | 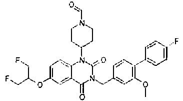 | 4-{6-[2-fluoro-1-(fluorométhyl)éthoxy]-3-[(4'-fluoro-2-méthoxybiphényl-4-yl)méthyl]-2,4-dioxo-3,4-dihydroquinazolin-1(2H)-yl}pipéridine-1-carbaldéhyde | 582 |
| 254 | 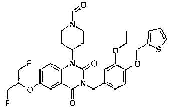 | 4-{3-[3-éthoxy-4-(thiophén-2-ylméthoxy)benzyl]-6-[2-fluoro-1-(fluorométhyl)éthoxy]-2,4-dioxo-3,4-dihydroquinazolin-1(2H)-yl}pipéridine-1-carbaldéhyde | 614 |
| 255 | 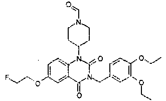 | 4-[3-(3,4-diéthoxybenzyl)-6-(2-fluoroéthoxy)-2,4-dioxo-3,4-dihydroquinazolin-1(2H)-yl]pipéridine-1-carbaldéhyde | 514 |
| 256 | 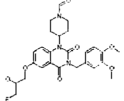 | 4-[3-(3,4-diméthoxybenzyl)-6-(3-fluoro-2-hydroxypropoxy)-2,4-d ioxo-3,4-dihydroquinazolin-1(2H)-yl]pipéridine-1-carbaldéhyde | 516 |
| 257 | 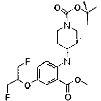 | 4-({4-[2-fluoro-1-(fluorométhyl)éthoxy]-2-(méthoxycarbonyl)phényl}amino)pipéridine-1-carboxylate de 1,1-diméthyléthyle | 429 |
| 258 | 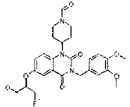 | 4-[3-(3,4-diméthoxybenzyl)-6-[2-fluoro-1-(hydroxyméthyl)éthoxy]-2,4-dioxo-3,4-dihydroquinazolin-1(2H)-yl]pipéridine-1-carbaldéhyde | 516 |
| 259 | 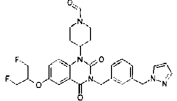 | 4-{6-[2-fluoro-1-(fluorométhyl)éthoxy]-2,4-dioxo-3-[3-(1H-pyrazol-1-ylméthyl)benzyl]-3,4-dihydroquinazolin-1(2H)-yl}pipéridine-1-carbaldéhyde | 538 |
| 260 | 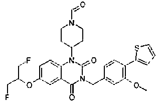 | 4-{6-[2-fluoro-1-(fluorométhyl)éthoxy]-3-(3-méthoxy-4-thiophén-2-ylbenzyl)-2,4-dioxo-3,4-dihydroquinazolin-1(2H)-yl}pipéridine-1-carbaldéhyde | 570 |
| 261 | 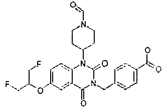 | acide 4-({6-[2-fluoro-1-(fluorométhyl)éthoxy]-1-(1-formylpipéridin-4-yl)-2,4-dioxo-1,4-dihydroquinazolin-3(2H)-yl}méthyl)benzoïque | 502 |
| 262 | 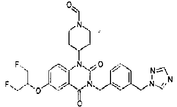 | 4-{6-[2-fluoro-1-(fluorométhyl)éthoxy]-2,4-dioxo-3-[3-(1H-1,2,4-triazol-1-ylméthyl)benzyl]-3,4-dihydroquinazolin-1(2H)-yl}pipéridine-1-carbaldéhyde | 539 |
| 263 | 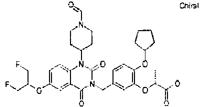 | acide (2R)-2-[2-(cyclopentyloxy)-5-({6-[2-fluoro-1-(fluorométhyl)éthoxy]-1-(1-formylpipéridin-4-yl)-2,4-dioxo-1,4-dihydroquinazolin-3(2H)-yl}méthyl)phénoxy]propanoïque | 630 |
| 264 | 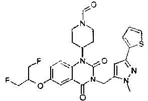 | 4-{6-[2-fluoro-1-(fluorométhyl)éthoxy]-3-[(1-méthyl-3-thiophén-2-yl-1H-pyrazol-5-yl)méthyl]-2,4-dioxo-3,4-dihydroquinazolin-1(2H)-yl}pipéridine-1-carbaldéhyde | 544 |
| 265 | 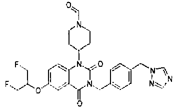 | 4-{6-[2-fluoro-1-(fluorométhyl)éthoxy]-2,4-dioxo-3-[4-(1H-1,2,4-triazol-1-ylméthyl)benzyl]-3,4-dihydroquinazolin-1(2H)-yl}pipéridine-1-carbaldéhyde | 539 |
| 266 | 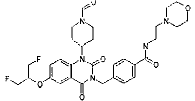 | 4-({6-[2-fluoro-1-(fluorométhyl)éthoxy]-1-(1-formylpipéridin-4-yl)-2,4-dioxo-1,4-dihydroquinazolin-3(2H)-yl}méthyl)-N-(2-morpholin-4-yléthyl)benzamide | 614 |
| 267 | 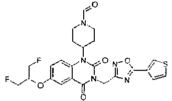 | 4-{6-[2-fluoro-1-(fluorométhyl)éthoxy]-2,4-dioxo-3-[(5-thiophén-3-yl-1,2,4-oxadiazol-3-yl)méthyl]-3,4-dihydroquinazolin-1(2H)-yl}pipéridine-1-carbaldéhyde | 532 |
| 268 | 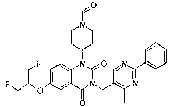 | 4-{6-[2-fluoro-1-(fluorométhyl)éthoxy]-3-[(4-méthyl-2-phénylpyrimidin-5-yl)méthyl]-2,4-dioxo-3,4-dihydroquinazolin-1(2H)-yl}pipéridine-1-carbaldéhyde | 550 |
| 269 | 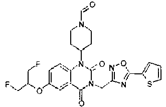 | 4-{6-[2-fluoro-1-(fluorométhyl)éthoxy]-2,4-dioxo-3-[(5-thiophén-2-yl-1,2,4-oxadiazol-3-yl)méthyl]-3,4-dihydroquinazolin-1(2H)-yl}pipéridine-1-carbaldéhyde | 532 |
| 270 | 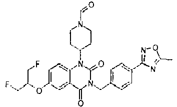 | 4-{6-[2-fluoro-1-(fluorométhyl)éthoxy]-3-[4-(5-méthyl-1,2,4-oxadiazol-3-yl)benzyl]-2,4-dioxo-3,4-dihydroquinazolin-1(2H)-yl}pipéridine-1-carbaldéhyde | 540 |
| 271 | 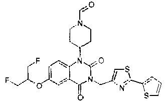 | 4-{6-[2-fluoro-1-(fluorométhyl)éthoxy]-2,4-dioxo-3-[(2-thiophén-2-yl-1,3-thiazol-4-yl)méthyl]-3,4-dihydroquinazolin-1(2H)-yl}pipéridine-1-carbaldéhyde | 547 |
| 272 | 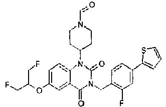 | 4-{6-[2-fluoro-1-(fluorométhyl)éthoxy]-3-(2-fluoro-4-thiophén-2-ylbenzyl)-2,4-dioxo-3,4-dihydroquinazolin-1(2H)-yl}pipéridine-1-carbaldéhyde | 558 |
| 273 | 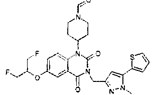 | 4-{6-[2-fluoro-1-(fluorométhyl)éthoxy]-3-[(1-méthyl-5-thiophén-2-yl-1H-pyrazol-3-yl)méthyl]-2,4-dioxo-3,4-dihydroquinazolin-1(2H)-yl}pipéridine-1-carbaldéhyde | 544 |
| 274 | 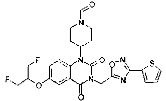 | 4-{6-[2-fluoro-1-(fluorométhyl)éthoxy]-2,4-dioxo-3-[(3-thiophén-2-yl-1,2,4-oxadiazol-5-yl)méthyl]-3,4-dihydroquinazolin-1(2H)-yl}pipéridine-1-carbaldéhyde | 532 |
| 275 | 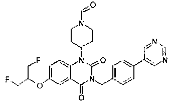 | 4-{6-[2-fluoro-1-(fluorométhyl)éthoxy]-2,4-dioxo-3-(4-pyrimidin-5-ylbenzyl)-3,4-dihydroquinazolin-1(2H)-yl}pipéridine-1-carbaldéhyde | 536 |
| 276 | 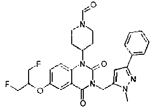 | 4-{6-[2-fluoro-1-(fluorométhyl)éthoxy]-3-[(1-méthyl-3-phényl-1H-pyrazol-5-yl)méthyl]-2,4-dioxo-3,4-dihydroquinazolin-1(2H)-yl}pipéridine-1-carbaldéhyde | 538 |
| 277 | 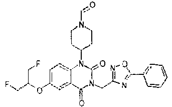 | 4-{6-[2-fluoro-1-(fluorométhyl)éthoxy]-2,4-dioxo-3-[(5-phényl-1,2,4-oxadiazol-3-yl)méthyl]-3,4-dihydroquinazolin-1(2H)-yl}pipéridine-1-carbaldéhyde | 526 |
| 278 | 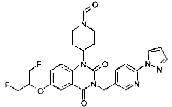 | 4-{6-[2-fluoro-1-(fluorométhyl)éthoxy]-2,4-dioxo-3-{[6-(1H-pyrazol-1-yl)pyridin-3-yl]méthyl}-3,4-dihydroquinazolin-1(2H)-yl}pipéridine-1-carbaldéhyde | 525 |
| 279 | 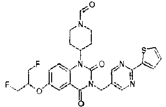 | 4-{6-[2-fluoro-1-(fluorométhyl)éthoxy]-2,4-dioxo-3-[(2-thiophén-2-ylpyrimidin-5-yl)méthyl]-3,4-dihydroquinazolin-1(2H)-yl}pipéridine-1-carbaldéhyde | 542 |
| 280 | 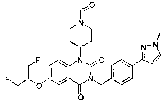 | 4-{6-[2-fluoro-1-(fluorométhyl)éthoxy]-3-[4-(1-méthyl-1H-pyrazol-3-yl)benzyl]-2,4-dioxo-3,4-dihydroquinazolin-1(2H)-yl}pipéridine-1-carbaldéhyde | 538 |
| 281 | 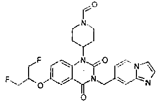 | 4-{6-[2-fluoro-1-(fluorométhyl)éthoxyl-3-(imidazo[1,2-a]pyridin-7-ylméthyl)-2,4-dioxo-3,4-dihydroquinazolin-1(2H)-yl}pipéridine-1-carbaldéhyde | 598 |
| 282 | 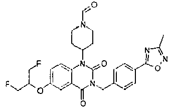 | 4-{6-[2-fluoro-1-(fluorométhyl)éthoxy]-3-[4-(3-méthyl-1,2,4-oxadiazol-5-yl)benzyl]-2,4-dioxo-3,4-dihydroquinazolin-1(2H)-yl}pipéridine-1-carbaldéhyde | 540 |
| 283 | 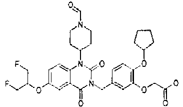 | acide [2-(cyclopentyloxy)-5-({6-[2-fluoro-1-(fluorométhyl)éthoxy]-1-(1-formylpipéridin-4-yl)-2,4-dioxo-1,4-dihydroquinazolin-3(2H)-yl}méthyl)phénoxy]acétique | 616 |
| 284 | 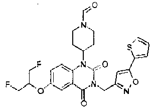 | 4-{6-[2-fluoro-1-(fluorométhyl)éthoxy]-2,4-dioxo-3-[(5-thiophén-2-ylisoxazol-3-yl)méthyl]-3,4-dihydroquinazolin-1(2H)-yl}pipéridine-1-carbaldéhyde | 531 |
| 285 | 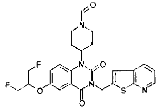 | 4-{6-[2-fluoro-1-(fluorométhyl)éthoxy]-2,4-dioxo-3-(thiéno[2,3-b]pyridin-2-ylméthyl)-3,4-dihydroquinazolin-1(2H)-yl}pipéridine-1-carbaldéhyde | 514 |
| 286 | 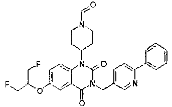 | 4-{6-[2-fluoro-1-(fluorométhyl)éthoxy]-2,4-dioxo-3-[(6-phénylpyridin-3-yl)méthyl]-3,4-dihydroquinazolin-1(2H)-yl}pipéridine-1-carbaldéhyde | 535 |
| 287 | 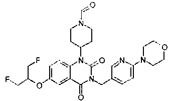 | 4-{6-[2-fluoro-1-(fluorométhyl)éthoxy]-3-[(6-morpholin-4-ylpyridin-3-yl)méthyl]-2,4-dioxo-3,4-dihydroquinazolin-1(2H)-yl}pipéridine-1-carbaldéhyde | 544 |
| 288 | 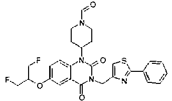 | 4-{6-[2-fluoro-1-(fluorométhyl)éthoxy]-2,4-dioxo-3-[(2-phényl-1,3-thiazol-4-yl)méthyl]-3,4-dihydroquinazolin-1(2H)-yl}pipéridine-1-carbaldéhyde | 541 |
| 289 | 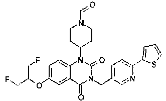 | 4-{6-[2-fluoro-1-(fluorométhyl)éthoxy]-2,4-dioxo-3-[(6-thiophén-2-ylpyridin-3-yl)méthyl]-3,4-dihydroquinazolin-1(2H)-yl}pipéridine-1-carbaldéhyde | 541 |
| 290 | 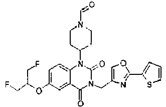 | 4-{6-[2-fluoro-1-(fluorométhyl)éthoxy]-2,4-dioxo-3-[(2-thiophén-2-yl-1,3-oxazol-4-yl)méthyl]-3,4-dihydroquinazolin-1(2H)-yl}pipéridine-1-carbaldéhyde | 531 |
| 291 | 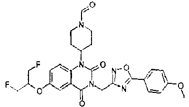 | 4-{6-[2-fluoro-1-(fluorométhyl)éthoxy]-3-{[5-(4-méthoxyphényl)-1,2,4-oxadiazol-3-yl]méthyl}-2,4-dioxo-3,4-dihydroquinazolin-1(2H)-yl}pipéridine-1-carbaldéhyde | 556 |
| 292 | 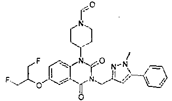 | 4-{6-[2-fluoro-1-(fluorométhyl)éthoxy]-3-[(1-méthyl-5-phényl-1H-pyrazol-3-yl)méthyl]-2,4-dioxo-3,4-dihydroquinazolin-1(2H)-yl}pipéridine-1-carbaldéhyde | 538 |
| 293 | 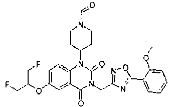 | 4-{6-[2-fluoro-1-(fluorométhyl)éthoxy]-3-{[5-(2-méthoxyphényl)-1,2,4-oxadiazol-3-yl]méthyl}-2,4-dioxo-3,4-dihydroquinazolin-1(2H)-yl}pipéridine-1-carbaldéhyde | 556 |
| 294 | 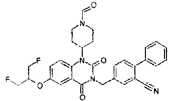 | 4-({6-[2-fluoro-1-(fluorométhyl)éthoxy]-1-(1-formylpipéridin-4-yl)-2,4-dioxo-1,4-dihydroquinazolin-3(2H)-yl}méthyl)biphényl-2-carbonitrile | 559 |
| 295 | 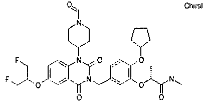 | (2R)-2-[2-(cyclopentyloxy)-5-({6-[2-fluoro-1-(fluorométhyl)éthoxy]-1-(1-formylpipéridin-4-yl)-2,4-dioxo-1,4-dihydroquinazolin-3(2H)-yl}méthyl)phénoxy]-N-méthylpropanamide | 643 |
| 296 | 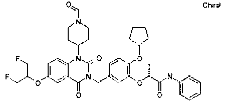 | (2R)-2-[2-(cyclopentyloxy)-5-({6-[2-fluoro-1-(fluorométhyl)éthoxy]-1-(1-formylpipéridin-4-yl)-2,4-dioxo-1,4-dihydroquinazolin-3(2H)-yl}méthyl)phénoxy]-N-phénylpropanamide | 705 |
| 297 | 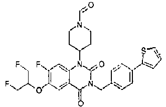 | 4-{7-fluoro-6-[2-fluoro-1-(fluorométhyl)éthoxy]-2,4-dioxo-3-(4-thiophén-2-ylbenzyl)-3,4-dihydroquinazolin-1(2H)-yl}pipéridine-1-carbaldéhyde | 558 |
| 298 | 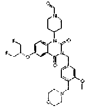 | 4-{6-[2-fluoro-1-(fluorométhyl)éthoxy]-3-[3-méthoxy-4-(morpholin-4-ylméthyl)benzyl]-2,4-dioxo-3,4-dihydroquinazolin-1(2H)-yl}pipéridine-1-carbaldéhyde | 587 |
| 299 | 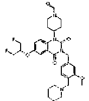 | 4-{6-[2-fluoro-1-(fluorométhyl)éthoxy]-3-[3-méthoxy-4-(pipéridin-1-ylméthyl)benzyl]-2,4-dioxo-3,4-dihydroquinazolin-1(2H)-yl}pipéridine-1-carbaldéhyde | 585 |
| 300 | 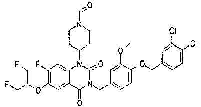 | 4-[3-{4-[(3,4-dichlorobenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde | 680 |
| 301 | 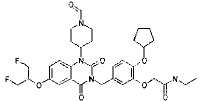 | 2-[2-(cyclopentyloxy)-5-({6-[2-fluoro-1-(fluorométhyl)éthoxy]-1-(1-formylpipéridin-4-yl)-2,4-dioxo-1,4-dihydroquinazolin-3(2H)-yl}méthyl)phénoxy]-N-éthylacétamide | 643 |
| 302 | 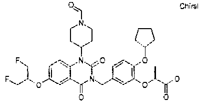 | acide (2S)-2-[2-(cyclopentyloxy)-5-({6-[2-fluoro-1-(fluorométhyl)éthoxy]-1-(1-formylpipéridin-4-yl)-2,4-dioxo-1,4-dihydroquinazolin-3(2H)-yl}méthyl)phénoxy]propanoïque | 630 |
| 303 | 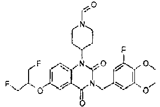 | 4-[3-(3-fluoro-4,5-diméthoxybenzyl)-6-[2-fluoro-1-(fluorométhyl)éthoxy]-2,4-dioxo-3,4-dihydroquinazolin-1(2H)-yl]pipéridine-1-carbaldéhyde | 536 |
| 304 | 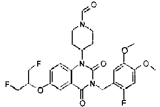 | 4-[3-(2-fluoro-4,5-diméthoxybenzyl)-6-[2-fluoro-1-(fluorométhyl)éthoxy]-2,4-dioxo-3,4-dihydroquinazolin-1(2H)-yl]pipéridine-1-carbaldéhyde | 536 |
| 305 | 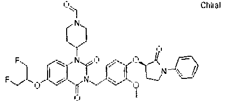 | 4-{6-[2-fluoro-1-(fluorométhyl)éthoxyl-3-(3-méthoxy-4-{[(3R)-2-oxo-1-phénylpyrrolidin-3-yl]oxy}benzyl)-2,4-dioxo-3,4-dihydroquinazolin-1(2H)-yl}pipéridine-1-carbaldéhyde | 663 |
| 306 | 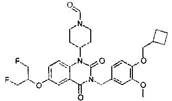 | 4-{3-[4-(cyclobutylméthoxy)-3-méthoxybenzyl]-6-[2-fluoro-1-(fluorométhyl)éthoxy]-2,4-dioxo-3,4-dihydroquinazolin-1(2H)-yl}pipéridine-1-carbaldéhyde | 572 |
| 307 | 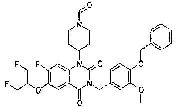 | 4-{3-[4-(benzyloxy)-3-méthoxybenzyl]-7-fluoro-6-[2-fluoro-1-(fluorométhyl)éthoxy]-2,4-dioxo-3,4-dihydroquinazolin-1(2H)-yl}pipéridine-1-carbaldéhyde | 612 |
| 308 | 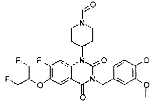 | 4-{7-fluoro-6-[2-fluoro-1-(fluorométhyl)éthoxy]-3-(4-hydroxy-3-méthoxybenzyl)-2,4-dioxo-3,4-dihydroquinazolin-1(2H)-yl}pipéridine-1-carbaldéhyde | 522 |
| 309 | 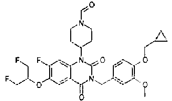 | 4-{3-[4-(cyclopropylméthoxy)-3-méthoxybenzyl]-7-fluoro-6-[2-fluoro-1-(fluorométhyl)éthoxy]-2,4-dioxo-3,4-dihydroquinazolin-1(2H)-yl}pipéridine-1-carbaldéhyde | 576 |
| 310 | 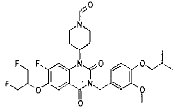 | 4-{7-fluoro-6-[2-fluoro-1-(fluorométhyl)éthoxy]-3-[3-méthoxy-4-(2-méthylpropoxy)benzyl]-2,4-dioxo-3,4-dihydroquinazolin-1(2H)-yl}pipéridine-1-carbaldéhyde | 578 |
| 311 | 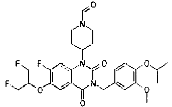 | 4-{7-fluoro-6-[2-fluoro-1-(fluorométhyl)éthoxy]-3-[3-méthoxy-4-(1-méthyléthoxy)benzyl]-2,4-dioxo-3,4-dihydroquinazolin-1(2H)-yl}pipéridine-1-carbaldéhyde | 564 |
| 312 | 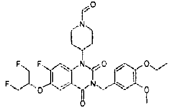 | 4-[3-(4-éthoxy-3-méthoxybenzyl)-7-fluoro-6-[2-fluoro-1-(fluorométhyl)éthoxy]-2,4-dioxo-3,4-dihydroquinazolin-1 (2H)-yl]pipéridine-1-carbaldéhyde | 550 |
| 313 | 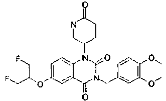 | 3-(3,4-diméthoxybenzyl)-6-[2-fluoro-1-(fluorométhyl)éthoxy]-1-(6-oxopipéridin-3-yl)quinazoline-2,4(1H,3H)-dione | 504 |
| 314 | 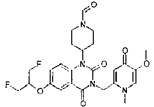 | 4-{6-[2-fluoro-1-(fluorométhyl)éthoxy]-3-[(5-méthoxy-1-méthyl-4-oxo-1,4-dihydropyridin-2-yl)méthyl]-2,4-dioxo-3,4-dihydroquinazolin-1(2H)-yl}pipéridine-1-carbaldéhyde | 519 |
| 315 | 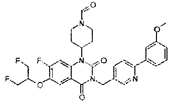 | 4-{7-fluoro-6-[2-fluoro-1-(fluorométhyl)éthoxy]-3-{[6-(3-méthoxyphényl)pyridin-3-yl]méthyl}-2,4-dioxo-3,4-dihydroquinazolin-1(2H)-yl}pipéridine-1-carbaldéhyde | 583 |
| 316 | 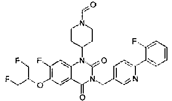 | 4-{7-fluoro-6-[2-fluoro-1-(fluorométhyl)éthoxy]-3-{[6-(2-fluorophényl)pyridin-3-yl]méthyl}-2,4-dioxo-3,4-dihydroquinazolin-1(2H)-yl}pipéridine-1-carbaldéhyde | 571 |
| 317 | 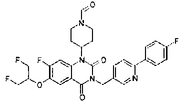 | 4-{7-fluoro-6-[2-fluoro-1-(fluorométhyl)éthoxy]-3-{[6-(4-fluorophényl)pyridin-3-yl]méthyl}-2,4-dioxo-3,4-dihydroquinazolin-1(2H)-yl}pipéridine-1-carbaldéhyde | 571 |
| 318 | 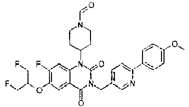 | 4-{7-fluoro-6-[2-fluoro-1-(fluorométhyl)éthoxy]-3-{[6-(4-méthoxyphényl)pyridin-3-yl]méthyl}-2,4-dioxo-3,4-dihydroquinazolin-1(2H)-yl}pipéridine-1-carbaldéhyde | 583 |
| 319 | 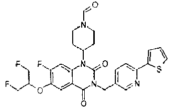 | 4-{7-fluoro-6-[2-fluoro-1-(fluorométhyl)éthoxy]-2,4-dioxo-3-[(6-thiophén-2-ylpyridin-3-yl)méthyl]-3,4-dihydroquinazolin-1(2H)-yl}pipéridine-1-carbaldéhyde | 559 |
| 320 | 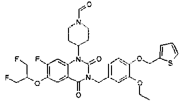 | 4-{3-[3-éthoxy-4-(thiophén-2-ylméthoxy)benzyl]-7-fluoro-6-[2-fluoro-1-(fluorométhyl)éthoxy]-2,4-dioxo-3,4-dihydroquinazolin-1(2H)-yl}pipéridine-1-carbaldéhyde | 632 |
| 321 | 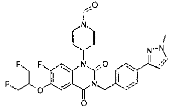 | 4-{7-fluoro-6-[2-fluoro-1-(fluorométhyl)éthoxy]-3-[4-(1-méthyl-1H-pyrazol-3-yl)benzyl]-2,4-dioxo-3,4-dihydroquinazolin-1(2H)-yl}pipéridine-1-carbaldéhyde | 556 |
| 322 | 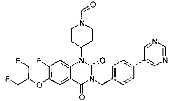 | 4-{7-fluoro-6-[2-fluoro-1-(fluorométhyl)éthoxy]-2,4-dioxo-3-(4-pyrimidin-5-ylbenzyl)-3,4-dihydroquinazolin-1(2H)-yl}pipéridine-1-carbaldéhyde | 554 |
| 323 | 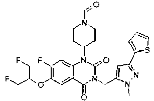 | 4-{7-fluoro-6-[2-fluoro-1-(fluorométhyl)éthoxy]-3-[(1-méthyl-3-thiophén-2-yl-1H-pyrazol-5-yl)méthyl]-2,4-dioxo-3,4-dihydroquinazolin-1(2H)-yl}pipéridine-1-carbaldéhyde | 562 |
| 324 | 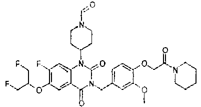 | 4-{7-fluoro-6-[2-fluoro-1-(fluorométhyl)éthoxy]-3-[3-méthoxy-4-(2-oxo-2-pipéridin-1-yléthoxy)benzyl]-2,4-dioxo-3,4-dihydroquinazolin-1(2H)-yl}pipéridine-1-carbaldéhyde | 647 |
| 325 | 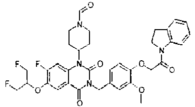 | 4-[3-{4-[2-(2,3-dihydro-1H-indol-1-yl)-2-oxoéthoxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde | 681 |
| 326 | 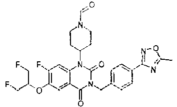 | 4-{7-fluoro-6-[2-fluoro-1-(fluorométhyl)éthoxy]-3-[4-(5-méthyl-1,2,4-oxadiazol-3-yl)benzyl]-2,4-dioxo-3,4-dihydroquinazolin-1(2H)-yl}pipéridine-1-carbaldéhyde | 558 |
| 327 | 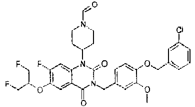 | 4-[3-{4-[(3-chlorobenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde | 646 |
| 328 | 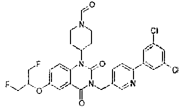 | 4-[3-{[6-(3,5-dichlorophényl)pyridin-3-yl]méthyl}-6-[2-fluoro-1-(fluorométhyl)éthoxy]-2,4-dioxo-3,4-dihydroquinazolin-1(2H)-yl]pipéridine-1-carbaldéhyde | 603 |
| 329 | 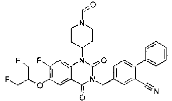 | 4-({7-fluoro-6-[2-fluoro-1-(fluorométhyl)éthoxy]-1-(1-formylpipéridin-4-yl)-2,4-dioxo-1,4-dihydroquinazolin-3(2H)-yl}méthyl)biphényl-2-carbonitrile | 577 |
| 330 | 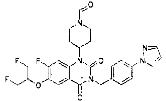 | 4-{7-fluoro-6-[2-fluoro-1-(fluorométhyl)éthoxy]-2,4-dioxo-3-[4-(1H-pyrazol-1-yl)benzyl]-3,4-dihydroquinazolin-1(2H)-yl}pipéridine-1-carbaldéhyde | 542 |
| 331 | 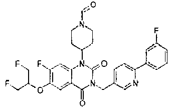 | 4-{7-fluoro-6-[2-fluoro-1-(fluorométhyl)éthoxy]-3-{[6-(3-fluorophényl)pyridin-3-yl]méthyl}-2,4-dioxo-3,4-dihydroquinazolin-1(2H)-yl}pipéridine-1-carbaldéhyde | 571 |
| 332 | 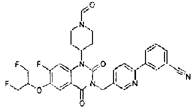 | 3-[5-({7-fluoro-6-[2-fluoro-1-(fluorométhyl)éthoxy]-1-(1-formylpipéridin-4-yl)-2,4-dioxo-1,4-dihydroquinazolin-3(2H)-yl}méthyl)pyridin-2-yl]benzonitrile | 578 |
| 333 | 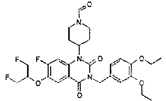 | 4-[3-(3,4-diéthoxybenzyl)-7-fluoro-6-[2-fluoro-1-(fluorométhyl)éthoxy]-2,4-dioxo-3,4-dihydroquinazolin-1(2H)-yl]pipéridine-1-carbaldéhyde | 564 |
| 334 | 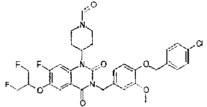 | 4-[3-{4-[(4-chlorobenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde | 646 |
| 335 | 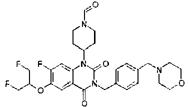 | 4-{7-fluoro-6-[2-fluoro-1-(fluorométhyl)éthoxy]-3-[4-(morpholin-4-ylméthyl)benzyl]-2,4-dioxo-3,4-dihydroquinazolin-1(2H)-yl}pipéridine-1-carbaldéhyde | 575 |
| 336 | 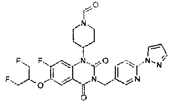 | 4-{7-fluoro-6-[2-fluoro-1-(fluorométhyl)éthoxy]-2,4-dioxo-3-{[6-(1H-pyrazo1-1-yl)pyridin-3-yl]méthyl}-3,4-dihydroquinazolin-1(2H)-yl}pipéridine-1-carbaldéhyde | 543 |
| 337 | 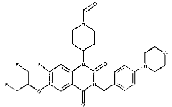 | 4-{7-fluoro-6-[2-fluoro-1-(fluorométhyl)éthoxy]-3-(4-morpholin-4-ylbenzyl)-2,4-dioxo-3,4-dihydroquinazolin-1(2H)-yl}pipéridine-1-carbaldéhyde | 561 |
| 338 | 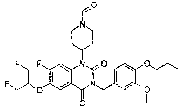 | 4-{7-fluoro-6-[2-fluoro-1-(fluorométhyl)éthoxy]-3-(3-méthoxy-4-propoxybenzyl)-2,4-dioxo-3,4-dihydroquinazolin-1(2H)-yl}pipéridine-1-carbaldéhyde | 564 |
| 339 | 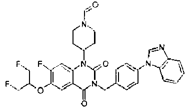 | 4-{3-[4-(1H-benzimidazol-1-yl)benzyl]-7-fluoro-6-[2-fluoro-1-(fluorométhyl)éthoxy]-2,4-dioxo-3,4-dihydroquinazolin-1(2H)-yl}pipéridine-1-carbaldéhyde | 592 |
| 340 | 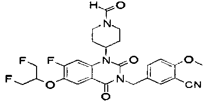 | 5-({7-fluoro-6-[2-fluoro-1-(fluorométhyl)éthoxy]-1-(1-formylpipéridin-4-yl)-2,4-dioxo-1,4-dihydroquinazolin-3(2H)-yl}méthyl)-2-méthoxybenzonitrile | 531 |
| 341 | 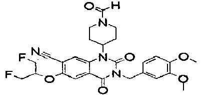 | 3-(3,4-diméthoxybenzyl)-6-[2-fluoro-1-(fluorométhyl)éthoxy]-1-(1-formylpipéridin-4-yl)-2,4-dioxo-1,2,3,4-tétrahydroquinazoline-7-carbonitrile | 543 |
| 342 | 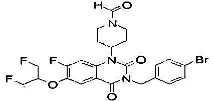 | 4-[3-(4-bromobenzyl)-7-fluoro-6-[2-fluoro-1-(fluorométhyl)éthoxy]-2,4-dioxo-3,4-dihydroquinazolin-1(2H)-yl]pipéridine-1-carbaldéhyde | 554 |
| 343 | 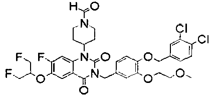 | 4-[3-{4-[(3,4-dichlorobenzyl)oxy]-3-(2-méthoxyéthoxy)benzyl}-7-fluoro-6-[2-fluoro-1-(fluorométhyl)éthoxy]-2,4-dioxo-3,4-dihydroquinazolin-1(2H)-yl]pipéridine-1-carbaldéhyde | 724 |
| 344 | 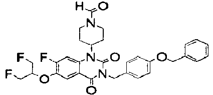 | 4-{3-[4-(benzyloxy)benzyl]-7-fluoro-6-[2-fluoro-1-(fluorométhyl)éthoxy]-2,4-dioxo-3,4-dihydroquinazolin-1(2H)-yl}pipéridine-1-carbaldéhyde | 582 |
| 345 | 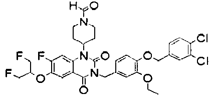 | 4-[3-{4-[(3,4-dichlorobenzyl)oxy]-3-éthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde | 694 |
| 346 | 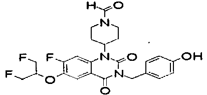 | 4-{7-fluoro-6-[2-fluoro-1-(fluorométhyl)éthoxy]-3-(4-hydroxybenzyl)-2,4-dioxo-3,4-dihydroquinazolin-1(2H)-yl}pipéridine-1-carbaldéhyde | 492 |
| 347 | 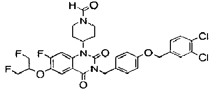 | 4-[3-{4-[(3,4-dichlorobenzyl)oxy]benzyl}-7-fluoro-6-[2-fluoro-1-(fluorométhyl)éthoxy]-2,4-dioxo-3,4-dihydroquinazolin-1(2H)-yl]pipéridine-1-carbaldéhyde | 650 |
| 348 | 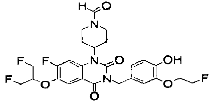 | 4-{7-fluoro-3-[3-(2-fluoroéthoxy)-4-hydroxybenzyl]-6-[2-fluoro-1-(fluorométhyl)éthoxy]-2,4-dioxo-3,4-dihydroquinazolin-1(2H)-yl}pipéridine-1-carbaldéhyde | 554 |
| 349 | 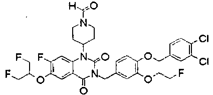 | 4-[3-{4-[(3,4-dichlorobenzyl)oxy]-3-(2-fluoroéthoxy)benzyl]-7-fluoro-6-[2-fluoro-1-(fluorométhyl)éthoxy]-2,4-dioxo-3,4-dihydroquinazolin-1(2H)-yl]pipéridine-1-carbaldéhyde | 712 |
| 350 | 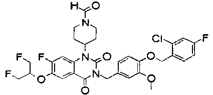 | 4-[3-{4-[(2-chloro-4-fluorobenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde | 664 |
| 351 | 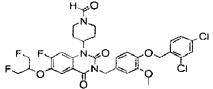 | 4-[3-{4-[(2,4-dichlorobenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde | 680 |
| 352 | 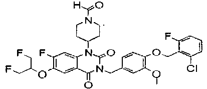 | 4-[3-{4-[(2-chloro-6-fluorobenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde | 664 |
| 353 | 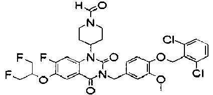 | 4-[3-{4-[(2,6-dichlorobenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde | 680 |
| 354 | 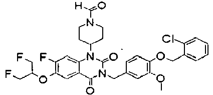 | 4-[3-{4-[(2-chlorobenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde | 646 |
| 355 | 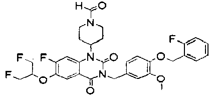 | 4-[7-fluoro-3-{4-[(2-fluorobenzyl)oxy]-3-méthoxybenzyl}-6-[2-fluoro-1-(fluorométhyl)éthoxy]-2,4-dioxo-3,4-dihydroquinazolin-1(2H)-yl]pipéridine-1-carbaldéhyde | 630 |
| 356 | 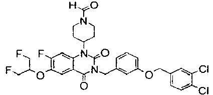 | 4-[3-{3-[(3,4-dichlorobenzyl)oxy]benzyl)-7-fluoro-6-[2-fluoro-1-(fluorométhyl)éthoxy]-2,4-dioxo-3,4-dihydroquinazolin-1(2H)-yl]pipéridine-1-carbaldéhyde | 650 |
| 357 | 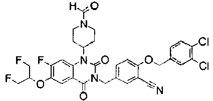 | 2-[(3,4-dichlorobenzyl)oxy]-5-({7-fluoro-6-[2-fluoro-1-(fluorométhyl)éthoxy]-1-(1-formylpipéridin-4-yl)-2,4-dioxo-1,4-dihydroquinazolin-3(2H)-yl}méthyl)benzonitrile | 675 |
| 358 | 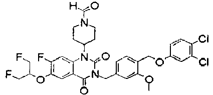 | 4-[3-{4-[(3,4-dichlorophénoxy)méthyl]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde | 680 |
| 359 | 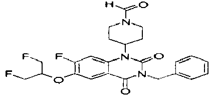 | 4-{3-benzyl-7-fluoro-6-[2-fluoro-1-(fluorométhyl)éthoxy]-2,4-dioxo-3,4-dihydroquinazolin-1(2H)-yl}pipéridine-1-carbaldéhyde | 476 |
| 360 | 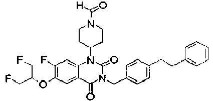 | 4-{7-fluoro-6-[2-fluoro-1-(fluorométhyl)éthoxyl-2,4-dioxo-3-[4-(2-phényléthyl)benzyl]-3,4-dihydroquinazolin-1(2H)-yl}pipéridine-1-carbaldéhyde | 580 |
| 361 | 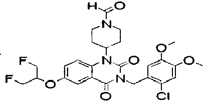 | 4-[3-(2-chloro-4,5-diméthoxybenzyl)-6-[2-fluoro-1-(fluorométhyl)éthoxy]-2,4-dioxo-3,4-dihydroquinazolin-1(2H)-yl]pipéridine-1-carbaldéhyde | 552 |
| 362 | 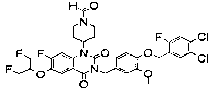 | 4-[3-{4-[(4,5-dichloro-2-fluorobenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde | 698 |
| 363 | 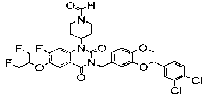 | 4-[3-{3-[(3,4-dichlorobenzyl)oxy]-4-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde | 680 |
| 364 | 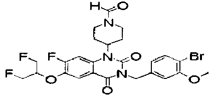 | 4-[3-(4-bromo-3-méthoxybenzyl)-7-fluoro-6-[2-fluoro-1-(fluorométhyl)éthoxy]-2,4-dioxo-3,4-dihydroquinazolin-1(2H)-yl]pipéridine-1-carbaldéhyde | 584 |
| 365 | 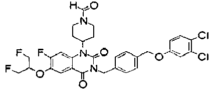 | 4-[3-{4-[(3,4-dichlorophenoxy)méthyl]benzyl}-7-fluoro-6-[2-fluoro-1-(fluorométhyl)éthoxy]-2,4-dioxo-3,4-dihydroquinazolin-1(2H)-yl]pipéridine-1-carbaldéhyde | 650 |
| 366 | 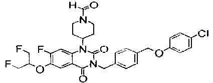 | 4-[3-{4-[(4-chlorophénoxy)méthyl]benzyl}-7-fluoro-6-[2-fluoro-1-(fluorométhyl)éthoxy]-2,4-dioxo-3,4-dihydroquinazolin-1(2H)-yl]pipéridine-1-carbaldéhyde | 616 |
| 367 | 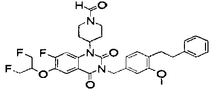 | 4-{7-fluoro-6-[2-fluoro-1-(fluorométhyl)éthoxy]-3-[3-méthoxy-4-(2-phényléthyl)benzyl]-2,4-dioxo-3,4-dihydroquinazolin-1(2H)-yl}pipéridine-1-carbaldéhyde | 610 |
| 368 | 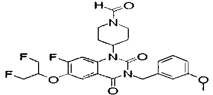 | 4-{7-fluoro-6-[2-fluoro-1-(fluorométhyl)éthoxyl)-3-(3-méthoxybenzyl)-2,4-dioxo-3,4-dihydroquinazolin-1(2H)-yl}pipéridine-1-carbaldéhyde | 506 |
| 369 | 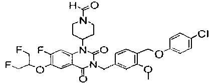 | 4-[3-{4-[(4-chlorophénoxy)méthyl]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde | 646 |
| 370 | 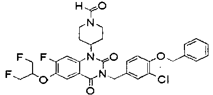 | 4-{3-[4-(benzyloxy)-3-chlorobenzyl]-7-fluoro-6-[2-fluoro-1-(fluorométhyl)éthoxy]-2,4-dioxo-3,4-dihydroquinazolin-1(2H)-yl}pipéridine-1-carbaldéhyde | 616 |
| 371 | 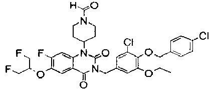 | 4-[3-{3-chloro-4-[(4-chlorobenzyl)oxy]-5-éthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde | 694 |
| 372 | 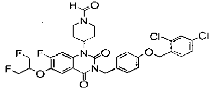 | 4-[3-{4-[(2,4-dichlorobenzyl)oxy]benzyl}-7-fluoro-6-[2-fluoro-1-(fluorométhyl)éthoxy]-2,4-dioxo-3,4-dihydroquinazolin-1(2H)-yl]pipéridine-1-carbaldéhyde | 650 |
| 373 | 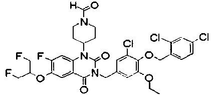 | 4-[3-{3-chloro-4-[(2,4-dichlorobenzyl)oxy]-5-éthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde | 728 |
| 374 | 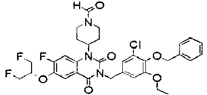 | 4-{3-[4-(benzyloxy)-3-chloro-5-éthoxybenzyl]-7-fluoro-6-[2-fluoro-1-(fluorométhyl)éthoxy]-2,4-dioxo-3,4-dihydroquinazolin-1(2H)-yl}pipéridine-1-carbaldéhyde | 660 |
| 375 | 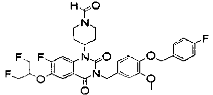 | 4-[7-fluoro-3-{4-[(4-fluorobenzyl)oxy]-3-méthoxybenzyl}-6-[2-fluoro-1-(fluorométhyl)éthoxy]-2,4-dioxo-3,4-dihydroquinazolin-1(2H)-yl]pipéridine-1-carbaldéhyde | 630 |
| 376 | 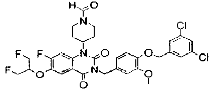 | 4-[3-{4-[(3,5-dichlorobenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde | 680 |
| 377 | 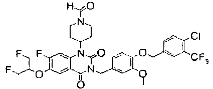 | 4-[3-(4-{[4-chloro-3-(trifluorométhyl)benzyl]oxy}-3-méthoxybenzyl)-7-fluoro-6-[2-fluoro-1-(fluorométhyl)éthoxy]-2,4-dioxo-3,4-dihydroquinazolin-1(2H)-yl]pipéridine-1-carbaldéhyde | 714 |
| 378 | 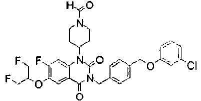 | 4-[3-{4-[(3-chlorophénoxy)méthyl]benzyl}-7-fluoro-6-[2-fluoro-1-(fluorométhyl)éthoxy]-2,4-dioxo-3,4-dihydroquinazolin-1(2H)-yl]pipéridine-1-carbaldéhyde | 616 |
| 379 | 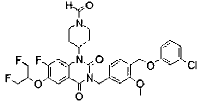 | 4-[3-{4-[(3-chlorophénoxy)méthyl]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde | 646 |
| 380 | 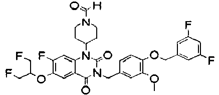 | 4-[3-{4-[(3,5-difluorobenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde | 648 |
| 381 | 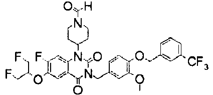 | 4-{3-[4-(benzyloxy)-3-méthoxybenzyl]-7-fluoro-6-[2-fluoro-1-(fluorométhyl)éthoxy]-2,4-dioxo-3,4-dihydroquinazolin-1(2H)-yl}pipéridine-1-carbaldéhyde | 680 |
| 382 | 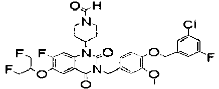 | 4-[3-{4-[(3-chloro-5-fluorobenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde | 664 |
| 383 | 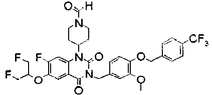 | 4-{7-fluoro-6-[2-fluoro-1-(fluorométhyl)éthoxy]-3-(3-méthoxy-4-{[4-(trifluorométhyl)benzyl]oxy}benzyl)-2,4-dioxo-3,4-dihydroquinazolin-1(2H)-yl}pipéridine-1-carbaldéhyde | 680 |
| 384 | 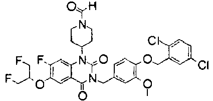 | 4-[3-{4-[(2,5-dichlorobenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde | 680 |
| 385 | 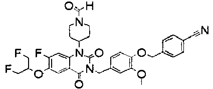 | 4-{[4-({7-fluoro-6-[2-fluoro-1-(fluorométhyl)éthoxy]-1-(1-formylpipéridin-4-yl)-2,4-dioxo-1,4-dihydroquinazolin-3(2H)-yl}méthyl)-2-méthoxyphénoxy]méthyl}benzonitrile | 637 |
| 386 | 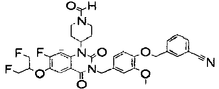 | 3-{[4-({7-fluoro-6-[2-fluoro-1-(fluorométhyl)éthoxy]-1-(1-formylpipéridin-4-yl)-2,4-dioxo-1,4-dihydroquinazolin-3(2H)-yl}méthyl)-2-méthoxyphénoxy]méthyl}benzonitrile | 637 |
| 387 | 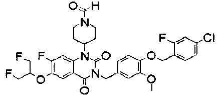 | 4-[3-{4-[(4-chloro-2-fluorobenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde | 664 |
| 388 | 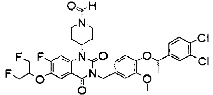 | 4-[3-{4-[1-(3,4-dichlorophényl)éthoxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde | 694 |
| 389 | 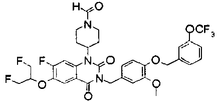 | 4-{7-fluoro-6-[2-fluoro-1-(fluorométhyl)éthoxy]-3-{4-[(3-hydroxybenzyl)oxy]-3-méthoxybenzyl}-2,4-dioxo-3,4-dihydroquinazolin-1(2H)-yl}pipéridine-1-carbaldéhyde | 696 |
| 390 | 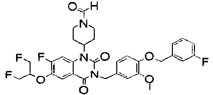 | 4-[7-fluoro-3-{4-[(3-fluorobenzyl)oxy]-3-méthoxybenzyl}-6-[2-fluoro-1-(fluorométhyl)éthoxy]-2,4-dioxo-3,4-dihydroquinazolin-1(2H)-yl]pipéridine-1-carbaldéhyde | 630 |
| 391 | 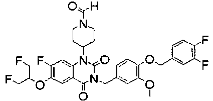 | 4-[3-{4-[(3,4-difluorobenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde | 648 |
| 392 | 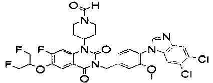 | 4-{3-[4-(5,6-dichloro-1H-benzimidazol-1-yl)-3-méthoxybenzyl]-7-fluoro-6-[2-fluoro-1-(fluorométhyl)éthoxy]-2,4-dioxo-3,4-dihydroquinazolin-1(2H)-yl}pipéridine-1-carbaldéhyde | 660 |
| 393 | 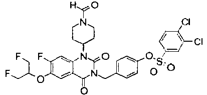 | 3,4-dichlorobenzènesulfonate de 4-({7-fluoro-6-[2-fluoro-1-(fluorométhyl)éthoxy]-1-(1-formylpipéridin-4-yl)-2,4-dioxo-1,4-dihydroquinazolin-3(2H)-yl}méthyl)phényle | 700 |
| 394 | 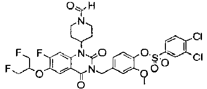 | 3,4-dichlorobenzènesulfonate de 4-({7-fluoro-6-[2-fluoro-1-(fluorométhyl)éthoxy]-1-(1-formylpipéridin-4-yl)-2,4-dioxo-1,4-dihydroquinazolin-3(2H)-yl}méthyl)-2-méthoxyphényle | 730 |
| 395 | 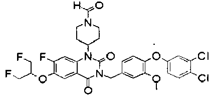 | 4-{3-[4-(3,4-dichlorophénoxy)-3-méthoxybenzyl]-7-fluoro-6-[2-fluoro-1-(fluorométhyl)éthoxy]-2,4-dioxo-3,4-dihydroquinazolin-1(2H)-yl}pipéridine-1-carbaldéhyde | 666 |
| 396 | 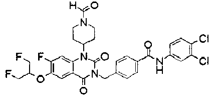 | N-(3,4-dichlorophényl)-4-({7-fluoro-6-[2-fluoro-1-(fluorométhyl)éthoxy]-1-(1-formylpipéridin-4-yl)-2,4-dioxo-1,4-dihydroquinazolin-3(2H)-yl}méthyl)benzamide | 645 |
| 397 | 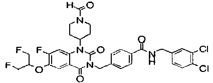 | N-(3,4-dichlorobenzyl)-4-({7-fluoro-6-[2-fluoro-1-(fluorométhyl)éthoxy]-1-(1-formylpipéridin-4-yl)-2,4-dioxo-1,4-dihydroquinazolin-3(2H)-yl}méthyl)benzamide | 659 |
| 398 | 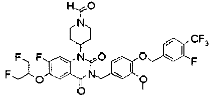 | 4-{7-fluoro-6-[2-fluoro-1-(fluorométhyl)éthoxy]-3-(4-{[3-fluoro-4-(trifluorométhyl)benzy]oxy}-3-méthoxybenzyl)-2,4-dioxo-3,4-dihydroquinazolin-1(2H)-yl}pipéridine-1-carbaldéhyde | 698 |
| 399 | 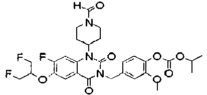 | carbonate de 4-({7-fluoro-6-[2-fluoro-1-(fluorométhyl)éthoxy]-1-(1-formylpipéridin-4-yl)-2,4-dioxo-1,4-dihydroquinazolin-3(2H)-yl}méthyl)-2-méthoxyphényle et de 1-méthyléthyle | 608 |
| 400 | 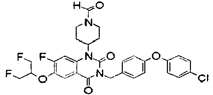 | 4-{3-[4-(4-chlorophénoxy)benzyl]-7-fluoro-6-[2-fluoro-1-(fluorométhyl)éthoxy]-2,4-dioxo-3,4-dihydroquinazolin-1(2H)-yl}pipéridine-1-carbaldéhyde | 602 |
| 401 | 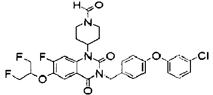 | 4-{3-[4-(3-chlorophénoxy)benzyl]-7-fluoro-6-[2-fluoro-1-(fluorométhyl)éthoxy]-2,4-dioxo-3,4-dihydroquinazolin-1(2H)-yl}pipéridine-1-carbaldéhyde | 602 |
| 402 | 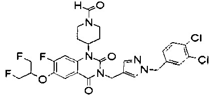 | 4-[3-{[1-(3,4-dichlorobenzyl)-1H-pyrazol-4-yl]méthyl}-7-fluoro-6-[2-fluoro-1-(fluorométhyl)éthoxy]-2,4-dioxo-3,4-dihydroquinazolin-1(2H)-yl]pipéridine-1-carbaldéhyde | 624 |
| 403 | 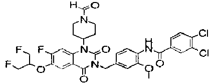 | 3,4-dichloro-N-[4-({7-fluoro-6-[2-fluoro-1-(fluorométhyl)éthoxy]-1-(1-formylpipéridin-4-yl)-2,4-dioxo-1,4-dihydroquinazolin-3(2H)-yl}méthyl)-2-méthoxyphényl]benzamide | 693 |
| 404 | 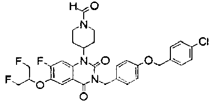 | 4-[3-{4-[(4-chlorobenzyl)oxy]benzyl}-7-fluoro-6-[2-fluoro-1-(fluorométhyl)éthoxy]-2,4-dioxo-3,4-dihydroquinazolin-1(2H)-yl]pipéridine-1-carbaldéhyde | 616 |
| 405 | 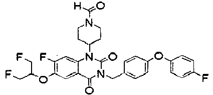 | 4-{7-fluoro-6-[2-fluoro-1-(fluorométhyl)éthoxy]-3-[4-(4-fluorophénoxy)benzyl]-2,4-dioxo-3,4-dihydroquinazolin-1(2H)-yl}pipéridine-1-carbaldéhyde | 586 |
| 406 | 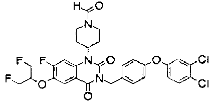 | 4-{3-[4-(3,4-dichlorophénoxy)benzyl]-7-fluoro-6-[2-fluoro-1-(fluorométhyl)éthoxy]-2,4-dioxo-3,4-dihydroquinazolin-1(2H)-yl}pipéridine-1-carbaldéhyde | 636 |
| 407 | 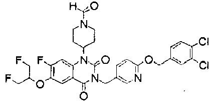 | 4-[3-({6-[(3,4-dichlorobenzyl)oxy]pyridin-3-yl}méthyl)-7-fluoro-6-[2-fluoro-1-(fluorométhyl)éthoxy]-2,4-dioxo-3,4-dihydroquinazolin-1(2H)-yl]pipéridine-1-carbaldéhyde | 651 |
| 408 | 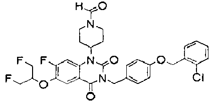 | 4-[3-{4-[(2-chlorobenzyl)oxy]benzyl}-7-fluoro-6-[2-fluoro-1-(fluorométhyl)éthoxy]-2,4-dioxo-3,4-dihydroquinazolin-1(2H)-yl]pipéridine-1-carbaldéhyde | 616 |
| 409 | 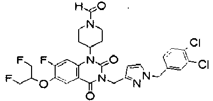 | 4-[3-{[1-(3,4-dichlorobenzyl)-1H-pyrazol-3-yl]méthyl}-7-fluoro-6-[2-fluoro-1-(fluorométhyl)éthoxy]-2,4-dioxo-3,4-dihydroquinazolin-1(2H)-yl]pipéridine-1-carbaldéhyde | 624 |
| 410 | 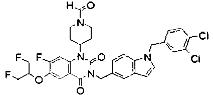 | 4-[3-{[1-(3,4-dichlorobenzyl)-1H-indol-5-yl]méthyl}-7-fluoro-6-[2-fluoro-1-(fluorométhyl)éthoxy]-2,4-dioxo-3,4-dihydroquinazolin-1(2H)-yl]pipéridine-1-carbaldéhyde | 673 |
| 411 | 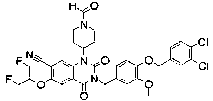 | 3-{4-[(3,4-dichlorobenzyl)oxy]-3-méthoxybenzyl}-6-[2-fluoro-1-(fluorométhyl)éthoxy]-1-(1-formylpipéridin-4-yl)-2,4-dioxo-1,2,3,4-tétrahydroquinazoline-7-carbonitrile | 687 |
| 412 | 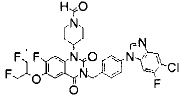 | 4-{3-[4-(5-chloro-6-fluoro-1H-benzimidazol-1-yl)benzyl]-7-fluoro-6-[2-fluoro-1-(fluorométhyl)éthoxy]-2,4-dioxo-3,4-dihydroquinazolin-1(2H)-yl}pipéridine-1-carbaldéhyde | 644 |
| 413 | 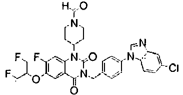 | 4-{3-[4-(5-chloro-1H-benzimidazol-1-yl)benzyl]-7-fluoro-6-[2-fluoro-1-(fluorométhyl)éthoxy]-2,4-dioxo-3,4-dihydroquinazolin-1(2H)-yl}pipéridine-1-carbaldéhyde | 626 |
| 414 | 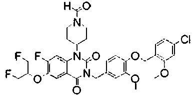 | 4-[3-{4-[(4-chloro-2-méthoxybenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde | 676 |
| 415 | 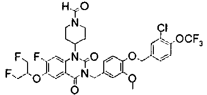 | 4-[3-{4-[(3-chloro-4-hydroxybenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde | 730 |

Les composés conformes à l'invention ont fait l'objet d'essais pharmacologiques qui ont montré leur intérêt comme substances thérapeutiquement actives.

### 1) Mesure de l'activité inhibitrice des composés conformes à l'invention vis-à-vis de la PDE7

La capacité des composés de formule (I) à inhiber la PDE7 est mesurée à l'aide d'un essai enzymatique basé sur la séparation de l'AMPc radioactif (substrat de la PDE7) du 5'-AMP radioactif (produit de la réaction enzymatique) par chromatographie en couche mince sur polyéthylèneimine (PEI) cellulose, après arrêt de la réaction enzymatique. Le 5'-AMP est extrait quantitativement de la PEI cellulose et sa radioactivité mesurée à l'aide d'un compteur à scintillation liquide.

L'activité inhibitrice des composés de formule (I) vis-à-vis de la PDE7 est représentée par la constante d'inhibition Cl₅₀, définie comme la concentration du composé (inhibiteur) testé dans l'essai qui permet de réduire de 50% l'activité enzymatique de la PDE7. Plus les valeurs de CI₅₀ sont faibles, plus les composés sont de puissants inhibiteurs.

### Matériel

Le [³H]-AMPc (NET 275; 25 à 40 Ci/mmole) a été acheté chez Perkin Elmer (NEN Life Sciences, Boston, Etats-Unis), le rolipram chez Sigma (St Louis, MO, Etats-Unis), les feuilles de polyéthylèneimine cellulose F en plastique pour chromatographie sur couche mince chez Merck (Darmstadt, Allemagne). Tous les autres produits utilisés sont d'origine commerciale.

### Enzyme

La PDE7 humaine a été partiellement purifiée à partir de la lignée cellulaire HUT-78 en suivant une méthode analogue à celle décrite par Bloom et Beavo (Proc Natl Acad Sci USA, (1996) 93, 14188-14192). La préparation d'enzyme obtenue est conservée à -80°C dans un tampon contenant 20 mM de Tris-HCl (pH 7,0), 5 mM de MgCl₂, 4 mM d'EDTA, 1 mM de dithiothréitol et 20% de glycérol. La PDE7 partiellement purifiée étant contaminée par de la PDE4, il est nécessaire d'ajouter 10 µM de rolipram (inhibiteur sélectif de PDE4) dans l'essai enzymatique pour inhiber totalement l'activité PDE4. La constante de Michaelis (Km) de la PDE7 pour l'AMPc, mesurée à l'aide de l'essai radiochimique décrit ci-après, est de 21 nM.

### Solutions de composés conformes à l'invention

Les composés de formule (I) à tester comme inhibiteurs de PDE7 sont mis en solution dans le DMSO à une concentration de 10 mM. Ces solutions sont ensuite diluées en cascade dans le DMSO pour obtenir des solutions de concentrations désirées. Ces dernières sont ensuite diluées au vingtième dans le tampon d'essai pour donner des solutions à 5% de DMSO. Ces dernières sont finalement diluées au cinquième dans l'essai enzymatique.

La solution de rolipram (ajouté dans l'essai pour inhiber totalement l'activité PDE4 contaminante) est préparée de manière identique et apporte 1% de DMSO dans l'essai enzymatique.

### Essai enzymatique PDE7

L'essai est réalisé dans des tubes Eppendorf de 1,5 ml contenant 40 mM de Tris-HCl (pH 7,5), 15 mM de MgCl₂, 1 mM d'EGTA, 0,5 mg/ml d'albumine de sérum de boeuf, 0,063 µCi de [³H]-AMPc (correspondant à une concentration d'AMPc comprise entre 15 et 25 nM), 10 µM de rolipram et la PDE7 dans un volume final de 100 µl. L'essai est réalisé en absence (échantillon témoin) ou en présence (échantillon traité) des composés testés comme inhibiteurs de PDE7. La concentration finale de DMSO dans l'essai est de 2%. La réaction est initiée par addition d'enzyme et les échantillons sont maintenus à température ambiante pendant 30 minutes. La dilution enzymatique est ajustée de façon à obtenir un taux de conversion de 10 à 15%. La réaction enzymatique est arrêtée par immersion des tubes Eppendorf bouchés dans un bain-marie à 100°C pendant 3 minutes. Des blancs (réaction arrêtée immédiatement après addition de l'enzyme) sont inclus dans chaque expérience. Les échantillons sont ensuite centrifugés à 10,000xg pendant 1 minute et une part aliquote de 10 µl de surnageant est déposée à 2 cm du bord inférieur d'une feuille de PEI cellulose sur laquelle 10 µg d'AMPc et 10 µg de 5'-AMP ont été préalablement déposés. Pour faciliter la migration et le découpage ultérieur des bandes de PEI cellulose contenant le 5'-AMP, 18 voies de migration d'1 cm de largeur sont délimitées par plaque en grattant la cellulose avec une spatule sur 1 mm de largeur. Les plaques sont développées sur toute leur longueur avec une solution 0,30 M de LiCl dans l'eau par chromatographie ascendante. Le 5'-AMP (Rf = 0,20) et l'AMPc (Rf = 0,47) sont visualisés sous lumière U.V. à 254 nm. Les bandes de PEI cellulose contenant le 5'-AMP sont découpées et le nucléotide est extrait quantitativement dans des fioles de comptage avec 2 ml d'une solution contenant 16 M d'acide formique et 2 M de formate d'ammonium dans l'eau (agitateur rotatif pendant 15 min). Après ajout de 10 ml de liquide à scintillation (OptiPhase HiSafe 3 de Perkin Elmer / Wallac), la radioactivité est comptée à l'aide d'un compteur à scintillation liquide (modèle 1414, Perkin Elmer / Wallac). Chaque essai est réalisé en double. La radioactivité spécifiquement associée au 5'-AMP formé dans la réaction enzymatique est obtenue en soustrayant la valeur moyenne des blancs de la valeur moyenne des témoins (ou des traités).

Le pourcentage d'inhibition de la PDE7 à une concentration donnée du composé testé (inhibiteur) est calculé à l'aide de l'équation : I% = [valeur moyenne des témoins - valeur moyenne des traités] x 100 / [valeur moyenne des témoins - valeur moyenne des blancs].

La Cl₅₀ est la concentration du composé (inhibiteur) testé dans l'essai qui permet de réduire de 50% l'activité enzymatique de la PDE7.

### Résultats

A titre d'exemples illustratifs et non limitatifs, les quinazolinediones suivantes inhibent la PDE7 avec les valeurs de Cl₅₀ indiquées ci-après:

| Composé | Cl₅₀ (µM) |
|---|---|
| n°16 : 4-[6-(2,2-difluoroéthoxy)-3-(3,4-diméthoxybenzyl)-2,4-dioxo-3,4-dihydroquinazolin-1(2H)-yl]pipéridine-1-carbaldéhyde | 0.015 |
| n°11 : 4-[3-(3,4-diméthoxybenzyl)-6-[2-fluoro-1-(fluorométhyl)éthoxy]-2,4-dioxo-3,4-dihydroquinazolin-1(2H)-yl]pipéridine-1-carbaldéhyde | 0.039 |
| n°72 : 4-[3-(3,4-diméthoxybenzyl)-6-(2-hydroxyéthoxy)-2,4-dioxo-3,4-dihydroquinazolin-1(2H)-yl]pipéridine-1-carbaldéhyde | 0.089 |
| n°77 : N-{[3-(3,4-diméthoxybenzyl)-1-(1-formylpyrrolidin-3-yl)-2,4-dioxo-1,2,3,4-tétrahydroquinazolin-6-yl]méthyl}acétamide | 0.82 |
| n°97 : 4-[5,7-dichloro-3-(3,4-diméthoxybenzyl)-2,4-dioxo-3,4-dihydroquinazolin-1(2H)-yl]pipéridine-1-carbaldéhyde | 0.061 |
| n°111 : 4-[6-(difluorométhoxy)-3-(3,4-diméthoxybenzyl)-2,4-dioxo-3,4-dihydroquinazolin-1(2H)-yl]pipéridine-1-carbaldéhyde | 0.0067 |

### 2) Mesure de l'activité inhibitrice des composés conformes à l'invention vis-à-vis de la PDE8

En utilisant pour PDE8 un essai enzymatique équivalent à celui décrit pour PDE7, on obtient les valeurs suivantes de Cl₅₀ :

| Composé | Cl₅₀ PDE7 (µM) | Cl₅₀ PDE8 (µM) |
|---|---|---|
| N° 11 | 0.039 | 0.14 |
| N° 251 | 0.046 | 0.39 |
| N° 294 | 0.037 | 0.015 |

### 3) Evaluation des composés conformes à l'invention dans un modèle murin d'infarctus du myocarde consécutif à un épisode d'ischémie-reperfusion

Des souris mâles C57B6 (23-26 g) sont anesthésiées (kétamine 1 g/kg + xylazine 0.02 g/kg, IP) et mises sous assistance respiratoire avant de subir une thoracotomie visant à exposer le coeur. L'ischémie cardiaque est alors induite par la ligature transitoire de l'artère coronaire gauche. Après 45 minutes d'occlusion coronaire, la ligature est levée pour permettre la reperfusion de la zone ischémiée. Après la fermeture du thorax et la restauration du vide pleurale, les animaux sont placés dans une cage de réveil.

Les composés n° 11, 56, 233 et 309 à tester sont administrés 5 minutes avant la reperfusion par voie intraveineuse à la dose de 3 mg/kg et comparés au véhicule (7.5% cremophor RH40 + 22.5% glycofurol dans une solution de sérum physiologique).

Après 24 heures de reperfusion cardiaque, les animaux sont de nouveau anesthésiés (kétamine 1g/kg + xylazine 0.02 g/kg, IP) avant de recevoir par voie intraveineuse du nitroprussiate de sodium (1 mg/ml à 1 ml/min pendant 4 minutes) pour dilater la vasculature coronaire. L'artère coronaire gauche est religaturée, et une solution de bleu Evans (3%, 200 à 300 µl) est injectée par rétroperfusion carotidienne pour déterminer la zone à risque ventriculaire. Le coeur est alors prélevé et plongé dans une solution de KCI saturée pour l'arrêter en diastole. Des coupes transversales de 1 mm sont réalisées, incubées dans du chlorure de triphenyltetrazolium (1% dans du PBS) à 37°C pendant 2 minutes, puis placées dans du formaldéhyde (4%) pendant une nuit. Le sel de tétrazolium permet de révéler la zone viable au sein de la zone à risque. La surface de l'infarctus par rapport à la zone à risque est déterminée par analyse d'images.

Aucun des composés testés ne modifie la zone à risque exprimée par rapport à la surface du ventricule gauche. Les composés n°11, 56, 233, et 309 réduisent significativement la taille de l'infarctus exprimé par rapport à la zone à risque de respectivement 43%, 33%, 40% et 32%.

Selon l'invention les composés de formule (I) définie plus haut, conformes à l'invention, peuvent être utilisés comme médicaments destiné(s) à traiter au moins une maladie cardiovasculaire et/ou à prévenir l'apparition d'au moins une maladie cardiovasculaire.

Comme maladies cardiovasculaires, on peut, par exemple, citer (i) les maladies coronaires, (ii) les maladies du muscle cardiaque, (iii) les maladies des valves cardiaques, (iv) les maladies du péricarde, (v) les maladies du rythme cardiaque et les maladies de la conduction cardiaque et (vi) les maladies des vaisseaux.

Selon l'invention, les composés de formule (I), conformes à l'invention, peuvent être utilisés comme médicaments destinés à traiter au moins une maladie cardiovasculaire et/ou à prévenir l'apparition d'au moins une maladie cardiovasculaire choisie parmi l'infarctus du myocarde, en particulier les dysfonctions cardiaques contractiles consécutives à un infarctus du myocarde, les maladies associées à des lésions de reperfusion du muscle cardiaque et/ou squelettique, l'hypertension pulmonaire, la fibrose hépatique, la resténose artérielle après angioplastie avec ou sans pose de stent, l'athérosclérose et ses complications telles que, par exemple, la rupture de plaque, l'anévrisme et les maladies coronaires, l'insuffisance cardiaque, les cardiopathies dilatées et les myocardites d'origine virale et/ou bactérienne.

Les composés de formule générale (I), conformes à l'invention, pour le traitement et/ou la prévention d' au moins une maladie mentionnée ci-dessus, fait partie intégrante de l'invention, et en particulier les composés de formule (I) choisis parmi les composés n° 1 à 6, 11 à 14, 16, 20, 22 à 25, 32 à 43, 47 à 52, 55 à 59, 72, 74, 76, 78, 79, 89 à 91, 97, 102, 108, 111, 112, 114, 116 à 118, 124, 130, 131, 133 à 135, 143, 145, 155, 158, 160, 165 à 167, 170, 175, 178, 183 à 186, 188, 189, 190, 193, 194, 200, 201, 203, 206, 207, 212, 213, 215, 216, 218, 223, 224, 226, 228, 230, 232 à 234, 239, 240, 242, 243, 245, 246, 250, 251, 254, 258, 263, 264, 270, 275, 276, 278 à 280, 282, 283, 285 à 287, 289, 292, 294, 295, 297 à 302, 305 à 312, 315 à 345, 349 à 355, 357, 358, 360, 362, 369, 371, 373, 375 à 377, 379 à 394, et 403.

L'invention a également pour objet un composé répondant à la formule générale (I) telle que définie plus haut, en particulier les composés de formule (I) choisis parmi les composés n° 1 à 6, 11 à 14, 16, 20, 22 à 25, 32 à 43, 47 à 52, 55 à 59, 72, 74, 76, 78, 79, 89 à 91, 97, 102, 108, 111, 112, 114, 116 à 118, 124, 130, 131, 133 à 135, 143, 145, 155, 158, 160, 165 à 167, 170, 175, 178, 183 à 186, 188, 189, 190, 193, 194, 200, 201, 203, 206, 207, 212, 213, 215, 216, 218, 223, 224, 226, 228, 230, 232 à 234, 239, 240, 242, 243, 245, 246, 250, 251, 254, 258, 263, 264, 270, 275, 276, 278 à 280, 282, 283, 285 à 287, 289, 292, 294, 295, 297 à 302, 305 à 312, 315 à 345, 349 à 355, 357, 358, 360, 362, 369, 371, 373, 375 à 377, 379 à 394, et 403, destiné à traiter au moins une maladie cardiovasculaire et/ou à prévenir l'apparition d'au moins une maladie cardiovasculaire, avantageusement telle que définie ci-dessus.

Des compositions pharmaceutiques comprenant, en tant que principe actif, au moins un composé conforme à l'invention sont décrites ici. Ces compositions pharmaceutiques contiennent une dose efficace d'au moins un composé de formule (I) conforme à l'invention, ainsi qu'au moins un excipient pharmaceutiquement acceptable.

Lesdits excipients sont choisis selon la forme pharmaceutique et le mode d'administration souhaité, parmi les excipients habituels qui sont connus de l'homme du métier.

Dans les compositions pharmaceutiques décrites ici pour l'administration orale, sublinguale, sous-cutanée, intramusculaire, intra-veineuse, topique, locale, intratrachéale, intranasale, transdermique ou rectale, le principe actif de formule (I) ci-dessus, ou son sel, solvat ou hydrate éventuel, peut être administré sous forme unitaire d'administration, en mélange avec des excipients pharmaceutiques classiques, aux animaux et aux êtres humains pour la prophylaxie ou le traitement des maladies définies ci-dessus.

Les formes unitaires d'administration appropriées comprennent les formes par voie orale telles que les comprimés, les gélules molles ou dures, les poudres, les granules et les solutions ou suspensions orales, les formes d'administration sublinguale, buccale, intratrachéale, intraoculaire, intranasale, par inhalation, les formes d'administration topique, transdermique, sous-cutanée, intramusculaire ou intraveineuse, les formes d'administration rectale et les implants. Pour l'application topique, on peut utiliser les composés selon l'invention dans des crèmes, gels, pommades ou lotions.

A titre d'exemple, une forme unitaire d'administration d'un composé conforme à l'invention sous forme de comprimé peut comprendre les composants suivants :

| | |
|---|---|
| Composé n°1 (2-{[3-(3,4-diméthoxybenzyl)-1-(1-formylpipéridin-4-yl)-2,4-dioxo-1,2,3,4-tétrahydroquinazolin-6-yl]oxy}propanenitrile) | 50,0 mg |
| Mannitol | 223,75 mg |
| Croscaramellose sodique | 6,0 mg |
| Amidon de maïs | 15,0 mg |
| Hydroxypropyl-méthylcellulose | 2,25 mg |
| Stéarate de magnésium | 3,0 mg |

Lesdites formes unitaires sont dosées pour permettre une administration journalière de 0,5 mg à 800 mg de principe actif par individu, plus particulièrement de 0,5 mg à 200 mg, selon la forme galénique.

Il peut y avoir des cas où des dosages plus élevés ou plus faibles sont appropriés ; de tels dosages ne sortent pas du cadre de l'invention. Selon la pratique habituelle, le dosage approprié à chaque patient est déterminé par le médecin selon le mode d'administration, le poids et la réponse dudit patient.

Une méthode de traitement et/ou de prévention des maladies cardiovasculaires indiquées ci-dessus comprend l'administration, à un patient, d'une dose efficace d'un composé conforme à l'invention, ou d'un de ses hydrates ou solvats.

## Revendications

1. Composé répondant à la formule générale (I) 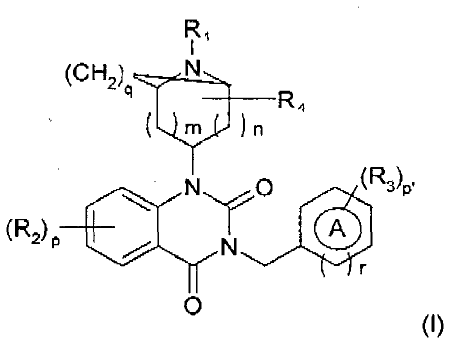 dans laquelle
- A représente un groupe aryle ou un groupe hétéroaryle;
- R₁ représente :
▪ un atome d'hydrogène,
▪ -C(O)R dans lequel R est un atome d'hydrogène, un groupe (C₁-C₆) alkoxy, un groupe aryle, un groupe (C₃-C₆) cycloalkyle, ou un groupe (C₁-C₆) alkyle, ledit alkyle étant éventuellement substitué par :
. un ou plusieurs groupe(s) hydroxy,
. un groupe benzyloxy,
. un groupe (C₁-C₆) alkoxy, éventuellement substitué par un aryle, ou
. un groupe (C₃-C₆) cycloalkyle,
▪ un groupe (C₁-C₆) alkyle éventuellement substitué;
- R₂ représente :
▪ un atome d'hydrogène,
▪ un atome d'halogène,
▪ un groupe cyano,
▪ un groupe nitro,
▪ un groupe (C₁-C₆) alkyle éventuellement substitué par un -NH₂, ou bien par un groupe -NHC(O)Rb, avec Rb tel que défini plus bas,
▪ un groupe -ORa dans lequel Ra représente :
. un atome d'hydrogène,
. un groupe (C₁-C₆) alkyle éventuellement substitué par un ou plusieurs atome(s) d'halogène, par un ou plusieurs groupe(s) hydroxy, par un groupe aryle et/ou par un ou plusieurs groupe(s) cyano,
. un groupe (C₂-C₆) alcynyle,
. un groupe aryle ;
- R₃ représente :
▪ un atome d'hydrogène,
▪ un atome d'halogène,
▪ un groupe hydroxy,
▪ un groupe cyano,
▪ un groupe -SCF₃,
▪ un groupe nitro,
▪ un groupe -S(O)₀₋₂alkyle, un groupe -S(O)₀₋₂-hétérocycloalkyle, un groupe - O- SO₂-aryle éventuellement substitué par un ou plusieurs atome(s) d'halogène ;
▪ un groupe -alkyl-amino-alkyle ou -cycloalkyl-amino-alkyle, chacun éventuellement substitué sur l'alkyle terminal,
▪ un groupe sulfonamide éventuellement substitué,
▪ un groupe aryle ou un groupe hétéroaryle, ledit groupe étant monocyclique ou polycyclique et étant de plus éventuellement substitué par un groupe (C₁-C₆) alkyle, par un ou plusieurs atome(s) d'halogène, ou par un groupe (C₁-C₆) alkoxy,
▪ un groupe hétérocycloalkyle éventuellement substitué par un groupe (C₁-C₆) alkyle,
▪ un groupe (C₁-C₆) alkyle éventuellement substitué par :
- un ou plusieurs atome(s) d'halogène,
- un groupe aryle pouvant être substitué par un ou plusieurs atome(s) d'halogène, ou par un ou plusieurs groupe(s) hydroxy,
- un groupe hétéroaryle,
- un ou plusieurs groupe(s) hydroxy pouvant être substitué(s) par un groupe aryle lui-même éventuellement substitué par un ou plusieurs atome(s) d'halogène, ou
- un groupe hétérocycloalkyle éventuellement sunstitué par un groupe CO(O)Ra, ou par un groupe (C₁-C₆) alkyle, avec Ra tel que défini précédemment,
▪ un groupe -C(O)NRbRc, avec Rb et Rc tels que définis plus bas,
▪ un groupe -C(O)ORc, ou un groupe -O-C(O)ORc, avec Rc tel que défini plus bas,
▪ un groupe (C₁-C₆) alkoxy, éventuellement substitué par
- un groupe amino-alkyle,
- un groupe amino-cycloalkyle,
- un groupe cycloalkyle,
- un groupe hétérocycloalkyle
- un groupe hétéroaryle monocyclique ou polycyclique,
- un ou plusieurs groupement(s) hydroxy,
- un ou plusieurs atome(s) d'halogène,
- un groupe (C₁-C₆) alkoxy,
- un groupe -C(O)ORc, avec Rc tel que défini plus bas,
- un groupe -C(O)NRbRc, avec Rb et Rc tels que définis plus bas, et/ou
- un groupe aryle, lui-même éventuellement substitué par un ou plusieurs atome(s) d'halogène, un groupe cyano, un groupe (C₁-C₆) alkoxy, un groupe -O- halogénoalkyle et/ou par un groupe halogénoalkyle,
▪ un groupe -O-cycloalkyle, un groupe -O-aryle, ou un groupe -O-hétérocycloalkyle, chacun éventuellement substitué par
- un groupe aryle, lui-même éventuellement substitué par un ou plusieurs atome(s) d'halogène, ou par un groupe (C₁-C₆) alkyle,
- un ou plusieurs atome(s) d'halogène, et/ou
- un groupe (C₁-C₆) alkyle, lui-même pouvant être substitué par un groupe aryle
▪ un groupe -NH-CO-NH-aryle, un groupe -NH-CO-NH-hétéroaryle, ou un groupe -NH-CO-NH-(C₁-C₆) alkyle, chacun étant éventuellement substitué par un ou plusieurs atome(s) d'halogène, par un groupe cyano, par un groupe nitro, par un ou plusieurs groupe(s) hydroxy, ou par un groupe (C₁-C₆) alkoxy,
▪ un groupe -N-(C₁-C₆) alkyle, le groupe (C₁-C₆) alkyle pouvant être substitué par
un ou plusieurs groupe(s) aryle éventuellement substitué(s) par un ou plusieurs atome(s) d'halogène et/ou par un groupe SO₂,
▪ un groupe -NH-CO-aryle, un groupe -NH-CO-hétéroaryle, chacun étant éventuellement substitué par un ou plusieurs atome(s) d'halogène ;
ou bien R₃ forme avec A un groupe hétéroaryle polycyclique éventuellement substitué par un groupe (C₁-C₆) alkoxy, un groupe (C₁-C₆) alkyle éventuellement substitué par un groupe aryle pouvant lui-même être substitué par un ou plusieurs atome(s) d'halogène ;
- R₄ représente un atome d'hydrogène un groupe (C₁-C₆) alkyle ;
- Rb représente ;
. un atome d'hydrogène,
. un groupe (C₁-C₆) alkyle éventuellement substitué par un ou plusieurs atome(s) d'halogène, par un ou plusieurs groupe(s) hydroxy, cyano, amino, hétérocycloalkyle, (C₁-C₆) alkoxy, ou par un groupe aryle éventuellement substitué par un ou plusieurs atome(s) d'halogène,
. un groupe (C₃-C₆) cycloalkyle,
- un groupe (C₂-C₆) alcynyle,
. un groupe (C₁-C₆) alkoxy,
. un groupe aryle éventuellement substitué par un ou plusieurs atome(s) d'halogène;
- Rc représente un atome d'hydrogène, ou un groupe (C₁-C₆) alkyle éventuellement substitué par un ou plusieurs atome(s) d'halagène ;
ou alors Rb et Rc forment ensemble avec l'atome d'azote auquel ils sont attachés un groupe hétéroaryle polycyclique, ou un groupe hétérocycloalkyle ;
- m et n représentent indépendamment l'un de l'autre la valeur 0, 1 ou 2, étant entendu que m+n ≤ 3 ;
- p et p' représentent indépendamment l'un de l'autre la valeur 1, 2 ou 3, étant entendu que lorsque p est supérieur ou égal à 2, alors les groupes R₂ sont sur des atomes de carbone distincts et peuvent être différents les uns des autres, et lorsque p' est supérieur ou égal à 2 alors les groupes R₃ sont sur des atomes de carbone distincts et peuvent être différents les uns des autres;
- q représente la valeur 0 ou 2, étant entendu que lorsque q = 0, alors le groupe hétérocyclique azoté rattaché à l'azote situé en position 1 du noyau 2,4-dioxo-1,2,3,4 tétrahydroquinazoline n'est plus ponté et est du type : 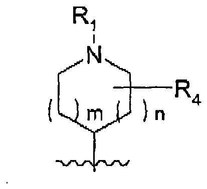
avec R₁, R₄, m et n tels que définis précédemment,
- r représente la valeur 0 ou 1 ;
à l'état de bases ou de sels d'addition à des acides ;
pour le traitement d'au moins une maladie cardiovasculaire et/ou pour prévenir l'apparition d'au moins une maladie cardiovasculaire.

2. Composé pour l'utilisation selon la revendication précédente, **caractérisé en ce que** la maladie cardiovasculaire est choisie parmi (i) les maladies coronaires, (ii) les maladies du muscle cardiaque, (iii) les maladies des valves cardiaques, (iv) les maladies du péricarde, (v) les maladies du rythme cardiaque et les maladies de la conduction cardiaque et (vi) les maladies des vaisseaux.

3. Composé pour l'utilisation selon l'une quelconque des revendications 1 ou 2, **caractérisé en ce que** la maladie cardiovasculaire est choisie parmi l'infarctus du myocarde, en particulier les dysfonctions cardiaques contractiles consécutives à un infarctus du myocarde, les maladies associées à des lésions de reperfusion du muscle cardiaque et/ou squelettique, l'hypertension pulmonaire, la fibrose hépatique, la resténose artérielle après angioplastie avec ou sans pose de stent, l'athérosclérose et ses complications, l'insuffisance cardiaque, les cardiopathies dilatées et les myocardites d'origine virale et/ou bactérienne.

4. Composé pour l'utilisation selon l'une quelconque des revendications précédentes, **caractérisée en ce que** A représente un groupe phényle ou un groupe pyridyle.

5. Composé pour l'utilisation selon l'une quelconque des revendications précédentes, **caractérisée en ce que** q = 0, m et n représente chacun 1.

6. Composé pour l'utilisation selon l'une quelconque des revendications précédentes, **caractérisée en ce que** R₂ représente un groupe (C₁-C₆) alkyle, en particulier un méthyle, substitué par un groupe -NH-C(O)-Rb, Rb étant tel que défini dans les revendications 1 ou 2.

7. Composé pour l'utilisation selon l'une quelconque des revendications 1 à 5, **caractérisée en ce que** R₂ représente un groupe -ORa, Ra étant tel que défini dans les revendications 1 ou 2.

8. Composé pour l'utilisation selon l'une quelconque des revendications 1 à 5, **caractérisée en ce que** R₂ est un atome d'halogène, ou un cyano, ou un hydrogène, ou un hydroxyle, ou un (C₁-C₆) alkyle éventuellement substitué par un -NH₂, ou bien par un groupe -NHC(O)Rb, Rb étant tel que défini dans les revendications 1 ou 2.

9. Composé pour l'utilisation selon l'une des revendications 1 à 8, **caractérisé en ce que** :
- A est tel que défini dans la revendication 4 ;
- q,m et n sont tels que définis dans la revendication 5 ;
- R₂ est tel que défini dans la revendication 6, 7 ou 8.

10. Composé pour l'utilisation selon l'une des revendications 1 à 3 **caractérisé en ce que** :
A est un phényle, R₁ est un groupe -C(O)R dans lequel R représente un atome d'hydrogène, q est égal à 0, n et m valent chacun 1, et R₂ représente -ORa, Ra étant tel que défini dans les revendications 1 ou 2.

11. Composé pour l'utilisation selon l'une des revendications 1 à 3 **caractérisé en ce que** :
A est un phényle, R₁ est un groupe -C(O)R dans lequel R représente un atome d'hydrogène, q est égal à 0, n et m valent chacun 1, p=1 et R₂ représente un méthyle substitué par un groupe -NH-CO-Rb, Rb étant tel que défini dans les revendications 1 ou 2.

12. Composé pour l'utilisation selon l'une des revendications 1 à 3 **caractérisé en ce que** :
A est un phényle, R₁ est un groupe -C(O)R dans lequel R représente un atome d'hydrogène, q est égal à 0, n et m valent chacun 1, p est égal à 2, l'un des R₂ est - ORa, Ra étant tel que défini dans les revendications 1 ou 2, et l'autre des R₂ est un atome d'halogène.

13. Composé pour l'utilisation selon l'une des revendications 1 à 3 **caractérisé en ce que** :
le groupe R₂ est en position 6 du noyau 2,4-dioxo-1,2,3,4 tétrahydroquinazoline, et **en ce qu'**il peut de plus y avoir un groupe R2, identique ou différent dudit groupe R2 précédemment mentionné, en position 7 du noyau 2,4-dioxo-1,2,3,4 tétrahydroquinazoline, ledit composé de formule (I) étant à l'état de base, d'hydrate, de solvat, d'isomères ou de leurs mélanges.

14. Composé pour l'utilisation selon l'une des revendications 1 à 3 choisi parmi:
n°1: 2-{[3-(3,4-diméthoxybenzyl)-1-(1-formylpipéridin-4-yl)-2,4-dioxo-1,2,3,4-tétrahydroquinazolin-6-yl]oxy}propanenitrile
n°2 : 1-(1-acétylpipéridin-4-yl)-3-(3,4-diméthoxybenzyl)-6-hydroxyquinazoline-2,4(1H,3H)-dione
n°3: {[1-(1-acétylpipéridin-4-yl)-3-(3,4-diméthoxybenzyl)-2,4-dioxo-1,2,3,4-tétrahydroquinazolin-6-yl]oxy}açétonitrile
n°4: 2-{[1-(1-acétylpipéridin-4-yl)-3-(3,4-diméthoxybenzyl)-2,4-dioxo-1,2,3,4-tétrahydroquinazolin-6-yl]oxy}propanenitrile
n°5: {[3-(3,4-diméthoxybenzyl)-1-(1-formylpipéridin-4-yl)-2,4-dioxo-1,2,3,4-tétrahydroquinazolin-6-yl]oxy}acétonitrile
n°6: {[3-(3,4-diméthoxybenzyl)-1-(1-formylpipéridin-4-yl)-2,4-dioxo-1,2,3,4-tétrahydroquinazolin-6-yl]oxy}acétonitrile
n°11: 4-[3-(3,4-diméthoxybenzyl)-6-[2-fluoro-1-(fluorométhyl)éthoxy]-2,4-dioxo-3,4-dihydroquinazolin-1(2H)-yl]pipéridine-1-carbaldéhyde
n°12: 1-(1-acétylpipéridin-4-yl)-3-(3,4-diméthoxybenzyl)-6-[2-fluoro-1-(fluorométhyl)éthoxy]quinazoline-2,4(1H,3H)-dione
n°13: 4-[3-(3,4-diméthoxybenzyl)-2,4-dioxo-6-(2,2,2-trifluoroéthoxy)-3,4-dihydroquinazolin-1(2H)-yl]pipéridine-1-carbaldéhyde
n°14: 1-(1-acétylpipéridin-4-yl)-6-(2,2-difluoroéthoxy)-3-(3,4-diméthoxybenzyl)quinazoline-2,4(1H,3H)-dione
n°16 : 4-[6-(2,2-difluoroéthoxy)-3-(3,4-diméthoxybenzyl)-2,4-dioxo-3,4-dihydroquinazolin-1(2H)-yl]pipéridine-1-carbaldéhyde
n°20: N-{[3-(3,4-diméthoxybenzyl)-1-(1-formylpipéridin-4-yl)-2,4-dioxo-1,2,3,4-tétrahydroquinazolin-6-yl]méthyl}acétamide
n°22: chlorhydrate de 1-(1-acétylpipéridin-4-yl)-6-(aminométhyl)-3-(3,4-diméthoxybenzyl)quinazoline-2,4(1H,3H)-dione
n°23: N-{[3-(3,4-diméthoxybenzyl)-(1-formylpipéridin-4-yl)-2,4-dioxo-1,2,3,4-tétrahydroquinazolin-6-yl]méthyl}formamide
n°24: N-{[1-(1-acétylpipéridin-4-yl)-3-(3,4-diméthoxybenzyl)-2,4-dioxo-1,2,3,4-tétrahydroquinazolin-6-yl]méthyl}formamide
n°25: N-{[1-(1-acéty!pîpéridin-4-y|)-3-(3,4-diméthoxybenzyl)-2,4-dioxa-1,2,3,4-tétrahydroquinazolin-6-yl]méthyl}acétamide
n°32: 4-[6-(2,2-difluoroéthoxy)-2,4-dioxo-3,4-dihydroquirazolin-1(2H)-yl]pipéridine-1-carbaldéhyde
n°33: 4-[3-(3,4-dichlorobenzyl)-6-(2,2-difluoroéthoxy)-2,4-dioxo-3,4-dihydroquinazolin-1 (2H)-yl]pipéridine-1-carbaldéhyde
n°34: 4-[3-(4-chlorobenzyl)-6-(2,2-difluoroéthoxy)-2,4-dioxo-3,4-dihydroquinazolin-1(2H)-yl]pipéridine-1-carbaldéhyde
n°35: 4-{[6-(2,2-difluoroéthoxy)-1-(1-formylpipéridin-4-yl)-2,4-dioxo-1,4-dihydroquinazolin-3(2H)-yl]méthyl}benzoate de méthyle
n°36: acide 4-{[6-(2,2-difluoroéthoxy)-1-(1-formylpipéridin-4-yl)-2,4-dioxo-1,4-dihydroquinazolin-3(2H)-yl]méthyl}benzoïque
n°37: 4-{[6-(2,2-difluoroéthoxy)-1-(1-formylpipéridin-4-yl)-2,4-dioxo-1,4-dihydroquinazolin-3(2H)-y1]méthyl}-N-(2-méthoxyéthyl)benzamide
n°38: 4-[3-(3,4-diméthoxybenzyl)-6-méthyl-2,4-dioxo-3,4-dihydroquinazolin-1(2H)-yl]pipéridine-1-carbaldéhyde
n°39: 4-[6-(2,2-difluoroéthoxy)-3-(3-hydroxy-4-méthoxybenzyl)-2,4-dioxo-3,4-dihydroquinazolin-1(2H)-yl]pipéridine-1-carbaldéhyde
n°40: 4-[6-(2,2-difluoroéthoxy)-3-[3-(2-hydroxyéthoxy)-4-méthoxybenzyl]-2,4-dioxo-3,4-dihydroquinazolin-1(2H)-yl]pipéridine-1-carbaldéhyde
n°41: 4-[6-(2,2-difluoroéthoxy)-3-(3-éthoxy-4-méthoxybenzyl)-2,4-dioxo-3,4-dihydroquinazolin-1(2H)-yl]pipéridine-1-carbaldéhyde
n°42: 4-[6-(2,2-difluoroéthoxy)-3-[4-méthoxy-3-(2-méthoxyéthoxy)benzyl]-2,4-dioxo-3,4-dihydroquinazolin-1(2H)-yl]pipéridine-1-carbaldéhyde
n°43: 4-[6-(2,2-difluoroéthoxy)-3-(3,4-diméthoxybenzyl)-2,4-dioxo-3,4-dihydroquinazolin-1(2H)-yl]azépane-1-carbaldéhyde
n°47: 4-[6-(2,2-difluoroéthoxy)-3-[3-(3-hydroxypropoxy)-4-méthoxybenzyl]-2,4-dioxo-3,4-dihydroquinazolin-1(2H)-yl]pipéridine-1-carbaldéhyde
n°48: 4-[5-chloro-3-(3,4-diméthoxybenzyl)-2,4-dioxo-3,4-dihydroquinazolin-1(2H)-yl]pipéridine-1-carbaldéhyde
n°49: 4-{3-[3-(cyclopentyloxy)-4-méthoxybenzyl]-6-(2,2-difluoroéthoxy)-2,4-dioxo-3,4-dihydroquinazolin-1(2H)-yl}pipéridine-1-carbaldéhyde
n°50: 2-(5-{[6-(2,2-difluoroéthoxy)-1-(1-formylpipéridin-4-yl)-2,4-dioxo-1,4-dihydroquinazolin-3(2H)-yl]méthyl}-2-méthoxyphénoxy)acétamide
n°51: 4-[6-(2,2-difluoroéthoxy)-3-(3,4-diméthoxybenzyl)-2,4-dioxo-3,4-dihydroquinazolin-1(2H)-yl]-3-méthylpipéridine-1-carbaldéhyde
n°52: 3-[6-(2,2-difluoroéthoxy)-3-(3,4-diméthoxybenzyl)-2,4-dioxo-3,4-dihydroquinazolin-1(2H)-yl]-8-azabicyclo[3.2.1]octane-8-carbaldéhyde
n° 55: 4-{6-[2-fluoro-1-(fluorométhyl)éthoxy]-2,4-dioxo-3,4-dihydroquinazolin-1(2H)-yl}pipéridine-1-carbaldéhyde
n°56: 4-{3-[4-(cyclopentyloxy)-3-méthoxybenzyl]-6-[2-fluoro-1-(fluorométhyl)éthoxy]-2,4-dioxo-3,4-dihydroquinazolin-1(2H)-yl}pipéridine-1-carbaldéhyde
n°57: 4-[3-(3-chlorobenzyl)-6-[2-fluoro-1-(fluorométhyl)éthoxy]-2,4-dioxo-3,4-dihydroquinazolin-1(2H)-yl]pipéridine-1-carbaldéhyde
n°58: 4-[3-(4-chlorobenzyl)-6-[2-fluoro-1-(fluorométhyl)éthoxy]-2,4-dioxo-3,4-dihydroquinazolin-1(2H)-yl]pipéridine-1-carbaldéhyde
n°59: 4-{3-[3-(cyclopentyloxy)-4-méthoxybenzyl]-6-[2-fluoro-1-(fluorométhyl)éthoxy]-2,4-dioxo-3,4-dihydroquinazolin-1(2H)-yl}pipéridine-1-carbaldéhyde
n°72 : 4-[3-(3,4-diméthoxybenzyl)-6-(2-hydroxyéthoxy)-2,4-dioxo-3,4-dihydroquinazolin-1(2H)-yl]pipéridine-1-carbaldéhyde
n°74 : 4-[3-(3,4-dichlorobenzyl)-6-[2-fluoro-1-(fluorométhyl)éthoxy]-2,4-dioxo-3,4-dihydroquinazolin-1(2H)-yl]pipéridine-1-carbaldéhyde
n°76 : 4-{3-[(6-chloropyridin-3-yl)méthyl]-6-[2-fluoro-1-(fluorométhyl)éthoxy]-2,4-dioxo-3,4-dihydroquinazalin-1(2H)-yl}pipéridine-1-carbaldéhyde
n°78 : 4-[3-(3-chloro-4-méthoxybenzyl)-6-[2-fluoro-1-(fluorométhyl)éthoxy]-2,4-dioxo-3,4-dihydroquinazolin-1(2H)-yl]pipéridine-1-carbaldéhyde
n°79 : 4-[3-(3,4-diméthoxybenzyl)-6-(2-fluoroéthoxy)-2,4-dioxo-3,4-dihydroquinazolin-1(2H)-yl]pipéridine-1-carbaldéhyde
n°89 : 2-[5-({6-[2-fluoro-1-(fluorométhyl)éthoxy]-1-(1-formylpipéridin-4-yl)-2,4-dioxo-1,4-dihydroquinazolin-3(2H)-yl}méthyl)-2-méthoxyphénoxy]acétamide
n°90 : 4-{6-[2-fluoro-1-(fluorométhyl)éthoxy]-3-(3-hydroxy-4-méthoxybenzyl)-2,4-dioxo-3,4-dihydroquinazolin-1(2H)-yl}pipéridine-1-carbaldéhyde
n°91: 4-[3-(3,4-diméthoxybenzyl)-6-éthoxy-2,4-dioxo-3,4-dihydroquinazolin-1(2H)-yl]pipéridine-1-carbaldéhyde
n°97: 4-[5,7-dichloro-3-(3,4-diméthoxybenzyl)-2,4-dioxo-3,4-dihydroquinazolin-1(2H)-yl]pipéridine-1-carbaldéhyde
n°102: 4-[7-chloro-3-(3,4-diméthoxybenzyl)-2,4-dioxo-3,4-dihydroquinazolin-1(2H)-yl]pipéridine-1-carbaldéhyde
n°108 :4-{6-[2-fluoro-1-(fluorométhyl)éthoxy]-3-(3-fluoro-4-méthoxybenzyl)-2,4-dioxo-3,4-dihydroquinazolin-1(2H)-yl}pipéridine-1-carbaldéhyde
n°111 : 4-[6-(difluorométhoxy)-3-(3,4-diméthoxybenzyl)-2,4-dioxo-3,4-dihydroquinazolin-1(2H)-yl]pipéridine-1-carbaldéhyde
n°112 : 4-[3-(3,4-diméthoxybenzyl)-6-(1-méthyléthoxy)-2,4-dioxo-3,4-dihydroquinazolin-1(2H)-yl]pipéridine-1-carbaldéhyde
n°114 : 4-{8-[2-fluoro-1-(fluorométhyl)éthoxy]-3-[4-méthoxy-3-(1-méthyléthoxy)benzyl]-2,4-dioxo-3,4-dihydroquinazolin-1(2H)-yl}pipéridine-1-carbaldéhyde
n°116 : 4-{6-[2-fluoro-1-(fluorométhyl)éthoxy]-3-(3-méthoxybenzyl)-2,4-dioxo-3,4-dihydroquinazolin-1(2H)-yl}pipéridine-1-carbaldéhyde
n°117 : 4-{3-[3,5-bis(trifluorométhyl)benzyl]-6-[2-fluoro-1-(fluorométhyl)éthoxy]-2,4-dioxo-3,4-dihydroquinazolin-1(2H)-yl}pipéridine-7-carbaldéhyde
n°118 : 4-[3-(3-éthoxybenzyl)-6-[2-fluoro-1-(fluorométhyl)éthoxyl-2,4-dioxo-3,4-dihydroquinazolin-1(2H)-yl]pipéridine-1-carbaldéhyde
n°124 :4-{3-[3-chloro-4-(2-méthoxyéthoxy)benzyl]-6-[2-fluoro-1-(fluorométhyl)éthoxy]-2,4-dioxo-3,4-dihydroquinazolin-1(2H)-yl}pipéridine-1-carbaldéhyde
n°130 : 4-[3-(3,4-diéthoxybenzyl)-6-[2-fluoro-1-(fluorométhyl)éthoxy]-2,4-dioxo-3,4-dihydroquinazolin-1(2H)-yl]pipéridine-1-carbaldéhyde
n°131 : 4-[3-(4-éthoxy-3-méthoxybenzyl)-6-[2-fluoro-1-(fluorométhyl)éthoxy]-2,4-dioxo-3,4-dihydroquinazolin-1(2H)-yl]pipéridine-1-carbaldéhyde
n°133 : 4-{6-[2-fluoro-1-(fluorométhyl)éthoxy]-3-(4-méthoxy-3-méthylbenzyl)-2,4-dioxo-3,4-dihydroquinazolin-1(2H)-yl}pipéridine-1-carbaldéhyde
n°134 : 4-{6-[2-fluoro-1-(fluorométhyl)éthoxy]-2,4-dioxo-3-[4-(trifluorométhyl)benzyl]-3,4-dihydroquinazolin-1(2H)-yl}pipéridine-1-carbaldéhyde
n°135 : 4-{6-[2-fluoro-1-(fluorométhyl)éthoxy]-2,4-dioxo-3-[4-(trifluorométhyl)benzyl]-3,4-dihydroquinazolin-1(2H)-yl}pipéridine-1-carbaldéhyde
n°143 : 4-{3-[4-(benzyloxy)-3-méthoxybenzyl]-6-[2-fluoro-1-(fluorométhyl)éthoxy]-2,4-dioxo-3,4-dihydroquinazolin-1(2H)-yl}pipéridine-1-carbaldéhyde
n°145 : 4-{6-[2-fluoro-1-{fluorométhyl)éthoxy]-3-(3-méthoxy-4-nitrobenzyl)-2,4-dioxo-3,4-dihydroquinazolin-1(2H)-yl}pipéridine-1-carbaldéhyde
n°155 :4-[3-(4-éthoxybenzyl)-6-[2-fluoro-1-(fluorométhyl)éthoxy]-2,4-dioxo-3,4-dihydroquinazolin-1(2H)-yl]pipéridine-1-carbaldéhyde
n°158 : 4-{6-[2-fluoro-1-(fluorométhyl)éthoxy]-3-[4-(morpholin-4-ylméthyl)benzyl]-2,4-dioxo-3,4-dihydroquinazolin-1(2H)-yl}pipéridine-1-carbaldéhyde
n°160 : 4-{6-[2-fluoro-1-(fluorométhyl)éthoxy]-3-(4-morpholin-4-ylbenzyl)-2,4-dioxo-3,4-dihydroquinazolin-1(2H)-yl}pipéridine-1-carbaldéhyde
n°165 : 4-[3-(biphényl-4-ylméthyl)-6-[2-fluoro-1-(fluorométhyl)éthoxy]-2,4-dioxo-3,4-dihydroquinazolin-1(2H)-yl]pipéridine-1-carbaldéhyde
n°166 : 4-{6-[2-fluoro-1-(fluorométhyl)éthoxy]-3-[4-(méthylsulfanyl)benzyl]-2,4-dioxo-3,4-dihydroquinazolin-1(2H)-yl}pipéridine-1-carbaldéhyde
n°167 : 4-{6-[2-fluoro-1-{fluorométhyl)éthoxy]-2,4-dioxo-3-(4-pyridin-3-ylbenzyl)-3,4-dihydroquinazolin-1(2H)-yl}pipéridine-1-carbaldéhyde
n°170 : 4-{6-[2-fluoro-1-(fluorométhyl)éthoxy]-3-(3-méthoxy-4-méthylbenzyl)-2,4-dioxo-3,4-dihydroquinazolin-1(2H)-yl}pipéridine-1-carbaldéhyde
n°175 : 2-[2-(cyclopentyloxy)-5-({6-[2-fluoro-1-(fluorométhyl)éthoxy]-1-(1-formylpipéridin-4-yl)-2,4-dioxo-1,4-dihydroquinazolin-3(2H)-yl}méthyl)phénoxy]acétamide
n°178 : 4-{6-[2-fluoro-1-(fluorométhyl)éthoxy]-3-(3-méthoxy-4-propoxybenzyl)-2,4-dioxo-3,4-dihydroquinazolin-1(2H)-yl}pipéridine-1-carbaldéhyde
n°183: 2-[2-(cyclopentyloxy)-5-({6-[2-fluoro-1-(fluorométhyl)éthoxy]-1-(1-formylpipéridin-4-yl)-2,4-dioxo-1,4-dihydroquinazolin-3(2H)-yl}méthyl)phénoxyl-N-méthylacétamide
n°184 : 2-[2-(cyclopentyloxy)-5-({6-[2-fluoro-1-(fluorométhyl)éthoxy]-1-{1-formylpipéridin-4-yl)-2,4-dioxo-1,4-dihydroquinazolin-3(2H)-yl}méthyl)phén6xy]-N,N-diméthylacétamide
n°185 : 2-[2-(cyclopentyloxy)-5-({6-[2-fluoro-1-(fluorométhyl)éthoxy]-1-(1-formylpipéridin-4-yl)-2,4-dioxo-1,4-dihydroquinazolin-3(2H)-yl}méthyl)phénoxy]-N-méthoxy-N-méthylacétamide
n°186 ; 4-{3-[4-(cyclopentyloxy)-3-éthoxybenzyl]-6-[2-fluoro-1-(fluorométhyl)éthoxy]-2,4-dioxo-3.4-dihydroquinazolin-1(2H)-yl}pipéridine-1-carbaldéhyde
n°188 : 4-{3-[4-(cyclopentyloxy)-3-(1-méthyléthoxy)benzyl]-6-[2-fluoro-1-(fluorométhyl)éthoxy]-2,4-dioxo-3,4-dihydroquinazolin-1(2H)-yl}pipéiïdine-1-carbaldéhyde
n°189 : 4-{3-[4-(cyclopentyloxy)-3-propoxybenzyl]-6-[2-fluoro-1-(fluorométhyl)éthoxy]-2,4-dioxo-3,4-dihydroquinazolin-1(2H)-yl}pipéridine-1-carbaldéhyde
n°190 : 4-{3-[4-(cyclopentyloxy)-3-hydroxybenzyl]-6-[2-fluoro-1-(fluorométhyl)éthoxy]-2,4-dloxo-3,4-dihydroquinazolin-1(2H)-yl}pipéridine-1-carbaldéhyde
n°193 : 4-{3-[4-(difluorométhoxy)-3-méthoxybenzyl]-6-[2-fluoro-1-(fluorométhyl)éthoxy]-2,4-dioxo-3,4-dihydroquinazolin-1(2H)-yl}pipéridine-1-carbaldéhyde
n°194 : 4-{3-[4-(difluorométhoxy)-3-éthoxybenzyl]-6-[2-fluoro-1-(fluorométhyl)éthoxy]-2,4-dioxo-3,4-dihydroquinazolin-1(2H)-yl}pipéridine-1-carbaldéhyde
n°200 : 4-{6-[2-fluoro-1-(fluorométhyl)éthoxy]-2,4-dioxo-3-(4-thiophén-3-ylbenzyl)-3,4-dihydroquinazolin-1(2H)-yl}pipéridine-1-carbaldéhyde
n°201 :4-{6-[2-fluoro-1-(fluorométhyl)éthoxy]-2,4-dioxo-3-(4-pyridin-4-ylbenzyl)-3,4-dihydroquinazolin-1(2H)-yl}pipéridine-1-carbaldéhyde
n°203 : 4-{6-[2-Fluoro-1-(fluorométhyl)éthoxy]-3-[(1-méthyl-1H-indol-6-yl)méthyl]-2,4-dioxo-3,4-dihydroquinazolin-1(2H)-yl}pipéridine-1-carbaldéhyde
n°206 : 4-{3-[4-(cyclopropylméthoxy)-3-méthoxybenzyl]-6-[2-fluoro-1-(fluorométhyl)éthoxy]-2,4-dioxo-3,4-dihydroquinazolin-1(2H)-yl}pipéridine-1-carbaldéhyde
n°207 :2-[4-({6-[2-fluoro-(fluorométhyl)éthoxy]-1-(1-formylpipéridin-4-yl)-2,4-dioxo-1,4-dihydroquinazolin-3(2H)-yl}méthyl)-2-méthoxyphénoxy]-N-méthylacétamide
n°212 ; 4-{6-[2-fluoro-1-(fluorométhyl)éthoxy]-2,4-dioxo-3-[4-(1H-pyrazol-1-yl)benzyl]-3,4-dihydroquinazolin-1(2H)-yl}pipéridine-1-carbaldéhyde
n°213 :4-{6-[2-fluoro-1-(fluorométhyl)éthoxy]-2,4-dioxo-3-(4-pyridin-2-ylbenzyl)-3,4-dihydroquinazolin-1(2H)-yl}pipéridine-1-carbaldéhyde
n°215 : 4-{6-[2-fluoro-1-(fluorométhyl)éthoxy]-2,4-dioxo-3-(4-thiophén-2-ylbenzyl)-3,4-dihydroquinazolin-1(2H)-yl}pipéridine-1-carbaldéhyde
n°216 : 4-{6-[2-fluoro-1-(fluorométhyl)éthoxy]-2,4-dioxo-3-(quinoléin-7-ylméthyl)-3,4-dihydroquinazolin-1(2H)-yl}pipéridine-1-carbaldéhyde
n°218 :4-{6-[2-fluoro-1-(fluorométhyl)éthoxy]-3-[(6-méthoxynaphtalén-2-yl)méthyl]-2,4-dioxo-3,4-dihydroquinazolin-1(2H)-yl}pipéridine-1-carbaldéhyde
n° 223 : 4-{3-[4-(1H-benzimidazol-1-yl)benzyl]-6-[2-fluoro-1-(fluorométhyl)éthoxy]-2,4-dioxo-3,4-dihydroquinazolin-1(2H)-yl}pipéridine-1-carbaldéhyde
n°224 : 4-{6-[2-fluoro-1-(fluorométhyl)éthoxy]-3-[3-méthoxy-4-(2-méthylpropoxy)benzyl]-2,4-dioxo-3,4-dihydroquinazolin-1(2H)-yl}pipéridine-1-carbaldéhyde
n°226 : 4-{6-[2-fluoro-1-(fluorométhyl)éthoxy]-3-[3-méthoxy-4-(tétrahydrofuran-3-yloxy)benzyl]-2,4-dioxo-3,4-dihydroquinazolin-1(2H)-yl}pipéridine-1-carbaldéhyde
n°228 4-[3-{4-[(1-benzylpyrrolidin-3-yl)oxy]-3-méthoxybenzyl}-6-[2-fluoro-1-(fluorométhyl)éthoxy]-2,4-dioxo-3,4-dihydroquinazolin-1(2H)-yl]pipéridine-1-carbaldéhyde
n°230 :4-[3-(1-benzothiophén-5-ylméthyl)-6-[2-fluoro-1-(fluorométhyl)éthoxy]-2,4-dioxo-3,4-dihydroquinazolin-1(2H)-yl]pipéridine-1-carbaldéhyde
n°232 : 4-{6-[2-fluoro-1-(fluorométhyl)éthoxy]-3-[3-méthoxy-4-(1-méthyléthoxy)benzyl]-2,4-dioxo-3,4-dihydroquinazolin-1(2H)-yl}pipéridine-1-carbaldéhyde
n°233 :4-[3-(3,4-diméthoxybenzyl)-7-fluoro-6-[2-fluoro-1-(fluorométhyl)éthoxy]-2,4-dioxo-3,4-dihydroquinazolin-1(2H)-yl]pipéridine-1-carbaldéhyde
n°234 : 4-[3-{4-[(1-acétylpyrrolidin-3-yl)oxy]-3-méthoxybenzyl}-6-[2-fluoro-7-(fluorométhyl)éthoxy]-2,4-dioxo-3,4-dihydroquinazolin-1(2H)-yl]pipéridine-1-carbaldéhyde
n°239 : 4-[3-{4-[(4-fluorobenzyl)oxy]-3-méthoxybenzyl}-6-[2-fluoro-1-(fluorométhyl)éthoxy]-2,4-dioxo-3,4-dihydroquinazolin-1(2H)-yl]pipéridine-1-carbaldéhyde
n°240 : 4-[3-{4-[(4-chlorobenzyl)oxy]-3-méthoxybenzyl}-6-[2-fluoro-1-(fluorométhyl)éthoxy]-2,4-dioxo-3,4-dihydroquinazolin-1(2H)-yl]pipéridine-1-carbaldéhyde
n°242 : 4-[3-{4-[(3-chlorobenzyl)oxy]-3-méthoxybenzyl}-6-[2-fluoro-1-(fluorométhyl)éthoxy]-2,4-dioxo-3,4-dihydroquinazolin-1(2H)-yl]pipéridine-1-carbaldéhyde
n°243:4-{6-[2-fluoro-1-(fluorométhyl)éthoxy]-2,4-dioxo-3-(3-thiophén-3-ylbenzyl)-3,4-dihydroquinazolin-1(2H)-yl}pipéridine-1-carbaldéhyde
n°245 : 4-[3-(4-éthoxy-3-méthoxybenzyl)-6-(2-hydroxyéthoxy)-2,4-dioxo-3,4-dihydroquinazolin-1(2H)-yl]pipéridine-1-carbaldéhyde
n°246 : 4-[3-{4-[2-(2,3-dihydro-1H-indol-1-yl)-2-oxoéthoxy]-3-méthoxybenzyl}-6-[2-fluoro-1-(fluorométhyl)éthoxy]-2,4-dioxo-3,4-dihydroquinazolin-1(2H)-yl]pipéridine-1-carbaldéhyde
n°250 : 4-[3-{4-[{3,4-dichforobenzyl)oxy]-3-méthoxybenzyl}-6-[2-fluoro-1-(fluorométhyl)éthoxy]-2,4-dioxo-3,4-dihydroquinazolin-1(2H)-yl]pipéridine-1-carbaldéhyde
n°251 : 4-{6-[2-fluoro-1-(fluorométhyl)éthoxy]-3-[3-méthoxy-4-(2-oxo-2-pipéridin-1-yléthoxy)benzyl]-2,4-dioxo-3,4-dihydroquiriazolin-1(2H)-yl}pipéridine-1-carbaldéhyde
n°254 : 4-{3-[3-éthoxy-4-(thiophén-2-ylméthoxy)benzyl]-6-[2-fluoro-1-(fluorométhyl)éthoxy]-2,4-dioxo-3,4-dihydroquinazolin-1(2H)-yl}pipéridine-1-carbaldéhyde
n°258 4-[3-(3,4-diméthoxybenzyl)-6-[2-fluoro-1-(hydroxyméthyl)éthoxy]-2,4-dioxo-3,4-dihydroquinazolin-1(2H)-yl]pipéridine-1-carbaldéhyde
n°263: acide (2R)-2-[2-(cyclopentyloxy)-5-({6-[2-fluoro-1-(fluorométhyl)éthoxy]-1-(1-formylpipéridin-4-yl)-2,4-dioxo-1,4-dihydroquinazolin-3(2H)-yl}méthyl)phénoxy]propanoïque
n°264: 4-{6-[2-fluoro-1-(fluorométhyl)éthoxy]-3-[(1-méthyl-3-thiophén-2-yl-1H-pyrazol-5-yl)méthyl]-2,4-dioxo-3,4-dihydroquinazolin-1(2H)-yl}pipéridine-1-carbaldéhyde
n°270: 4-{6-[2-fluoro-1-(fluorométhyl)éthoxy]-3-[4-(5-méthyl-1,2,4-oxadiazol-3-yl)benzyl]-2,4-dioxo-3,4-dihydroquinazolin-1(2H)-yl}pipéridine-1-carbaldéhyde
n°275: 4-{6-[2-fluoro-1-(fluorométhyl)éthoxy]-2,4-dioxo-3-(4-pyrimidin-5-ylbenzyl)-3,4-dihydroquinazolin-1(2H)-yl}pipéridine-1-carbaldéhyde
n°276- 4-{6-[2-fluoro-1-(fluorométhyl)éthoxy]-3-[(1-méthyl-3-phényl-1H-pyrazol-5-yl)méthyl]-2,4-dioxo-3,4-dihydroquinazolin-1(2H)-yl}pipéridine-1-carbaldéhyde
n278: 4-{6-{2-fluoro-1-(fluorométhyl)éthoxy]-2,4-dioxo-3-{[6-(1H-pyrazol-1-yl)pyridin-3-yl]méthyl}-3,4-dihydroquinazolin-1(2H)-yl}pipéridine-1-carbaldéhyde
n°279: 4-{6-[2-fluoro-1-(fluorométhyl)éthoxy]-2,4-dioxo-3-[(2-thiophén-2-ylpyrimidin-5-yl)méthyl]-3,4-dihydroquinazolin-1(2H)-yl}pipéridine-1-carbaldéhyde
n°280: 4-{6-[2-fluoro-1-(fluorométhyl)éthoxy]-3-[4-(1-méthyl-1H-pyrazol-3-yl)benzyl]-2,4-dioxo-3,4-dihydroquinazolin-1(2H)-yl}pipéridine-1-carbaldéhyde
n°282: 4-{6-[2-fluoro-1-(fluorométhyl)éthoxy]-3-[4-(3-méthyl-1,2,4-oxadiazol-5-yl)benzyl]-2,4-dioxo-3,4-dihydroquinazolin-1(2H)-yl}pipéridine-1-carbaldéhyde
n°283: acide [2-(cyclopentyloxy)-5-({6-[2-fluoro-1-(fluorométhyl)éthoxy]-1-(1-formylpipéridin-4-yl)-2,4-dioxo-1,4-dihydroquinazolin-3(2H)-yl}méthyl)phénoxy]acétique
n°285: 4-{6-[2-fluoro-1-(fluorométhyl)éthoxy]-2,4-dioxo-3-(thiéno[2,3-b]pyridin-2-ylméthyl)-3,4-dihydroquinazolin-1(2H)-yl}pipéridine-1-carbaldéhyde
n°286: 4-{6-[2-fluoro-1-(fluorométhyl)éthoxy]-2,4-dioxo-3-[(6-phénylpyridin-3-yl)méthyl]-3,4-dihydroquinazolin-1(2H)-yl}pipéridine-1-carbaldéhyde
n°287: 4-{6-[2-fluoro-1-(fluorométhyl)éthoxy]-3-[(6-morpholin-4-ylpyridin-3-yl)méthyl]-2,4-dioxo-3,4-dihydroquinazolin-1(2H)-yl}pipéridine-1-carbaldéhyde
n°289: 4-{6-[2-fluoro-1-(fluorométhyl)éthoxy]-2,4-dioxo-3-[(6-thiophén-2-ylpyridin-3-yl)méthyl]-3,4-dihydroquinazolin-1(2H)-yl}pipéridine-1-carbaldéhyde
n°292: 4-{6-[2-fluoro-1-(fluorométhyl)éthoxy]-3-[(1-méthyl-5-phényl-1H-pyrazol-3-yl)méthyl]-2,4-dioxo-3,4-dihydroquinazolin-1(2H)-yl}pipéridine-1-carbaldéhyde
n°294: 4-({6-[2-fluoro-1-(fluorométhyl)éthoxy]-1-(1-formylpipéridin-4-yl)-2,4-dioxo-1,4-dihydroquinazolin-3(2H)-yl}méthyl)biphényl-2-carbonitrile
n°295: (2R)-2-[2-(cyclopentyloxy)-5-({6-[2-fluoro-1-(fluorométhyl)éthoxy]-1-(1-formylpipéridin-4-yl)-2,4-dioxo-1,4-dihydroquinazolin-3(2H)-yl}méthyl)phénoxy]-N-méthylpropanamide
n°297 : 4-{7-fluoro-6-[2-fluoro-1-(fluorométhyl)éthoxy]-2,4-dioxo-3-(4-thiophén-2-ylbenzyl)-3,4-dihydroquinazolin-1(2H)-yl}pipéridine-1-carbaldéhyde
n°298 : 4-{6-[2-fluoro-1-(fluorométhyl)éthoxy]-3-[3-méthoxy-4-(morpholin-4-ylméthyl)benzyl]-2,4-dioxo-3,4-dihydroquinazolin-1(2H)-yl}pipéridine-1-carbaldéhyde
n°299: 4-{6-[2-fluoro-1-(fluorométhyl)éthoxy]-3-[3-méthoxy-4-(pipéridin-1-ylméthyl)benzyl]-2,4-dioxo-3,4-dihydroquinazolin-1(2H)-yl}pipéridine-1-carbaldéhyde
n°300: 4-[3-{4-[(3,4-dichlorobenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde
n°301: 2-[2-(cyclopentyloxy)-5-({6-[2-fluoro-1-(fluorométhyl)éthoxy]-1-(1-formylpipéridin-4-yl)-2,4-dioxo-1,4-dihydroquinazolin-3(2H)-yl}méthyl)phénoxy]-N-éthylacétamide
n°302: acide (2S)-2-[2-(cyclopentyloxy)-5-({6-[2-fluoro-1-(fluorométhyl)éthoxy]-1-(1-formylpipéridin-4-yl)-2,4-dioxo-1,4-dihydroquinazolin-3(2H)-yl}méthyl)phénoxy]propanoïque
n°305: 4-{6-[2-fluoro-1-(fluorométhyl)éthoxy]-3-(3-méthoxy-4-{[(3R)-2-oxo-1-phénylpyrrolidin-3-yl]oxy}benzyl)-2,4-dioxo-3,4-dihydroquinazolin-1(2H)-yl}pipéridine-1-carbaldéhyde
n°306: 4-{3-[4-(cyclobutylméthoxy)-3-méthoxybenzyl]-6-[2-fluoro-1-(fluorométhyl)éthoxy]-2,4-dioxo-3,4-dihydroquinazolin-1(2H)-yl}pipéridine-1-carbaldéhyde
n°307: 4-{3-[4-(benzyloxy)-3-méthoxybenzyl]-7-fluoro-6-[2-fluoro-1-(fluorométhyl)éthoxy]-2,4-dioxo-3,4-dihydroquinazolin-1(2H)-yl}pipéridine-1-carbaldéhyde
n°308: 4-{7-fluoro-6-[2-fluoro-1-(fluorométhyl)éthoxy]-3-(4-hydroxy-3-méthoxybenzyl)-2,4-dioxo-3,4-dihydroquinazolin-1(2H)-yl}pipéridine-1-carbaldéhyde
n°309: 4-{3-[4-(cyclopropylméthoxy)-3-méthoxybenzyl]-7-fluoro-6-[2-fluoro-1-(fluorométhyl)éthoxy]-2,4-dioxo-3,4-dihydroquinazolin-1(2H)-yl}pipéridine-1-carbaldéhyde
n°310: 4-{7-fluoro-6-[2-fluoro-1-(fluorométhyl)éthoxy]-3-[3-méthoxy-4-(2-méthylpropoxy)benzyl]-2,4-dioxo-3,4-dihydroquinazolin-1(2H)-yl}pipéridine-1-carbaldéhyde
n°311: 4-{7-fluoro-6-[2-fluoro-1-(fluorométhyl)éthoxy]-3-[3-méthoxy-4-(1-méthyléthoxy)benzyl]-2,4-dioxo-3,4-dihydroquinazolin-1(2H)-yl}pipéridine-1-carbaldéhyde
n°312: 4-[3-(4-éthoxy-3-méthoxybenzyl)-7-fluoro-6-[2-fluoro-1-(fluorométhyl)éthoxy]-2,4-dioxo-3,4-dihydroquinazolin-1(2H)-yl]pipéridine-1-carbaldéhyde
n°315: 4-{7-fluoro-6-[2-fluoro-1-(fluorométhyl)éthoxy]-3-{[6-(3-méthoxyphényl)pyridin-3-yl]méthyl}-2,4-dioxo-3.4-dihydroquinazolin-1(2H)-yl}pipéridine-1-carbaldéhyde
n°316: 4-{7-fluoro-6-[2-fluoro-1-(fluorométhyl)éthoxy]-3-{[6-(2-fluorophényl)pyridin-3-yl]méthyl}-2,4-dioxo-3,4-dihydroquinazolin-1(2H)-yl}pipéridine-1-carbaldéhyde
n°317: 4-{7-fluoro-6-[2-fluoro-1-(fluorométhyl)éthoxy]-3-{[6-(4-fluorophényl)pyridin-3-yl]méthyl}-2,4-dioxo-3,4-dihydroquinazolin-1(2H)-yl}pipéridine-1-carbaldéhyde
n°318: 4-{7-fluoro-6-[2-fluoro-1-(fluorométhyl)éthoxy]-3-{[6-(4-méthoxyphényl)pyridin-3-yl]méthyl}-2.4-dioxo-3,4-dihydroquinazolin-1(2H)-yl}pipéridine-1-carbaldéhyde
n°319: 4-{7-fluoro-6-[2-fluoro-1-(fluorométhyl)éthoxy]-2,4-dioxo-3-[(6-thiophén-2-ylpyridin-3-yl)méthyl]-3,4-dihydroquinazolin-1(2H)-yl}pipéridine-1-carbaldéhyde
n°320: 4-{3-[3-éthoxy-4-(thiophén-2-ylméthoxy)benzyl]-7-fluoro-6-[2-fluoro-1-(fluorométhyl)éthoxy]-2,4-dioxo-3,4-dihydroquinazolin-1(2H)-yl}pipéridine-1-carbaldéhyde
n°321: 4-{7-fluoro-6-[2-fluoro-1-(fluorométhyl)éthoxy]-3-[4-(1-méthyl-1H-pyrazol-3-yl)benzyl]-2,4-dioxo-3,4-dihydroquinazolin-1(2H)-yl}pipéridine-1-carbaldéhyde
n°322; 4-{7-fluoro-6-[2-fluoro-1-(fluorométhyl)éthoxy]-2,4-dioxo-3-(4-pyrimidin-5-ylbenzyl)-3,4-dihydroquinazolin-1(2H)-yl}pipéridine-1-carbaldéhyde
n°323: 4-{7-fluoro-6-[2-fluoro-1-(fluorométhyl)éthoxy]-3-[(1-méthyl-3-thiophén-2-yl-1H-pyrazol-5-yl)méthyl]-2,4-dioxo-3,4-dihydroquinazolin-1(2H)-yl}pipéridin-1-carbaldéhyde
n°324: 4-{7-fluoro-6-[2-fluoro-1-(fluorométhyl)éthoxy]-3-[3-méthoxy-4-(2-oxo-2-pipéridin-1-yléthoxy)benzyl]-2,4-dioxo-3,4-dihydroquinazolin-1(2H)-yl}pipéridine-1-carbaldéhyde
n°325: 4-[3-{4-[2-(2,3-dihydro-1H-indol-1-yl)-2-oxoéthoxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde
n°326: 4-{7-fluoro-6-[2-fluoro-1-(fluorométhyl)éthoxy]-3-[4-(5-méthyl-1,2,4-oxadiazol-3-yl)benzyl]-2,4-dioxo-3,4-dihydroquinazolin-1(2H)-yl}pipéridine-1-carbaldéhyde
n°327: 4-[3-{4-[(3-chlorobenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde
n°328: 4-[3-{[6-(3,5-dichlorophényl)pyridin-3-yl]méthyl}-6-[2-fluoro-1-(fluorométhyl)éthoxy]-2,4-dioxo-3,4-dihydroquinazolin-1(2H)-yl]pipéridine-1-carbaldéhyde
n°329: 4-({7-fluoro-6-[2-fluoro-1-(fluorométhyl)éthoxy]-1-(1-formylpipéridin-4-yl)-2,4-dioxo-1,4-dihydroquinazolin-3(2H)-yl}méthyl)biphényl-2-carbonitrile
n°330: 4-{7-fluoro-6-[2-fluoro-1-(fluorométhyl)éthoxy]-2,4-dioxo-3-[4-(1H-pyrazol-1-yl)benzyl]-3,4-dihydroquinazolin-1(2H)-yl}pipéridine-1-carbaldéhyde
n°331: 4-{7-fluoro-6-[2-fluoro-1-(fluorométhy)éthoxy]-3-{[6-(3-fluorophényl)pyridin-3-yl]méthyl}-2,4-dioxo-3,4-dihydroquinazolin-1(2H)-yl}pipéridine-1-carbaldéhyde
n°332: 3-[5-({7-fluoro-6-[2-fluoro-1-(fluorométhyl)éthoxy]-1-(1-formylpipéridin-4-yl)-2,4-dioxo-1,4-dihydroquinazolin-3(2H)-yl}méthyl)pyridin-2-yl]benzonitrile
n°333: 4-[3-(3,4-diéthoxybenzyl)-7-fluoro-6-[2-fluoro-1-(fluorométhyl)éthoxyl-2,4-dioxo-3,4-dihydroquinazolin-1(2H)-yl]pipéridine-1-carbaldéhyde
n°334: 4-[3-{4-[(4-chlorobenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde
n°335: 4-{7-fluoro-6-[2-fluoro-1-(fluorométhyl)éthoxy]-3-[4-(morpholin-4-ylméthyl)benzyl]-2,4-dioxo-3,4-dihydroquinazolin-1(2H)-yl}pipéridine-1-carbaldéhyde
n°336: 4-{7-fluoro-6-[2-fluoro-1-(fluorométhyl)éthoxy]-2,4-dioxo-3-{[6-(1H-pyrazol-1-yl)pyridin-3-yl]méthyl}-3,4-dihydroquinazolin-1(2H)-yl}pipéridine-1-carbaldéhyde
n°337: 4-{7-fluoro-6-[2-fluoro-1-(fluorométhyl)éthoxy]-3-(4-morpholin-4-ylbenzyl)-2,4-dioxo-3,4-dihydroquinazolin-1(2H)-yl}pipéridine-1-carbaldéhyde
n°338: 4-{7-fluoro-6-[2-fluoro-1-(fluorométhyl)éthoxy]-3-(3-méthoxy-4-propoxybenzyl)-2,4-dioxo-3,4-dihydroquinazolin-1(2H)-yl}pipéridine-1-carbaldéhyde
n°339: 4-{3-[4-(1H-benzimidazol-1-yl)benzyl]-7-fluoro-6-[2-fluoro-1-(fluorométhyl)éthoxy]-2,4-dioxo-3,4-dihydroquinazolin-1(2H)-yl}pipéridine-1-carbaldéhyde
n°340: 5-({7-fluoro-5-[2-fluoro-1-(fluorométhyl)éthoxy]-1-(1-formylpipéridin-4-yl)-2,4-dioxo-1,4-dihydroquinazolin-3(2H)-yl}méthyl)-2-méthoxybenzonitrile
n°341: 3-(3,4-diméthoxybenzyl)-6-[2-fluoro-1-(fluorométhyl)éthoxy]-1-(1-formylpipéridin-4-yl)-2,4-dioxo-1,2,3,4-tétrahydroquinazoline-7-carbonitrile
n°342: 4-[3-(4-bromobenzyl)-7-fluoro-6-[2-fluoro-1-(fluorométhyl)éthoxy]-2,4-dioxo-3,4-dihydroquinazolin-1(2H)-yl]pipéridine-1-carbaldéhyde
n°343: 4-[3-{4-[(3,4-dichlorobenzyl)oxyl-3-(2-méthoxyéthoxy)benzyl}-7-fluoro-6-[2-fluoro-1-(fluorométhyl)éthoxy]-2,4-dioxo-3,4-dihydroquinazolin-1(2H)-yl]pipéridine-1-carbaldéhyde
n°344: 4-{3-[4-(benzyloxy)benzyl]-7-fluoro-6-[2-fluoro-1-(fluorométhyl)éthoxy]-2,4-dioxo-3,4-dihydroquinazolin-1(2H)-yl}pipéridine-1-carbaldéhyde
n°345: 4-[3-{4-[(3,4-dichlorobenzyl)oxy]-3-éthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde
n°349 : 4-[3-{4-[(3,4-dichlorobenzyl)oxy]-3-(2-fluoroéthoxy)benzyl}-7-fluoro-6-[2-fluoro-1-(fluorométhyl)éthoxy]-2,4-dioxo-3,4-dihydroquinazolin-1(2H)-yl]pipéridine-1-carbaldéhyde
n°350 : 4-[3-{4-[(2-chloro-4-fluorobenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde
n°351 : 4-[3-{4-[(2,4-dichlorobenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde
n°352 : 4-[3-{4-[(2-chloro-6-fluorobenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde
n°353 : 4-[3-{4-[(2,6-dichlorobenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde
n°354 : 4-[3-{4-[(2-chlorobenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde
n°355 : 4-[7-fluoro-3-{4-[(2-fluorobenzyl)oxy]-3-méthoxybenzyl}-6-[2-fluoro-1-(fluorométhyl)éthoxy]-2,4-dioxo-3,4-dihydroquinazolin-1(2H)-yl]pipéridine-1-carbaldéhyde
n°357 : 2-[(3,4-dichlorobenzyl)oxy]-5-({7-fluoro-6-[2-fluoro-1-(fluorométhyl)éthoxy]-1-(1-formylpipéridin-4-yl)-2,4-dioxo-1,4-dihydroquinazolin-3(2H)-yl}méthyl)benzonitrile
n°358 : 4-[3-{4-[(3,4-dichlorophénoxy)méthyl]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde
n°360 : 4-{7-fluoro-6-[2-fluoro-1-(fluorométhyl)éthoxy]-2,4-dioxo-3-[4-(2-phényléthyl)benzyl]-3,4-dihydroquinazolin-1(2H)-yl}pipéridine-1-carbaldéhyde
n°362: 4-[3-{4-[(4,5-dichloro-2-fluorobenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde
n°369: 4-[3-{4-[(4-chlorophénoxy)méthyl]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde
n°371: 4-[3-{3-chloro-4-[(4-chlorobenzyl)oxy]-5-éthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde
n°373: 4-[3-{3-chloro-4-[(2,4-dichlorobenzyl)oxy]-5-éthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde
n°375: 4-[7-fluoro-3-{4-[(4-fluorobenzyl)oxy]-3-méthoxybenzyl}-6-[2-fluoro-1-(fluorométhyl)éthoxy]-2,4-dioxo-3,4-dihydroquinazolin-1(2H)-yl]pipéridine-1-carbaldéhyde
n° 376: 4-[3-{4-[(3,5-dichlorobenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde
n°377: 4-[3-(4-{[4-chloro-3-(trifluorométhyl)benzyl]oxy}-3-méthoxybenzyl)-7-fluoro-6-[2-fluoro-1-(fluorométhyl)éthoxy]-2,4-dioxo-3,4-dihydroquinazolin-1(2H)-yl]pipéridine-1-carbaldéhyde
n°379: 4-[3-{4-[(3-chlorophénoxy)méthyl]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde
n°380: 4-[3-{4-[(3,5-difluorobenzyl)oxy]-3-méthoxybenzyl}-7-fluoro-o-[2-fluoro-1-(fluorométhyl)éthoxy]-2,4-dioxo-3,4-dihydroquinazolin-1(2H)-yl]pipéridine-1-carbaldéhyde
n°381: 4-{3-[4-(benzyloxy)-3-méthoxybenzyl]-7-fluoro-6-[2-fluoro-1-(fluorométhyl)éthoxy]-2,4-dioxo-3,4-dihydroquinazolin-1(2H)-yl}pipéridine-1-carbaldéhyde
n°382: 4-[3-{4-[(3-chloro-5-fluorobenzyl)oxy]-3-méthoxybenzyl]-7-fluoro-6-[2-fluoro-1-(fluorométhyl)éthoxy]-2,4-dioxo-3,4-dihydroquinazolin-1(2H)-yl]pipéridine-1-carbaldéhyde
n°383; 4-{7-fluoro-6-[2-fluoro-1-(fluorométhyl)éthoxy]-3-(3-méthoxy-4-{[4-(trifluorométhyl)benzyl]oxy}benzyl)-2,4-dioxo-3,4-dihydroquinazolin-1(2H)-yl}pipéridine-1-carbaldéhyde
n°384: 4-[3-{4-[(2,5-dichlorobenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde
n°385: 4-{[4-({7-fluoro-6-[2-fluoro-1-(fluorométhyl)éthoxyl]-1-(1-formylpipéridin-4-yl)-2,4-dioxo-1,4-dihydroquinazolin-3(2H)-yl}méthyl)-2-méthoxyphénoxy]méthyl}benzonitrile
n°386: 3-{[4-({7-fluoro-6-[2-fluoro-1-(fluorométhyl)éthoxy]-1-(1-formylpipéridin-4-yl)-2,4-dioxo-1,4-dihydroquinazolin-3(2H)-yl}méthyl)-2-méthoxyphénoxy]méthyl}benzonitrile
n°387: 4-[3-{4-[(4-chloro-2-fluorobenzyl)oxy]-3-méthoxybenzyl}-7-fluoro-6-[2-fluoro-1-(fluoromèthyl)éthoxy]-2,4-dioxo-3,4-dihydroquinazolin-1(2H)-yl]pipéridine-1-carbaldéhyde
n°388: 4-[3-{4-[1-(3,4-dichlorophényl)éthoxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde
n°389: 4-{7-fluoro-6-[2-fluoro-1-(fluorométhyl)éthoxy]-3-{4-[(3-hydroxybenzyl)oxy]-3-méthoxybenzyl}-2,4-dioxo-3,4-dihydroquinazolin-1(2H)-yl}pipéridine-1-carbaldéhyde
n°390: 4-[7-fluoro-3-{4-[(3-fluorobenzyl)oxy]-3-méthoxybenzyl}-6-[2-fluoro-1-(fluorométhyl)éthoxy]-2,4-dioxo-3,4-dihydroquinazolin-1(2H)-yl]pipéridine-1-carbaldéhyde
n°391: 4-[3-{4-[(3,4-difluorobenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde
n°392: 4-{3-[4-(5,6-dichloro-1H-benzimidazol-1-yl)-3-méthoxybenzyl]-7-fluoro-6-[2-fluoro-1-(fluorométhyl)éthoxy]-2,4-dioxo-3,4-dihydroquinazolin-1(2H)-yl}pipéridine-1-carbaldéhyde
n°393: 3,4-dichlorobenzènesulfonate de 4-({7-f(uoro-6-[2-fluoro-1-(fluorométhyl)éthoxy]-1-(1-formylpipéridin-4-yl)-2,4-dioxo-1,4-dihydroquinazolin-3(2H)-yl}méthyl)phényle
n°394 : 3,4-dichlorobenzènesulfonate de 4-({7-fluoro-6-[2-fluoro-1-(fluorométhyl)éthoxy]-1-(1-formylpipéridin-4-yl)-2,4-dioxo-1,4-dihydroquinazolin-3(2H)-yl}méthyl)-2-méthoxyphényle
n°403: 3,4-dichloro-N-[4-({7-fluoro-6-[2-fluoro-1-(fluorométhyl)éthoxy]-1-(1-formylpipéridin-4-yl)-2,4-dioxo-1,4-dihydroquinazolin-3(2H)-yl}méthyl)-2-méthoxyphényl]benzamide
à l'état de base ou de sel d'addition à un acide.

## Patentansprüche

1. Verbindung mit der allgemeinen Formel (I) 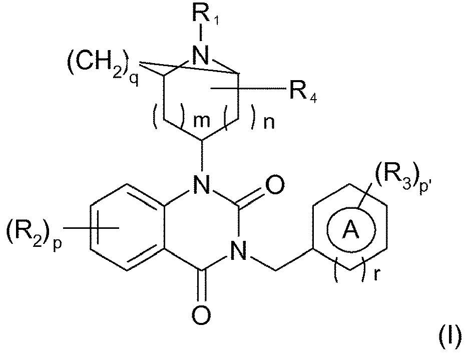 worin
- A für eine Arylgruppe oder eine Heteroarylgruppe steht;
- R₁ für Folgendes steht:
▪ ein Wasserstoffatom,
▪ -C(0)R, worin R ein Wasserstoffatom, eine (C₁-C₆)-Alkoxygruppe, eine Arylgruppe, eine (C₃-C₆)-Cycloalkylgruppe oder eine (C₁-C₆)-Alkylgruppe ist, wobei das Alkyl gegebenenfalls substituiert ist mit:
. einer oder mehreren Hydroxygruppen,
. einer Benzyloxygruppe,
. einer (C₁-C₆) -Alkoxygruppe, gegebenenfalls substituiert mit einem Aryl, oder
. einer (C₃-C₆)-Cycloalkylgruppe,
▪ eine gegebenenfalls substituierte (C₁-C₆)-Alkylgruppe;
- R₂ für Folgendes steht:
▪ ein Wasserstoffatom,
▪ ein Halogenatom,
▪ eine Cyanogruppe,
▪ eine Nitrogruppe,
▪ eine (C₁-C₆) -Alkylgruppe, gegebenenfalls substituiert mit einer -NH₂-Gruppe oder auch mit einer -NHC(0)Rb-Gruppe, wobei Rb wie weiter unten definiert ist,
▪ eine -ORa-Gruppe, worin Ra für Folgendes steht:
. ein Wasserstoffatom,
. eine (C₁-C₆)-Alkylgruppe, gegebenenfalls substituiert mit einem oder mehreren Halogenatomen, mit einer oder mehreren Hydroxygruppen, mit einer Arylgruppe und/oder mit einer oder mehreren Cyanogruppen,
. eine (C₂-C₆) -Alkinylgruppe,
. eine Arylgruppe;
- R₃ für Folgendes steht:
▪ ein Wasserstoffatom,
▪ ein Halogenatom,
▪ eine Hydroxygruppe,
▪ eine Cyanogruppe,
▪ eine -SCF₃-Gruppe,
▪ eine Nitrogruppe,
▪ eine -S(O)₀₋₂-Alkylgruppe, eine -S(O)₀₋₂-Heterocycloalkylgruppe, eine -O-SO₂- Arylgruppe, gegebenenfalls substituiert mit einem oder mehreren Halogenatomen;
▪ eine -Alkylaminoalkyl- oder -Cycloalkylaminoalkylgruppe, die gegebenenfalls jeweils am terminalen Alkyl substituiert ist,
▪ eine gegebenenfalls substituierte Sulfonamidgruppe,
▪ eine Arylgruppe oder eine Heteroarylgruppe, wobei die Gruppe monocyclisch oder polycyclisch ist und ferner gegebenenfalls mit einer (C₁-C₆) - Alkylgruppe, mit einem oder mehreren Halogenatomen oder mit einer (C₁-C₆) - Alkoxygruppe substituiert ist,
▪ eine Heterocycloalkylgruppe, gegebenenfalls substituiert mit einer (C₁-C₆) -Alkylgruppe,
▪ eine (C₁-C₆)-Alkylgruppe, gegebenenfalls substituiert mit:
- einem oder mehreren Halogenatomen,
- einer Arylgruppe, die mit einem oder mehreren Halogenatomen oder mit einer oder mehreren Hydroxygruppen substituiert sein kann,
- einer Heteroarylgruppe,
- einer oder mehreren Hydroxygruppen, die mit einer Arylgruppe substituiert sein können, die ihrerseits gegebenenfalls mit einem oder mehreren Halogenatomen substituiert ist, oder
- einer Heterocycloalkylgruppe, gegebenenfalls substituiert mit einer CO(O)Ra-Gruppe oder mit einer (C₁-C₆) -Alkylgruppe, wobei Ra wie oben definiert ist,
▪ eine -C(O)NRbRc-Gruppe, wobei Rb und Rc wie weiter unten definiert sind,
▪ eine -C(O)ORc-Gruppe oder eine -0-C(O)ORc-Gruppe, wobei Rc wie weiter unten definiert ist,
▪ eine (C₁-C₆)-Alkoxygruppe, gegebenenfalls substituiert mit:
- einer Aminoalkylgruppe,
- einer Aminocycloalkylgruppe,
- einer Cycloalkylgruppe,
- einer Heterocycloalkylgruppe,
- einer monocyclischen oder polycyclischen Heteroarylgruppe,
- einer oder mehreren Hydroxygruppen,
- einer oder mehreren Halogenatomen,
- einer (C₁-C₆) -Alkoxygruppe,
- einer -C(O)ORc-Gruppe, wobei Rc ist, wie weiter unten definiert,
- einer -C(O)NRbRc-Gruppe, wobei Rb und Rc wie weiter unten definiert sind, und/oder
- einer Arylgruppe, ihrerseits gegebenenfalls substituiert mit einem oder mehreren Halogenatomen, einer Cyanogruppe, einer (C₁-C₆) - Alkoxygruppe, einer -O- Halogenoalkylgruppe und/oder mit einer Halogenoalkylgruppe,
▪ eine -O-Cycloalkylgruppe, eine -O- Arylgruppe oder eine -O- Heterocycloalkylgruppe, gegebenenfalls jeweils substituiert mit:
- einer Arylgruppe, ihrerseits gegebenenfalls substituiert mit einem oder mehreren Halogenatomen oder mit einer (C₁-C₆)-Alkylgruppe,
- einem oder mehreren Halogenatomen, und/oder
- einer (C₁-C₆) -Alkylgruppe, die ihrerseits mit einer Arylgruppe substituiert sein kann,
▪ eine -NH-CO-NH-Arylgruppe, eine -NH-CO-NH-Heteroarylgruppe oder eine -NH-CO-NH-(C₁-C₆)-Alkylgruppe, gegebenenfalls jeweils substituiert mit einem oder mehreren Halogenatomen, mit einer Cyanogruppe, mit einer Nitrogruppe, mit einer oder mehreren Hydroxygruppen oder mit einer (C₁-C₆)-Alkoxygruppe,
▪ eine -N-(C₁-C₆)-Alkylgruppe, wobei die (C₁-C₆) -Alkylgruppe mit einer oder mehreren Arylgruppen substituiert sein kann, gegebenenfalls substituiert mit einem oder mehreren Halogenatomen und/oder mit einer SO₂-Gruppe,
▪ eine -NH-CO-Arylgruppe, eine -NH-CO- Heteroarylgruppe, die jeweils gegebenenfalls mit einem oder mehreren Halogenatomen substituiert sind;
oder R₃ bildet mit A eine polycyclische Heteroarylgruppe, gegebenenfalls substituiert mit einer (C₁-C₆) -Alkoxygruppe, einer (C₁-C₆) - Alkylgruppe, gegebenenfalls substituiert mit einer Arylgruppe, die ihrerseits mit einem oder mehreren Halogenatomen substituiert sein kann;
- R₄ für ein Wasserstoffatom oder eine (C₁-C₆) - Alkylgruppe steht;
- Rb für Folgendes steht:
. ein Wasserstoffatom,
. eine (C₁-C₆) -Alkylgruppe, gegebenenfalls substituiert mit einem oder mehreren Halogenatomen, mit einer oder mehreren Hydroxy-, Cyano-, Amino-, Heterocycloalkyl-, (C₁-C₆)-Alkoxygruppen oder mit einer Arylgruppe, gegebenenfalls substituiert mit einem oder mehreren Halogenatomen,
. eine (C₃-C₆)-Cycloalkylgruppe,
. eine (C₂-C₆) -Alkinylgruppe,
. eine (C₁-C₆) -Alkoxygruppe,
. eine Arylgruppe, gegebenenfalls substituiert mit einem oder mehreren Halogenatomen;
- Rc für ein Wasserstoffatom oder eine (C₁-C₆)-Alkylgruppe steht, gegebenenfalls substituiert mit einem oder mehreren Halogenatomen;
oder Rb und Rc dann zusammen mit dem Stickstoffatom, an das sie gebunden sind, eine polycyclische Heteroarylgruppe oder eine Heterocycloalkylgruppe bilden;
- m und n unabhängig voneinander für den Wert 0, 1 oder 2 stehen, wobei gilt, dass m+n ≤ 3;
- p und p' unabhängig voneinander für den Wert 1, 2 oder 3 stehen, wobei gilt, dass, wenn p größer oder gleich 2 ist, sich die Gruppen R₂ dann an verschiedenen Kohlenstoffatomen befinden und voneinander verschieden sein können, und wenn p' größer oder gleich 2 ist, sich die Gruppen R₃ dann an verschiedenen Kohlenstoffatomen befinden und voneinander verschieden sein können;
- q für den Wert 0 oder 2 steht, wobei gilt, dass wenn q = 0, dass dann die heterocyclische Stickstoffgruppe, die mit dem Stickstoff verknüpft ist, der an Position 1 des 2,4-Dioxo-1,2,3,4-tetrahydrochinazolinkerns liegt, nicht mehr verbrückt wird und folgenden Typs ist: 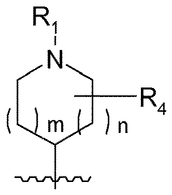
wobei R₁, R₄, m und n wie oben definiert sind,
- r für den Wert 0 oder 1 steht;
in Form einer Base oder eines Säureadditionssalzes;
zur Behandlung mindestens einer kardiovaskulären Erkrankung und/oder um das Auftreten mindestens einer kardiovaskulären Erkrankung zu verhindern.

2. Verbindung zur Verwendung nach dem vorhergehenden Anspruch, **dadurch gekennzeichnet, dass** die kardiovaskuläre Erkrankung ausgewählt ist aus (i) den koronaren Herzerkrankungen, (ii) den Herzmuskelerkrankungen, (iii) den Herzklappenerkrankungen, (iv) den Herzbeutelerkrankungen, (v) den Herzrhythmusstörungen und den Erregungsleitungsstörungen und (vi) den Gefäßerkrankungen.

3. Verbindung zur Verwendung nach einem der Ansprüche 1 oder 2, **dadurch gekennzeichnet, dass** die kardiovaskuläre Erkrankung ausgewählt ist aus Myokardinfarkt, insbesondere den kardialen Kontraktionsstörungen infolge eines Myokardinfarkts, Erkrankungen, die mit Reperfusionsverletzungen des Herz- und/oder Skelettmuskels assoziiert sind, pulmonaler Hypertonie, Leberfibrose, arterieller Restenose nach Angioplastie mit oder ohne Stenteinlage, Atherosklerose und deren Komplikationen, Herzinsuffizienz, erweiterten Kardiomyopathien und Herzmuskelentzündungen viralen und/oder bakteriellen Ursprungs.

4. Verbindung zur Verwendung nach einem der vorhergehenden Ansprüche, **dadurch gekennzeichnet, dass** A für eine Phenylgruppe oder eine Pyridylgruppe steht.

5. Verbindung zur Verwendung nach einem der vorhergehenden Ansprüche, **dadurch gekennzeichnet, dass** q = 0 ist, m und n jeweils für 1 stehen.

6. Verbindung zur Verwendung nach einem der vorhergehenden Ansprüche, **dadurch gekennzeichnet, dass** R₂ für eine (C₁-C₆)-Alkylgruppe, insbesondere ein Methyl steht, das mit einer -NH-C(0)-Rb-Gruppe substituiert ist, wobei Rb wie in den Ansprüchen 1 oder 2 definiert ist.

7. Verbindung zur Verwendung nach einem der Ansprüche
1 bis 5, **dadurch gekennzeichnet, dass** R₂ für eine -ORa-Gruppe steht, wobei Ra wie in den Ansprüchen 1 oder 2 definiert ist.

8. Verbindung zur Verwendung nach einem der Ansprüche 1 bis 5, **dadurch gekennzeichnet, dass** R₂ ein Halogenatom oder eine Cyano- oder eine Wasserstoff- oder eine Hydroxyl- oder eine (C₁-C₆)-Alkylgruppe ist, gegebenenfalls substituiert mit einer -NH₂-Gruppe oder auch mit einer -NHC(0)Rb-Gruppe, wobei Rb wie in den Ansprüchen 1 oder 2 definiert ist.

9. Verbindung zur Verwendung nach einem der Ansprüche 1 bis 8, **dadurch gekennzeichnet, dass**:
- A wie in Anspruch 4 definiert ist;
- q, m und n wie in Anspruch 5 definiert sind;
- R₂ wie in Anspruch 6, 7 oder 8 definiert ist.

10. Verbindung zur Verwendung nach einem der Ansprüche 1 bis 3, **dadurch gekennzeichnet, dass**:
A ein Phenyl ist, R₁ eine -C(0)R-Gruppe ist, worin R für ein Wasserstoffatom steht, q gleich 0 ist, n und m jeweils den Wert 1 haben, und R₂ für -ORa steht, wobei Ra wie in den Ansprüchen 1 oder 2 definiert ist.

11. Verbindung zur Verwendung nach einem der Ansprüche 1 bis 3, **dadurch gekennzeichnet, dass**:
A ein Phenyl ist, R₁ eine -C(O)R-Gruppe ist, worin R für ein Wasserstoffatom steht, q gleich 0 ist, n und m jeweils den Wert 1 haben, p=1 ist, und R₂ für ein Methyl steht, das mit einer -NH-CO-Rb-Gruppe substituiert ist, wobei Rb wie in den Ansprüchen 1 oder 2 definiert ist.

12. Verbindung zur Verwendung nach einem der Ansprüche 1 bis 3, **dadurch gekennzeichnet, dass**:
A ein Phenyl ist, R₁ eine -C(O)R-Gruppe ist, worin R für ein Wasserstoffatom steht, q gleich 0 ist, n und m jeweils den Wert 1 haben, p gleich 2 ist, einer der Reste R₂ für -ORa steht, wobei Ra wie in den Ansprüchen 1 oder 2 definiert ist, und der andere Rest R₂ ein Halogenatom ist.

13. Verbindung zur Verwendung nach einem der Ansprüche 1 bis 3, **dadurch gekennzeichnet, dass**:
die Gruppe R₂ an Position 6 des 2,4-Dioxo-1,2,3,4-tetrahydrochinazolinkerns ist, und dadurch, dass es dort ferner eine Gruppe R₂ geben kann, gleich oder verschieden von der vorgenannten Gruppe R₂, an Position 7 des 2,4-Dioxo-1,2,3,4-tetrahydrochinazolinkerns, wobei die Verbindung mit der Formel (I) in Form einer Base, eines Hydrats, eines Solvats, von Isomeren oder deren Gemischen vorliegt.

14. Verbindung zur Verwendung nach einem der Ansprüche 1 bis 3, ausgewählt aus:
Nr. 1: 2-{[3-(3,4-Dimethoxybenzyl)-1-(1-formylpiperidin-4-yl)-2,4-dioxo-1,2,3,4-tetrahydrochinazolin-6-yl]oxy}propannitril
Nr. 2: 1-(1-Acetylpiperidin-4-yl)-3-(3,4-dimethoxybenzyl)-6-hydroxychinazolin-2,4(1H,3H)-dion
Nr. 3: {[1-(1-Acetylpiperidin-4-yl)-3-(3,4-dimethoxybenzyl)-2,4-dioxo-1,2,3,4-tetrahydrochinazolin-6-yl]oxy}acetonitril
Nr. 4: 2-{[1-(1-Acetylpiperidin-4-yl)-3-(3,4-dimethoxybenzyl)-2,4-dioxo-1,2,3,4-tetrahydrochinazolin-6-yl]oxy}propannitril
Nr. 5: {[3-(3,4-Dimethoxybenzyl)-1-(1-formylpiperidin-4-yl)-2,4-dioxo-1,2,3,4-tetrahydrochinazolin-6-yl]oxy}acetonitril
Nr. 6: {[3-(3,4-Dimethoxybenzyl)-1-(1-formylpiperidin-4-yl)-2,4-dioxo-1,2,3,4-tetrahydrochinazolin-6-yl]oxy}acetonitril
Nr. 11: 4-[3-(3,4-Dimethoxybenzyl)-6-[2-fluor-1-(fluormethyl)ethoxy]-2,4-dioxo-3,4-dihydrochinazolin-1(2H)-yl]piperidin-1-carbaldehyd
Nr. 12: 1-(1-Acetylpiperidin-4-yl)-3-(3,4-dimethoxybenzyl)-6-[2-fluor-1-(fluormethyl)ethoxy]chinazolin-2,4(1H,3H)-dion
Nr. 13: 4-[3-(3,4-Dimethoxybenzyl)-2,4-dioxo-6-(2,2,2-trifluorethoxy)-3,4-dihydrochinazolin-1(2H)-yl]piperidin-1-carbaldehyd
Nr. 14: 1-(1-Acetylpiperidin-4-yl)-6-(2,2-difluorethoxy)-3-(3,4-dimethoxybenzyl)chinazolin-2,4(1H,3H)-dion
Nr. 16: 4-[6-(2,2-Difluorethoxy)-3-(3,4-dimethoxybenzyl)-2,4-dioxo-3,4-dihydrochinazolin-1(2H)-yl]piperidine-1-carbaldehyde
Nr. 20: N-{[3-(3,4-Dimethoxybenzyl)-1-(1-formylpiperidin-4-yl)-2,4-dioxo-1,2,3,4-tetrahydrochinazolin-6-yl]methyl}acetamid
Nr. 22: 1-(1-Acetylpiperidin-4-yl)-6-(aminomethyl)-3-(3,4-dimethoxybenzyl)chinazolin-2,4(1H,3H)-dionhydrochlorid
Nr. 23: N-{[3-(3,4-Dimethoxybenzyl)-1-(1-formylpiperidin-4-yl)-2,4-dioxo-1,2,3,4-tetrahydrochinazolin-6-yl]methyl}formamid
Nr. 24: N-{[1-(1-Acetylpiperidin-4-yl)-3-(3,4-dimethoxybenzyl)-2,4-dioxo-1,2,3,4-tetrahydrochinazolin-6-yl]methyl}formamid
Nr. 25: N-{[1-(1-Acetylpiperidin-4-yl)-3-(3,4-dimethoxybenzyl)-2,4-dioxo-1,2,3,4-tetrahydrochinazolin-6-yl]methyl}acetamid
Nr. 32: 4-[6-(2,2-Difluorethoxy)-2,4-dioxo-3,4-dihydrochinazolin-1(2H)-yl]piperidin-1-carbaldehyd
Nr. 33: 4-[3-(3,4-Dichlorbenzyl)-6-(2,2-difluorethoxy)-2,4-dioxo-3,4-dihydrochinazolin-1(2H)-yl]piperidin-1-carbaldehyd
Nr. 34: 4-[3-(4-Chlorbenzyl)-6-(2,2-difluorethoxy)-2,4-dioxo-3,4-dihydrochinazolin-1(2H)-yl]piperidin-1-carbaldehyd
Nr. 35: Methyl-4-{[6-(2,2-difluorethoxy)-1-(1-formylpiperidin-4-yl)-2,4-dioxo-1,4-dihydrochinazolin-3(2H)-yl]methyl}-benzoat
Nr. 36: 4-{[6-(2,2-Difluorethoxy)-1-(1-formylpiperidin-4-yl)-2,4-dioxo-1,4-dihydrochinazolin-3(2H)-yl]methyl}benzoesäure
Nr. 37: 4-{[6-(2,2-Difluorethoxy)-1-(1-formylpiperidin-4-yl)-2,4-dioxo-1,4-dihydrochinazolin-3(2H)-yl]methyl}-N-(2-methoxyethyl)benzamid
Nr. 38: 4-[3-(3,4-Dimethoxybenzyl)-6-methyl-2,4-dioxo-3,4-dihydrochinazolin-1(2H)-yl]piperidin-1-carbaldehyd
Nr. 39: 4-[6-(2,2-Difluorethoxy)-3-(3-hydroxy-4-methoxybenzyl)-2,4-dioxo-3,4-dihydrochinazolin-1(2H)-yl]piperidin-1-carbaldehyd
Nr. 40: 4-[6-(2,2-Difluorethoxy)-3-[3-(2-hydroxyethoxy)-4-methoxybenzyl]-2,4-dioxo-3,4-dihydrochinazolin-1(2H)-yl]piperidin-1-carbaldehyd
Nr. 41: 4-[6-(2,2-Difluorethoxy)-3-(3-ethoxy-4-methoxybenzyl)-2,4-dioxo-3,4-dihydrochinazolin-1(2H)-yl]piperidin-1-carbaldehyd
Nr. 42: 4-[6-(2,2-Difluorethoxy)-3-[4-methoxy-3-(2-methoxyethoxy)benzyl]-2,4-dioxo-3,4-dihydrochinazolin-1(2H)-yl]piperidin-1-carbaldehyd
Nr. 43: 4-[6-(2,2-Difluorethoxy)-3-(3,4-dimethoxybenzyl)-2,4-dioxo-3,4-dihydrochinazolin-1(2H)-yl]azepan-1-carbaldehyd
Nr. 47: 4-[6-(2,2-Difluorethoxy)-3-[3-(3-hydroxypropoxy)-4-methoxybenzyl]-2,4-dioxo-3,4-dihydrochinazolin-1(2H)-yl]piperidin-1-carbaldehyd
Nr. 48: 4-[5-Chlor-3-(3,4-dimethoxybenzyl)-2,4-dioxo-3,4-dihydrochinazolin-1(2H)-yl]piperidin-1-carbaldehyd
Nr. 49: 4-{3-[3-(Cyclopentyloxy)-4-methoxybenzyl]-6-(2,2-difluorethoxy)-2,4-dioxo-3,4-dihydrochinazolin-1(2H)-yl}piperidin-1-carbaldehyd
Nr. 50: 2-(5-{[6-(2,2-Difluorethoxy)-1-(1-formylpiperidin-4-yl)-2,4-dioxo-1,4-dihydrochinazolin-3(2H)-yl]methyl}-2-methoxyphenoxy)acetamid
Nr. 51: 4-[6-(2,2-Difluorethoxy)-3-(3,4-dimethoxybenzyl)-2,4-dioxo-3,4-dihydrochinazolin-1(2H)-yl]-3-methylpiperidin-1-carbaldehyd
Nr. 52: 3-[6-(2,2-Difluorethoxy)-3-(3,4-dimethoxybenzyl)-2,4-dioxo-3,4-dihydrochinazolin-1(2H)-yl]-8-azabicyclo[3.2.1]octan-8-carbaldehyd
Nr. 55: 4-{6-[2-Fluor-1-(fluormethyl)ethoxy]-2,4-dioxo-3,4-dihydrochinazolin-1(2H)-yl}piperidin-1-carbaldehyd
Nr. 56: 4-{3-[4-(Cyclopentyloxy)-3-methoxybenzyl]-6-[2-fluor-1-(fluormethyl)ethoxy]-2,4-dioxo-3,4-dihydrochinazolin-1(2H)-yl}piperidin-1-carbaldehyd
Nr. 57: 4-[3-(3-Chlorbenzyl)-6-[2-fluor-1-(fluormethyl)ethoxy]-2,4-dioxo-3,4-dihydrochinazolin-1(2H)-yl]piperidin-1-carbaldehyd
Nr. 58: 4-[3-(4-Chlorbenzyl)-6-[2-fluor-1-(fluormethyl)ethoxy]-2,4-dioxo-3,4-dihydrochinazolin-1(2H)-yl]piperidin-1-carbaldehyd
Nr. 59: 4-{3-[3-(Cyclopentyloxy)-4-methoxybenzyl]-6-[2-fluor-1-(fluormethyl)ethoxy]-2,4-dioxo-3,4-dihydrochinazolin-1(2H)-yl}piperidin-1-carbaldehyd
Nr. 72: 4-[3-(3,4-Dimethoxybenzyl)-6-(2-hydroxyethoxy)-2,4-dioxo-3,4-dihydrochinazolin-1(2H)-yl]piperidin-1-carbaldehyd
Nr. 74: 4-[3-(3,4-Dichlorbenzyl)-6-[2-fluor-1-(fluormethyl)ethoxy]-2,4-dioxo-3,4-dihydrochinazolin-1(2H)-yl]piperidin-1-carbaldehyd
Nr. 76: 4-{3-[(6-Chlorpyridin-3-yl)methyl]-6-[2-fluor-1-(fluormethyl)ethoxy]-2,4-dioxo-3,4-dihydrochinazolin-1(2H)-yl}piperidin-1-carbaldehyd
Nr. 78: 4-[3-(3-Chlor-4-methoxybenzyl)-6-[2-fluor-1-(fluormethyl)ethoxy]-2,4-dioxo-3,4-dihydrochinazolin-1(2H)-yl]piperidin-1-carbaldehyd
Nr. 79: 4-[3-(3,4-Dimethoxybenzyl)-6-(2-fluorethoxy)-2,4-dioxo-3,4-dihydrochinazolin-1(2H)-yl]piperidin-1-carbaldehyd
Nr. 89: 2-[5-({6-[2-Fluor-1-(fluormethyl)ethoxy]-1-(1-formylpiperidin-4-yl)-2,4-dioxo-1,4-dihydrochinazolin-3(2H)-yl}methyl)-2-methoxyphenoxy]acetamid
Nr. 90: 4-{6-[2-Fluor-1-(fluormethyl)ethoxy]-3-(3-hydroxy-4-methoxybenzyl)-2,4-dioxo-3,4-dihydrochinazolin-1(2H)-yl}piperidin-1-carbaldehyd
Nr. 91: 4-[3-(3,4-Dimethoxybenzyl)-6-ethoxy-2,4-dioxo-3,4-dihydrochinazolin-1(2H)-yl]piperidin-1-carbaldehyd
Nr. 97: 4-[5,7-Dichlor-3-(3,4-dimethoxybenzyl)-2,4-dioxo-3,4-dihydrochinazolin-1(2H)-yl]piperidin-1-carbaldehyd
Nr. 102: 4-[7-Chlor-3-(3,4-dimethoxybenzyl)-2,4-dioxo-3,4-dihydrochinazolin-1(2H)-yl]piperidin-1-carbaldehyd
Nr. 108: 4-{6-[2-Fluor-1-(fluormethyl)ethoxy]-3-(3-fluor-4-methoxybenzyl)-2,4-dioxo-3,4-dihydrochinazolin-1(2H)-yl}piperidin-1-carbaldehyd
Nr. 111: 4-[6-(Difluormethoxy)-3-(3,4-dimethoxybenzyl)-2,4-dioxo-3,4-dihydrochinazolin-1(2H)-yl]piperidin-1-carbaldehyd
Nr. 112: 4-[3-(3,4-Dimethoxybenzyl)-6-(1-methylethoxy)-2,4-dioxo-3,4-dihydrochinazolin-1(2H)-yl]piperidin-1-carbaldehyd
Nr. 114: 4-{6-[2-Fluor-1-(fluormethyl)ethoxy]-3-[4-methoxy-3-(1-methylethoxy)benzyl]-2,4-dioxo-3,4-dihydrochinazolin-1(2H)-yl}piperidin-1-carbaldehyd
Nr. 116: 4-{6-[2-Fluor-1-(fluormethyl)ethoxy]-3-(3-methoxybenzyl)-2,4-dioxo-3,4-dihydrochinazolin-1(2H)-yl}piperidin-1-carbaldehyd
Nr. 117: 4-{3-[3,5-Bis(trifluormethyl)benzyl]-6-[2-fluor-1-(fluormethyl)ethoxy]-2,4-dioxo-3,4-dihydrochinazolin-1(2H)-yl}piperidin-1-carbaldehyd
Nr. 118: 4-[3-(3-Ethoxybenzyl)-6-[2-fluor-1-(fluormethyl)ethoxy]-2,4-dioxo-3,4-dihydrochinazolin-1(2H)-yl]piperidin-1-carbaldehyd
Nr. 124: 4-{3-[3-Chlor-4-(2-methoxyethoxy)benzyl]-6-[2-fluor-1-(fluormethyl)ethoxy]-2,4-dioxo-3,4-dihydrochinazolin-1(2H)-yl}piperidin-1-carbaldehyd
Nr. 130: 4-[3-(3,4-Diethoxybenzyl)-6-[2-fluor-1-(fluormethyl)ethoxy]-2,4-dioxo-3,4-dihydrochinazolin-1(2H)-yl]piperidin-1-carbaldehyd
Nr. 131: 4-[3-(4-Ethoxy-3-methoxybenzyl)-6-[2-fluor-1-(fluormethyl)ethoxy]-2,4-dioxo-3,4-dihydrochinazolin-1(2H)-yl]piperidin-1-carbaldehyd
Nr. 133: 4-{6-[2-Fluor-1-(fluormethyl)ethoxy]-3-(4-methoxy-3-methylbenzyl)-2,4-dioxo-3,4-dihydrochinazolin-1(2H)-yl}piperidin-1-carbaldehyd
Nr. 134: 4-{6-[2-Fluor-1-(fluormethyl)ethoxy]-2,4-dioxo-3-[4-(trifluormethyl)benzyl]-3,4-dihydrochinazolin-1(2H)-yl}piperidin-1-carbaldehyd
Nr. 135: 4-{6-[2-Fluor-1-(fluormethyl)ethoxy]-2,4-dioxo-3-[4-(trifluormethyl)benzyl]-3,4-dihydrochinazolin-1(2H)-yl}piperidin-1-carbaldehyd
Nr. 143: 4-{3-[4-(Benzyloxy)-3-methoxybenzyl]-6-[2-fluor-1-(fluormethyl)ethoxy]-2,4-dioxo-3,4-dihydrochinazolin-1(2H)-yl}piperidin-1-carbaldehyd
Nr. 145: 4-{6-[2-Fluor-1-(fluormethyl)ethoxy]-3-(3-methoxy-4-nitrobenzyl)-2,4-dioxo-3,4-dihydrochinazolin-1(2H)-yl}piperidin-1-carbaldehyd
Nr. 155: 4-[3-(4-Ethoxybenzyl)-6-[2-fluor-1-(fluormethyl)ethoxy]-2,4-dioxo-3,4-dihydrochinazolin-1(2H)-yl]piperidin-1-carbaldehyd
Nr. 158: 4-{6-[2-Fluor-1-(fluormethyl)ethoxy]-3-[4-(morpholin-4-ylmethyl)benzyl]-2,4-dioxo-3,4-dihydrochinazolin-1(2H)-yl}piperidin-1-carbaldehyd
Nr. 160: 4-{6-[2-Fluor-1-(fluormethyl)ethoxy]-3-(4-morpholin-4-ylbenzyl)-2,4-dioxo-3,4-dihydrochinazolin-1(2H)-yl}piperidin-1-carbaldehyd
Nr. 165: 4-[3-(Biphenyl-4-ylmethyl)-6-[2-fluor-1-(fluormethyl)ethoxy]-2,4-dioxo-3,4-dihydrochinazolin-1(2H)-yl]piperidin-1-carbaldehyd
Nr. 166: 4-{6-[2-Fluor-1-(fluormethyl)ethoxy]-3-[4-(methylsulfanyl)benzyl]-2,4-dioxo-3,4-dihydrochinazolin-1(2H)-yl}piperidin-1-carbaldehyd
Nr. 167: 4-{6-[2-Fluor-1-(fluormethyl)ethoxy]-2,4-dioxo-3-(4-pyridin-3-ylbenzyl)-3,4-dihydrochinazolin-1(2H)-yl}piperidin-1-carbaldehyd
Nr. 170: 4-{6-[2-Fluor-1-(fluormethyl)ethoxy]-3-(3-methoxy-4-methylbenzyl)-2,4-dioxo-3,4-dihydrochinazolin-1(2H)-yl}piperidin-1-carbaldehyd
Nr. 175: 2-[2-(Cyclopentyloxy)-5-({6-[2-fluor-1-(fluormethyl)ethoxy]-1-(1-formylpiperidin-4-yl)-2,4-dioxo-1,4-dihydrochinazolin-3(2H)-yl}methyl)phenoxy]acetamid
Nr. 178: 4-{6-[2-Fluor-1-(fluormethyl)ethoxy]-3-(3-methoxy-4-propoxybenzyl)-2,4-dioxo-3,4-dihydrochinazolin-1(2H)-yl}piperidin-1-carbaldehyd
Nr. 183: 2-[2-(Cyclopentyloxy)-5-({6-[2-fluor-1-(fluormethyl)ethoxy]-1-(1-formylpiperidin-4-yl)-2,4-dioxo-1,4-dihydrochinazolin-3(2H)-yl}methyl)phenoxy]-N-methylacetamid
Nr. 184: 2-[2-(Cyclopentyloxy)-5-({6-[2-fluor-1-(fluormethyl)ethoxy]-1-(1-formylpiperidin-4-yl)-2,4-dioxo-1,4-dihydrochinazolin-3(2H)-yl}methyl)phenoxy]-N,N-dimethylacetamid
Nr. 185: 2-[2-(Cyclopentyloxy)-5-({6-[2-fluor-1-(fluormethyl)ethoxy]-1-(1-formylpiperidin-4-yl)-2,4-dioxo-1,4-dihydrochinazolin-3(2H)-yl}methyl)phenoxy]-N-methoxy-N-methylacetamid
Nr. 186: 4-{3-[4-(Cyclopentyloxy)-3-ethoxybenzyl]-6-[2-fluor-1-(fluormethyl)ethoxy]-2,4-dioxo-3,4-dihydrochinazolin-1(2H)-yl}piperidin-1-carbaldehyd
Nr. 188: 4-{3-[4-(Cyclopentyloxy)-3-(1-methylethoxy)benzyl]-6-[2-fluor-1-(fluormethyl)ethoxy]-2,4-dioxo-3,4-dihydrochinazolin-1(2H)-yl}piperidin-1-carbaldehyd
Nr. 189: 4-{3-[4-(Cyclopentyloxy)-3-propoxybenzyl]-6-[2-fluor-1-(fluormethyl)ethoxy]-2,4-dioxo-3,4-dihydrochinazolin-1(2H)-yl}piperidin-1-carbaldehyd
Nr. 190: 4-{3-[4-(Cyclopentyloxy)-3-hydroxybenzyl]-6-[2-fluor-1-(fluormethyl)ethoxy]-2,4-dioxo-3,4-dihydrochinazolin-1(2H)-yl}piperidin-1-carbaldehyd
Nr. 193: 4-{3-[4-(Difluormethoxy)-3-methoxybenzyl]-6-[2-fluor-1-(fluormethyl)ethoxy]-2,4-dioxo-3,4-dihydrochinazolin-1(2H)-yl}piperidin-1-carbaldehyd
Nr. 194: 4-{3-[4-(Difluormethoxy)-3-ethoxybenzyl]-6-[2-fluor-1-(fluormethyl)ethoxy]-2,4-dioxo-3,4-dihydrochinazolin-1(2H)-yl}piperidin-1-carbaldehyd
Nr. 200: 4-{6-[2-Fluor-1-(fluormethyl)ethoxy]-2,4-dioxo-3-(4-thiophen-3-ylbenzyl)-3,4-dihydrochinazolin-1(2H)-yl}piperidin-1-carbaldehyd
Nr. 201: 4-{6-[2-Fluor-1-(fluormethyl)ethoxy]-2,4-dioxo-3-(4-pyridin-4-ylbenzyl)-3,4-dihydrochinazolin-1(2H)-yl}piperidin-1-carbaldehyd
Nr. 203: 4-{6-[2-Fluor-1-(fluormethyl)ethoxy]-3-[(1-methyl-1H-indol-6-yl)methyl]-2,4-dioxo-3,4-dihydrochinazolin-1(2H)-yl}piperidin-1-carbaldehyd
Nr. 206: 4-{3-[4-(Cyclopropylmethoxy)-3-methoxybenzyl]-6-[2-fluor-1-(fluormethyl)ethoxy]-2,4-dioxo-3,4-dihydrochinazolin-1(2H)-yl}piperidin-1-carbaldehyd
Nr. 207: 2-[4-({6-[2-Fluor-1-(fluormethyl)ethoxy]-1-(1-formylpiperidin-4-yl)-2,4-dioxo-1,4-dihydrochinazolin-3(2H)-yl}methyl)-2-methoxyphenoxy]-N-methylacetamid
Nr. 212: 4-{6-[2-Fluor-1-(fluormethyl)ethoxy]-2,4-dioxo-3-[4-(1H-pyrazol-1-yl)benzyl]-3,4-dihydrochinazolin-1(2H)-yl}piperidin-1-carbaldehyd
Nr. 213: 4-{6-[2-Fluor-1-(fluormethyl)ethoxy]-2,4-dioxo-3-(4-pyridin-2-ylbenzyl)-3,4-dihydrochinazolin-1(2H)-yl}piperidin-1-carbaldehyd
Nr. 215: 4-{6-[2-Fluor-1-(fluormethyl)ethoxy]-2,4-dioxo-3-(4-thiophen-2-ylbenzyl)-3,4-dihydrochinazolin-1(2H)-yl}piperidin-1-carbaldehyd
Nr. 216: 4-{6-[2-Fluor-1-(fluormethyl)ethoxy]-2,4-dioxo-3-(chinolein-7-ylmethyl)-3,4-dihydrochinazolin-1(2H)-yl}piperidin-1-carbaldehyd
Nr. 218: 4-{6-[2-Fluor-1-(fluormethyl)ethoxy]-3-[(6-methoxynaphtalin-2-yl)methyl]-2,4-dioxo-3,4-dihydrochinazolin-1(2H)-yl}piperidin-1-carbaldehyd
Nr. 223: 4-{3-[4-(1H-Benzimidazol-1-yl)benzyl]-6-[2-fluor-1-(fluormethyl)ethoxy]-2,4-dioxo-3,4-dihydrochinazolin-1(2H)-yl}piperidin-1-carbaldehyd
Nr. 224: 4-{6-[2-Fluor-1-(fluormethyl)ethoxy]-3-[3-methoxy-4-(2-methylpropoxy)benzyl]-2,4-dioxo-3,4-dihydrochinazolin-1(2H)-yl}piperidin-1-carbaldehyd
Nr. 226: 4-{6-[2-Fluor-1-(fluormethyl)ethoxy]-3-[3-methoxy-4-(tetrahydrofuran-3-yloxy)benzyl]-2,4-dioxo-3,4-dihydrochinazolin-1(2H)-yl}piperidin-1-carbaldehyd
Nr. 228: 4-[3-{4-[(1-Benzylpyrrolidin-3-yl)oxy]-3-methoxybenzyl}-6-[2-fluor-1-(fluormethyl)ethoxy]-2,4-dioxo-3,4-dihydrochinazolin-1(2H)-yl]piperidin-1-carbaldehyd
Nr. 230: 4-[3-(1-Benzothiophen-5-ylmethyl)-6-[2-fluor-1-(fluormethyl)ethoxy]-2,4-dioxo-3,4-dihydrochinazolin-1(2H)-yl]piperidin-1-carbaldehyd
Nr. 232: 4-{6-[2-Fluor-1-(fluormethyl)ethoxy]-3-[3-methoxy-4-(1-methylethoxy)benzyl]-2,4-dioxo-3,4-dihydrochinazolin-1(2H)-yl}piperidin-1-carbaldehyd
Nr. 233: 4-[3-(3,4-Dimethoxybenzyl)-7-fluor-6-[2-fluor-1-(fluormethyl)ethoxy]-2,4-dioxo-3,4-dihydrochinazolin-1(2H)-yl]piperidin-1-carbaldehyd
Nr. 234: 4-[3-{4-[(1-Acetylpyrrolidin-3-yl)oxy]-3-methoxybenzyl}-6-[2-fluor-1-(fluormethyl)ethoxy]-2,4-dioxo-3,4-dihydrochinazolin-1(2H)-yl]piperidin-1-carbaldehyd
Nr. 239: 4-[3-{4-[(4-Fluorbenzyl)oxy]-3-methoxybenzyl}-6-[2-fluor-1-(fluormethyl)ethoxy]-2,4-dioxo-3,4-dihydrochinazolin-1(2H)-yl]piperidin-1-carbaldehyd
Nr. 240: 4-[3-{4-[(4-Chlorbenzyl)oxy]-3-methoxybenzyl}-6-[2-fluor-1-(fluormethyl)ethoxy]-2,4-dioxo-3,4-dihydrochinazolin-1(2H)-yl]piperidin-1-carbaldehyd
Nr. 242: 4-[3-{4-[(3-Chlorbenzyl)oxy]-3-methoxybenzyl}-6-[2-fluor-1-(fluormethyl)ethoxy]-2,4-dioxo-3,4-dihydrochinazolin-1(2H)-yl]piperidin-1-carbaldehyd
Nr. 243: 4-{6-[2-Fluor-1-(fluormethyl)ethoxy]-2,4-dioxo-3-(3-thiophen-3-ylbenzyl)-3,4-dihydrochinazolin-1(2H)-yl}piperidin-1-carbaldehyd
Nr. 245: 4-[3-(4-Ethoxy-3-methoxybenzyl)-6-(2-hydroxyethoxy)-2,4-dioxo-3,4-dihydrochinazolin-1(2H)-yl]piperidin-1-carbaldehyd
Nr. 246: 4-[3-{4-[2-(2,3-Dihydro-1H-indol-1-yl)-2-oxoethoxy]-3-methoxybenzyl}-6-[2-fluor-1-(fluormethyl)ethoxy]-2,4-dioxo-3,4-dihydrochinazolin-1(2H)-yl]piperidin-1-carbaldehyd
Nr. 250: 4-[3-{4-[(3,4-Dichlorbenzyl)oxy]-3-methoxybenzyl}-6-[2-fluor-1-(fluormethyl)ethoxy]-2,4-dioxo-3,4-dihydrochinazolin-1(2H)-yl]piperidin-1-carbaldehyd
Nr. 251: 4-{6-[2-Fluor-1-(fluormethyl)ethoxy]-3-[3-methoxy-4-(2-oxo-2-piperidin-1-ylethoxy)benzyl]-2,4-dioxo-3,4-dihydrochinazolin-1(2H)-yl}piperidin-1-carbaldehyd
Nr. 254: 4-{3-[3-Ethoxy-4-(thiophen-2-ylmethoxy)benzyl]-6-[2-fluor-1-(fluormethyl)ethoxy]-2,4-dioxo-3,4-dihydrochinazolin-1(2H)-yl}piperidin-1-carbaldehyd
Nr. 258: 4-[3-(3,4-Dimethoxybenzyl)-6-[2-fluor-1-(hydroxymethyl)ethoxy]-2,4-dioxo-3,4-dihydrochinazolin-1(2H)-yl]piperidin-1-carbaldehyd
Nr. 263: (2R)-2-[2-(Cyclopentyloxy)-5-({6-[2-fluor-1-(fluormethyl)ethoxy]-1-(1-formylpiperidin-4-yl)-2,4-dioxo-1,4-dihydrochinazolin-3(2H)-yl}methyl)phenoxy]propansäure
Nr. 264: 4-{6-[2-Fluor-1-(fluormethyl)ethoxy]-3-[(1-methyl-3-thiophen-2-yl-1H-pyrazol-5-yl)methyl]-2,4-dioxo-3,4-dihydrochinazolin-1(2H)-yl}piperidin-1-carbaldehyd
Nr. 270: 4-{6-[2-Fluor-1-(fluormethyl)ethoxy]-3-[4-(5-methyl-1,2,4-oxadiazol-3-yl)benzyl]-2,4-dioxo-3,4-dihydrochinazolin-1(2H)-yl}piperidin-1-carbaldehyd
Nr. 275: 4-{6-[2-Fluor-1-(fluormethyl)ethoxy]-2,4-dioxo-3-(4-pyrimidin-5-ylbenzyl)-3,4-dihydrochinazolin-1(2H)-yl}piperidin-1-carbaldehyd
Nr. 276: 4-{6-[2-Fluor-1-(fluormethyl)ethoxy]-3-[(1-methyl-3-phenyl-1H-pyrazol-5-yl)methyl]-2,4-dioxo-3,4-dihydrochinazolin-1(2H)-yl}piperidin-1-carbaldehyd
Nr. 278: 4-{6-[2-Fluor-1-(fluormethyl)ethoxy]-2,4-dioxo-3-{[6-(1H-pyrazol-1-yl)pyridin-3-yl]methyl}-3,4-dihydrochinazolin-1(2H)-yl}piperidin-1-carbaldehyd
Nr. 279: 4-{6-[2-Fluor-1-(fluormethyl)ethoxy]-2,4-dioxo-3-[(2-thiophen-2-ylpyrimidin-5-yl)methyl]-3,4-dihydrochinazolin-1(2H)-yl}piperidin-1-carbaldehyd
Nr. 280: 4-{6-[2-Fluor-1-(fluormethyl)ethoxy]-3-[4-(1-methyl-1H-pyrazol-3-yl)benzyl]-2,4-dioxo-3,4-dihydrochinazolin-1(2H)-yl}piperidin-1-carbaldehyd
Nr. 282: 4-{6-[2-Fluor-1-(fluormethyl)ethoxy]-3-[4-(3-methyl-1,2,4-oxadiazol-5-yl)benzyl]-2,4-dioxo-3,4-dihydrochinazolin-1(2H)-yl}piperidin-1-carbaldehyd
Nr. 283: [2-(Cyclopentyloxy)-5-({6-[2-fluor-1-(fluormethyl)ethoxy]-1-(1-formylpiperidin-4-yl)-2,4-dioxo-1,4-dihydrochinazolin-3(2H)-yl}methyl)phenoxy]essigsäure
Nr. 285: 4-{6-[2-Fluor-1-(fluormethyl)ethoxy]-2,4-dioxo-3-(thieno[2,3-b]pyridin-2-ylmethyl)-3,4-dihydrochinazolin-1(2H)-yl}piperidin-1-carbaldehyd Nr. 286: 4-{6-[2-Fluor-1-(fluormethyl)ethoxy]-2,4-dioxo-3-[(6-phenylpyridin-3-yl)methyl]-3,4-dihydrochinazolin-1(2H)-yl}piperidin-1-carbaldehyd
Nr. 287: 4-{6-[2-Fluor-1-(fluormethyl)ethoxy]-3-[(6-morpholin-4-ylpyridin-3-yl)methyl]-2,4-dioxo-3,4-dihydrochinazolin-1(2H)-yl}piperidin-1-carbaldehyd
Nr. 289: 4-{6-[2-Fluor-1-(fluormethyl)ethoxy]-2,4-dioxo-3-[(6-thiophen-2-ylpyridin-3-yl)methyl]-3,4-dihydrochinazolin-1(2H)-yl}piperidin-1-carbaldehyd
Nr. 292: 4-{6-[2-Fluor-1-(fluormethyl)ethoxy]-3-[(1-methyl-5-phenyl-1H-pyrazol-3-yl)methyl]-2,4-dioxo-3,4-dihydrochinazolin-1(2H)-yl}piperidin-1-carbaldehyd
Nr. 294: 4-({6-[2-Fluor-1-(fluormethyl)ethoxy]-1-(1-formylpiperidin-4-yl)-2,4-dioxo-1,4-dihydrochinazolin-3(2H)-yl}methyl)biphenyl-2-carbonitril
Nr. 295: (2R)-2-[2-(Cyclopentyloxy)-5-({6-[2-fluor-1-(fluormethyl)ethoxy]-1-(1-formylpiperidin-4-yl)-2,4-dioxo-1,4-dihydrochinazolin-3(2H)-yl}methyl)phenoxy]-N-methylpropanamid
Nr. 297: 4-{7-Fluor-6-[2-fluor-1-(fluormethyl)ethoxy]-2,4-dioxo-3-(4-thiophen-2-ylbenzyl)-3,4-dihydrochinazolin-1(2H)-yl}piperidin-1-carbaldehyd
Nr. 298: 4-{6-[2-Fluor-1-(fluormethyl)ethoxy]-3-[3-methoxy-4-(morpholin-4-ylmethyl)benzyl]-2,4-dioxo-3,4-dihydrochinazolin-1(2H)-yl}piperidin-1-carbaldehyd
Nr. 299: 4-{6-[2-Fluor-1-(fluormethyl)ethoxy]-3-[3-methoxy-4-(piperidin-1-ylmethyl)benzyl]-2,4-dioxo-3,4-dihydrochinazolin-1(2H)-yl}piperidin-1-carbaldehyd
Nr. 300: 4-[3-{4-[(3,4-Dichlorbenzyl)oxy]-3-methoxybenzyl}-7-fluor-6-[2-fluor-1-(fluormethyl)ethoxy]-2,4-dioxo-3,4-dihydrochinazolin-1(2H)-yl]piperidin-1-carbaldehyd
Nr. 301: 2-[2-(Cyclopentyloxy)-5-({6-[2-fluor-1-(fluormethyl)ethoxy]-1-(1-formylpiperidin-4-yl)-2,4-dioxo-1,4-dihydrochinazolin-3(2H)-yl}methyl)phenoxy]-N-ethylacetamid
Nr. 302: (2S)-2-[2-(Cyclopentyloxy)-5-({6-[2-fluor-1-(fluormethyl)ethoxy]-1-(1-formylpiperidin-4-yl)-2,4-dioxo-1,4-dihydrochinazolin-3(2H)-yl}methyl)phenoxy]propansäure
Nr. 305: 4-{6-[2-Fluor-1-(fluormethyl)ethoxy]-3-(3-methoxy-4-{[(3R)-2-oxo-1-phenylpyrrolidin-3-yl]oxy}benzyl)-2,4-dioxo-3,4-dihydrochinazolin-1(2H)-yl}piperidin-1-carbaldehyd
Nr. 306: 4-{3-[4-(Cyclobutylmethoxy)-3-methoxybenzyl]-6-[2-fluor-1-(fluormethyl)ethoxy]-2,4-dioxo-3,4-dihydrochinazolin-1(2H)-yl}piperidin-1-carbaldehyd
Nr. 307: 4-{3-[4-(Benzyloxy)-3-methoxybenzyl]-7-fluor-6-[2-fluor-1-(fluormethyl)ethoxy]-2,4-dioxo-3,4-dihydrochinazolin-1(2H)-yl}piperidin-1-carbaldehyd
Nr. 308: 4-{7-Fluor-6-[2-fluor-1-(fluormethyl)ethoxy]-3-(4-hydroxy-3-methoxybenzyl)-2,4-dioxo-3,4-dihydrochinazolin-1(2H)-yl}piperidin-1-carbaldehyd
Nr. 309: 4-{3-[4-(Cyclopropylmethoxy)-3-methoxybenzyl]-7-fluor-6-[2-fluor-1-(fluormethyl)ethoxy]-2,4-dioxo-3,4-dihydrochinazolin-1(2H)-yl}piperidin-1-carbaldehyd
Nr. 310: 4-{7-Fluor-6-[2-fluor-1-(fluormethyl)ethoxy]-3-[3-methoxy-4-(2-methylpropoxy)benzyl]-2,4-dioxo-3,4-dihydrochinazolin-1(2H)-yl}piperidin-1-carbaldehyd
Nr. 311: 4-{7-Fluor-6-[2-fluor-1-(fluormethyl)ethoxy]-3-[3-methoxy-4-(1-methylethoxy)benzyl]-2,4-dioxo-3,4-dihydrochinazolin-1(2H)-yl}piperidin-1-carbaldehyd
Nr. 312: 4-[3-(4-Ethoxy-3-methoxybenzyl)-7-fluor-6-[2-fluor-1-(fluormethyl)ethoxy]-2,4-dioxo-3,4-dihydrochinazolin-1(2H)-yl]piperidin-1-carbaldehyd
Nr. 315: 4-{7-Fluor-6-[2-fluor-1-(fluormethyl)ethoxy]-3-{[6-(3-methoxyphenyl)pyridin-3-yl]methyl}-2,4-dioxo-3,4-dihydrochinazolin-1(2H)-yl}piperidin-1-carbaldehyd
Nr. 316: 4-{7-Fluor-6-[2-fluor-1-(fluormethyl)ethoxy]-3-{[6-(2-fluorphenyl)pyridin-3-yl]methyl}-2,4-dioxo-3,4-dihydrochinazolin-1(2H)-yl}piperidin-1-carbaldehyd
Nr. 317: 4-{7-Fluor-6-[2-fluor-1-(fluormethyl)ethoxy]-3-{[6-(4-fluorphenyl)pyridin-3-yl]methyl}-2,4-dioxo-3,4-dihydrochinazolin-1(2H)-yl}piperidin-1-carbaldehyd
Nr. 318: 4-{7-Fluor-6-[2-fluor-1-(fluormethyl)ethoxy]-3-{[6-(4-methoxyphenyl)pyridin-3-yl]methyl}-2,4-dioxo-3,4-dihydrochinazolin-1(2H)-yl}piperidin-1-carbaldehyd
Nr. 319: 4-{7-Fluor-6-[2-fluor-1-(fluormethyl)ethoxy]-2,4-dioxo-3-[(6-thiophen-2-ylpyridin-3-yl)methyl]-3,4-dihydrochinazolin-1(2H)-yl}piperidin-1-carbaldehyd
Nr. 320: 4-{3-[3-Ethoxy-4-(thiophen-2-ylmethoxy)benzyl]-7-fluor-6-[2-fluor-1-(fluormethyl)ethoxy]-2,4-dioxo-3,4-dihydrochinazolin-1(2H)-yl}piperidin-1-carbaldehyd
Nr. 321: 4-{7-Fluor-6-[2-fluor-1-(fluormethyl)ethoxy]-3-[4-(1-methyl-1H-pyrazol-3-yl)benzyl]-2,4-dioxo-3,4-dihydrochinazolin-1(2H)-yl}piperidin-1-carbaldehyd
Nr. 322: 4-{7-Fluor-6-[2-fluor-1-(fluormethyl)ethoxy]-2,4-dioxo-3-(4-pyrimidin-5-ylbenzyl)-3,4-dihydrochinazolin-1(2H)-yl}piperidin-1-carbaldehyd
Nr. 323: 4-{7-Fluor-6-[2-fluor-1-(fluormethyl)ethoxy]-3-[(1-methyl-3-thiophen-2-yl-1H-pyrazol-5-yl)methyl]-2,4-dioxo-3,4-dihydrochinazolin-1(2H)-yl}piperidin-1-carbaldehyd
Nr. 324: 4-{7-Fluor-6-[2-fluor-1-(fluormethyl)ethoxy]-3-[3-methoxy-4-(2-oxo-2-piperidin-1-ylethoxy)benzyl]-2,4-dioxo-3,4-dihydrochinazolin-1(2H)-yl}piperidin-1-carbaldehyd
Nr. 325: 4-[3-{4-[2-(2,3-Dihydro-1H-indol-1-yl)-2-oxoethoxy]-3-methoxybenzyl}-7-fluor-6-[2-fluor-1-(fluormethyl)ethoxy]-2,4-dioxo-3,4-dihydrochinazolin-1(2H)-yl]piperidin-1-carbaldehyd
Nr. 326: 4-{7-Fluor-6-[2-fluor-1-(fluormethyl)ethoxy]-3-[4-(5-methyl-1,2,4-oxadiazol-3-yl)benzyl]-2,4-dioxo-3,4-dihydrochinazolin-1(2H)-yl}piperidin-1-carbaldehyd
Nr. 327: 4-[3-{4-[(3-Chlorbenzyl)oxy]-3-methoxybenzyl}-7-fluor-6-[2-fluor-1-(fluormethyl)ethoxy]-2,4-dioxo-3,4-dihydrochinazolin-1(2H)-yl]piperidin-1-carbaldehyd
Nr. 328: 4-[3-{[6-(3,5-Dichlorphenyl)pyridin-3-yl]methyl}-6-[2-fluor-1-(fluormethyl)ethoxy]-2,4-dioxo-3,4-dihydrochinazolin-1(2H)-yl]piperidin-1-carbaldehyd
Nr. 329: 4-({7-Fluor-6-[2-fluor-1-(fluormethyl)ethoxy]-1-(1-formylpiperidin-4-yl)-2,4-dioxo-1,4-dihydrochinazolin-3(2H)-yl}methyl)biphenyl-2-carbonitril
Nr. 330: 4-{7-Fluor-6-[2-fluor-1-(fluormethyl)ethoxy]-2,4-dioxo-3-[4-(1H-pyrazol-1-yl)benzyl]-3,4-dihydrochinazolin-1(2H)-yl}piperidin-1-carbaldehyd
Nr. 331: 4-{7-Fluor-6-[2-fluor-1-(fluormethyl)ethoxy]-3-{[6-(3-fluorphenyl)pyridin-3-yl]methyl}-2,4-dioxo-3,4-dihydrochinazolin-1(2H)-yl}piperidin-1-carbaldehyd
Nr. 332: 3-[5-({7-Fluor-6-[2-fluor-1-(fluormethyl)ethoxy]-1-(1-formylpiperidin-4-yl)-2,4-dioxo-1,4-dihydrochinazolin-3(2H)-yl}methyl)pyridin-2-yl]benzonitril
Nr. 333: 4-[3-(3,4-Diethoxybenzyl)-7-fluor-6-[2-fluor-1-(fluormethyl)ethoxy]-2,4-dioxo-3,4-dihydrochinazolin-1(2H)-yl]piperidin-1-carbaldehyd
Nr. 334: 4-[3-{4-[(4-Chlorbenzyl)oxy]-3-methoxybenzyl}-7-fluor-6-[2-fluor-1-(fluormethyl)ethoxy]-2,4-dioxo-3,4-dihydrochinazolin-1(2H)-yl]piperidin-1-carbaldehyd
Nr. 335: 4-{7-Fluor-6-[2-fluor-1-(fluormethyl)ethoxy]-3-[4-(morpholin-4-ylmethyl)benzyl]-2,4-dioxo-3,4-dihydrochinazolin-1(2H)-yl}piperidin-1-carbaldehyd
Nr. 336: 4-{7-Fluor-6-[2-fluor-1-(fluormethyl)ethoxy]-2,4-dioxo-3-{[6-(1H-pyrazol-1-yl)pyridin-3-yl]methyl}-3,4-dihydrochinazolin-1(2H)-yl}piperidin-1-carbaldehyd
Nr. 337: 4-{7-Fluor-6-[2-fluor-1-(fluormethyl)ethoxy]-3-(4-morpholin-4-ylbenzyl)-2,4-dioxo-3,4-dihydrochinazolin-1(2H)-yl}piperidin-1-carbaldehyd
Nr. 338: 4-{7-Fluor-6-[2-fluor-1-(fluormethyl)ethoxy]-3-(3-methoxy-4-propoxybenzyl)-2,4-dioxo-3,4-dihydrochinazolin-1(2H)-yl}piperidin-1-carbaldehyd
Nr. 339: 4-{3-[4-(1H-Benzimidazol-1-yl)benzyl]-7-fluor-6-[2-fluor-1-(fluormethyl)ethoxy]-2,4-dioxo-3,4-dihydrochinazolin-1(2H)-yl}piperidin-1-carbaldehyd
Nr. 340: 5-({7-Fluor-6-[2-fluor-1-(fluormethyl)ethoxy]-1-(1-formylpiperidin-4-yl)-2,4-dioxo-1,4-dihydrochinazolin-3(2H)-yl}methyl)-2-methoxybenzonitril
Nr. 341: 3-(3,4-Dimethoxybenzyl)-6-[2-fluor-1-(fluormethyl)ethoxy]-1-(1-formylpiperidin-4-yl)-2,4-dioxo-1,2,3,4-tetrahydrochinazolin-7-carbonitril
Nr. 342: 4-[3-(4-Brombenzyl)-7-fluor-6-[2-fluor-1-(fluormethyl)ethoxy]-2,4-dioxo-3,4-dihydrochinazolin-1(2H)-yl]piperidin-1-carbaldehyd
Nr. 343: 4-[3-{4-[(3,4-Dichlorbenzyl)oxy]-3-(2-methoxyethoxy)benzyl}-7-fluor-6-[2-fluor-1-(fluormethyl)ethoxy]-2,4-dioxo-3,4-dihydrochinazolin-1(2H)-yl]piperidin-1-carbaldehyd
Nr. 344: 4-{3-[4-(Benzyloxy)benzyl]-7-fluor-6-[2-fluor-1-(fluormethyl)ethoxy]-2,4-dioxo-3,4-dihydrochinazolin-1(2H)-yl}piperidin-1-carbaldehyd
Nr. 345: 4-[3-{4-[(3,4-Dichlorbenzyl)oxy]-3-ethoxybenzyl}-7-fluor-6-[2-fluor-1-(fluormethyl)ethoxy]-2,4-dioxo-3,4-dihydrochinazolin-1(2H)-yl]piperidin-1-carbaldehyd
Nr. 349: 4-[3-{4-[(3,4-Dichlorbenzyl)oxy]-3-(2-fluorethoxy)benzyl}-7-fluor-6-[2-fluor-1-(fluormethyl)ethoxy]-2,4-dioxo-3,4-dihydrochinazolin-1(2H)-yl]piperidin-1-carbaldehyd
Nr. 350: 4-[3-{4-[(2-Chlor-4-fluorbenzyl)oxy]-3-methoxybenzyl}-7-fluor-6-[2-fluor-1-(fluormethyl)ethoxy]-2,4-dioxo-3,4-dihydrochinazolin-1(2H)-yl]piperidin-1-carbaldehyd
Nr. 351: 4-[3-{4-[(2,4-Dichlorbenzyl)oxy]-3-methoxybenzyl}-7-fluor-6-[2-fluor-1-(fluormethyl)ethoxy]-2,4-dioxo-3,4-dihydrochinazolin-1(2H)-yl]piperidin-1-carbaldehyd
Nr. 352: 4-[3-{4-[(2-Chlor-6-fluorbenzyl)oxy]-3-methoxybenzyl}-7-fluor-6-[2-fluor-1-(fluormethyl)ethoxy]-2,4-dioxo-3,4-dihydrochinazolin-1(2H)-yl]piperidin-1-carbaldehyd
Nr. 353: 4-[3-{4-[(2,6-Dichlorbenzyl)oxy]-3-methoxybenzyl}-7-fluor-6-[2-fluor-1-(fluormethyl)ethoxy]-2,4-dioxo-3,4-dihydrochinazolin-1(2H)-yl]piperidin-1-carbaldehyd
Nr. 354: 4-[3-{4-[(2-Chlorbenzyl)oxy]-3-methoxybenzyl}-7-fluor-6-[2-fluor-1-(fluormethyl)ethoxy]-2,4-dioxo-3,4-dihydrochinazolin-1(2H)-yl]piperidin-1-carbaldehyd
Nr. 355: 4-[7-Fluor-3-{4-[(2-fluorbenzyl)oxy]-3-methoxybenzyl}-6-[2-fluor-1-(fluormethyl)ethoxy]-2,4-dioxo-3,4-dihydrochinazolin-1(2H)-yl]piperidin-1-carbaldehyd
Nr. 357: 2-[(3,4-Dichlorbenzyl)oxy]-5-({7-fluor-6-[2-fluor-1-(fluormethyl)ethoxyl-1-(1-formylpiperidin-4-yl)-2,4-dioxo-1,4-dihydrochinazolin-3(2H)-yl}methyl)benzonitril
Nr. 358: 4-[3-{4-[(3,4-Dichlorphenoxy)methyl]-3-methoxybenzyl}-7-fluor-6-[2-fluor-1-(fluormethyl)ethoxy]-2,4-dioxo-3,4-dihydrochinazolin-1(2H)-yl]piperidin-1-carbaldehyd
Nr. 360: 4-{7-Fluor-6-[2-fluor-1-(fluormethyl)ethoxy]-2,4-dioxo-3-[4-(2-phenylethyl)benzyl]-3,4-dihydrochinazolin-1(2H)-yl}piperidin-1-carbaldehyd
Nr. 362: 4-[3-{4-[(4,5-Dichlor-2-fluorbenzyl)oxy]-3-methoxybenzyl}-7-fluor-6-[2-fluor-1-(fluormethyl)ethoxy]-2,4-dioxo-3,4-dihydrochinazolin-1(2H)-yl]piperidin-1-carbaldehyd
Nr. 369: 4-[3-{4-[(4-Chlorphenoxy)methyl]-3-methoxybenzyl}-7-fluor-6-[2-fluor-1-(fluormethyl)ethoxy]-2,4-dioxo-3,4-dihydrochinazolin-1(2H)-yl]piperidin-1-carbaldehyd
Nr. 371: 4-[3-{3-Chlor-4-[(4-chlorbenzyl)oxy]-5-ethoxybenzyl}-7-fluor-6-[2-fluor-1-(fluormethyl)ethoxy]-2,4-dioxo-3,4-dihydrochinazolin-1(2H)-yl]piperidin-1-carbaldehyd
Nr. 373: 4-[3-{3-Chlor-4-[(2,4-dichlorbenzyl)oxy]-5-ethoxybenzyl}-7-fluor-6-[2-fluor-1-(fluormethyl)ethoxy]-2,4-dioxo-3,4-dihydrochinazolin-1(2H)-yl]piperidin-1-carbaldehyd
Nr. 375: 4-[7-Fluor-3-{4-[(4-fluorbenzyl)oxy]-3-methoxybenzyl}-6-[2-fluor-1-(fluormethyl)ethoxy]-2,4-dioxo-3,4-dihydrochinazolin-1(2H)-yl]piperidin-1-carbaldehyd
Nr. 376: 4-[3-{4-[(3,5-Dichlorbenzyl)oxy]-3-methoxybenzyl}-7-fluor-6-[2-fluor-1-(fluormethyl)ethoxy]-2,4-dioxo-3,4-dihydrochinazolin-1(2H)-yl]piperidin-1-carbaldehyd
Nr. 377: 4-[3-(4-{[4-Chlor-3-(trifluormethyl)benzyl]oxy}-3-methoxybenzyl)-7-fluor-6-[2-fluor-1-(fluormethyl)ethoxy]-2,4-dioxo-3,4-dihydrochinazolin-1(2H)-yl]piperidin-1-carbaldehyd
Nr. 379: 4-[3-{4-[(3-Chlorphenoxy)methyl]-3-methoxybenzyl}-7-fluor-6-[2-fluor-1-(fluormethyl)ethoxy]-2,4-dioxo-3,4-dihydrochinazolin-1(2H)-yl]piperidin-1-carbaldehyd
Nr. 380: 4-[3-{4-[(3,5-Difluorbenzyl)oxy]-3-methoxybenzyl}-7-fluor-6-[2-fluor-1-(fluormethyl)ethoxy]-2,4-dioxo-3,4-dihydrochinazolin-1(2H)-yl]piperidin-1-carbaldehyd
Nr. 381: 4-{3-[4-(Benzyloxy)-3-methoxybenzyl]-7-fluor-6-[2-fluor-1-(fluormethyl)ethoxy]-2,4-dioxo-3,4-dihydrochinazolin-1(2H)-yl}piperidin-1-carbaldehyd
Nr. 382: 4-[3-{4-[(3-Chlor-5-fluorbenzyl)oxy]-3-methoxybenzyl}-7-fluor-6-[2-fluor-1-(fluormethyl)ethoxy]-2,4-dioxo-3,4-dihydrochinazolin-1(2H)-yl]piperidin-1-carbaldehyd
Nr. 383: 4-{7-Fluor-6-[2-fluor-1-(fluormethyl)ethoxy]-3-(3-methoxy-4-{[4-(trifluormethyl)benzyl]oxy}benzyl)-2,4-dioxo-3,4-dihydrochinazolin-1(2H)-yl}piperidin-1-carbaldehyd
Nr. 384: 4-[3-{4-[(2,5-Dichlorbenzyl)oxy]-3-methoxybenzyl}-7-fluor-6-[2-fluor-1-(fluormethyl)ethoxy]-2,4-dioxo-3,4-dihydrochinazolin-1(2H)-yl]piperidin-1-carbaldehyd
Nr. 385: 4-{[4-({7-Fluor-6-[2-fluor-1-(fluormethyl)ethoxy]-1-(1-formylpiperidin-4-yl)-2,4-dioxo-1,4-dihydrochinazolin-3(2H)-yl}methyl)-2-methoxyphenoxy]methyl}benzonitril
Nr. 386: 3-{[4-({7-Fluor-6-[2-fluor-1-(fluormethyl)ethoxy]-1-(1-formylpiperidin-4-yl)-2,4-dioxo-1,4-dihydrochinazolin-3(2H)-yl}methyl)-2-methoxyphenoxy]methyl}benzonitril
Nr. 387: 4-[3-{4-[(4-Chlor-2-fluorbenzyl)oxy]-3-methoxybenzyl}-7-fluor-6-[2-fluor-1-(fluormethyl)ethoxy]-2,4-dioxo-3,4-dihydrochinazolin-1(2H)-yl]piperidin-1-carbaldehyd
Nr. 388: 4-[3-{4-[1-(3,4-Dichlorphenyl)ethoxy]-3-methoxybenzyl}-7-fluor-6-[2-fluor-1-(fluormethyl)ethoxy]-2,4-dioxo-3,4-dihydrochinazolin-1(2H)-yl]piperidin-1-carbaldehyd
Nr. 389: 4-{7-Fluor-6-[2-fluor-1-(fluormethyl)ethoxy]-3-{4-[(3-hydroxybenzyl)oxy]-3-methoxybenzyl}-2,4-dioxo-3,4-dihydrochinazolin-1(2H)-yl}piperidin-1-carbaldehyd
Nr. 390: 4-[7-Fluor-3-{4-[(3-fluorbenzyl)oxy]-3-methoxybenzyl}-6-[2-fluor-1-(fluormethyl)ethoxy]-2,4-dioxo-3,4-dihydrochinazolin-1(2H)-yl]piperidin-1-carbaldehyd
Nr. 391: 4-[3-{4-[(3,4-Difluorbenzyl)oxy]-3-methoxybenzyl}-7-fluor-6-[2-fluor-1-(fluormethyl)ethoxy]-2,4-dioxo-3,4-dihydrochinazolin-1(2H)-yl]piperidin-1-carbaldehyd
Nr. 392: 4-{3-[4-(5,6-Dichlor-1H-benzimidazol-1-yl)-3-methoxybenzyl]-7-fluor-6-[2-fluor-1-(fluormethyl)ethoxy]-2,4-dioxo-3,4-dihydrochinazolin-1(2H)-yl}piperidin-1-carbaldehyd
Nr. 393: 4-({7-Fluor-6-[2-fluor-1-(fluormethyl)ethoxy]-1-(1-formylpiperidin-4-yl)-2,4-dioxo-1,4-dihydrochinazolin-3(2H)-yl}methyl)phenyl-3,4-dichlorbenzensulfonat
Nr. 394: 4-({7-Fluor-6-[2-fluor-1-(fluormethyl)ethoxy]-1-(1-formylpiperidin-4-yl)-2,4-dioxo-1,4-dihydrochinazolin-3(2H)-yl}methyl)-2-methoxyphenyl-3,4-dichlorbenzensulfonat
Nr. 403: 3,4-Dichlor-N-[4-({7-fluor-6-[2-fluor-1-(fluormethyl)ethoxy]-1-(1-formylpiperidin-4-yl)-2,4-dioxo-1,4-dihydrochinazolin-3(2H)-yl}methyl)-2-methoxyphenyl]benzamid;
in Form einer Base oder eines Säureadditionssalzes.

## Claims

1. Compound corresponding to the general formula (I) 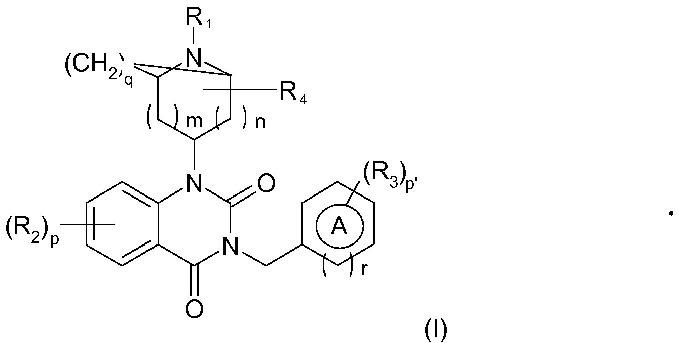 in which
- A represents an aryl group or a heteroaryl group;
- R₁ represents:
▪ a hydrogen atom,
▪ -C(O)R in which R is a hydrogen atom, a (C₁-C₆) alkoxy group, an aryl group, a (C₃-C₆) cycloalkyl group or a (C₁-C₆) alkyl group, the said alkyl optionally being substituted by:
• one or more hydroxyl group(s),
• a benzyloxy group,
• a (C₁-C₆) alkoxy group, optionally substituted by an aryl, or
• a (C₃-C₆) cycloalkyl group,
▪ an optionally substituted (C₁-C₆) alkyl group;
- R₂ represents:
▪ a hydrogen atom,
▪ a halogen atom,
▪ a cyano group,
▪ a nitro group,
▪ a (C₁-C₆) alkyl group optionally substituted by an - NH₂ or else by an -NHC(O)Rb group, with Rb as defined below,
▪ an -ORa group in which Ra represents:
▪ a hydrogen atom,
▪ a (C₁-C₆) alkyl group optionally substituted by one or more halogen atom(s), by one or more hydroxyl group(s), by an aryl group and/or by one or more cyano group(s),
• a (C₂-C₆) alkynyl group,
• an aryl group;
- R₃ represents:
▪ a hydrogen atom,
▪ a halogen atom,
▪ a hydroxyl group,
▪ a cyano group,
▪ an -SCF₃ group,
▪ a nitro group,
▪ an -S(O)₀₋₂-alkyl group, an -S(O)₀₋₂-heterocycloalkyl group, an -O-SO₂-aryl group optionally substituted by one or more halogen atom(s);
▪ an -alkylaminoalkyl or -cycloalkylaminoalkyl group, each optionally substituted on the end alkyl,
▪ an optionally substituted sulphonamide group,
▪ an aryl group or a heteroaryl group, the said group being monocyclic or polycyclic and in addition optionally being substituted by a (C₁-C₆) alkyl group, by one or more halogen atom(s) or by a (C₁-C₆) alkoxy group,
▪ a heterocycloalkyl group optionally substituted by a (C₁-C₆) alkyl group,
▪ a (C₁-C₆) alkyl group optionally substituted by:
- one or more halogen atom(s),
- an aryl group which can be substituted by one or more halogen atom(s) or by one or more hydroxyl group(s),
- a heteroaryl group,
- one or more hydroxyl group(s) which can be substituted by an aryl group itself optionally substituted by one or more halogen atom(s), or
- a heterocycloalkyl group optionally substituted by a CO(O)Ra group or by a (C₁-C₆) alkyl group, with Ra as defined above,
▪ a -C(O)NRbRc group, with Rb and Rc as defined below,
▪ a -C(O)ORc group or an -O-C(O)ORc group, with Rc as defined below,
▪ a (C₁-C₆) alkoxy group, optionally substituted by
- an aminoalkyl group,
- an aminocycloalkyl group,
- a cycloalkyl group,
- a heterocycloalkyl group,
- a monocyclic or polycyclic heteroaryl group,
- one or more hydroxyl group(s),
- one or more halogen atom(s),
- a (C₁-C₆) alkoxy group,
- a -C(O)ORc group, with Rc as defined below,
- a -C(0)NRbRc group, with Rb and Rc as defined below, and/or
- an aryl group, itself optionally substituted by one or more halogen atom(s), a cyano group, a (C₁-C₆) alkoxy group, an -O-haloalkyl group and/or a haloalkyl group,
▪ an -O-cycloalkyl group, an -O-aryl group or an -0-heterocycloalkyl group, each optionally substituted by
- an aryl group, itself optionally substituted by one or more halogen atom(s) or by a (C₁-C₆) alkyl group,
- one or more halogen atom(s), and/or
- a (C₁-C₆) alkyl group, which can itself be substituted by an aryl group,
▪ an -NH-CO-NH-aryl group, an -NH-CO-NH-heteroaryl group or an -NH-CO-NH-(C₁-C₆) alkyl group, each optionally being substituted by one or more halogen atom(s), by a cyano group, by a nitro group, by one or more hydroxyl group(s) or by a (C₁-C₆) alkoxy group,
▪ an -N-(C₁-C₆) alkyl group, it being possible for the (C₁-C₆) alkyl group to be substituted by
one or more aryl group(s) optionally substituted by one or more halogen atom(s) and/or by an SO₂ group,
▪ an -NH-CO-aryl group or an -NH-CO-heteroaryl group, each optionally being substituted by one or more halogen atom(s);
or else R₃ forms, with A, a polycyclic heteroaryl group optionally substituted by a (C₁-C₆) alkoxy group or a (C₁-C₆) alkyl group optionally substituted by an aryl group which can itself be substituted by one or more halogen atom(s);
- R₄ represents a hydrogen atom or a (C₁-C₆) alkyl group;
- Rb represents:
• a hydrogen atom,
• a (C₁-C₆) alkyl group optionally substituted by one or more halogen atom(s), by one or more hydroxyl, cyano, amino, heterocycloalkyl or (C₁-C₆) alkoxy group(s) or by an aryl group optionally substituted by one or more halogen atom(s),
• a (C₃-C₆) cycloalkyl group,
• a (C₂-C₆) alkynyl group,
• a (C₁-C₆) alkoxy group,
• an aryl group optionally substituted by one or more halogen atom(s);
- Rc represents a hydrogen atom or a (C₁-C₆) alkyl group optionally substituted by one or more halogen atom(s);
or then Rb and Rc form, together with the nitrogen atom to which they are attached, a polycyclic heteroaryl group or a heterocycloalkyl group;
- m and n represent, independently of one another, the value 0, 1 or 2, it being understood that m+n≤3;
- p and p' represent, independently of one another, the value 1, 2 or 3, it being understood that, when p is greater than or equal to 2, then the R₂ groups are on separate carbon atoms and can be different from one another and, when p' is greater than or equal to 2, then the R₃ groups are on separate carbon atoms and can be different from one another;
- q represents the value 0 or 2, it being understood that, when q = 0, then the nitrogenous heterocyclic group attached to the nitrogen situated in the 1 position of the 2,4-dioxo-1,2,3,4-tetrahydroquinazoline ring system is no longer bridged and is of the type: 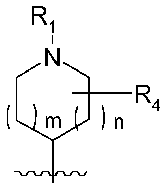
with R₁, R₄, m and n as defined above,
- r represents the value 0 or 1;
in the form of bases or of addition salts with acids;
for the treatment of at least one cardiovascular disease and/or for preventing the appearance of at least one cardiovascular disease.

2. Compound for the use according to the preceding claim, **characterized in that** the cardiovascular disease is chosen from (i) coronary diseases, (ii) diseases of the cardiac muscle, (iii) diseases of the heart valves, (iv) diseases of the pericardium, (v) diseases of the heart rhythm and diseases of cardiac conduction, and (vi) diseases of the vessels.

3. Compound for the use according to either one of Claims 1 and 2, **characterized in that** the cardiovascular disease is chosen from myocardial infarction, in particular contractile cardiac dysfunction resulting from a myocardial infarction, diseases associated with reperfusion injuries of the cardiac and/or skeletal muscle, pulmonary hypertension, hepatic fibrosis, post-angioplasty arterial restenosis, with or without fitting a stent, atherosclerosis and its complications, cardiac insufficiency, dilated cardiopathy and myocarditis of viral and/or bacterial origin.

4. Compound for the use according to any one of the preceding claims, **characterized in that** A represents a phenyl group or a pyridyl group.

5. Compound for the use according to any one of the preceding claims, **characterized in that** q = 0, and m and n each represents 1.

6. Compound for the use according to any one of the preceding claims, **characterized in that** R₂ represents a (C₁-C₆) alkyl group, in particular a methyl substituted by an -NH-CO-Rb group, Rb being as defined in Claims 1 or 2.

7. Compound for the use according to any one of Claims 1 to 5, **characterized in that** R₂ represents an -ORa group, Ra being as defined in Claims 1 or 2.

8. Compound for the use according to any one of Claims 1 to 5, **characterized in that** R₂ is a halogen atom or a cyano or a hydrogen or a hydroxyl or a (C₁-C₆) alkyl optionally substituted by an -NH₂ or else by an-NHC(O)Rb group, Rb being as defined in Claims 1 or 2.

9. Compound for the use according to one of Claims 1 to 8, **characterized in that**:
- A is as defined in Claim 4;
- q, m and n are as defined in Claim 5;
- R₂ is as defined in Claim 6, Claim 7 or Claim 8.

10. Compound for the use according to one of Claims 1 to 3, **characterized in that** A is a phenyl, R₁ is a - C(0)R group in which R represents a hydrogen atom, q is equal to 0, n and m each have the value 1 and R₂ represents -ORa, Ra being as defined in Claims 1 or 2.

11. Compound for the use according to one of Claims 1 to 3, **characterized in that** A is a phenyl, R₁ is a - C(0)R group in which R represents a hydrogen atom, q is equal to 0, n and m each have the value 1, p = 1 and R₂ represents a methyl substituted by an -NH-CO-Rb group, Rb being as defined in Claims 1 or 2.

12. Compound for the use according to one of Claims 1 to 3, **characterized in that** A is a phenyl, R₁ is a - C(0)R group in which R represents a hydrogen atom, q is equal to 0, n and m each have the value 1, p is equal to 2, one of the R₂ groups is -ORa, Ra being as defined in Claims 1 or 2, and the other of the R₂ groups is a halogen atom.

13. Compound for the use according to one of Claims 1 to 3, **characterized in that** the R₂ group is in the 6 position of the 2,4-dioxo-1,2,3,4-tetrahydroquinazoline ring system and **in that** there may additionally be an R₂ group, identical to or different from the said R₂ group mentioned above, in the 7 position of the 2,4-dioxo-1,2,3,4-tetrahydroquinazoline ring system, the said compound of formula (I) being in the base, hydrate or solvate form, in the form of isomers or in the form of their mixtures.

14. Compound for the use according to one of Claims 1 to 3, chosen from:
No. 1: 2-{[3-(3,4-dimethoxybenzyl)-1-(1-formylpiperidin-4-yl)-2,4-dioxo-1,2,3,4-tetrahydroquinazolin-6-yl]oxy}propanenitrile
No. 2: 1-(1-acetylpiperidin-4-yl)-3-(3,4-dimethoxybenzyl)-6-hydroxyquinazoline-2,4(1H,3H)-dione
No. 3: {[1-(1-acetylpiperidin-4-yl)-3-(3,4-dimethoxybenzyl)-2,4-dioxo-1,2,3,4-tetrahydroquinazolin-6-yl]oxy}acetonitrile
No. 4: 2-{[1-(1-acetylpiperidin-4-yl)-3-(3,4-dimethoxybenzyl)-2,4-dioxo-1,2,3,4-tetrahydroquinazolin-6-yl]oxy}propanenitrile
No. 5: {[3-(3,4-dimethoxybenzyl)-1-(1-formylpiperidin-4-yl)-2,4-dioxo-1,2,3,4-tetrahydroquinazolin-6-yl]oxy}acetonitrile
No. 6: {[3-(3,4-dimethoxybenzyl)-1-(1-formylpiperidin-4-yl)-2,4-dioxo-1,2,3,4-tetrahydroquinazolin-6-yl]oxy}acetonitrile
No. 11: 4-[3-(3,4-dimethoxybenzyl)-6-[2-fluoro-1-(fluoromethyl)ethoxy]-2,4-dioxo-3,4-dihydroquinazolin-1(2H)-yl]piperidine-1-carbaldehyde
No. 12: 1-(1-acetylpiperidin-4-yl)-3-(3,4-dimethoxybenzyl)-6-[2-fluoro-1-(fluoromethyl)ethoxy]quinazoline-2,4(1H,3H)-dione
No. 13: 4-[3-(3,4-dimethoxybenzyl)-2,4-dioxo-6-(2,2,2-trifluoroethoxy)-3,4-dihydroquinazolin-1(2H)-yl]piperidine-1-carbaldehyde
No. 14: 1-(1-acetylpiperidin-4-yl)-6-(2,2-difluoroethoxy)-3-(3,4-dimethoxybenzyl)quinazoline-2,4(1H,3H)-dione
No. 16: 4-[6-(2,2-difluoroethoxy)-3-(3,4-dimethoxybenzyl)-2,4-dioxo-3,4-dihydroquinazolin-1(2H)-yl]piperidine-1-carbaldehyde
No. 20: N-{[3-(3,4-dimethoxybenzyl)-1-(1-formylpiperidin-4-yl)-2,4-dioxo-1,2,3,4-tetrahydroquinazolin-6-yl]methyl}acetamide
No. 22: 1-(1-acetylpiperidin-4-yl)-6-(aminomethyl)-3-(3,4-dimethoxybenzyl)quinazoline-2,4(1H,3H)-dione hydrochloride
No. 23: N-{[3-(3,4-dimethoxybenzyl)-1-(1-formylpiperidin-4-yl)-2,4-dioxo-1,2,3,4-tetrahydroquinazolin-6-yl]methyl}formamide
No. 24: N-{[1-(1-acetylpiperidin-4-yl)-3-(3,4-dimethoxybenzyl)-2,4-dioxo-1,2,3,4-tetrahydroquinazolin-6-yl]methyl}formamide
No. 25: N-{[1-(1-acetylpiperidin-4-yl)-3-(3,4-dimethoxybenzyl)-2,4-dioxo-1,2,3,4-tetrahydroquinazolin-6-yl]methyl}acetamide
No. 32: 4-[6-(2,2-difluoroethoxy)-2,4-dioxo-3,4-dihydroquinazolin-1(2H)-yl]piperidine-1-carbaldehyde
No. 33: 4-[3-(3,4-dichlorobenzyl)-6-(2,2-difluoroethoxy)-2,4-dioxo-3,4-dihydroquinazolin-1(2H)-yl]piperidine-1-carbaldehyde
No. 34: 4-[3-(4-chlorobenzyl)-6-(2,2-difluoroethoxy)-2,4-dioxo-3,4-dihydroquinazolin-1(2H)-yl]piperidine-1-carbaldehyde
No. 35: methyl 4-{[6-(2,2-difluoroethoxy)-1-(1-formylpiperidin-4-yl)-2,4-dioxo-1,4-dihydroquinazolin-3(2H)-yl]methyl}benzoate
No. 36: 4-{[6-(2,2-difluoroethoxy)-1-(1-formylpiperidin-4-yl)-2,4-dioxo-1,4-dihydroquinazolin-3(2H)-yl]methyl}benzoic acid
No. 37: 4-{[6-(2,2-difluoroethoxy)-1-(1-formylpiperidin-4-yl)-2,4-dioxo-1,4-dihydroquinazolin-3(2H)-yl]methyl}-N-(2-methoxyethyl)benzamide
No. 38: 4-[3-(3,4-dimethoxybenzyl)-6-methyl-2,4-dioxo-3,4-dihydroquinazolin-1(2H)-yl]piperidine-1-carbaldehyde
No. 39: 4-[6-(2,2-difluoroethoxy)-3-(3-hydroxy-4-methoxybenzyl)-2,4-dioxo-3,4-dihydroquinazolin-1(2H)-yl]piperidine-1-carbaldehyde
No. 40: 4-[6-(2,2-difluoroethoxy)-3-[3-(2-hydroxyethoxy)-4-methoxybenzyl]-2,4-dioxo-3,4-dihydroquinazolin-1(2H)-yl]piperidine-1-carbaldehyde
No. 41: 4-[6-(2,2-difluoroethoxy)-3-(3-ethoxy-4-methoxybenzyl)-2,4-dioxo-3,4-dihydroquinazolin-1(2H)-yl]piperidine-1-carbaldehyde
No. 42: 4-[6-(2,2-difluoroethoxy)-3-[4-methoxy-3-(2-methoxyethoxy)benzyl]-2,4-dioxo-3,4-dihydroquinazolin-1(2H)-yl]piperidine-1-carbaldehyde
No. 43: 4-[6-(2,2-difluoroethoxy)-3-(3,4-dimethoxybenzyl)-2,4-dioxo-3,4-dihydroquinazolin-1(2H)-yl]azepane-1-carbaldehyde
No. 47: 4-[6-(2,2-difluoroethoxy)-3-[3-(3-hydroxypropoxy)-4-methoxybenzyl]-2,4-dioxo-3,4-dihydroquinazolin-1(2H)-yl]piperidine-1-carbaldehyde
No. 48: 4-[5-chloro-3-(3,4-dimethoxybenzyl)-2,4-dioxo-3,4-dihydroquinazolin-1(2H)-yl]piperidine-1-carbaldehyde
No. 49: 4-{3-[3-(cyclopentyloxy)-4-methoxybenzyl]-6-(2,2-difluoroethoxy)-2,4-dioxo-3,4-dihydroquinazolin-1(2H)-yl}piperidine-1-carbaldehyde
No. 50: 2-(5-{[6-(2,2-difluoroethoxy)-1-(1-formylpiperidin-4-yl)-2,4-dioxo-1,4-dihydroquinazolin-3(2H)-yl]methyl}-2-methoxyphenoxy)acetamide
No. 51: 4-[6-(2,2-difluoroethoxy)-3-(3,4-dimethoxybenzyl)-2,4-dioxo-3,4-dihydroquinazolin-1(2H)-yl]-3-methylpiperidine-1-carbaldehyde
No. 52: 3-[6-(2,2-difluoroethoxy)-3-(3,4-dimethoxybenzyl)-2,4-dioxo-3,4-dihydroquinazolin-1(2H)-yl]-8-azabicyclo[3.2.1]octane-8-carbaldehyde
No. 55: 4-{6-[2-fluoro-1-(fluoromethyl)ethoxy]-2,4-dioxo-3,4-dihydroquinazolin-1(2H)-yl}piperidine-1-carbaldehyde
No. 56: 4-{3-[4-(cyclopentyloxy)-3-methoxybenzyl]-6-[2-fluoro-l-(fluoromethyl)ethoxy]-2,4-dioxo-3,4-dihydroquinazolin-1(2H)-yl}piperidine-1-carbaldehyde
No. 57: 4-[3-(3-chlorobenzyl)-6-[2-fluoro-1-(fluoromethyl)ethoxy]-2,4-dioxo-3,4-dihydroquinazolin-1(2H)-yl]piperidine-1-carbaldehyde
No. 58: 4-[3-(4-chlorobenzyl)-6-[2-fluoro-1-(fluoromethyl)ethoxy]-2,4-dioxo-3,4-dihydroquinazolin-1(2H)-yl]piperidine-1-carbaldehyde
No. 59: 4-{3-[3-(cyclopentyloxy)-4-methoxybenzyl]-6-[2-fluoro-1-(fluoromethyl)ethoxy]-2,4-dioxo-3,4-dihydroquinazolin-1(2H)-yl}piperidine-1-carbaldehyde
No. 72: 4-[3-(3,4-dimethoxybenzyl)-6-(2-hydroxyethoxy)-2,4-dioxo-3,4-dihydroquinazolin-1(2H)-yl]piperidine-1-carbaldehyde
No. 74: 4-[3-(3,4-dichlorobenzyl)-6-[2-fluoro-1-(fluoromethyl)ethoxy]-2,4-dioxo-3,4-dihydroquinazolin-1(2H)-yl]piperidine-1-carbaldehyde
No. 76: 4-{3-[(6-chloropyridin-3-yl)methyl]-6-[2-fluoro-1-(fluoromethyl)ethoxy]-2,4-dioxo-3,4-dihydroquinazolin-1(2H)-yl}piperidine-1-carbaldehyde
No. 78: 4-[3-(3-chloro-4-methoxybenzyl)-6-[2-fluoro-1-(fluoromethyl)ethoxy]-2,4-dioxo-3,4-dihydroquinazolin-1(2H)-yl]piperidine-1-carbaldehyde
No. 79: 4-[3-(3,4-dimethoxybenzyl)-6-(2-fluoroethoxy)-2,4-dioxo-3,4-dihydroquinazolin-1(2H)-yl]piperidine-1-carbaldehyde
No. 89: 2-[5-({6-[2-fluoro-1-(fluoromethyl)ethoxy]-1-(1-formylpiperidin-4-yl)-2,4-dioxo-1,4-dihydroquinazolin-3(2H)-yl}methyl)-2-methoxyphenoxy]acetamide
No. 90: 4-{6-[2-fluoro-1-(fluoromethyl)ethoxy]-3-(3-hydroxy-4-methoxybenzyl)-2,4-dioxo-3,4-dihydroquinazolin-1(2H)-yl}piperidine-1-carbaldehyde
No. 91: 4-[3-(3,4-dimethoxybenzyl)-6-ethoxy-2,4-dioxo-3,4-dihydroquinazolin-1(2H)-yl]piperidine-1-carbaldehyde
No. 97: 4-[5,7-dichloro-3-(3,4-dimethoxybenzyl)-2,4-dioxo-3,4-dihydroquinazolin-1(2H)-yl]piperidine-1-carbaldehyde
No. 102: 4-[7-chloro-3-(3,4-dimethoxybenzyl)-2,4-dioxo-3,4-dihydroquinazolin-1(2H)-yl]piperidine-1-carbaldehyde
No. 108: 4-{6-[2-fluoro-1-(fluoromethyl)ethoxy]-3-(3-fluoro-4-methoxybenzyl)-2,4-dioxo-3,4-dihydroquinazolin-1(2H)-yl}piperidine-1-carbaldehyde
No. 111 : 4-[6-(difluoromethoxy)-3-(3,4-dimethoxybenzyl)-2,4-dioxo-3,4-dihydroquinazolin-1(2H)-yl]piperidine-1-carbaldehyde
No. 112: 4-[3-(3,4-dimethoxybenzyl)-6-(1-methylethoxy)-2,4-dioxo-3,4-dihydroquinazolin-1(2H)-yl]piperidine-1-carbaldehyde
No. 114: 4-{6-[2-fluoro-1-(fluoromethyl)ethoxy]-3-[4-methoxy-3-(1-methylethoxy)benzyl]-2,4-dioxo-3,4-dihydroquinazolin-1(2H)-yl}piperidine-1-carbaldehyde
No. 116: 4-{6-[2-fluoro-1-(fluoromethyl)ethoxy]-3-(3-methoxybenzyl)-2,4-dioxo-3,4-dihydroquinazolin-1(2H)-yl}piperidine-1-carbaldehyde
No. 117: 4-{3-[3,5-bis(trifluoromethyl)benzyl]-6-[2-fluoro-1-(fluoromethyl)ethoxy]-2,4-dioxo-3,4-dihydroquinazolin-1(2H)-yl}piperidine-1-carbaldehyde
No. 118: 4-[3-(3-ethoxybenzyl)-6-[2-fluoro-1-(fluoromethyl)ethoxy]-2,4-dioxo-3,4-dihydroquinazolin-1(2H)-yl]piperidine-1-carbaldehyde
No. 124: 4-{3-[3-chloro-4-(2-methoxyethoxy)benzyl]-6-[2-fluoro-1-(fluoromethyl)ethoxy]-2,4-dioxo-3,4-dihydroquinazolin-1(2H)-yl}piperidine-1-carbaldehyde
No. 130: 4-[3-(3,4-diethoxybenzyl)-6-[2-fluoro-1-(fluoromethyl)ethoxy]-2,4-dioxo-3,4-dihydroquinazolin-1(2H)-yl]piperidine-1-carbaldehyde
No. 131: 4-[3-(4-ethoxy-3-methoxybenzyl)-6-[2-fluoro-1-(fluoromethyl)ethoxy]-2,4-dioxo-3,4-dihydroquinazolin-1(2H)-yl]piperidine-l-carbaldehyde
No. 133: 4-{6-[2-fluoro-1-(fluoromethyl)ethoxy]-3-(4-methoxy-3-methylbenzyl)-2,4-dioxo-3,4-dihydroquinazolin-1(2H)-yl}piperidine-1-carbaldehyde
No. 134: 4-{6-[2-fluoro-1-(fluoromethyl)ethoxy]-2,4-dioxo-3-[4-(trifluoromethyl)benzyl]-3,4-dihydroquinazolin-1(2H)-yl}piperidine-1-carbaldehyde
No. 135: 4-{6-[2-fluoro-1-(fluoromethyl)ethoxy]-2,4-dioxo-3-[4-(trifluoromethyl)benzyl]-3,4-dihydroquinazolin-1(2H)-yl}piperidine-1-carbaldehyde
No. 143: 4-{3-[4-(benzyloxy)-3-methoxybenzyl]-6-[2-fluoro-1-(fluoromethyl)ethoxy]-2,4-dioxo-3,4-dihydroquinazolin-1(2H)-yl}piperidine-1-carbaldehyde
No. 145: 4-{6-[2-fluoro-1-(fluoromethyl)ethoxy]-3-(3-methoxy-4-nitrobenzyl)-2,4-dioxo-3,4-dihydroquinazolin-l(2H)-yl}piperidine-l-carbaldehyde
No. 155: 4-[3-(4-ethoxybenzyl)-6-[2-fluoro-1-(fluoromethyl)ethoxy]-2,4-dioxo-3,4-dihydroquinazolin-1(2H)-yl]piperidine-1-carbaldehyde
No. 158: 4-{6-[2-fluoro-1-(fluoromethyl)ethoxy]-3-[4-(morpholin-4-ylmethyl)benzyl]-2,4-dioxo-3,4-dihydroquinazolin-1(2H)-yl}piperidine-1-carbaldehyde
No. 160: 4-{6-[2-fluoro-1-(fluoromethyl)ethoxy]-3-(4-(morpholin-4-yl)benzyl)-2,4-dioxo-3,4-dihydroquinazolin-1(2H)-yl}piperidine-1-carbaldehyde
No. 165: 4-[3-(biphenyl-4-ylmethyl)-6-[2-fluoro-1-(fluoromethyl)ethoxy]-2,4-dioxo-3,4-dihydroquinazolin-1(2H)-yl]piperidine-1-carbaldehyde
No. 166: 4-{6-[2-fluoro-1-(fluoromethyl)ethoxy]-3-[4-(methylsulphanyl)benzyl]-2,4-dioxo-3,4-dihydroquinazolin-1(2H)-yl}piperidine-1-carbaldehyde
No. 167: 4-{6-[2-fluoro-1-(fluoromethyl)ethoxy]-2,4-dioxo-3-(4-(pyridin-3-yl)benzyl)-3,4-dihydroquinazolin-1(2H)-yl}piperidine-1-carbaldehyde
No. 170: 4-{6-[2-fluoro-1-(fluoromethyl)ethoxy]-3-(3-methoxy-4-methylbenzyl)-2,4-dioxo-3,4-dihydroquinazolin-1(2H)-yl}piperidine-1-carbaldehyde
No. 175: 2-[2-(cyclopentyloxy)-5-({6-[2-fluoro-1-(fluoromethyl)ethoxy]-1-(1-formylpiperidin-4-yl)-2,4-dioxo-1,4-dihydroquinazolin-3(2H)-yl}methyl)phenoxy]acetamide
No. 178: 4-{6-[2-fluoro-1-(fluoromethyl)ethoxy]-3-(3-methoxy-4-propoxybenzyl)-2,4-dioxo-3,4-dihydroquinazolin-l(2H)-yl}piperidine-1-carbaldehyde
No. 183: 2-[2-(cyclopentyloxy)-5-({6-[2-fluoro-1-(fluoromethyl)ethoxy]-1-(1-formylpiperidin-4-yl)-2,4-dioxo-1,4-dihydroquinazolin-3(2H)-yl}methyl)phenoxy]-N-methylacetamide
No. 184: 2-[2-(cyclopentyloxy)-5-({6-[2-fluoro-1-(fluoromethyl)ethoxy]-1-(1-formylpiperidin-4-yl)-2,4-dioxo-1,4-dihydroquinazolin-3(2H)-yl}methyl)phenoxy]-N,N-dimethylacetamide
No. 185: 2-[2-(cyclopentyloxy)-5-({6-[2-fluoro-1-(fluoromethyl)ethoxy]-1-(1-formylpiperidin-4-yl)-2,4-dioxo-1,4-dihydroquinazolin-3(2H)-yl}methyl)phenoxy]-N-methoxy-N-methylacetamide
No. 186: 4-{3-[4-(cyclopentyloxy)-3-ethoxybenzyl]-6-[2-fluoro-1-(fluoromethyl)ethoxy]-2,4-dioxo-3,4-dihydroquinazolin-1(2H)-yl}piperidine-1-carbaldehyde
No. 188: 4-{3-[4-(cyclopentyloxy)-3-(1-methylethoxy)benzyl]-6-[2-fluoro-1-(fluoromethyl)ethoxy]-2,4-dioxo-3,4-dihydroquinazolin-1(2H)-yl}piperidine-1-carbaldehyde
No. 189: 4-{3-[4-(cyclopentyloxy)-3-propoxybenzyl]-6-[2-fluoro-1-(fluoromethyl)ethoxy]-2,4-dioxo-3,4-dihydroquinazolin-1(2H)-yl}piperidine-1-carbaldehyde
No. 190: 4-{3-[4-(cyclopentyloxy)-3-hydroxybenzyl]-6-[2-fluoro-1-(fluoromethyl)ethoxy]-2,4-dioxo-3,4-dihydroquinazolin-1(2H)-yl}piperidine-1-carbaldehyde
No. 193: 4-{3-[4-(difluoromethoxy)-3-methoxybenzyl]-6-[2-fluoro-1-(fluoromethyl)ethoxy]-2,4-dioxo-3,4-dihydroquinazolin-1(2H)-yl}piperidine-1-carbaldehyde
No. 194: 4-{3-[4-(difluoromethoxy)-3-ethoxybenzyl]-6-[2-fluoro-1-(fluoromethyl)ethoxy]-2,4-dioxo-3,4-dihydroquinazolin-1(2H)-yl}piperidine-1-carbaldehyde
No. 200: 4-{6-[2-fluoro-1-(fluoromethyl)ethoxy]-2,4-dioxo-3-(4-(thiophen-3-yl)benzyl)-3,4-dihydroquinazolin-1(2H)-yl}piperidine-1-carbaldehyde
No. 201: 4-{6-[2-fluoro-1-(fluoromethyl)ethoxy]-2,4-dioxo-3-(4-(pyridin-4-yl)benzyl)-3,4-dihydroquinazolin-1(2H)-yl}piperidine-1-carbaldehyde
No. 203: 4-{6-[2-fluoro-1-(fluoromethyl)ethoxy]-3-[(1-methyl-1H-indol-6-yl)methyl]-2,4-dioxo-3,4-dihydroquinazolin-1(2H)-yl}piperidine-1-carbaldehyde
No. 206: 4-{3-[4-(cyclopropylmethoxy)-3-methoxybenzyl]-6-[2-fluoro-1-(fluoromethyl)ethoxy]-2,4-dioxo-3,4-dihydroquinazolin-1(2H)-yl}piperidine-1-carbaldehyde
No. 207: 2-[4-({6-[2-fluoro-1-(fluoromethyl)ethoxy]-1-(1-formylpiperidin-4-yl)-2,4-dioxo-1,4-dihydroquinazolin-3(2H)-yl}methyl)-2-methoxyphenoxy]-N-methylacetamide
No. 212: 4-{6-[2-fluoro-1-(fluoromethyl)ethoxy]-2,4-dioxo-3-[4-(1H-pyrazol-1-yl)benzyl]-3,4-dihydroquinazolin-1(2H)-yl}piperidine-1-carbaldehyde
No. 213: 4-{6-[2-fluoro-1-(fluoromethyl)ethoxy]-2,4-dioxo-3-(4-(pyridin-2-yl)benzyl)-3,4-dihydroquinazolin-1(2H)-yl}piperidine-1-carbaldehyde
No. 215: 4-{6-[2-fluoro-1-(fluoromethyl)ethoxy]-2,4-dioxo-3-(4-(thiophen-2-yl)benzyl)-3,4-dihydroquinazolin-1(2H)-yl}piperidine-1-carbaldehyde
No. 216: 4-{6-[2-fluoro-1-(fluoromethyl)ethoxy]-2,4-dioxo-3-(quinolin-7-ylmethyl)-3,4-dihydroquinazolin-1(2H)-yl}piperidine-1-carbaldehyde
No. 218: 4-{6-[2-fluoro-1-(fluoromethyl)ethoxy]-3-[(6-methoxynaphthalen-2-yl)methyl]-2,4-dioxo-3,4-dihydroquinazolin-1(2H)-yl}piperidine-1-carbaldehyde
No. 223: 4-{3-[4-(1H-benzimidazol-1-yl)benzyl]-6-[2-fluoro-1-(fluoromethyl)ethoxy]-2,4-dioxo-3,4-dihydroquinazolin-1(2H)-yl}piperidine-1-carbaldehyde
No. 224: 4-{6-[2-fluoro-1-(fluoromethyl)ethoxy]-3-[3-methoxy-4-(2-methylpropoxy)benzyl]-2,4-dioxo-3,4-dihydroquinazolin-1(2H)-yl}piperidine-1-carbaldehyde
No. 226: 4-{6-[2-fluoro-1-(fluoromethyl)ethoxy]-3-[3-methoxy-4-(tetrahydrofuran-3-yloxy)benzyl]-2,4-dioxo-3,4-dihydroquinazolin-1(2H)-yl}piperidine-1-carbaldehyde
No. 228: 4-[3-{4-[(1-benzylpyrrolidin-3-yl)oxy]-3-methoxybenzyl}-6-[2-fluoro-1-(fluoromethyl)ethoxy]-2,4-dioxo-3,4-dihydroquinazolin-1(2H)-yl]piperidine-1-carbaldehyde
No. 230: 4-[3-(1-benzothiophen-5-ylmethyl)-6-[2-fluoro-1-(fluoromethyl)ethoxy]-2,4-dioxo-3,4-dihydroquinazolin-1(2H)-yl]piperidine-1-carbaldehyde
No. 232: 4-{6-[2-fluoro-1-(fluoromethyl)ethoxy]-3-[3-methoxy-4-(1-methylethoxy)benzyl]-2,4-dioxo-3,4-dihydroquinazolin-1(2H)-yl}piperidine-1-carbaldehyde
No. 233: 4-[3-(3,4-dimethoxybenzyl)-7-fluoro-6-[2-fluoro-1-(fluoromethyl)ethoxy]-2,4-dioxo-3,4-dihydroquinazolin-1(2H)-yl]piperidine-1-carbaldehyde
No. 234: 4-[3-{4-[(1-acetylpyrrolidin-3-yl)oxy]-3-methoxybenzyl}-6-[2-fluoro-1-(fluoromethyl)ethoxy]-2,4-dioxo-3,4-dihydroquinazolin-1(2H)-yl]piperidine-1-carbaldehyde
No. 239: 4-[3-{4-[(4-fluorobenzyl)oxy]-3-methoxybenzyl}-6-[2-fluoro-1-(fluoromethyl)ethoxy]-2,4-dioxo-3,4-dihydroquinazolin-1(2H)-yl]piperidine-1-carbaldehyde
No. 240: 4-[3-{4-[(4-chlorobenzyl)oxy]-3-methoxybenzyl}-6-[2-fluoro-1-(fluoromethyl)ethoxy]-2,4-dioxo-3,4-dihydroquinazolin-1(2H)-yl]piperidine-1-carbaldehyde
No. 242: 4-[3-{4-[(3-chlorobenzyl)oxy]-3-methoxybenzyl}-6-[2-fluoro-1-(fluoromethyl)ethoxy]-2,4-dioxo-3,4-dihydroquinazolin-1(2H)-yl]piperidine-1-carbaldehyde
No. 243: 4-{6-[2-fluoro-1-(fluoromethyl)ethoxy]-2,4-dioxo-3-(3-(thiophen-3-yl)benzyl)-3,4-dihydroquinazolin-1(2H)-yl}piperidine-1-carbaldehyde
No. 245: 4-[3-(4-ethoxy-3-methoxybenzyl)-6-(2-hydroxyethoxy)-2,4-dioxo-3,4-dihydroquinazolin-1(2H)-yl]piperidine-1-carbaldehyde
No. 246: 4-[3-{4-[2-(2,3-dihydro-1H-indol-1-yl)-2-oxoethoxy]-3-methoxybenzyl}-6-[2-fluoro-1-(fluoromethyl)ethoxy]-2,4-dioxo-3,4-dihydroquinazolin-1(2H)-yl]piperidine-1-carbaldehyde
No. 250: 4-[3-{4-[(3,4-dichlorobenzyl)oxy]-3-methoxybenzyl}-6-[2-fluoro-1-(fluoromethyl)ethoxy]-2,4-dioxo-3,4-dihydroquinazolin-1(2H)-yl]piperidine-1-carbaldehyde
No. 251: 4-{6-[2-fluoro-1-(fluoromethyl)ethoxy]-3-[3-methoxy-4-(2-oxo-2-(piperidin-1-yl)ethoxy)benzyl]-2,4-dioxo-3,4-dihydroquinazolin-1(2H)-yl}piperidine-1-carbaldehyde
No. 254: 4-{3-[3-ethoxy-4-(thiophen-2-ylmethoxy)benzyl]-6-[2-fluoro-1-(fluoromethyl)ethoxy]-2,4-dioxo-3,4-dihydroquinazolin-1(2H)-yl}piperidine-1-carbaldehyde
No. 258: 4-[3-(3,4-dimethoxybenzyl)-6-[2-fluoro-1-(hydroxymethyl)ethoxy]-2,4-dioxo-3,4-dihydroquinazolin-1(2H)-yl]piperidine-1-carbaldehyde
No. 263: (2R)-2-[2-(cyclopentyloxy)-5-({6-[2-fluoro-1-(fluoromethyl)ethoxy]-1-(1-formylpiperidin-4-yl)-2,4-dioxo-1,4-dihydroquinazolin-3(2H)-yl}methyl)phenoxy]propanoic acid
No. 264: 4-{6-[2-fluoro-1-(fluoromethyl)ethoxy]-3-[(1-methyl-3-(thiophen-2-yl)-1H-pyrazol-5-yl)methyl]-2,4-dioxo-3,4-dihydroquinazolin-1(2H)-yl}piperidine-1-carbaldehyde
No. 270: 4-{6-[2-fluoro-1-(fluoromethyl)ethoxy]-3-[4-(5-methyl-1,2,4-oxadiazol-3-yl)benzyl]-2,4-dioxo-3,4-dihydroquinazolin-1(2H)-yl}piperidine-1-carbaldehyde
No. 275: 4-{6-[2-fluoro-1-(fluoromethyl)ethoxy]-2,4-dioxo-3-(4-(pyrimidin-5-yl)benzyl)-3,4-dihydroquinazolin-1(2H)-yl}piperidine-1-carbaldehyde
No. 276: 4-{6-[2-fluoro-1-(fluoromethyl)ethoxy]-3-[(1-methyl-3-phenyl-1H-pyrazol-5-yl)methyl]-2,4-dioxo-3,4-dihydroquinazolin-1(2H)-yl}piperidine-1-carbaldehyde
No. 278: 4-{6-[2-fluoro-1-(fluoromethyl)ethoxy]-2,4-dioxo-3-{[6-(1H-pyrazol-1-yl)pyridin-3-yl]methyl}-3,4-dihydroquinazolin-1(2H)-yl}piperidine-1-carbaldehyde
No. 279: 4-{6-[2-fluoro-1-(fluoromethyl)ethoxy]-2,4-dioxo-3-[(2-(thiophen-2-yl)pyrimidin-5-yl)methyl]-3,4-dihydroquinazolin-1(2H)-yl}piperidine-1-carbaldehyde
No. 280: 4-{6-[2-fluoro-1-(fluoromethyl)ethoxy]-3-[4-(1-methyl-1H-pyrazol-3-yl)benzyl]-2,4-dioxo-3,4-dihydroquinazolin-1(2H)-yl}piperidine-1-carbaldehyde
No. 282: 4-{6-[2-fluoro-1-(fluoromethyl)ethoxy]-3-[4-(3-methyl-1,2,4-oxadiazol-5-yl)benzyl]-2,4-dioxo-3,4-dihydroquinazolin-1(2H)-yl}piperidine-1-carbaldehyde
No. 283: [2-(cyclopentyloxy)-5-({6-[2-fluoro-1-(fluoromethyl)ethoxy]-1-(1-formylpiperidin-4-yl)-2,4-dioxo-1,4-dihydroquinazolin-3(2H)-yl}methyl)phenoxy]acetic acid
No. 285: 4-{6-[2-fluoro-1-(fluoromethyl)ethoxy]-2,4-dioxo-3-(thieno[2,3-b]pyridin-2-ylmethyl)-3,4-dihydroquinazolin-1(2H)-yl}piperidine-1-carbaldehyde
No. 286: 4-{6-[2-fluoro-1-(fluoromethyl)ethoxy]-2,4-dioxo-3-[(6-phenylpyridin-3-yl)methyl]-3,4-dihydroquinazolin-1(2H)-yl}piperidine-1-carbaldehyde
No. 287: 4-{6-[2-fluoro-1-(fluoromethyl)ethoxy]-3-[(6-(morpholin-4-yl)pyridin-3-yl)methyl]-2,4-dioxo-3,4-dihydroquinazolin-1(2H)-yl}piperidine-1-carbaldehyde
No. 289: 4-{6-[2-fluoro-1-(fluoromethyl)ethoxy]-2,4-dioxo-3-[(6-(thiophen-2-yl)pyridin-3-yl)methyl]-3,4-dihydroquinazolin-1(2H)-yl}piperidine-1-carbaldehyde
No. 292: 4-{6-[2-fluoro-1-(fluoromethyl)ethoxy]-3-[(1-methyl-5-phenyl-1H-pyrazol-3-yl)methyl]-2,4-dioxo-3,4-dihydroquinazolin-1(2H)-yl}piperidine-1-carbaldehyde
No. 294: 4-({6-[2-fluoro-1-(fluoromethyl)ethoxy]-1-(1-formylpiperidin-4-yl)-2,4-dioxo-1,4-dihydroquinazolin-3(2H)-yl}methyl)biphenyl-2-carbonitrile
No. 295: (2R)-2-[2-(cyclopentyloxy)-5-({6-[2-fluoro-1-(fluoromethyl)ethoxy]-1-(1-formylpiperidin-4-yl)-2,4-dioxo-1,4-dihydroquinazolin-3(2H)-yl}methyl)phenoxy]-N-methylpropanamide
No. 297: 4-{7-fluoro-6-[2-fluoro-1-(fluoromethyl)ethoxy]-2,4-dioxo-3-(4-(thiophen-2-yl)benzyl)-3,4-dihydroquinazolin-1(2H)-yl}piperidine-1-carbaldehyde
No. 298: 4-{6-[2-fluoro-1-(fluoromethyl)ethoxy]-3-[3-methoxy-4-(morpholin-4-ylmethyl)benzyl]-2,4-dioxo-3,4-dihydroquinazolin-1(2H)-yl}piperidine-1-carbaldehyde
No. 299: 4-{6-[2-fluoro-1-(fluoromethyl)ethoxy]-3-[3-methoxy-4-(piperidin-1-ylmethyl)benzyl]-2,4-dioxo-3,4-dihydroquinazolin-1(2H)-yl}piperidine-1-carbaldehyde
No. 300: 4-[3-{4-[(3,4-dichlorobenzyl)oxy]-3-methoxybenzyl}-7-fluoro-6-[2-fluoro-1-(fluoromethyl)ethoxy]-2,4-dioxo-3,4-dihydroquinazolin-1(2H)-yl]piperidine-1-carbaldehyde
No. 301: 2-[2-(cyclopentyloxy)-5-({6-[2-fluoro-1-(fluoromethyl)ethoxy]-1-(1-formylpiperidin-4-yl)-2,4-dioxo-1,4-dihydroquinazolin-3(2H)-yl}methyl)phenoxy]-N-ethylacetamide
No. 302: (2S)-2-[2-(cyclopentyloxy)-5-({6-[2-fluoro-1-(fluoromethyl)ethoxy]-1-(1-formylpiperidin-4-yl)-2,4-dioxo-1,4-dihydroquinazolin-3(2H)-yl}methyl)phenoxy]propanoic acid
No. 305: 4-{6-[2-fluoro-1-(fluoromethyl)ethoxy]-3-(3-methoxy-4-{[(3R)-2-oxo-1-phenylpyrrolidin-3-yl]oxy}benzyl)-2,4-dioxo-3,4-dihydroquinazolin-1(2H)-yl}piperidine-1-carbaldehyde
No. 306: 4-{3-[4-(cyclobutylmethoxy)-3-methoxybenzyl]-6-[2-fluoro-1-(fluoromethyl)ethoxy]-2,4-dioxo-3,4-dihydroquinazolin-1(2H)-yl}piperidine-1-carbaldehyde
No. 307: 4-{3-[4-(benzyloxy)-3-methoxybenzyl]-7-fluoro-6-[2-fluoro-1-(fluoromethyl)ethoxy]-2,4-dioxo-3,4-dihydroquinazolin-1(2H)-yl}piperidine-1-carbaldehyde
No. 308: 4-{7-fluoro-6-[2-fluoro-1-(fluoromethyl)ethoxy]-3-(4-hydroxy-3-methoxybenzyl)-2,4-dioxo-3,4-dihydroquinazolin-1(2H)-yl}piperidine-1-carbaldehyde
No. 309: 4-{3-[4-(cyclopropylmethoxy)-3-methoxybenzyl]-7-fluoro-6-[2-fluoro-1-(fluoromethyl)ethoxy]-2,4-dioxo-3,4-dihydroquinazolin-1(2H)-yl}piperidine-1-carbaldehyde
No. 310: 4-{7-fluoro-6-[2-fluoro-1-(fluoromethyl)ethoxy]-3-[3-methoxy-4-(2-methylpropoxy)benzyl]-2,4-dioxo-3,4-dihydroquinazolin-1(2H)-yl}piperidine-1-carbaldehyde
No. 311: 4-{7-fluoro-6-[2-fluoro-1-(fluoromethyl)ethoxy]-3-[3-methoxy-4-(1-methylethoxy)benzyl]-2,4-dioxo-3,4-dihydroquinazolin-1(2H)-yl}piperidine-1-carbaldehyde
No. 312: 4-[3-(4-ethoxy-3-methoxybenzyl)-7-fluoro-6-[2-fluoro-1-(fluoromethyl)ethoxy]-2,4-dioxo-3,4-dihydroquinazolin-1(2H)-yl]piperidine-1-carbaldehyde
No. 315: 4-{7-fluoro-6-[2-fluoro-1-(fluoromethyl)ethoxy]-3-{[6-(3-methoxyphenyl)pyridin-3-yl]methyl}-2,4-dioxo-3,4-dihydroquinazolin-l(2H)-yl}piperidine-1-carbaldehyde
No. 316: 4-{7-fluoro-6-[2-fluoro-1-(fluoromethyl)ethoxy]-3-{[6-(2-fluorophenyl)pyridin-3-yl]methyl}-2,4-dioxo-3,4-dihydroquinazolin-1(2H)-yl}piperidine-1-carbaldehyde
No. 317: 4-{7-fluoro-6-[2-fluoro-1-(fluoromethyl)ethoxy]-3-{[6-(4-fluorophenyl)pyridin-3-yl]methyl}-2,4-dioxo-3,4-dihydroquinazolin-1(2H)-yl}piperidine-1-carbaldehyde
No. 318: 4-{7-fluoro-6-[2-fluoro-1-(fluoromethyl)ethoxy]-3-{[6-(4-methoxyphenyl)pyridin-3-yl]methyl}-2,4-dioxo-3,4-dihydroquinazolin-1(2H)-yl}piperidine-1-carbaldehyde
No. 319: 4-{7-fluoro-6-[2-fluoro-1-(fluoromethyl)ethoxy]-2,4-dioxo-3-[(6-(thiophen-2-yl)pyridin-3-yl)methyl]-3,4-dihydroquinazolin-1(2H)-yl}piperidine-1-carbaldehyde
No. 320: 4-{3-[3-ethoxy-4-(thiophen-2-ylmethoxy)benzyl]-7-fluoro-6-[2-fluoro-1-(fluoromethyl)ethoxy]-2,4-dioxo-3,4-dihydroquinazolin-1(2H)-yl}piperidine-1-carbaldehyde
No. 321: 4-{7-fluoro-6-[2-fluoro-1-(fluoromethyl)ethoxy]-3-[4-(1-methyl-1H-pyrazol-3-yl)benzyl]-2,4-dioxo-3,4-dihydroquinazolin-1(2H)-yl}piperidine-1-carbaldehyde
No. 322: 4-{7-fluoro-6-[2-fluoro-1-(fluoromethyl)ethoxy]-2,4-dioxo-3-(4-(pyrimidin-5-yl)benzyl)-3,4-dihydroquinazolin-1(2H)-yl}piperidine-1-carbaldehyde
No. 323: 4-{7-fluoro-6-[2-fluoro-1-(fluoromethyl)ethoxy]-3-[(1-methyl-3-(thiophen-2-yl)-1H-pyrazol-5-yl)methyl]-2,4-dioxo-3,4-dihydroquinazolin-1(2H)-yl}piperidine-1-carbaldehyde
No. 324: 4-{7-fluoro-6-[2-fluoro-1-(fluoromethyl)ethoxy]-3-[3-methoxy-4-(2-oxo-2-(piperidin-1-yl)ethoxy)benzyl]-2,4-dioxo-3,4-dihydroquinazolin-1(2H)-yl}piperidine-1-carbaldehyde
No. 325: 4-[3-{4-[2-(2,3-dihydro-1H-indol-1-yl)-2-oxoethoxy]-3-methoxybenzyl}-7-fluoro-6-[2-fluoro-1-(fluoromethyl)ethoxy]-2,4-dioxo-3,4-dihydroquinazolin-1(2H)-yl]piperidine-1-carbaldehyde
No. 326: 4-{7-fluoro-6-[2-fluoro-1-(fluoromethyl)ethoxy]-3-[4-(5-methyl-1,2,4-oxadiazol-3-yl)benzyl]-2,4-dioxo-3,4-dihydroquinazolin-1(2H)-yl}piperidine-1-carbaldehyde
No. 327: 4-[3-{4-[(3-chlorobenzyl)oxy]-3-methoxybenzyl}-7-fluoro-6-[2-fluoro-1-(fluoromethyl)ethoxy]-2,4-dioxo-3,4-dihydroquinazolin-1(2H)-yl]piperidine-1-carbaldehyde
No. 328: 4-[3-{[6-(3,5-dichlorophenyl)pyridin-3-yl]methyl}-6-[2-fluoro-1-(fluoromethyl)ethoxy]-2,4-dioxo-3,4-dihydroquinazolin-1(2H)-yl]piperidine-1-carbaldehyde
No. 329: 4-({7-fluoro-6-[2-fluoro-1-(fluoromethyl)ethoxy]-1-(1-formylpiperidin-4-yl)-2,4-dioxo-1,4-dihydroquinazolin-3(2H)-yl}methyl)biphenyl-2-carbonitrile
No. 330: 4-{7-fluoro-6-[2-fluoro-1-(fluoromethyl)ethoxy]-2,4-dioxo-3-[4-(1H-pyrazol-1-yl)benzyl]-3,4-dihydroquinazolin-1(2H)-yl}piperidine-1-carbaldehyde
No. 331: 4-{7-fluoro-6-[2-fluoro-1-(fluoromethyl)ethoxy]-3-{[6-(3-fluorophenyl)pyridin-3-yl]methyl}-2,4-dioxo-3,4-dihydroquinazolin-1(2H)-yl}piperidine-1-carbaldehyde
No. 332: 3-[5-({7-fluoro-6-[2-fluoro-1-(fluoromethyl)ethoxy]-1-(1-formylpiperidin-4-yl)-2,4-dioxo-1,4-dihydroquinazolin-3(2H)-yl}methyl)pyridin-2-yl]benzonitrile
No. 333: 4-[3-(3,4-diethoxybenzyl)-7-fluoro-6-[2-fluoro-1-(fluoromethyl)ethoxy]-2,4-dioxo-3,4-dihydroquinazolin-1(2H)-yl]piperidine-1-carbaldehyde
No. 334: 4-[3-{4-[(4-chlorobenzyl)oxy]-3-methoxybenzyl}-7-fluoro-6-[2-fluoro-1-(fluoromethyl)ethoxy]-2,4-dioxo-3,4-dihydroquinazolin-1(2H)-yl]piperidine-1-carbaldehyde
No. 335: 4-{7-fluoro-6-[2-fluoro-1-(fluoromethyl)ethoxy]-3-[4-(morpholin-4-ylmethyl)benzyl]-2,4-dioxo-3,4-dihydroquinazolin-1(2H)-yl}piperidine-1-carbaldehyde
No. 336: 4-{7-fluoro-6-[2-fluoro-1-(fluoromethyl)ethoxy]-2,4-dioxo-3-{[6-(1H-pyrazol-1-yl)pyridin-3-yl]methyl}-3,4-dihydroquinazolin-1(2H)-yl}piperidine-1-carbaldehyde
No. 337: 4-{7-fluoro-6-[2-fluoro-1-(fluoromethyl)ethoxy]-3-(4-(morpholin-4-yl)benzyl)-2,4-dioxo-3,4-dihydroquinazolin-1(2H)-yl}piperidine-1-carbaldehyde
No. 338: 4-{7-fluoro-6-[2-fluoro-1-(fluoromethyl)ethoxy]-3-(3-methoxy-4-propoxybenzyl)-2,4-dioxo-3,4-dihydroquinazolin-1(2H)-yl}piperidine-1-carbaldehyde
No. 339: 4-{3-[4-(1H-benzimidazol-1-yl)benzyl]-7-fluoro-6-[2-fluoro-1-(fluoromethyl)ethoxy]-2,4-dioxo-3,4-dihydroquinazolin-1(2H)-yl}piperidine-1-carbaldehyde
No. 340: 5-({7-fluoro-6-[2-fluoro-1-(fluoromethyl)ethoxy]-1-(1-formylpiperidin-4-yl)-2,4-dioxo-1,4-dihydroquinazolin-3(2H)-yl}methyl)-2-methoxybenzonitrile
No. 341: 3-(3,4-dimethoxybenzyl)-6-[2-fluoro-1-(fluoromethyl)ethoxy]-1-(1-formylpiperidin-4-yl)-2,4-dioxo-1,2,3,4-tetrahydroquinazoline-7-carbonitrile
No. 342: 4-[3-(4-bromobenzyl)-7-fluoro-6-[2-fluoro-1-(fluoromethyl)ethoxy]-2,4-dioxo-3,4-dihydroquinazolin-1(2H)-yl]piperidine-1-carbaldehyde
No. 343: 4-[3-{4-[(3,4-dichlorobenzyl)oxy]-3-(2-methoxyethoxy)benzyl}-7-fluoro-6-[2-fluoro-1-(fluoromethyl)ethoxy]-2,4-dioxo-3,4-dihydroquinazolin-1(2H)-yl]piperidine-1-carbaldehyde
No. 344: 4-{3-[4-(benzyloxy)benzyl]-7-fluoro-6-[2-fluoro-1-(fluoromethyl)ethoxy]-2,4-dioxo-3,4-dihydroquinazolin-1(2H)-yl}piperidine-1-carbaldehyde
No. 345: 4-[3-{4-[(3,4-dichlorobenzyl)oxy]-3-ethoxybenzyl}-7-fluoro-6-[2-fluoro-1-(fluoromethyl)ethoxy]-2,4-dioxo-3,4-dihydroquinazolin-1(2H)-yl]piperidine-1-carbaldehyde
No. 349: 4-[3-{4-[(3,4-dichlorobenzyl)oxy]-3-(2-fluoroethoxy)benzyl}-7-fluoro-6-[2-fluoro-1-(fluoromethyl)ethoxy]-2,4-dioxo-3,4-dihydroquinazolin-1(2H)-yl]piperidine-1-carbaldehyde
No. 350: 4-[3-{4-[(2-chloro-4-fluorobenzyl)oxy]-3-methoxybenzyl}-7-fluoro-6-[2-fluoro-1-(fluoromethyl)ethoxy]-2,4-dioxo-3,4-dihydroquinazolin-1(2H)-yl]piperidine-1-carbaldehyde
No. 351: 4-[3-{4-[(2,4-dichlorobenzyl)oxy]-3-methoxybenzyl}-7-fluoro-6-[2-fluoro-1-(fluoromethyl)ethoxy]-2,4-dioxo-3,4-dihydroquinazolin-1(2H)-yl]piperidine-1-carbaldehyde
No. 352: 4-[3-{4-[(2-chloro-6-fluorobenzyl)oxy]-3-methoxybenzyl}-7-fluoro-6-[2-fluoro-1-(fluoromethyl)ethoxy]-2,4-dioxo-3,4-dihydroquinazolin-1(2H)-yl]piperidine-1-carbaldehyde
No. 353: 4-[3-{4-[(2,6-dichlorobenzyl)oxy]-3-methoxybenzyl}-7-fluoro-6-[2-fluoro-1-(fluoromethyl)ethoxy]-2,4-dioxo-3,4-dihydroquinazolin-1(2H)-yl]piperidine-1-carbaldehyde
No. 354: 4-[3-{4-[(2-chlorobenzyl)oxy]-3-methoxybenzyl}-7-fluoro-6-[2-fluoro-1-(fluoromethyl)ethoxy]-2,4-dioxo-3,4-dihydroquinazolin-1(2H)-yl]piperidine-1-carbaldehyde
No. 355: 4-[7-fluoro-3-{4-[(2-fluorobenzyl)oxy]-3-methoxybenzyl}-6-[2-fluoro-1-(fluoromethyl)ethoxy]-2,4-dioxo-3,4-dihydroquinazolin-1(2H)-yl]piperidine-1-carbaldehyde
No. 357: 2-[(3,4-dichlorobenzyl)oxy]-5-({7-fluoro-6-[2-fluoro-1-(fluoromethyl)ethoxy]-1-(1-formylpiperidin-4-yl)-2,4-dioxo-1,4-dihydroquinazolin-3(2H)-yl}methyl)benzonitrile
No. 358: 4-[3-{4-[(3,4-dichlorophenoxy)methyl]-3-methoxybenzyl}-7-fluoro-6-[2-fluoro-1-(fluoromethyl)ethoxy]-2,4-dioxo-3,4-dihydroquinazolin-1(2H)-yl]piperidine-1-carbaldehyde
No. 360: 4-{7-fluoro-6-[2-fluoro-1-(fluoromethyl)ethoxy]-2,4-dioxo-3-[4-(2-phenylethyl)benzyl]-3,4-dihydroquinazolin-1(2H)-yl}piperidine-1-carbaldehyde
No. 362: 4-[3-{4-[(4,5-dichloro-2-fluorobenzyl)oxy]-3-methoxybenzyl}-7-fluoro-6-[2-fluoro-1-(fluoromethyl)ethoxy]-2,4-dioxo-3,4-dihydroquinazolin-1(2H)-yl]piperidine-l-carbaldehyde
No. 369: 4-[3-{4-[(4-chlorophenoxy)methyl]-3-methoxybenzyl}-7-fluoro-6-[2-fluoro-1-(fluoromethyl)ethoxy]-2,4-dioxo-3,4-dihydroquinazolin-1(2H)-yl]piperidine-1-carbaldehyde
No. 371: 4-[3-{3-chloro-4-[(4-chlorobenzyl)oxy]-5-ethoxybenzyl}-7-fluoro-6-[2-fluoro-1-(fluoromethyl)ethoxy]-2,4-dioxo-3,4-dihydroquinazolin-1(2H)-yl]piperidine-1-carbaldehyde
No. 373: 4-[3-{3-chloro-4-[(2,4-dichlorobenzyl)oxy]-5-ethoxybenzyl}-7-fluoro-6-[2-fluoro-1-(fluoromethyl)ethoxy]-2,4-dioxo-3,4-dihydroquinazolin-1(2H)-yl]piperidine-1-carbaldehyde
No. 375: 4-[7-fluoro-3-{4-[(4-fluorobenzyl)oxy]-3-methoxybenzyl}-6-[2-fluoro-1-(fluoromethyl)ethoxy]-2,4-dioxo-3,4-dihydroquinazolin-1(2H)-yl]piperidine-1-carbaldehyde
No. 376: 4-[3-{4-[(3,5-dichlorobenzyl)oxy]-3-methoxybenzyl}-7-fluoro-6-[2-fluoro-1-(fluoromethyl)ethoxy]-2,4-dioxo-3,4-dihydroquinazolin-1(2H)-yl]piperidine-1-carbaldehyde
No. 377: 4-[3-(4-{[4-chloro-3-(trifluoromethyl)benzyl]oxy}-3-methoxybenzyl)-7-fluoro-6-[2-fluoro-1-(fluoromethyl)ethoxy]-2,4-dioxo-3,4-dihydroquinazolin-1(2H)-yl]piperidine-1-carbaldehyde
No. 379: 4-[3-{4-[(3-chlorophenoxy)methyl]-3-methoxybenzyl}-7-fluoro-6-[2-fluoro-1-(fluoromethyl)ethoxy]-2,4-dioxo-3,4-dihydroquinazolin-1(2H)-yl]piperidine-1-carbaldehyde
No. 380: 4-[3-{4-[(3,5-difluorobenzyl)oxy]-3-methoxybenzyl}-7-fluoro-6-[2-fluoro-1-(fluoromethyl)ethoxy]-2,4-dioxo-3,4-dihydroquinazolin-1(2H)-yl]piperidine-1-carbaldehyde
No. 381: 4-{3-[4-(benzyloxy)-3-methoxybenzyl]-7-fluoro-6-[2-fluoro-1-(fluoromethyl)ethoxy]-2,4-dioxo-3,4-dihydroquinazolin-1(2H)-yl}piperidine-1-carbaldehyde
No. 382: 4-[3-{4-[(3-chloro-5-fluorobenzyl)oxy]-3-methoxybenzyl}-7-fluoro-6-[2-fluoro-1-(fluoromethyl)ethoxy]-2,4-dioxo-3,4-dihydroquinazolin-1(2H)-yl]piperidine-1-carbaldehyde
No. 383: 4-{7-fluoro-6-[2-fluoro-1-(fluoromethyl)ethoxy]-3-(3-methoxy-4-{[4-(trifluoromethyl)benzyl]oxy}benzyl)-2,4-dioxo-3,4-dihydroquinazolin-1(2H)-yl}piperidine-1-carbaldehyde
No. 384: 4-[3-{4-[(2,5-dichlorobenzyl)oxy]-3-methoxybenzyl}-7-fluoro-6-[2-fluoro-1-(fluoromethyl)ethoxy]-2,4-dioxo-3,4-dihydroquinazolin-1(2H)-yl]piperidine-1-carbaldehyde
No. 385: 4-{[4-({7-fluoro-6-[2-fluoro-1-(fluoromethyl)ethoxy]-1-(1-formylpiperidin-4-yl)-2,4-dioxo-1,4-dihydroquinazolin-3(2H)-yl}methyl)-2-methoxyphenoxy]methyl}benzonitrile
No. 386: 3-{[4-({7-fluoro-6-[2-fluoro-1-(fluoromethyl)ethoxy]-1-(1-formylpiperidin-4-yl)-2,4-dioxo-1,4-dihydroquinazolin-3(2H)-yl}methyl)-2-methoxyphenoxy]methyl}benzonitrile
No. 387: 4-[3-{4-[(4-chloro-2-fluorobenzyl)oxy]-3-methoxybenzyl}-7-fluoro-6-[2-fluoro-1-(fluoromethyl)ethoxy]-2,4-dioxo-3,4-dihydroquinazolin-1(2H)-yl]piperidine-1-carbaldehyde
No. 388: 4-[3-{4-[1-(3,4-dichlorophenyl)ethoxy]-3-methoxybenzyl}-7-fluoro-6-[2-fluoro-1-(fluoromethyl)ethoxy]-2,4-dioxo-3,4-dihydroquinazolin-1(2H)-yl]piperidine-1-carbaldehyde
No. 389: 4-{7-fluoro-6-[2-fluoro-1-(fluoromethyl)ethoxy]-3-{4-[(3-hydroxybenzyl)oxy]-3-methoxybenzyl}-2,4-dioxo-3,4-dihydroquinazolin-1(2H)-yl}piperidine-1-carbaldehyde
No. 390: 4-[7-fluoro-3-{4-[(3-fluorobenzyl)oxy]-3-methoxybenzyl}-6-[2-fluoro-1-(fluoromethyl)ethoxy]-2,4-dioxo-3,4-dihydroquinazolin-1(2H)-yl]piperidine-1-carbaldehyde
No. 391: 4-[3-{4-[(3,4-difluorobenzyl)oxy]-3-methoxybenzyl}-7-fluoro-6-[2-fluoro-1-(fluoromethyl)ethoxy]-2,4-dioxo-3,4-dihydroquinazolin-1(2H)-yl]piperidine-1-carbaldehyde
No. 392: 4-{3-[4-(5,6-dichloro-1H-benzimidazol-1-yl)-3-methoxybenzyl]-7-fluoro-6-[2-fluoro-1-(fluoromethyl)ethoxy]-2,4-dioxo-3,4-dihydroquinazolin-1(2H)-yl}piperidine-1-carbaldehyde
No. 393: 4-({7-fluoro-6-[2-fluoro-1-(fluoromethyl)ethoxy]-1-(1-formylpiperidin-4-yl)-2,4-dioxo-1,4-dihydroquinazolin-3(2H)-yl}methyl)phenyl 3,4-dichlorobenzenesulphonate
No. 394: 4-({7-fluoro-6-[2-fluoro-1-(fluoromethyl)ethoxy]-1-(1-formylpiperidin-4-yl)-2,4-dioxo-1,4-dihydroquinazolin-3(2H)-yl}methyl)-2-methoxyphenyl 3,4-dichlorobenzenesulphonate
No. 403: 3,4-dichloro-N-[4-({7-fluoro-6-[2-fluoro-1-(fluoromethyl)ethoxy]-1-(1-formylpiperidin-4-yl)-2,4-dioxo-1,4-dihydroquinazolin-3(2H)-yl}methyl)-2-methoxyphenyl]benzamide;
in the form of the base or of an addition salt with an acid.
